# EUROPEAN PATENT APPLICATION

(11) **EP 2 423 333 A1**
(43) Date of publication of application: **29.02.2012**
(21) Application number: 11180581.8
(22) Date of filing: 23.08.2007
(51) Int. Cl.: C12Q 1/68

(54) **Prognostic markers and therapeutic targets for lung cancer**

(30) Priority: 25.08.2006 US 840376 P; 14.03.2007 US 894801 P
(62) Divisional of application: 07806302.1
(71) Applicant: Oncotherapy Science, Inc., Kawasaki-shi Kanagawa 213-0012 (JP)
(72) Inventor: Nakamura, Yusuke, Tokyo, 113-8654 (JP); Daigo, Yataro, Tokyo, 113-8654 (JP); Nakatsuru, Shuichi, Kanagawa, 213-0012 (JP)
(74) Representative: Vossius & Partner

(57) **Abstract**

The present invention provides a diagnostic marker to detecting lung cancer. In particular, the present invention provides lung cancer marker genes i.e. KIF4A, MAPJD, NPTX, or FGFR1OP. The present invention further provides methods and kit for identifying compounds for treating lung cancer as well as methods for predicting a prognosis or diagnosis of lung cancer. In particular, the present invention provides methods and kits for identifying inhibitors of the interaction between KIF4A/ZNF549, KIF4A/ZNF553, MAPJD/MYC, or FGFR1OP/WRNIP1 which find utility in the treatment and prevention of lung. Alternatively, the present invention provides MAPJD associated with HAT complex as therapeutic target.

## Description

### Cross- Reference to Related Applications

The present application claims the benefit of U.S. Provisional Application No. 60/840,376, filed August 25, 2006, and U.S. Provisional Application No. 60/894,801, filed March 14, 2007, the entire disclosures of each of which are hereby incorporated herein by reference for all purposes.

### Technical Field

The present invention relates to methods for detecting and diagnosing cancer as well as methods for treating and preventing cancer.

### Background Art

Lung cancer is one of the most common and fatal cancers in the world (Greenlee RT et al. CA Cancer J Clin 2001 ;51:15-36.). A number of genetic alterations associated with development and progression of lung cancer, have been reported, but its precise molecular mechanisms still remain unclear (Sozzi G. Eur J Cancer 37 Suppl 7: S63-73.). Two major histological-distinct types of lung cancer, non-small cell lung cancer (NSCLC) and small-cell lung cancer (SCLC) have different pathophysiological and clinical features that suggest differences in the mechanisms of their carcinogenesis. SCLC accounts for 15-20% of all lung cancers (Morita T et al. Acta Pathol Jpn 40: 665-75, 1990.; Simon GR et al. Chest 123 Suppl 1: S259-71, 2003.) and is categorized as neuroendocrine tumors of the lung with certain morphologic, ultra-structural, and immunohistochemical characteristics. However, detailed molecular characteristics of neuroendocrine tumors are still not well understood. Although patients with SCLC respond favorably to the 1st line multi-agent chemotherapy, they often relapse in a short time. Hence, only 20% of patients with limited-stage disease (LD) can be cured with combined modality therapy and less than 5% of those with extensive-disease (ED) can achieve 5-year survival after the initial diagnosis (Chute JP et al. J Clin Oncol 17: 1794-801, 1999.; Sander AB. Semin Oncol 30: 9-25, 2003.). Therefore, new therapeutic strategies such as molecular-targeted agents are eagerly awaited.

Systemic chemotherapy is the main treatment for the majority of patients with NSCLC because most are diagnosed with advanced inoperable disease. Within the last decade several newly-developed cytotoxic agents such as paclitaxel, docetaxel, gemcitabine, and vinorelbine have begun to offer multiple choices for treatment of patients with advanced lung cancer; however, each of those regimens confers only a modest survival benefit compared with cisplatin-based therapies (Schiller JH, et al. N Engl J Med 2002; 345:92-8.; Kelly K, et al. J Clin Oncol 2001;19:3210-8.). Hence, novel therapeutic strategies such as molecular-targeted drugs, antibodies, nucleic acid drugs and cancer vaccines, are eagerly being sought.

The long-term survival rate, even with complete clinical resections, remains unsatisfactory (Naruke T, et al. Ann Thorac Surg. 2001 Jun; 71(6): 1759-64.). Therefore, a better understanding of the molecular pathogenesis of lung cancer is an urgent issue in order to develop better diagnostic approaches and new molecular targeted therapies. Although the precise pathways involved in lung tumorigenesis remain unclear, some evidences indicate that tumor cells express cell-surface markers unique to each histological type at particular stages of differentiation. Since cell-surface proteins are considered more accessible to immune mechanisms and drug-delivery systems, identification of cancer-specific cell-surface and secretory proteins is likely to be an effective approach to development of novel diagnostic markers and therapeutic strategies.

The genome-wide cDNA microarray analysis is useful to obtain comprehensive gene expression profiles related to detailed phenotypic and biological information in cancer cells (Golub TR, et al. Science. 1999 Oct 15;286(5439):531-7.; Pomeroy SL, et al. Nature. 2002 Jan 24;415(6870):436-42.; van 't Veer LJ, et al. Nature. 2002 Jan 31;415(6871):530-6.). This approach is also useful to identify unknown molecules involved in the pathways of carcinogenesis. Through gene-expression profile analysis of SCLCs and NSCLCs coupled with purification of cancer cell population by laser-microbeam microdissection (LMM) on a cDNA microarray consisting of about 30,000 genes, the present inventors identified a number of potential molecular targets for diagnosis, treatment, and/or choice of therapy (Kikuchi T, et al. Oncogene. 2003 Apr 10;22(14):2192-205.; Int J Oncol. 2006 Apr;28(4):799-805.; Kakiuchi S, et al. Mol Cancer Res. 2003 May;1(7):485-99.; Hum Mol Genet. 2004 Dec 15;13(24):3029-43. Epub 2004 Oct 20.). To verify the biological and clinicopathological significance of the respective gene products, the present inventors have also been performing high-throughput screening of loss-of-function effects by means of the RNAi technique as well as tumor-tissue microarray analysis of clinical lung-cancer materials (Suzuki C, et al. Cancer Res. 2003 Nov 1;63(21):7038-41.; Cancer Res. 2005 Dec 15;65(24):11314-25.; Ishikawa N, et al. Clin Cancer Res. 2004 Dec 15;10(24):8363-70.; Cancer Res. 2005 Oct 15;65(20):9176-84.; Kato T, et al. Cancer Res. 2005;65(13):5638-46.; Furukawa C, et al. Cancer Res. 2005;65(16):7102-10.). This systematic approach revealed that KIF4A (kinesin family member 4A) (GenBank Accession No. NM_012310) was frequently over-expressed in the great majority of SCLCs as well as in 40% of NSCLCs, and was essential to growth or progression of lung-cancers, that MAPJD (Myc-associated protein with JmjC domain) (C14orf169, chromosome 14 open reading frame 169; alias FLJ21802/NO66 protein) (GenBank Accession NO. NM_024644) was over-expressed in the great majority of the NSCLCs, that Neural pentraxin I (NPTX1) (GenBank Accession No. NM_002522) was frequently transactivated in primary lung cancers, and that the gene encoding fibroblast growth factor receptor 1 oncogene partner (FGFR1OP alias FOP) (GenBank Accession No. NM_007045) was likely to over-express in the great majority of primary NSCLCs (WO2004/31413, WO2005/89735).

Recent years, a new approach of cancer therapy using gene-specific siRNA was attempted in clinical trials (Bumcrot D et al., Nat Chem Biol 2006 Dec, 2(12): 711-9). RNAi seems to have already earned a place among the major technology platforms (Putral LN et al., Drug News Perspect 2006 Jul-Aug, 19(6): 317-24; Frantz S, Nat Rev Drug Discov 2006 Jul, 5(7): 528-9; Dykxhoom DM et al., Gene Ther 2006 Mar, 13(6): 541-52). Nevertheless, there are several challenges that need to be faced before RNAi can be applied in clinical use. These challenges include poor stability of RNA *in vivo* (Hall AH et al., Nucleic Acids Res 2004 Nov 15, 32(20): 5991-6000, Print 2004; Amarzguioui M et al., Nucleic Acids Res 2003 Jan 15, 31 (2): 589-95), toxicity as an agent (Frantz S, Nat Rev Drug Discov 2006 Jul, 5(7): 528-9), mode of delivery, the precise sequence of the siRNA or shRNA used, and cell type specificity. It is a well-known fact that there are possible toxicities related to silencing of partially homologous genes or induction of the interferon response (Judge AD et al., Nat Biotechnol 2005 Apr, 23(4); 457-62, Epub 2005 Mar 20; Jackson AL & Linsley PS, Trends Genet 2004 Nov, 20(11): 521-4). So double-stranded molecules targeting cancer-specific genes must be devoid of adverse side-effects.

### Summary of the Invention

The present invention is based, at least in part, on the discovery of a specific expression pattern of a kinesin family member 4A (KIF4A) (GenBank Accession No. NM_012310), Myc-associated protein with JmjC domains (MAPJD) (GenBank Accession No. NM_024644), Neuronal pentraxin I (NPTX1) (GenBank Accession No. NM_002522) and fibroblast growth factor receptor 1 oncogene partner (FGFR1OP) (GenBank Accession No. NM_007045) gene in cancerous cells.

Through the present invention, the KIF4A, MAPJD, NPTX1 and FGFR1OP gene were revealed to be frequently up-regulated in a wide range of human tumors including lung cancer (small-cell lung carcinomas (SCLCs) and non-small cell lung cancers (NSCLCs)).

The KIF4A gene identified herein as well as its transcription and translation products find diagnostic utility as markers for cancer and as an oncogene target, the expression and/or activity of which may be altered to treat or alleviate a symptom of cancer. Similarly, by detecting the changes in the expression of the KIF4A gene in the presence of a test compound, various compounds can be identified as agents for treating or preventing cancer.

Accordingly, the present invention provides a method for diagnosing or determining a predisposition to cancer in a subject by determining the expression level of the KIF4A gene in a subject-derived biological sample, such as tissue sample (*e.g*., a lung tissue sample). Increased expression level of the gene as compared to a normal control level indicates that the subject suffers from or is at risk of developing cancer. The normal control level can be determined using a normal cell obtained from a non-cancerous tissue. In the present invention, the cancer being detected is typically lung cancer, *e.g*., SCLC or NSCLC.

In the context of the present invention, the phrase "control level" refers to the expression level of the KIF4A gene detected in a control sample and includes both normal control level and cancer control level. A control level can be a single expression pattern derived from a single reference population or from a plurality of expression patterns. For example, the control level can be a database of expression patterns from previously tested cells. A "normal control level" refers to a level of the KIF4A gene expression detected in a normal healthy individual or in a population of individuals known not to be suffering from cancer. A normal individual is one with no clinical symptom of cancer. On the other hand, a "cancer control level" refers to an expression level of the KIF4A gene found in an individual or population suffering from cancer.

An increase in the expression level of the KIF4A gene detected in a sample as compared to a normal control level indicates that the subject (from which the sample has been obtained) suffers from or is at risk of cancer.

Alternatively, expression levels of the KIF4A gene in a sample can be compared to cancer control levels of the same gene. A similarity between the expression levels of a sample and the cancer control levels indicates that the subject (from which the sample has been obtained) suffers from or is at risk of cancer.

Herein, gene expression levels are deemed to be "altered" when the gene expression increases by, for example, 10%, 25%, or 50% from, or at least 0.1 fold, at least 0.2 fold, at least 1 fold, at least 2 fold, at least 5 fold, or at least 10 fold or more compared to a control level The expression level of the KIF4A gene can be determined by detecting, *e.g*., hybridization intensity of nucleic acid probes to gene transcripts in a sample.

In the context of the present invention, subject-derived tissue samples may be any tissues obtained from test subjects, *e.g*., patients known to have or suspected of having cancer. For example, tissues may comprise epithelial cells. More particularly, tissues may be cancerous epithelial cells.

The present invention is also directed to the detection of elevated levels of NPTX in the blood of lung cancer patients. Thus, the NPTX1 gene and its protein are useful as diagnostic markers (*i.e*. whole blood, serum, or plasma). For example, sandwich ELISA can be conveniently used to detect serum NPTX1 in patients with lung cancer.

Accordingly, the present invention provides methods for diagnosing lung cancer in a subject comprising the steps of determining the level of NPTX1 in subject-derived blood samples and comparing this level to that found in a reference sample, typically a normal control. A high level of NPTX1 in a sample indicates that the subject either suffers from or is at risk for developing lung cancer. The term "normal control level" indicates a level associated with a normal healthy individual or a population of individuals known not to be suffering from lung cancer.

The level of NPTX1 may be determined by detecting the NPTX1 protein using immunoassay such as ELISA.

Subject-derived blood samples may be derived from whole blood, serum, and plasma derived from subjects, *e.g*., patients known to or suspected of having lung cancer.

In addition, the present invention provides the above-described methods further comprising the steps of determining the level of either of CEA and proGRP or both in a subject-derived blood samples and comparing the CEA and/or proGRP level to that found in a reference sample, typically a normal control. The evidence provided here also shows that patients with NSCLC cancer can be identified more sensitively by detecting both NPTX1 and CEA levels. Similarly, patients with SCLC cancer can be identified more sensitively by considering both NPTX1 and proGRP levels.

Furthermore, the present invention also provides immunoassay reagents for detecting NPTX1 comprising an anti-NPTX1 antibody or antibody mimic. The anti-NPTX1 antibody may be either polyclonal antibodies or a monoclonal antibody or at least two monoclonal antibodies recognizing different antigenic determinants of NPTX1 each other.

The present invention further provides kits for detecting a lung cancer comprising (i) an immunoassay reagent for determining a level of NPTX1 in a blood sample; and (ii) a positive control sample for NPTX1. The kits may further comprise (iii) an immunoassay reagent for determining a level of either of CEA and proGRP or both in a blood sample; and (iv) a positive control sample for either of CEA and proGRP or both.

Moreover, since the suppression of the KIF4A, NPTX1, FGFR1OP and/or WRNIP1 gene(s) by small interfering RNA (siRNA) resulted in growth, inhibition and/or cell death of lung cancer cells, e.g. SCLC and/or NSCLC, these genes are useful therapeutic targets for various types of human neoplasms.

Therapeutic methods of the present invention include methods for treating or preventing cancer in a subject comprising the step of administering a pharmaceutical composition comprising siRNA to the subject. In the context of the present invention, the pharmaceutical composition reduces the expression of the KIF4A, NPTX1, FGFR1OP and/or WRNIP1 gene(s). The inhibition of cancer cell proliferation by siRNA molecules of the invention is demonstrated in the Examples section.

The present invention, is also based, at least in part, on the discovery that the higher the expression level of the KIF4A, NPTX1 or FGFR1OP gene measured in the patient-derived biological sample, the poorer the prognosis for post-treatment remission, recovery, and/or survival and the higher the likelihood of poor clinical outcome. Thus, the present invention provides a method for determining or assessing the prognosis of a patient with cancer, in particular lung cancer, by detecting the expression level of the KIF4A, NPTX1 or FGFR1OP gene in a biological sample of the patient; comparing the detected expression level to a control level; and determining an increased expression level to the control level as indicative of poor prognosis (poor survival).

According to the present method, the "control level" used for comparison may be, for example, the expression level of the KIF4A, NPTX1 or FGFR1OP gene detected before any kind of treatment in an individual or a population of individuals who showed good or positive prognosis of cancer, after the treatment, which herein will be referred to as "good prognosis control level". Alternatively, the "control level" may be the expression level of the KIF4A, NPTX1 or FGFR1OP gene detected before any kind of treatment in an individual or a population of individuals who showed poor or negative prognosis of cancer, after the treatment, which herein will be referred to as "poor prognosis control level".

According to the present invention, a similarity in the expression level of the KIF4A, NPTX1 or FGFR1OP gene to the good prognosis control level indicates a more favorable prognosis of the patient and an increase in the expression level in comparison to the good prognosis control level indicates less favorable, poorer prognosis for post-treatment remission, recovery, survival, and/or clinical outcome. On the other hand, a decrease in the expression level of the KIF4A, NPTX1 or FGFR1OP gene in comparison to the poor prognosis control level indicates a more favorable prognosis of the patient and a similarity in the expression level to the poor prognosis control level indicates less favorable, poorer prognosis for post-treatment emission, recovery, survival, and/or clinical outcome.

The present invention also provides the use of gene-expression profiles of small-cell lung carcinomas (SCLCs) and non-small cell lung cancers (NSCLCs) to screen candidate molecules that might be useful as diagnostic biomarkers or for development of novel molecular-targeting therapies.

The present invention also identifies proteins that interact with the proteins noted above. As KIF4A interacting proteins, two zinc finger proteins, ZNF549 (GenBank Accession No. NM_153263) and ZNF553 (GenBank Accession No. NM_152652), which are also activated in lung cancers are identified here. Through interaction with MYC protein, MAPJD transactivates a set of genes including kinases and cell signal transducers that are possibly related to lung cancer cell proliferation. As the data demonstrate that MAPJD is a novel member of the MYC transcriptional complex and its activation is a common feature of lung-cancer, selective suppression of this pathway can be used in the therapeutic target for treatment of lung cancers. As FGFR1OP interacting proteins, the Werner helicase interacting protein 1 (WRNIP1) (GenBank Accession No. NM_020135) and the Abelson murine leukemia viral oncogene homolog 1 (ABL1) (GenBank Accession No. NM_007313) are identified here.

In addition, a high level of KIF4A, MAPJD, NPTX1 and FGFR1OP expression was associated with poor survival for patients with SCLC and/or NSCLC, demonstrating an important role of the proteins in development and progression of this disease. In addition, serum NPTX1 levels were higher in NSCLC as well as SCLC patients than in healthy controls.

According to the present invention, a method for assessing or determining the prognosis of a patient with lung cancer is provided. Specifically, the expression level of the KIF4A, NPTX1 and/or FGFR1OP gene is determined in a biological sample, such as sputum or blood, derived from the patient and compared to a control (expression) level of the gene. Herein, an increase of the expression level of the gene compared to a good prognosis control level indicates poor prognosis, *i.e*., poor survival of the patient. Such an increase may, for example, at least 10% greater than the control level. The present methods is particularly suited for assessing the prognosis of SCLC and/or NSCLC.

The present invention is based on the finding that target molecules and partner molecules interact in lung cancer cells. Accordingly, the present invention provides novel methods for identifying compounds that slow or arrest the progression of lung cancer, *e.g*., non-small cell lung cancer and small cell lung cancer, by interfering with target molecules / partner molecules interaction.

Accordingly, an objective of the present invention is to provide methods of screening for inhibitor of a binding or interaction of polypeptides of the present invention or compounds that are useful for inhibiting lung cancer cell growth or treating or preventing lung cancer. In some embodiments, the methods comprise the steps of:
(1) contacting a polypeptide comprising a partner molecule-binding domain of a target molecule with a polypeptide comprising a target molecule-binding domain of a partner molecule in the presence of a test compound;
(2) detecting binding between the peptides; and
(3) selecting a test compound that inhibits the binding,
wherein a combination of the target molecule and the partner molecules thereof is selected from the group consisting of KIF4A/ZNF549, KIF4A/ZNF553, MAPJD/MYC, FGFR1OP/WRNIP1, FGFR1OP/ABL1, and FGFR1OP/WRNIP1/ABL1.

In some embodiments, the polypeptide comprising the partner molecule-binding domain of a target molecule comprises a KIF4A polypeptide, MAPJD polypeptide, or FGFR1OP polypeptide. Similarly, in other embodiments, the polypeptide comprising the target molecule-binding domain comprises a ZNF553 polypeptide, MYC polypeptide, WRNIP1 polypeptide, or ABL1 polypeptide.

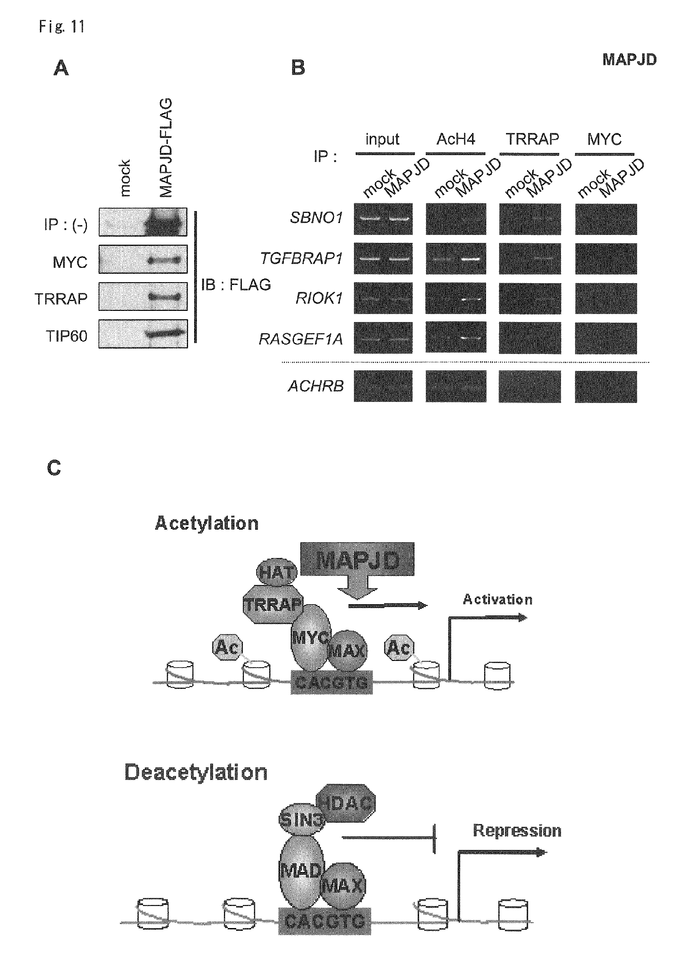

The present invention is also based, at least in part, on the discovery of a novel mechanism of MAPJD associated with HAT complex to acetylate a substrate *e.g.* histone H4 *in vitro* and *in vivo* (*see* Fig. 11C). Accordingly, the present invention provides methods of identifying compounds for preventing or treating lung cancer by identifying compounds that modulate the acetylation of histone H4 by, the MAPJD. In particular, the present invention provides methods of screening for modulator, e.g. inhibitor, of the MAPJD-mediated acetylation of histone H4, and compounds that are useful for inhibiting lung cancer cell growth or treating or preventing lung cancer, the methods comprise the steps of:
(1) contacting a test compound to a MAPJD polypeptide associated with a HAT complex and a substrate that is acetylated by the polypeptide under the condition capable of acetylation of the substrate;
(2) detecting a acetylation level of the substrate; and
(3) selecting the test compound that decreases the acetylation level of the substrate to be acetylated as compared to a control acetylation level detected in the absence of the test compound.

In some embodiments, the substrate to be acetylated is histone H4. In some embodiments, the MAPJD polypeptide and the HAT complex are expressed in a living cell.

In addition, the present invention relates to the discovery of a mechanism of regulation of genes contained E-box (CANNTG; provided that "N" may be any one base selected from group consisting of a, t, c, and g) in their transcriptional regulatory regions and the further identification of the genes as a novel downstream target of the MAPJD associated with HAT complex. In particular, the evidence demonstrates that elevated expression of MAPJD plays an important role in carcinogenesis of the lung.

Accordingly, the present invention provides methods of screening for a modulator, e.g. inhibitor, of a binding or interaction of a MAPJD/HAT complex and E-box motif, and compounds that are useful for inhibiting lung cancer cell growth or treating or preventing lung cancer, the methods comprise the steps of:
(1) contacting a test compound to a MAPJD polypeptide associated with a HAT complex or MYC and polynucleotide comprising E-box motif;
(2) detecting a binding between the polypeptide and the polynucleotide; and
(3) selecting a test compound that inhibits the binding.

In some embodiments, the polynucleotide containing E-box is a vector comprising the transcriptional regulatory region and a reporter gene that is expressed under the control of the transcriptional regulatory region, wherein the transcriptional regulatory region comprises at least one E-box motif, and wherein the binding is detected by measuring the expression level or activity of said reporter gene. In some embodiments, the polynucleotide comprises E-box motifs in the 5' flanking region of a gene selected from the group consisting of SBNO1, TGFBRAP1, RIOK1, and RASGEF1A as the transcriptional regulatory region.

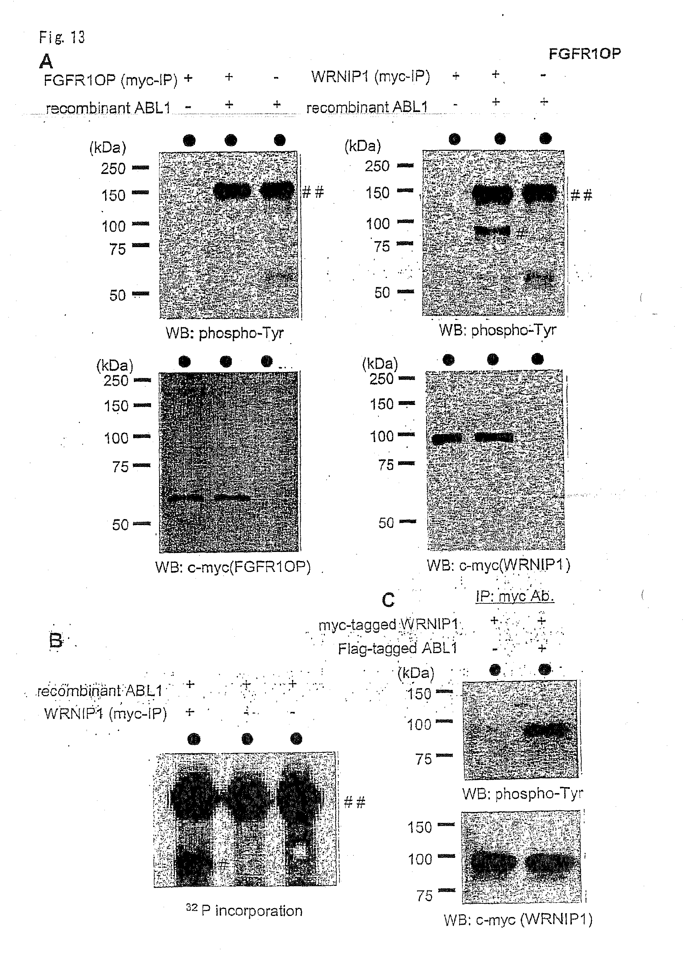

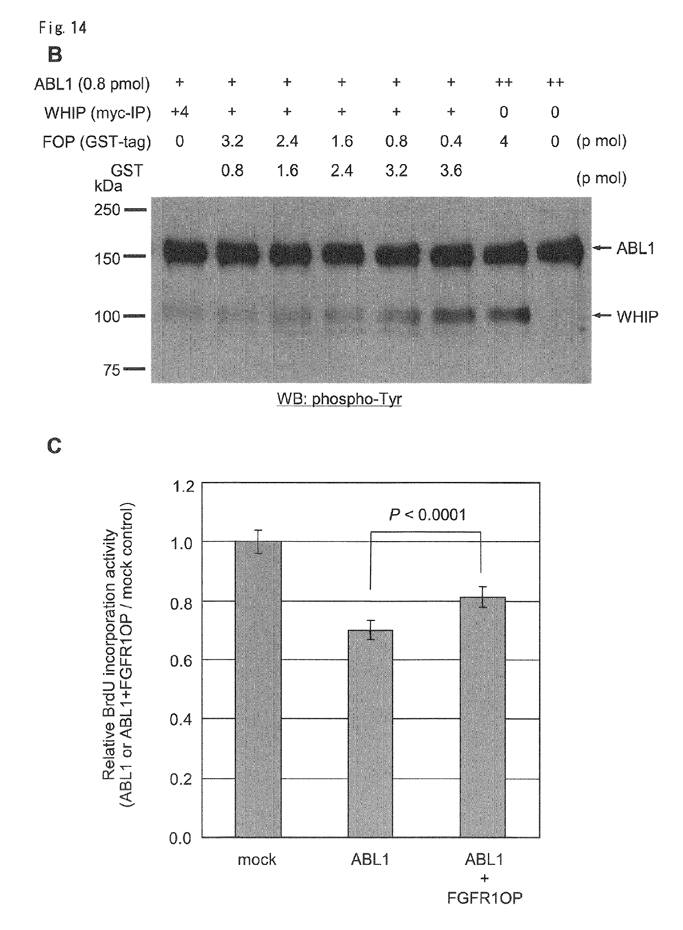

The present invention is also based, at least in part, on the discovery of a novel mechanism of an inhibition for the ABL1 -mediated phosphorylation of a WRNIP1 by an FGFR1OP *in vitro* and *in vivo* (*see* Fig. 13A). Accordingly, the present invention provides methods of identifying compounds for preventing or treating lung cancer by identifying compounds that modulate the phosphorylation of a WRNIP1 by an ABL1 associated with an FGFR1OP. Moreover the present invention is also based on the discovery that FGFR1OP significantly reduces ABL1-dependent phosphorylation of WRNIP1 and appears to promote cancer cell cycle progression (*see* Fig. 14C). Accordingly, the present invention provides methods of identifying compounds for preventing or treating lung cancer by identifying compounds that inhibite the reduction of ABL1 -dependent phosphorylation of WRNIP1 by FGFR1OP. So that, the present invention provides methods of identifying compounds that interrupt the association of ABL1 and FGFR1OP and result in enhancing the ABL1-mediated phosphorylation of WRNIP1. The present invention provides methods of identifying compounds for preventing or treating lung cancer by identifying compounds that increase a phosphorylation of WRNIP1 by an ABL1 associated with FGFR1OP. In particular, the present invention provides methods of screening for modulators, e.g. enhancer, of an interaction of the polypeptide of the invention and compounds that are useful for inhibiting lung cancer cell growth or treating or preventing lung cancer, the methods comprise the steps of:
(1) contacting a test compound to an ABL1 associated with an FGFR1OP polypeptide and a WRNIP1 polypeptide in the suitable condition for phosphorylation;
(2) detecting a phosphorylation level of the WRNIP1 polypeptide; and
(3) selecting the test compound that increases the phosphorylation level of the WRNIP1 polypeptide as compared to a control phosphorylation level detected in the absence of the test compound.

In some embodiments, the ABL1 polypeptide, the FGFR1OP polypeptide and WRNIP1 polypeptide are expressed in a living cell.

The present invention also provides methods for treating or preventing lung cancer in a subject. In some embodiments, the method comprises the step of administering a pharmaceutically effective amount of a compound that inhibits binding between a target molecule and a partner molecule thereof. In the present invention, a combination of these molecules can be selected from the group consisting of KIF4A/ZNF549, KIF4A/ZNF553, MAPJD/MYC. FGFR1OP/WRNIP1 FGFR1OP/ABL1 and FGFR1OP/WRNIP1/ABL1. In some embodiment, the method comprises the step of administering a pharmaceutically effective amount of a compound that inhibits the acetylation of histone H4 by MAPJD polypeptide associated with a HAT complex. Further, in some embodiments, the method comprises the step of administering a pharmaceutically effective amount of a compound that inhibits binding between a MAPJD polypeptide associated with a HAT complex or MYC and E-box motif. In some embodiments, the method comprises the step of administering a pharmaceutically effective amount of a compound that increases an ABL1-mediated phosphorylation of a WRNIP1 polypeptide.

Similarly, the present invention provides a kit for screening for inhibitors of a binding or interaction, and a compound useful in inhibiting lung cancer cell growth or treating or preventing lung cancer, the kit of the present invention may comprise:
(a) a polypeptide comprising a partner molecule-binding domain of a target molecule;
(b) a polypeptide comprising an target molecule-binding domain of a partner molecule, and
(c) reagent to detect the interaction between the polypeptides,
wherein a combination of the target molecule and the partner molecule thereof is selected from the group consisting of KIF4A/ZNF549, KIF4A/ZNF553, MAPJD/MYC, FGFR1OP/WRNIP1, and FGFR1OP/ABL1.

Alternatively, the present invention also provides a kit for screening for modulators, e.g. inhibitors, of MAPJD-mediated acetylation of histone H4 and a compound useful in inhibiting lung cancer cell growth or treating or preventing lung cancer, wherein the kit comprises:
(a) a cell expressing an MAPJD polypeptide and a HAT complex or MYC, and
(b) reagent to detect the acetylation level of histone H4.

Further, the present invention also provides a kit for screening for modulators, e.g. inhibitors, of MAPJD-mediated acetylation of histone H4 and a compound useful in inhibiting lung cancer cell growth or treating or preventing lung cancer, wherein the kit comprises:
(a) a cell expressing an MAPJD polypeptide and a HAT complex or MYC, wherein the cell is transfected with a vector comprising the transcriptional regulatory region and an reporter gene that is expressed under the control of the transcriptional regulatory region, wherein the transcriptional regulatory region comprises the E-box motif, and
(b) reagent to detect the expression level or the activity of the reporter gene.

Alternatively, the present invention also provides a kit for screening for modulators, e.g. enhancer, of ABL1-mediated phosphorylation of WRNIP1 and a compound useful in inhibiting lung cancer cell growth or treating or preventing lung cancer, wherein the kit comprises:
(a) a cell expressing an FGFR1OP polypeptide, a WRNIP1 polypeptide and an ABL1 polypeptide, and
(b) reagent to detect the phosphorylation level of the WRNIP polypeptide.

These and other objects and features of the invention will become more fully apparent when the following detailed description is read in conjunction with the accompanying figures and examples. However, it is to be understood that both the foregoing summary of the invention and the following detailed description are of a preferred embodiment, and not restrictive of the invention or other alternate embodiments of the invention.

One advantage of the methods described herein is that the disease is identified prior to detection of overt clinical symptoms of cancers. Other features and advantages of the invention will be apparent from the following detailed description, and from the claims.

### Brief Description of the Drawings

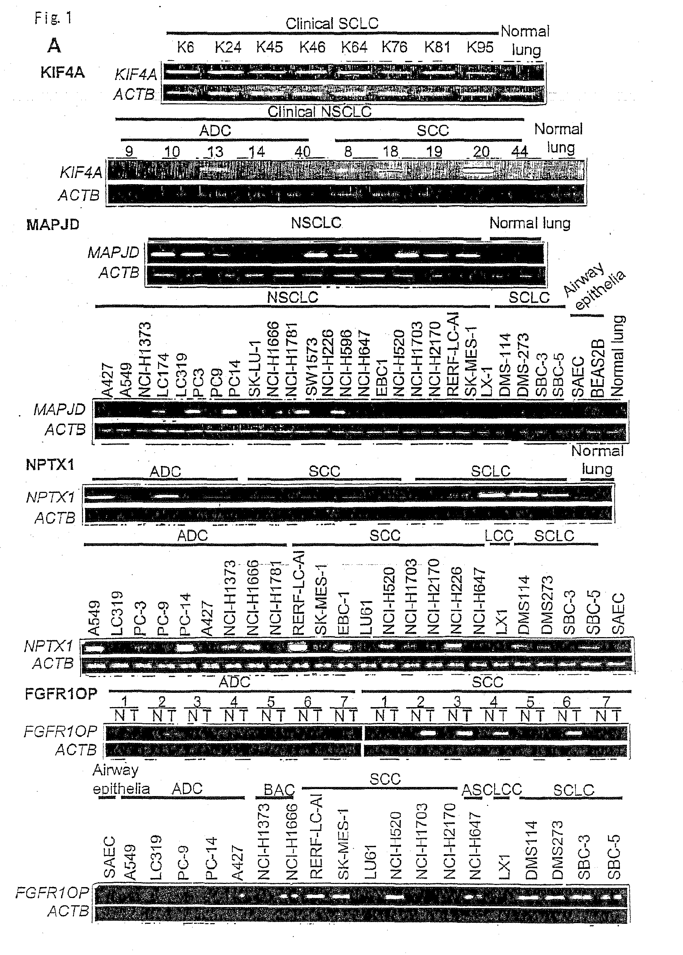

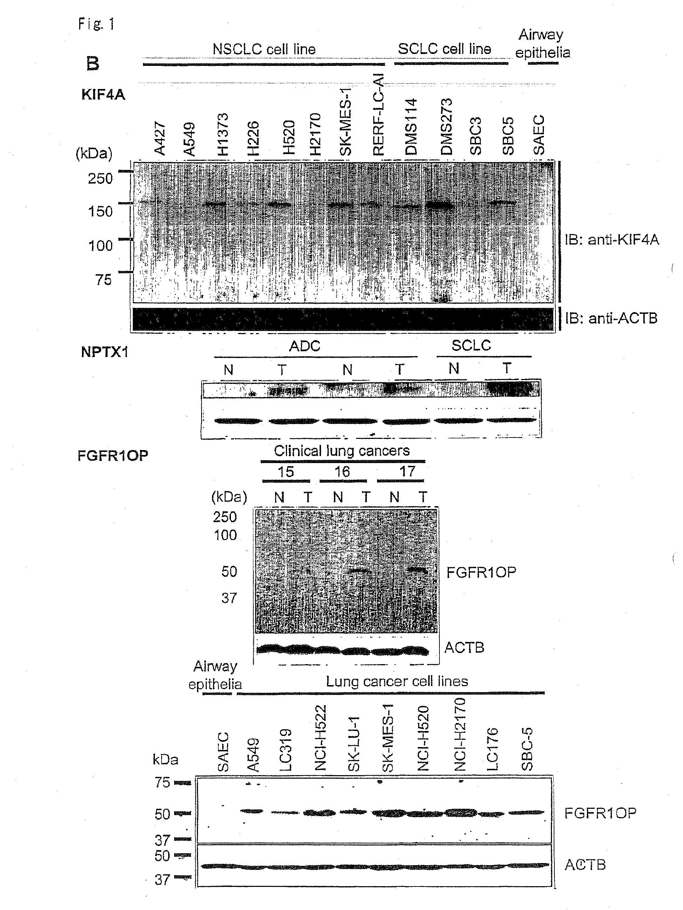

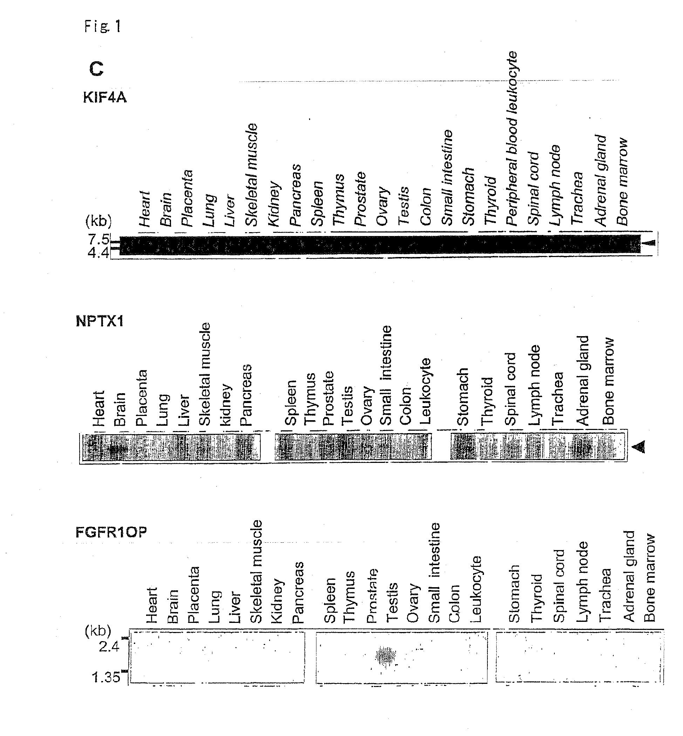

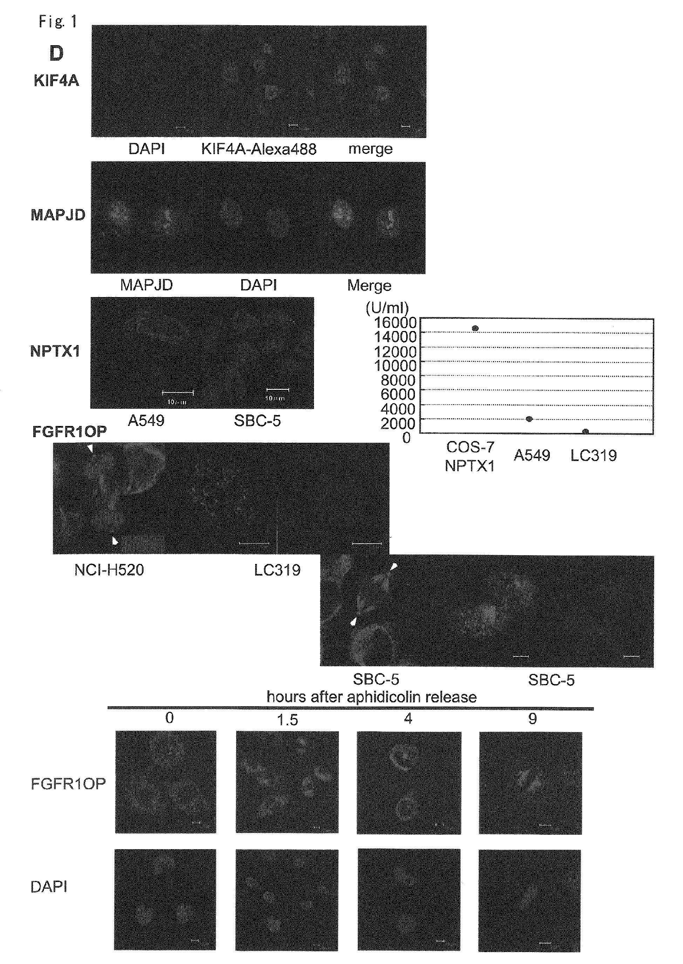

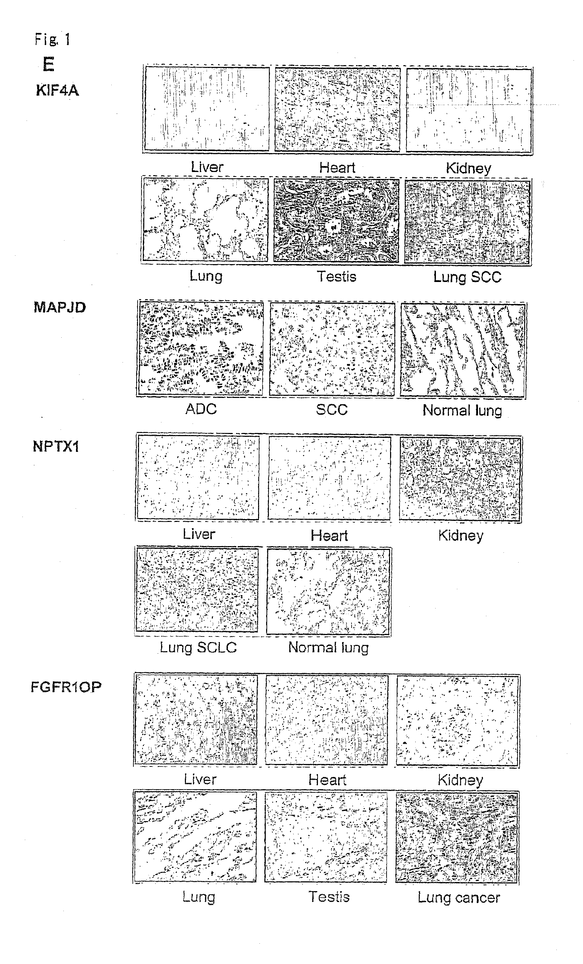

Figure 1 shows the KIF4A, MAPJD, NPTX1 and FGFR1OP expression in lung cancers and normal tissues. (A) KIF4A: Expression of *KIF4A* in clinical samples of SCLC and NSCLC, and normal lung tissues, examined by semi-quantitative RT-PCR. The present inventors prepared appropriate dilutions of each single-stranded cDNA prepared from mRNAs of lung-cancer samples, taking the level of beta-actin (ACTB) expression as a quantitative control. MAPJD: Expression of MAPJD gene in clinical samples of non-small cell lung cancer (NSCLC), examined by semi-quantitative RT-PCR. Expression of MAPJD gene in lung-cancer cell lines. NPTX1: Expression of NPTX1 in a normal lung tissue and 10 clinical non-small cell lung cancer (NSCLC) and 5 SCLC samples detected by semi-quantitative RT-PCR analysis. Expression of NPTX1 in small airway epithelial cells (SAEC) and 23 lung-cancer cell lines detected by semi-quantitative RT-PCR analysis. FGFR1OP: Expression of FGFR1OP in clinical samples of NSCLC (T) and corresponding normal lung tissues (N), lung-cancer cell lines, examined by semi-quantitative RT-PCR. Expression of FGFR1OP in lung-cancer cell lines, detected by semi-quantitative RT-PCR. (B) KIF4A: Expression of KJF4A protein in lung-cancer cell lines, examined by western-blot analysis. Expression of ACTB was served as a quantity control. NPTX1: Western-blot analysis of NPTX1 protein in three representative pairs of lung adenocarcinoma (ADC) and SCLC samples. FGFR1OP: Western blot analysis of FGFR1OP protein in three representative pairs of lung-cancer tissue samples and lung-cancer cell lines A549, NCI-H226, NCI-H522, NCI-H522, SK-LU-1, SK-MES-1, NCI-H520,NCI-H2170, LC176, SBC-5. (C) KIF4A: Expression of *KIF4A* in normal human tissues, detected by northern-blot analysis. NPTX1: Northern blot analysis of the NPTX1 transcript in 23 normal adult human tissues. FGFR1OP: Northern blot analysis of the FGFR1OP transcript in 23 normal adult human tissues. (D) KIF4A: Subcellular localization of endogenous KIF4A protein in DMS273 cells. KIF4A was stained at the cytoplasm and nucleus of the cell. MAPJD: Subcellular localization of endogenous MAPJD in lung cancer cells (LC319), detected by immunocytochemical staining with anti-MAPJD antibody. NPTX1: Subcellular localization of endogenous NPTX1 protein in A549 and SBC-5 cells. NPTX1 was stained at the cytoplasm of the cell with granular appearance. Specific detection of NPTX1 with ELISA in conditioned medium from NPTX1-expressing A549 and transiently expressed COS-7 cells, and non-expressing LC319 cells. FGFR1OP: Upper panels, Immunofluorescence staining of endogenous FGFR1OP and α-tubulin in NCI-H520 (upper left panel), SBC-5 (lower panel) and LC319 (upper right panel) cells. Cells were fixed; The FGFR1OP-Alexa594 (red), α-tubulin-Alexa488 (green), or cell nuclei (DAPI) were visualized. Arrows indicate localization of FGFR1OP at the centrosome. Lower panels, Immunocytochemical analysis of LC319 cells using anti-FGFR1OP antibody. LC319 cells were synchronized at the G1/S boundary by aphidicolin. At different time-points after the release from cell-cycle arrest, cells were immunostained with FGFR1OP-Alexa488 (green) or cell nuclei (DAPI) at individual time points. (E) KIF4A: Expression of KIF4A in normal human tissues and lung SCC, detected by immunohistochemical staining (x100). MAPJD: immunohistochemical evaluation of representative samples from surgically resected adenocarcinoma (ADC) and squamous-cell carcinoma (SCC) tissues, using anti-MAPJD polyclonal antibody. NPTX1: Immunohistochemical evaluation of NPTX1 protein in representative SCLC tissue and normal organ tissues; adult liver, heart, kidney, and lung tissues. FGFR1OP: Immunohistochemical evaluation of FGFR1OP protein in representative normal tissues; adult heart, liver, lung, kidney, testis and lung adenocarcinoma tissue. Magnification, x200.

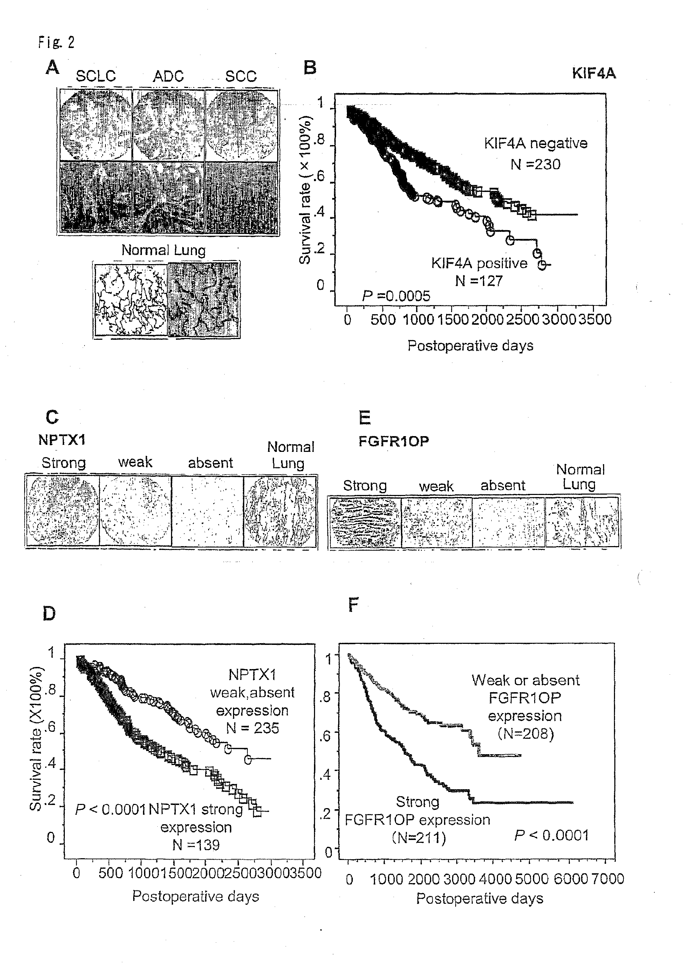

Figure 2 shows the association of KIF4A, NPTX1, or FGFR1OP over-expression with poor clinical outcomes among NSCLC patients. (A) Immunohistochemical evaluation of KIF4A expression and localization on lung cancer tissues (*upper panels* x100; *lower panels* x200). Positive staining appeared predominantly in the cytoplasm and nucleus. Examples are shown of KIF4A expression in SCLCs, lung ADCs, and lung SCCs, and of no expression in normal lung. (B) Kaplan-Meier analysis of tumor-specific, survival in patients with NSCLC according to KIF4A expression (*P* = 0.0005; Log-rank test). (C) Immunohistochemical evaluation of NPTX1 expression on tissue microarrays (x100). Examples are shown of strong, weak, and absent NPTX1 expression in NSCLCs, and of no expression in normal lung. (D) Kaplan-Meier analysis of tumor-specific survival in patients with NSCLC according to NPTX1 expression (P = 0.0001; Log-rank test). (E) Immunohistochemical evaluation of FGFR1OP protein expression on tissue microarrays. Examples are shown for strong, weak, or absent FGFR1OP expression in lung SCCs, and for no expression in normal lung. Magnification, x100. (F) Kaplan-Meier analysis of tumor-specific survival in 419 patients with NSCLCs according to the level of FGFR1OP expression (P < 0.0001; log-rank test).

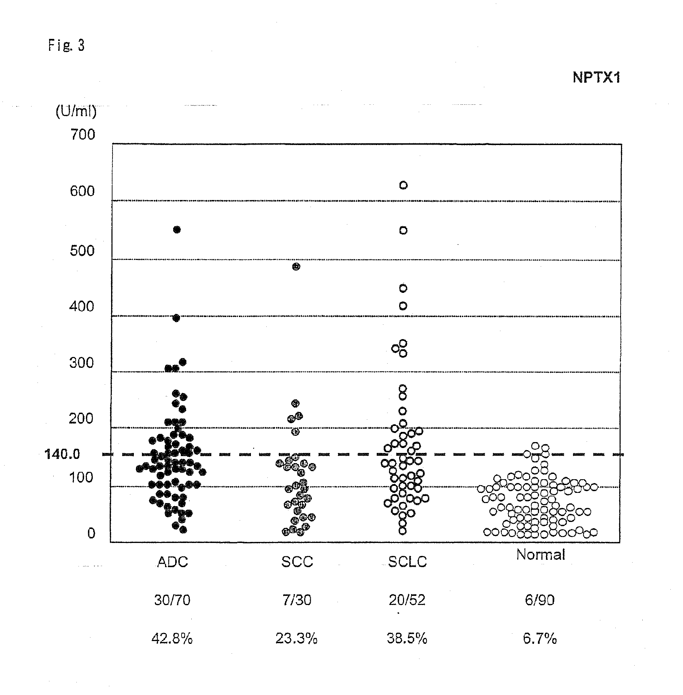

Figure 3 shows the serologic concentration of NPTX1 determined by ELISA in patients with lung cancers and in healthy controls. Distribution of NPTX1 in sera from patients with lung ADC, lung SCC, or SCLC. Difference were significant between ADC patients and healthy individuals (P < 0.001, Mann-Whitney U test), between SCC patients and healthy individuals (P < 0.001) and between SCLC patients and healthy individuals (P < 0.001).

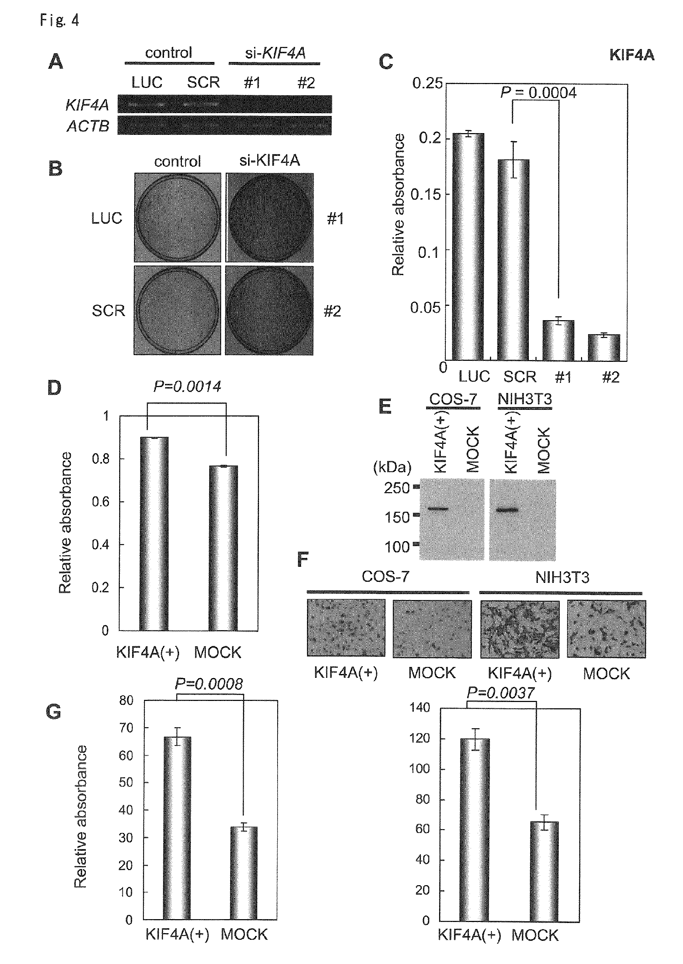

Figure 4 shows the results of the inhibition of growth of lung cancer cells by siRNA against *KIF4A.* (A) Expression of *KIF4A* in response to si-*KIF4A* (si-#1 or -#2) or control siRNAs (LUC or SCR) in SBC-5 cells, analyzed by semi-quantitative RT-PCR. (B) Colony-formation assays of SBC-5 cells transfected with specific siRNAs or control plasmids. (C) Viability of SBC-5 cells evaluated by MTT assay in response to si-*KIF4A* (si-#1 or -#2), - LUC, or -SCR. All assays were performed three times, and in triplicate wells. Growth-promoting and invasive effects of KIF4A in mammalian cells transfected with KIF4A-expressing plasmids. (D) Growth-promoting effect of KIF4A. The relative number of COS-7 cells evaluated by MTT assay. (E) Transient expression of KIF4A in COS-7 and NIH3T3 cells, detected by Western-Blotting. (F) and (G) Assays demonstrating the invasive nature of NIH3T3 and COS-7 cells in Matrigel matrix after transfection with expression plasmids for human KIF4A. Giemsa staining (F, x100), and the relative number of cells migrating through the Matrigel-coated filters (G). Assays were performed three times and in triplicate wells.

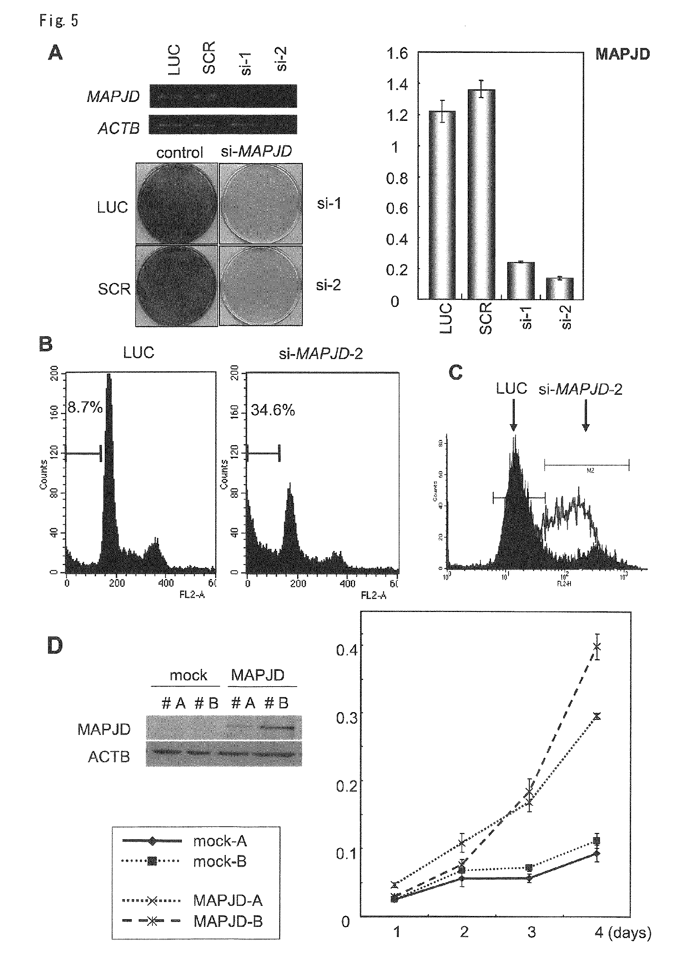

Figure 5 shows the effect of MAPJD on cell growth. (A)-(C) Inhibition of growth of NSCLC cells by siRNA against MAPJD. (A) Expression of MAPJD in response to siRN A-MAPJD-1 (si-1). -2 (si-2), or control siRNAs against luciferase (LUC) or scramble (SCR) in LC319 cells, analyzed by semi-quantitative RT-PCR (left upper panels). Colony-formation assays of LC319 cells transfected with the specific siRNAs for MAPJD (si-1 and -2) or control plasmids (left lower panels). Viability of LC319 cells evaluated by MTT assay in response to siRNA-MAPJD-2 (si-2), in comparison with control siRNAs (right panel). Assays were performed three times, and in triplicate wells. (B) The sub-G1 proportion of cells treated with control siRNA (LUC) (left panel) or si-2 (right panel), detected by flow-cytometric analysis. (C) Annexin V-binding assay using flow-cytometric analysis of LC319 cells treated with control siRNA or si-2. (D) Effect of MAPJD on growth of NIH3T3 cells. Expression of MAPJD in stable transfectants of NIH3T3 cells on western blots (upper panels). Cell viability of the stable transfectants evaluated by the MTT assay (lower panel). Assays were performed three times, and in triplicate wells.

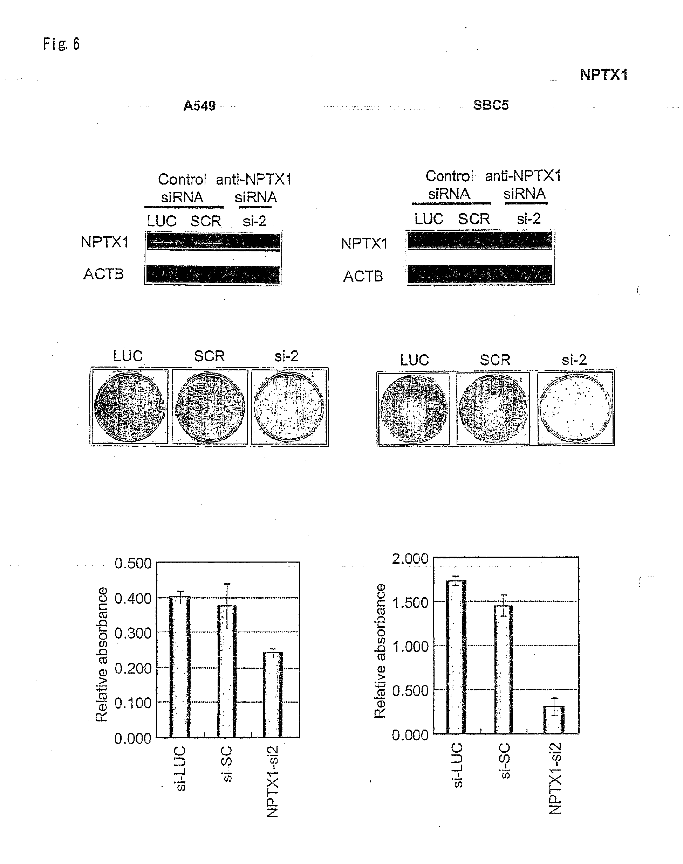

Figure 6 shows the growth effect of NPTX1 (Inhibition of growth of lung cancer cells by siRNA against NPTX1). Upper panels, expression of NPTX1 in response to si-NPTX1 (si-2) or control siRNAs (LUC or SCR) in A549 and SBC-5 cells, analyzed by RT-PCR analysis. Middle panels, the image of colonies examined by colony-formation assays of A549 and SBC-5 cells transfected with specific siRNAs for NPTX1 or control plasmid. Bottom panels, viability of A549 or SBC-5 cells evaluated by MTT assay in response to si-NPTX1 (si-2), -LUC or PCR All assays were performed three times, and in triplicate wells.

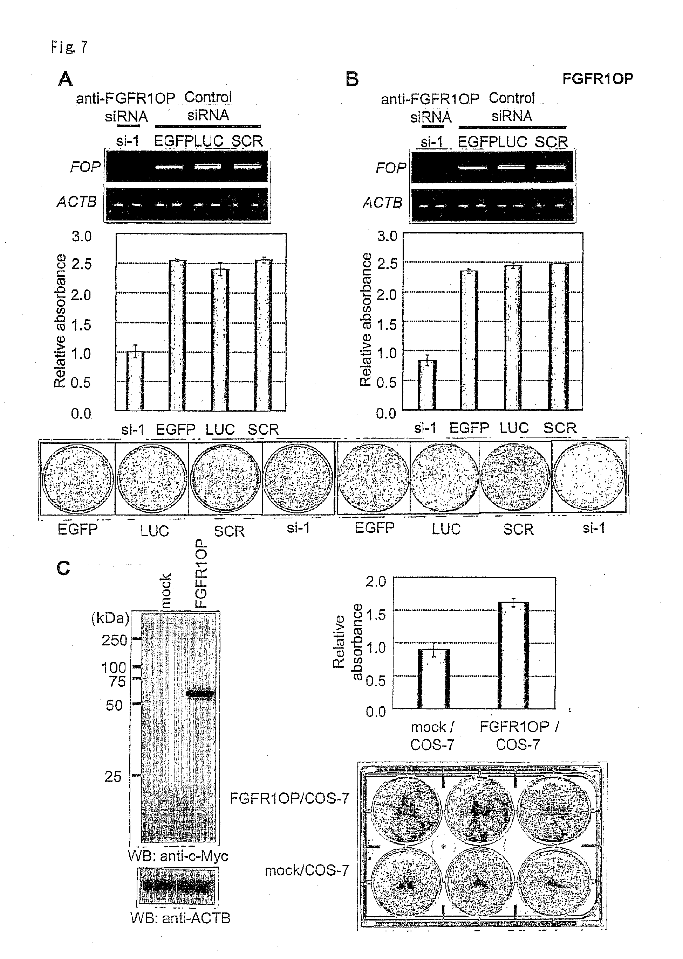

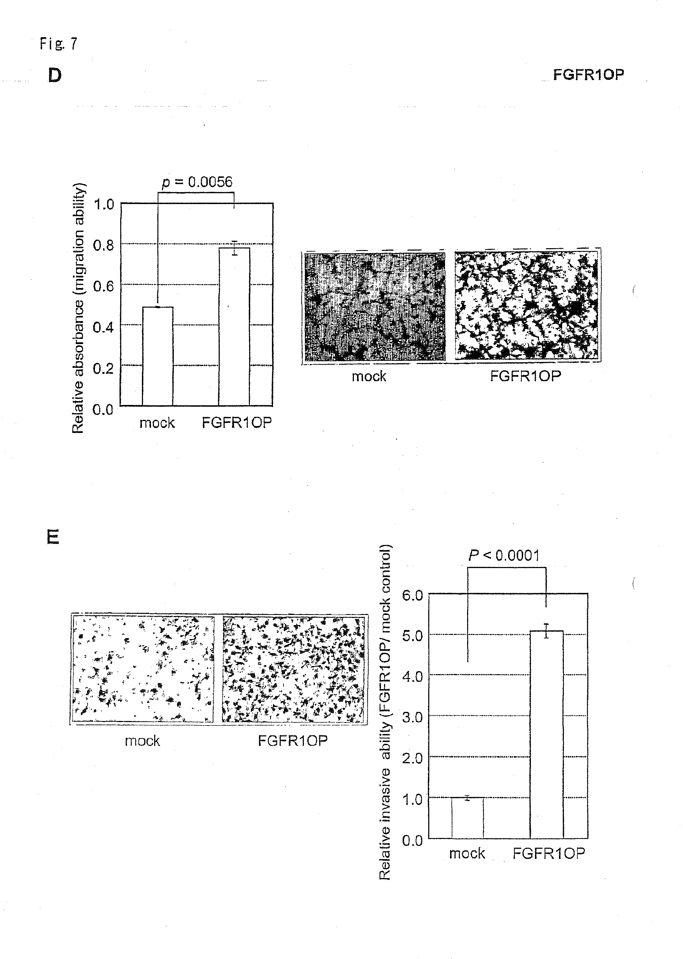

Figure 7 shows the effect of FGFR1OP on growth of cells. (A) and (B) Expression of PGFR1OP in response to si-FGFR1OPs (si-1 and -2) or control siRNAs (EGFP, luciferase (LUC), or scramble (SCR)) in LC319 (A) and SBC-5 (B) cells, analyzed by semi-quantitative RT-PCR (upper panels). Viability of LC319 or SBC-5 cells evaluated by MTT assay in response to si-1, si-2, si-EGFP, si-LUC, or si-SCR (middle panels). Colony-formation assays of LC319 and SBC-5 cells transected with specific siRNAs or control plasmids (lower panels). All experiments were done in triplicate. (C) Growth-promoting effect of FGFR1OP transiently over-expressed in mammalian cells. COS-7 cells that expressed a very low level of endogenous FGFR1OP, were transfected with pcDNA3.1-FGFR1OP-myc-His vector. Whole cell extracts from these cells were used for western-blot analysis with anti-c-Myc antibody (left panels). MTT assay (right upper panels) and Colony-formation assay demonstrating the growth promoting effect of FGFR1OP on COS-7 cells (right lower panels). (D) Cell migration assay demonstrating the increased motility of COS-7 cells transected with expression plasmids for c-myc-tagged FGFR1OP (right panels). Colorimetric measurements and Giemsa staining (x200) (left and right panels). Assays were performed three times, and each in triplicate wells. Whole cell extracts from these cells were used for western-blot analysis with anti-c-Myc antibody (left panels). (E) Assays demonstrating the invasive nature of COS-7 cells in Matrigel matrix after transfection with expression plasmids for FGFR1OP. Giemsa staining (x200) and the number of cells migrating through the Mangel-coated filters (left and right panels). Assays were performed three times, and each in triplicate wells.

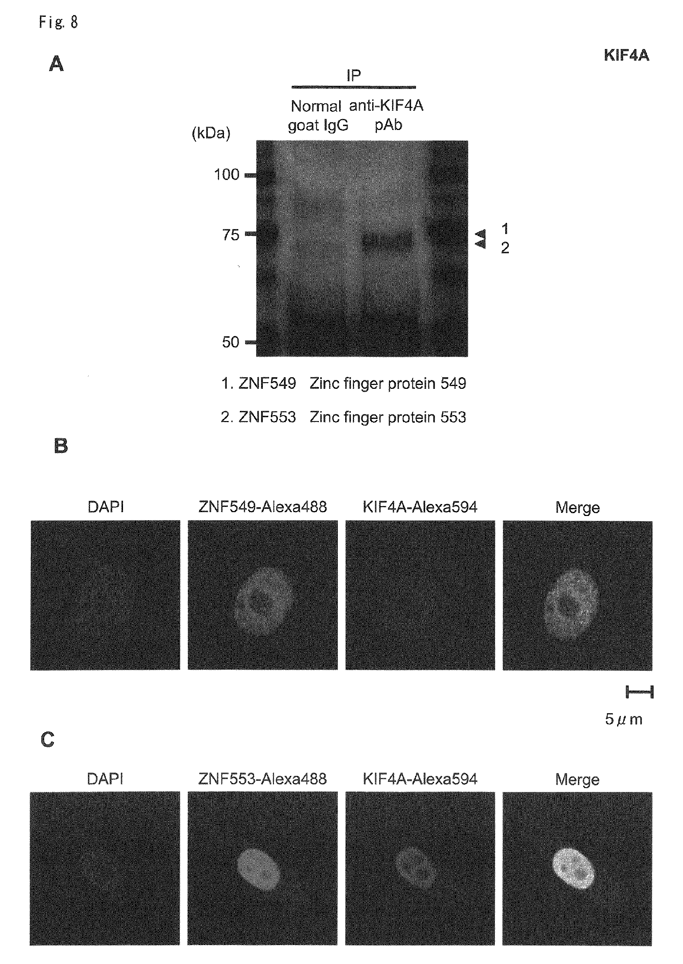

Figure 8 shows the identification of proteins, interacting with KIF4A in lung cancer. (A) Immunoprecipitation of endogenous KIF4A and two interacting proteins from extracts of lung-cancer cell line DMS273. Silver staining of cell extracts immunoprecipitated with anti-KIF4A antibodies, with two specific bands possibly corresponding to endogenous ZNF549 and ZNF553 proteins, which were determined later by MALDI-TOF mass spectrometric analysis. (B) Co-localization of endogenous KIF4A protein with ZNF549 in DMS273 cells. (C) Co-localization of endogenous KIF4A protein with ZNF553 in SBC-5 cells.

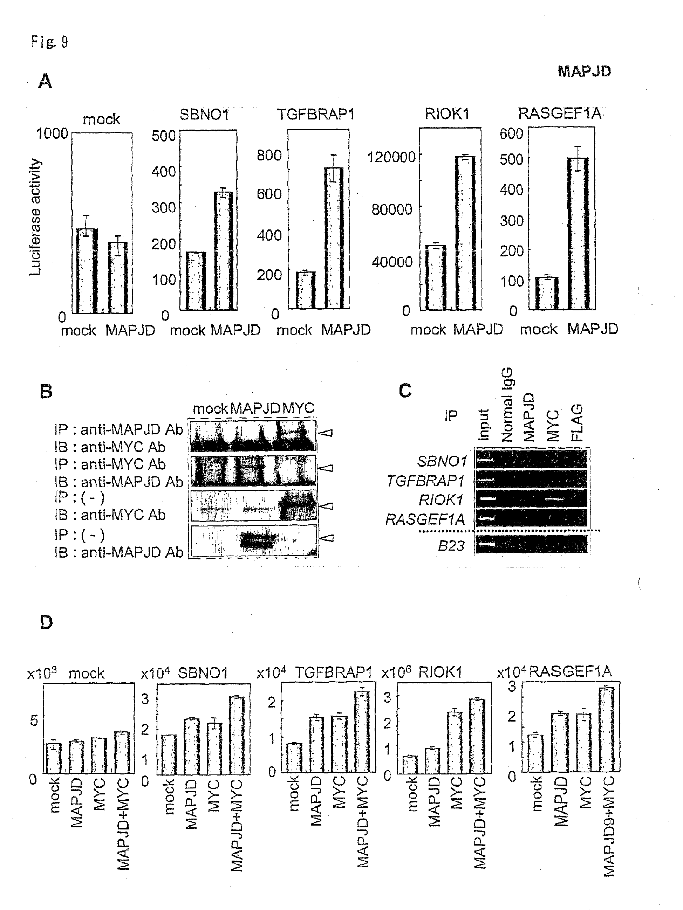

Figure 9 shows the identification of candidate downstream genes of KLAPJD, and interaction of MAPJD with MYC oncogene and their transcriptional regulation. (A) Effect of MAPJD on the luciferase activity of reporter plasmids containing the promoter region of each of the four candidate target genes in LC319 cells. (B) Association of MAPJD and MYC. Reciprocal immunoprecipitation (IP) followed by immunoblotting (IB)-with antibodies to MAPJD or MYC, using extracts of LC319 cells transected with MAPJD- or MYC-expression vectors. (C) Association of endogenous MAPJD or MYC with DNA fragments containing a 1-kb upstream region of the putative transcription start sequence (TSS) of the four candidate MAPJD-target genes and B23 gene (control for IP by representing the binding of MYC), detected by ChIP assay. DNA from LC319 cells was immunoprecipitated with indicated antibodies and served for PCR. (D) Effect of exogenous MAPJD and MYC on the luciferase activity of LC319 cells transfected with reporter genes containing the promoter region of each MAPJD-target gene.

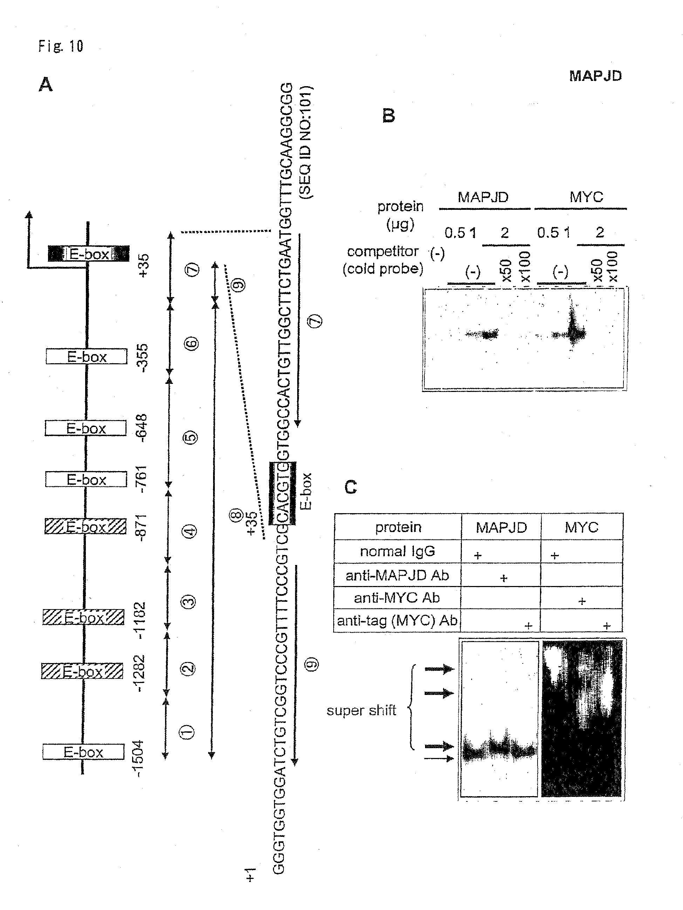

Figure 10 shows the interaction of MAPJD with E-box sequence of the RIOK1 gene. (A) Positions of E-box motifs in the 5' flanking region of the RIOK1 gene. (B) and (C) Sequence specific binding of MAPJD to E-box motif, detected by EMSA. MAPJD or MYC purified by immunoprecipitation methods was incubated with a DNA probe containing the E-box sequence in the genomic segment-7, with or without a competitor (B), as well as with or without anti-MAPJD or MYC antibody (C).
Figure 11 shows the enhancement of MYC-related HAT complex recruitment to the target genes and histone H4 acetylation by MAPJD. (A) Interaction of MAPJD with HAT containing MYC. Immunoprecipitation (IP) with antibodies to endogenous MYC, TRRAP, or TIP60; followed by immunoblotting (IB) with anti-FLAG antibody (M2), using extracts of LC319 cells transfected with FLAG-MAPJD-expressing or mock vectors, (B) Association of endogenous acetylated histone H4 (AcH4) or TRRAP with DNA containing the 5'-flanking region containing E-boxes of the four MAPJD-target genes, detected by ChIP assay. DNAs from LC319 cells transfected with MAPJD-expressing or mock vectors were immunoprecipitated with indicated antibodies and served for PCR. (C) Schematic model for the mechanism of histone acetylation by MYC-related HAT complex and MAPJD.

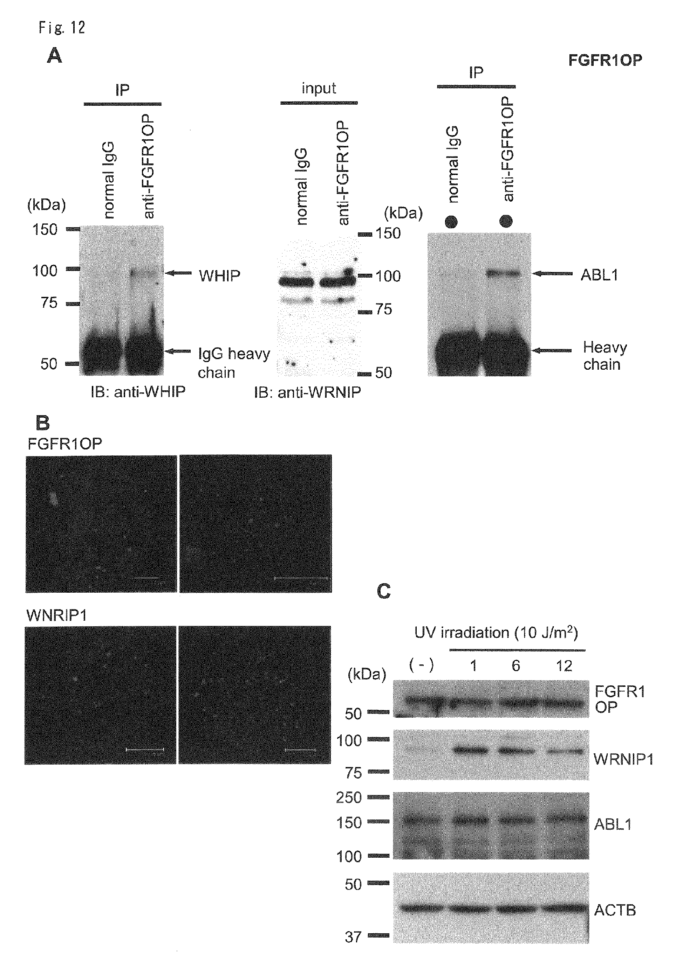

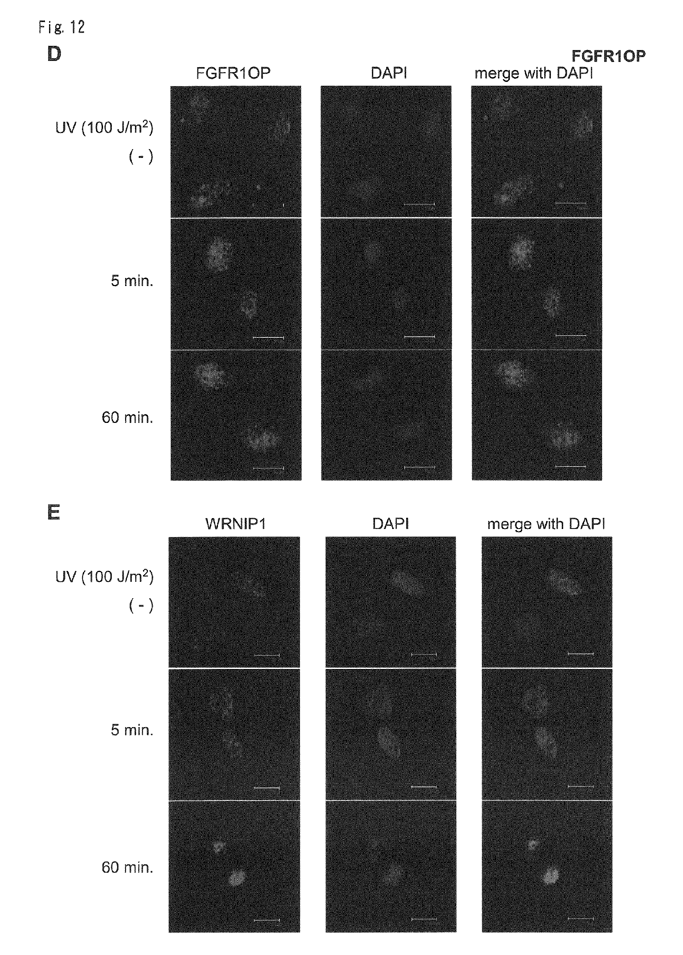

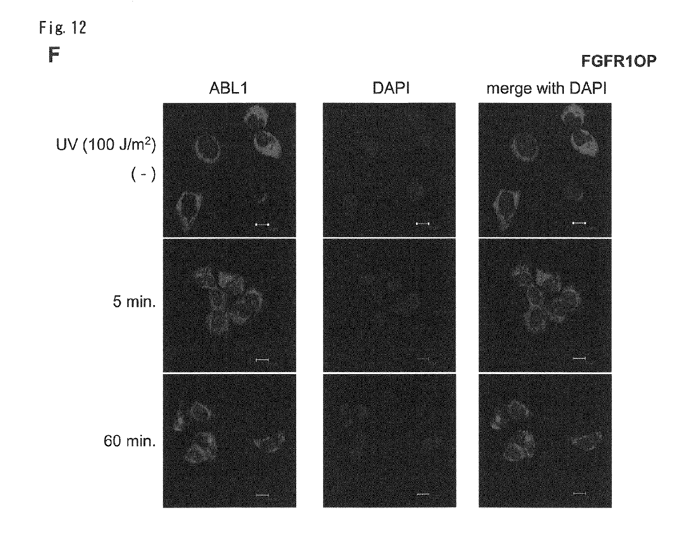

Figure 12 shows the identification of the novel molecules interacting with FGFR1OP. (A) Interaction of endogenous FGFR1OP and WRMIP1 in lung cancer cells. Immunoprecipitations were performed using anti-FGFR1OP antibodies and extracts from LC319 cells. Immunoprecipitants (left panels) and cell extracts (left panels) were subjected to western blot analysis to detect endogenous WRNIP1. IP, immunoprecipitation; IB, immunoblot. Cell extracts (lower right panel) were subjected to western blot analysis to detect endogenous ABL1. (B) Localization of FGFR1OP and WRNIP1 were observed in granular structures in the nucleus of LC319 (left panels) and SBC-5 cells (right panels), LC319 and SBC-5 cells were fixed. The FGFR1OP-Alexa594 (red) and cell nuclei (DAPI) were visualized in red and blue, respectively. (C) A549 cells were treated or not treated with 100J/m2 of UV radiation. At the indicated times after radiation, whole cell lysates were collected and subjected to evaluated by western-blot analysis using anti- FGFR1OP, WRNIP1, ABL1 antibodies. Blots were probed for beta-actin to verify equal loading. (D), (E) The intracellular distribution of FGFR1OP, WRNIP1 and ABL1 in response to DNA damage. The formation of UV-induced FGFR1OP and WRNIP1 foci. A549 cells were treated or not treated with 100J/m2 of UV radiation. At 1 hour after radiation, cells were fixed and Immunostained endogenous FGFR1OP (D) and WRNIP1 (E). The FGFR1OP- or WRNIP1-Alexa488, and cell nuclei (DAPI) were visualized in green (left panels) and blue (middle panels), respectively. Images were merged visualizations in A549 cells (right panels). (F) Nuclear translocation of ABL1 in response to UV radiation. A549 cells were treated or not treated with 100J/m2 of UV radiation. At 1 hour after radiation, cells were fixed and Immuno-stained endogenous ABL1. The ABL1-Alexa488 and cell nuclei (DAPI) were visualized in green (left panels) and blue (middle panels), respectively. Images were merged visualizations in A549 cells (light panels).
Figure 13 shows the phosphorylation of WRNIP1 (a novel molecule interacting with FGFR1OP) by ABL1 *in vitro* and *in vivo*. (A) Kinase assays were conducted by incubating c-myc-tagged FGFR1OP (anti-c-myc immunoprecipitates from COS-7 cells transfected with expression plasmids for c-myc-tagged FGFR1OP) or c-myc-tagged WRN1IP (anti-c-myc immunoprecipitates from COS-7 cells transfected with expression plasmids for c-myc-tagged WRNIP1) with recombinant ABL1 (as kinase). After the kinase reaction, samples were subjected to western-blot analysis with anti- pan-phospho-tyrosine antibodies (upper left and right panels). Blots were probed for c-myc to verify equal loading (lower left and right panels). The phosphorylation of c-myc-tagged FGFR1OP was not detected in the presence of recombinant ABL1 (upper left panel), while the phosphorylation of c-myc-tagged WRNIP1 was detected (upper right panel). The phosphorylated form of WRNIP1 by recombinant ABL1 (#) and the autophosphorylated form of recombinant ABL1 (# #) were indicated. (B) c-myc-tagged WRN1IP was incubated with recombinant ABL1 and [γ-³²P]ATP. The reaction samples were analyzed by SDS-PAGE and autoradiography. The phosphorylated form of WRNIP1 by recombinant ABL1 (#) and the autophosphorylated form of recombinant ABL1 (# #) were indicated. (C) COS-7 cells were co-transfected with expression plasmids for c-myc-tagged WRNIP1 and expression plasmids for Flag-tagged ABL1 or empty plasmids. The anti-c-myc immunoprecipitates (c-myc-tagged WRNIP1) were subjected to western-blot analysis with anti- pan-phospho-tyrosine antibodies (upper panel). Blots were probed for c-myc to verify equal loading (lower panel).

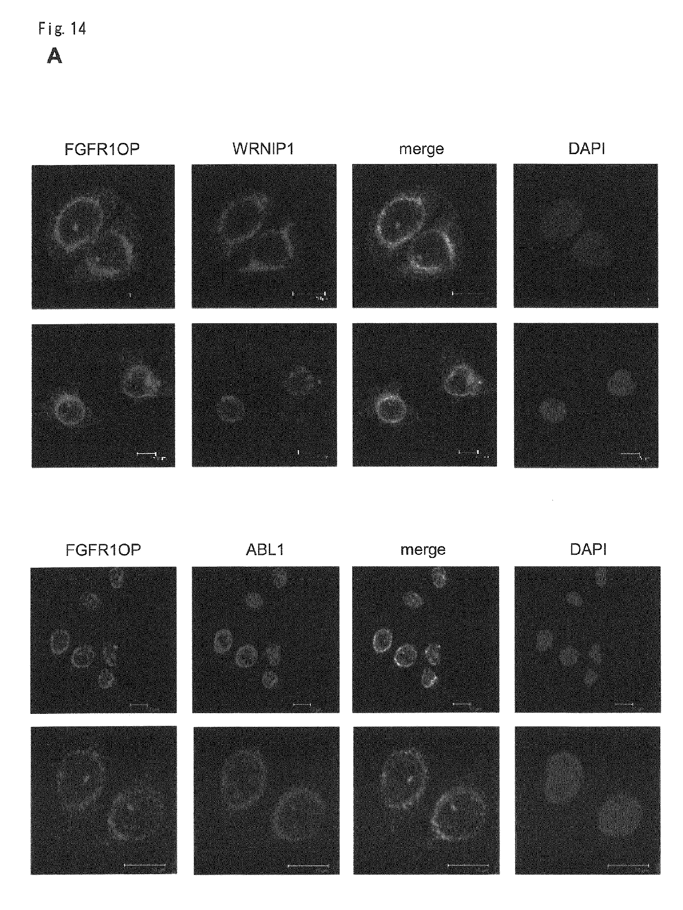

Figure 14 shows a significant reduction of ABL1-dependent phosphorylation of WRNIP1 by FGFR1OP. (A) Immunofluorescence staining of endogenous FGFR1OP and endogenous WRNIP1 in A549 cells. The FGFR1OP-Alexa488 (green), WRNIP1-Alexa594 (red), or cell nuclei (DAPI) were visualized. Co-localization of FGFR1OP and WRNIP1 was observed mainly in perinucleus (upper panels). Immunofluorescence staining of endogenous FGFR1OP and endogenous ABL1 in A549 cells. The FGFR1OP-Alexa488 (green), ABL1-Alexa594 (red), or cell nuclei (DAPI) were visualized. Co-localization of FGFR1OP and ABL1 was observed mainly in perinucleus (lower panels). (B) Inhibition of ABL kinase activity on WRNIP1 by FGFR1OP. Kinase assays were conducted by incubating c-myc-tagged WRN1IP (as substrate) with recombinant ABL1 (as kinase) in the presence of recombinant GST-tagged FGFR1OP or recombinant GST (0, 0.4, 0.8, 2, 4 pmol). After the L-inase reaction, samples were subjected to western-blot analysis with anti-pan-phospho-tyrosine antibodies. (C) The effect of FGFR1OP on ABL1-induced cell-cycle arrest was analyzed by BrdU incorporation assay. A549 cells were co-transfected with expression plasmids for Flag-tagged ABL1 and c-myc-tagged FGFR1OP. The cells were allowed to incorporate BrdU for last 8 hours, and its absorbancies were measured.

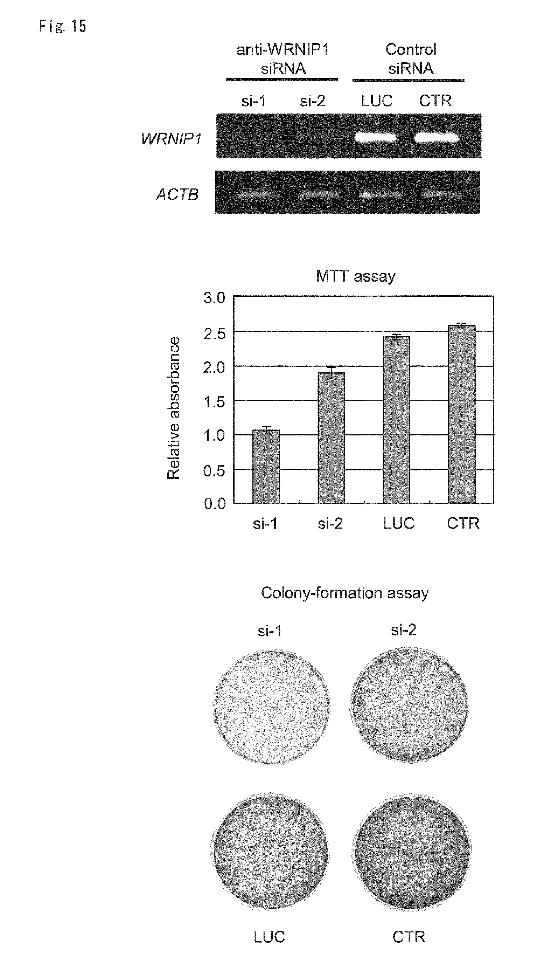

Figure 15 shows a growth promotive effect of WRNIP1. Inhibition of growth of a lung cancer cell line LC319 by siRNAs against WRNIP1. Upper panels, gene knockdown effect on WRNIP1 expression in LC319 cells by two si-WRNIP1 (si-WRNIP1-1 and si-WRNIP-2) and two control siRNAs (si-LUC and si-CTR), analyzed by RT-PCR. Middle and lower panels, MTT and colony formation assays of LC319 cells transfected with si-WRNIPs or control siRNA. Columns of the MTT assay (mid-panel), relative absorbance of triplicate assays; bars, S.D..

### Detailed description of the Intention

Molecular-targeted drugs are expected to be highly specific to malignant cells, with minimal adverse effects due to their well-defined mechanisms of action. As a promising strategy to identify appropriate molecular targets for development of such drugs, the present inventors combined the genome-wide expression analysis that could select genes over-expressed in cancer cells, with high-throughput screening of loss-of-function effects by means of the RNAi technique. In addition, the tissue microarray method was applied to analyze hundreds of archived clinical samples for validation of the potential target proteins. Using this kind of systematic approach, it is demonstrated herein that KIF4A, MAPJD, NPTX1 and FGFR1 OP are frequently over-expressed in clinical lung cancer samples as well as cell lines, and that their gene products play indispensable roles in the growth and progression of lung-cancer cells.

Kinesin superfamily proteins (KIFs), such as KIF4K are microtubule-based motor proteins that generate directional movement along microtubules. Kiss are key players or central proteins in the intracellular transport system, which is essential for cellular function and morphology, including cell division (Zhu C and Jiang W. Proc Nat Acad Sci U S A. 102: 343-8, 2005.). The KIF superfamily is also the first large protein family in mammals whose constituents have been completely identified and confirmed both *in silico* and *in vivo* (Miki H, et al. Proc Natl Acad Sci U S A. 2001 Jun 19;98(13):7004-11.). A large portion of human KIF4A is associated with the nuclear matrix during the interphase, while a small portion is found in the cytoplasm. During mitosis, it is associated with chromosomes throughout the entire process (Lee YM, et al. Biochem J. 2003 Sep 1,374(Pt 2):497-503.): However, the role(s) of KIF4A during carcinogenesis has not been clarified.

Analysis of the molecular roles of the KIF4A protein through interaction with several proteins in carcinogenesis and progression of lung cancer demonstrates that this molecule is a useful target for development of novel therapeutic drugs and prognostic markers for lung cancer. As demonstrated below, two zinc singer proteins, ZNF549 (GenBank Accession No. NM_153263) and ZNF553 (GenBank Accession No. NM_152652), which are also activated in lung cancers, were identified as KIF4A interacting proteins.

MAPJD is previously reported as a nuclear protein with a conserved JmjC domain that is commonly found in DNA- or chromatin-binding domains (Eilbracht J, et al. Mol Biol Cell 2004 Apr; 15(4): 1816-32.). JmjC domain-containing proteins are thought to have the Enzymatic activity that regulates chromatin remodeling and gene expressions. However, the role(s) of the members belonging to the JmjC-containing family during carcinogenesis have not been clarified.

The MYC (c-Myc) oncogene is one of the most frequently over-expressed genes in human cancer (Nesbit CE, et al. 1999, Oncogene 18:3004-16.). The expression level of MYC is tightly associated with cell proliferation in part by regulating genes involved in cell-cycle control MYC functions as a sequence-specific transcription factor belonging to the basic, helix-loop-helix, leucine zipper family. When dimerized with MYC associated factor X (MAX), MYC binds to CACGTG (CANNTG) motifs (E-box) in the genome and activates the transcription of various target genes (Blackwell TK. et al. Science 250: 149-51.; Blackwood EM and Eisenman RN. 1991. Science. 251:1211-7.). Recently, transcriptional activity of MYC/MAX heterodimers was linked to its ability to recruit cofactor complexes containing a transformation/transcription domain-associated protein (TRRAP) together with p300/CBP-associated factor (PCAF, alias GCN5) (McMahon SB, et al. Mol. Cell. 20, 556-62 (2000).) or TIP60 histone acetyltransferases (HATs) (Frank SR, et al. EMBO Rep. 4, 575-80 (2003).).

The present inventor reveals that MAPJD plays a significant role in pulmonary carcinogenesis by activating various downstream target genes through interaction with MYC (Mol Cancer Ther. 2007 Feb;6(2):542-51). Thus, MAPJD is a useful target for development of novel therapeutic drugs for lung cancer.

FGFR1OP was originally identified as a fusion partner for FGFR1 in a t(6;8)(q27;p11) chromosomal translocations giving rise to myeloproliferative disorders (MSD) (Popovici C, et al. Blood 1999;93:1381-9.; Guasch G, et al. Mol Cell Biol 2001;21:8129-42., Blood 2004;103:309-12.). Although it is well established that constitutive activation of tyrosine kinase activity is critical for oncogenesis in leukemia patients carrying some chimera kinase fusion proteins, the role(s) of FGFR1OP during lung carcinogenesis have not been clarified.

On the other hand, Werner helicase interacting protein I (WRNIP1 alias WHIP) (GenBank Accession No. NM_C20135) was known to physically interact with WRN (Werner syndrome protein), which encodes a member of the RecQ subfamily and the DEAH (Asp-Glu-Ala-His) subfamily of DNA and RNA helicases (Kawabe Yi, et al. J Biol Chem 2001:276:20364-9.). WRNIP1 shows homology to replication factor C family proteins, and is conserved from *E. coli* to human (Kawabe Yi, et al. J Biol Chem 2001;276:20364-9.). Studies in yeast and human cells suggest that this gene may affect the aging process and interact with the DNA replication machinery to modulate the function of DNA polymerase δ (POLD) during DNA replication or replication-associated repair. (Kawabe Yi, et al., J Biol Chem. 2001 Jun 8:276(23):20364-9. Epub 2001 Apr 11.; Hishida T, et al., Proc Natl Acad Sci U S A. 2001 Jul 17;99(15):8283-9.; Branzei D, et al., Mol Genet Genomics. 2002 Nov;268(3):371-86. Epub 2002 Oct 8.; Tsurimoto T, et al., Genes Cells. 2005 Jan;10(1):13-22.). Previous report has been shown that WRN roles a tumor suppressor (Nakayama H. Oncogene 2002;21:9008-21.; Agrelo R, et al. Proc Natl Acad Sci U S A 2006;103:8822-7.), but the role(s) of WRNIP1 have not been clarified in tumorigenesis.

On the other hand, the ubiquitously expressed ABL1 is non-receptor tyrosine kinase distributed in the nucleus and cytoplasm (Wen ST, et al., EMBO J. 1996 Apr 1;15(7):1583-95.; Taagepera S, et al., Proc Natl Acad Sci U S A. 1998 Jun 23;95(13):7457-62.). Nuclear ABL1 is activated by genotoxic stress, including DNA double strand breaks and cross-links, induces apoptosis (Kharbanda S, et al., Nature. 1995 Aug 31;376(6543):785-8,; Yuan ZM, et al., Proc Natl Acad Sci U S A. 1997 Feb 18;94(4):1437-40.). Activation of nuclear ABL1 by DNA damage contributes to apoptosis by mechanisms that partly depend on p53, p73 and Rad9 (Agami R, et al., Nature. 1999 Jun 24;399(673S):809-13.; Gong JG, et al., Nature. 1999 Jun 24:399(6738):806-9.; Yoshida K, et al., Mol Cell Biol. 2002 May;22(10):3292-300.; Yuan ZM, et al., J Biol Chem. 1996 Oct 25;271(43):26457-60.; Yuan ZM, et al., Nature. 1999 Jun 24;399(6738):814-7.). In addition, cellular responses to DNA damage involve interaction of ABL1 with DNA repair proteins, including Rad51, Rad52, BRCA1, and a UV-damaged DNA binding protein (Cong F, et al., J Biol Chem. 2002 Sep 20;277(38):34870-8. Epub 2002 Jul 9.; Foray N, et al., Mol Cell Biol. 2002 Jun;22(12):4020-32.; Kitao H & Yuan ZM., J Biol Chem. 2002 Dec 13;277(50):48944-8. Epub 2002 Oct 11.; Yuan ZM, et al., J Biol Chem. 1998 Feb 13;273(7):3799-802.). Also, tyrosine phosphorylation by ABL1 plays important regulatory roles in the DNA damage response, previous reports have been shown that phosphorylation of Rad51 by ABL1 inhibits its strand exchange activity (Yuan ZM, et al., J Biol Chem. 1998 Feb 13;273(7):3799-802.), and that BRCA1 is phosphorylated by ABL1 in an ATM dependent manner (Foray N, et al., Mol Cell Biol. 2002 Jun;22(12):4020-32.). Thus, ABL1 appears to function in coordinating recombinational DNA repair with the induction of apoptosis. Recent study, also, has identified that WRN is phosphorylated by ABL1, its tyrosine phosphorylation inhibits of both WRN exonuclease and helicase activities. Previous reports, WRN, WRNIP1 and POLD may form a ternary complex, function to regulate POLD-mediated DNA synthesis when the replication fork complex is stalled by DNA damage or structural stress (Kawabe Yi, et al., J Biol Chem. 2001 Jun 8;276(23):20364-9. Epub 2001 Apr 11.; Tsurimoto T, et al., Genes Cells. 2005 Jan;10(1):13-22.). While WRNIP1 can be serine- and tyrosine-phosphorylated (Beausoleil SA, et al., Proc Natl Acad Sci U S A. 2004 Aug 17;101(33):12130-5. Epub 2004 Aug 9.; Foray N, et al., Mol Cell Biol. 2002 Jun;22(12):4020-32.; Kitao H & Yuan ZM., J Biol Chem. 2002 Dec 13;277(30):48944-8. Epub 2002 Oct 11.; Yuan ZM, et al., J Biol Chem. 1998 Feb 13;273(7):3799-802.), the kinase which phosphorylates WRNIP1 directly has not been reported. In present invention, it has provided the first evidence that WRNIP1 tyrosine is phosphorylated by ABL1 *in vivo* and *in vitro*.

In the present invention, FGFR1OP is identified as a target for development of novel therapeutic drugs or prognostic markers for lung cancer. In particular, the present invention provides evidence that over-expression of FGFR1OP promotes growth of lung cancer cells. In addition, the invention shows that FGFR1OP interacts with WRNIP1 or ABL1, which play important roles in cell proliferation and differentiation. In this study, it describe that over-expression of FGFR1OP could contribute to the malignant nature of lung cancer cells and that FGFR1OP significantly reduces ABL1-dependent phosphorylation of WRNIP1 and appears to promote cancer cell cycle progression. Furthermore, it is shown that over-expressed FGFR1OP is an essential contributor to the cell migration/movement and genome surveillance pathway, and to aggressive features of lung cancers. Since these data imply the possible molecular roles of FGFR1OP in human pulmonary carcinogenesis, the present inventors suggest that targeting the FGFR1 OP molecule might bold promise for development of a new diagnostic and therapeutic strategy for clinical management of lung cancers.

In the present invention, NPTX1 is a member of a newly recognized subfamily of "long pentraxin" (Goodman AR, et al. Cytokine Growth Factor Rev. 1996 Aug;7(2): 191-202). NPTX1 gene encodes a secreted protein of 430 amino acids with an N-terminal signal sequence and C-terminal pentaxin domain. The "long pentraxins", a newly recognized subfamily of proteins, have several structural and functional characteristics that might play a role in promoting excitatory synapse formation and synaptic remodeling (Schlimgen AK, et al. Neuron. 1995 Mar;14(3):519-26.; Kirkpatrick LL, et al. J Biol Chem. 2000 Jun 9;275(23):17786-92.), Members of this subfamily include neuronal pentraxin 1 (NPTX1), neuronal pentraxin 2 (NPTX2), and neurosal pentraxin receptor (NPTXR) (Schlimgen AK, et al. Neuron. 1995 Mar; 14(3):519-26.; Kirkpatrick LL, et al. J Biol Chem. 2000 Jun 9;275(23);17786-92.; Goodman AR, et al. Cytokine Growth Factor Rev. 1996 Aug;7(2): 191-202, Dodds, J Biol Chem. 1997 Aug 22;272(34):21488-94.). NPTX1 and NPTX2 together have super-additive synaptogenic activity. However, the role of "long pentraxins" during carcinogenesis and its function have not been clarified.

In the study reported here, present inventors identified NPTX1 as a potential target for development of novel therapeutic drugs and diagnostic markers, and revealed possible roles for this molecule in human pulmonary carcinogenesis .

### Definitions:

The words "a", "an", and "the" as used herein mean "at least one" unless otherwise specifically indicated.

The term "polynucleotide" and "oligonucleotide" are used interchangeably herein unless otherwise specifically indicated and are referred to by their commonly accepted single-letter codes. The terms apply to nucleic acid (nucleotide) polymers in which one or more nucleic acids are linked by ester bonding. The polynucleotide or oligonucleotide may be composed of DNA, RNA or a combination thereof.

As use herein, the term "double-stranded molecule" refers to a nucleic acid molecule that inhibits expression of a target gene including, for example, short interfering RNA (siRNA; *e.g.*, double-stranded ribonucleic acid (dsRNA) or small hairpin RNA (shRNA)) and short interfering DNA/RNA (siD/R-NA; *e.g.* double-stranded chimera of DNA and RNA (dsD/R-NA) or small hairpin chimera of DNA and RNA (shD/R-NA)).

In the context of the present invention, a "KIF4A polypeptide" or "KTF4A" refers to a kinesin family member 4A. *See*, *e.g.*, Zhu C and Jiang W. Proc Nat Acad Sci U S A. 102: 343-8, 2005. incorporated by reference herein in its entirety. Amino acid sequence of KIF4A polypeptide can be referred as SEQ ID NO: 53 encoded by the nucleotide sequence of SEQ ID NO: 52. Herein, a "ZNF549 polypeptide" or "ZNF549", and "ZNF553 polypeptide" or "ZNF553" refers to the zinc finger protein 549 and 553, respectively. Examples of "ZNF549 and ZNF553 polypeptides include, *e.g.*, proteins substantially identical to SEQ ID NO: 87 and SEQ ID NO: 89, which are encoded by the nucleotide sequence of SEQ ID NO: 86 and SEQ ID NO: 88, respectively.

In the context of the present invention, an "MAPJD polypeptide" or " MAPJD" refers to a Myc-associated protein with JmjC domain. *See*, *e.g.*, Eilbracht J, et al, Mol Biol Cell. 2004 Apr; 15(4):1816-32. incorporated by reference herein in its entirety. Amino acid sequence of MAPJD polypeptide can be referred as SEQ ID NO: 55 encoded by the nucleotide sequence of SEQ ID NO: 54.

Herein, an "HAT complex" refers to the complex comprising of MYC, TRRAP, and TIP60. In the context of the present invention, a "MYC" refers to c-Myc oncogene which is one of the most frequently over-expressed genes in human cancer. *See*, *e.g.* Nesbit C.E., et al. 1999, Oncogene 18:3004-16. incorporated by reference herein in its entirety. In addition, "TRRAP" refers to transformation/transcription domain-associated protein. *See*, *e.g.* McMahon SB., et al. Mol. Cell. 20, 556-62 (2000). Further, "TIP60" refers to TAT interacting proteins 60 which was originally identified as a coactivator of HIV TAT protein. *See e.g.* Brady ME., et al., 1999. J. Biol. Chem. 274: 17599-604. The amino acid sequence of MYC, TRRAP, and TTP60 polypeptides and nucleotide sequence encoding thereof are available *e.g.* from GenBank Accession numbers NM_002467 for MYC, NM_03496 for TRRAP, and NM_182710, NM_006388 or NM_182709 for TIP60.

In the context of the present invention, an "FGFR1OP polypeptide" or "FGFR1OP" refers to a fibroblast growth factor receptor 1 oncogene partner. FGFR1OP consists of 399 amino acids with a LisH domain. It is suggested that LisH motifs contribute to the regulation of microtubule dynamics, either by mediating dimerization, or else by binding cytoplasmic dynein heavy chain or microtubules directly. *See*, *e.g.* Sapir T, et al. Eur J Biochem 1999;265:181-8.; and Cahana A, et al. Proc Natl Acad Sci U S A 2001;98: 6429-34. incorporated by reference herein in its entirety. Examples of "FGFR1OP polypeptide" includes, *e.g.*, proteins substantially identical to SEQ ID NO: 59, which is encoded by the nucleotide sequence of SEQ ID NO: 58, and also available from GenBank Accession number NM_007045.

Herein, a "WRNIP1 polypeptide" or "WRNEP1" refers to Werner helicase interacting protein 1. Examples of "WRNIP1 polypeptide" includes, *e.g.*, proteins substantially identical to SEQ ID NO: 91, which is encoded by the nucleotide sequence of SEQ ID NO: 90, and also available from GenBank Accession No. NM_020135.

Herein, an "ABL1 polypeptide" or "ABL1" refers to v-abl Abelson murine leukemia viral oncogene homolog 1. Examples of "ABL1 polypeptide" includes, *e.g.*, proteins substantially identical to SEQ ID NO: 92, which is encoded by the nucleotide sequence of SEQ ID NO: 93, and also available from GenBask Accession No. NM_007313.

In the context of the present invention, "target molecule" refers to any one molecule selected from group consisting of KIF4A, MAPJD, NPTX1 and FGFR1OP, or functional equivalents thereof. Further, in the context of the present invention, "partner molecule" refers to any one molecule ZNF549, 2NF553, MYC, WRNIP1, and ABL1, or functional equivalent thereof. In addition, in the present invention, a combination of the target molecule and partner molecule thereof is selected from the group consisting of KIF4A/ZNF549, KIF4A/ZNF553, MAPJD/MYC, FGFR1OP/WRNIP1, FGFR1OP/ABL1 and FGFR1OP/WRNIP1/ABL1.

In the context of the present invention, inhibition of binding" between two proteins refers to at least reducing binding between the proteins. Thus, in some cases, the percentage of binding pairs in a sample will be decreased compared to an appropriate (*e.g.*, not treated with test compound or from a non-cancer sample, or from a cancer sample) control. The reduction in the amount of proteins bound may be, *e.g.*, less than 90%, 80%, 70%, 60%, 50%, 40%, 25%, 10%, 5%, 1% or less (*e.g.*, 0%), than the pairs bound in a control sample.

The term "test compound" refers to any (*e.g.*, chemically or recombinantly-produced) molecule that may disrupt the protein-protein interaction between target molecule and partner molecule thereof, as discussed in detail herein. In some embodiments, the test compounds have a molecular weight of less than 1,500 daltons, and in some cases less than 1,000, 800, 600, 500, or 400 daltons.

A "pharmaceutically effective amount" of a compound is a quantity that is sufficient to treat and/or ameliorate a target molecule-mediated disease in an individual. An example of a pharmaceutically effective amount may an amount needed to decrease the interaction between target molecule and partner molecule, when administered to an animal. The decrease in interaction may be, *e.g*., at least a 5%, 10%, 20%, 30%, 40%, 50%, 75%, 80%, 90%, 95%, 99%, or 100% change in binding. Alternatively, the amount may comprise an amount that, when administered, results in detectably decreased nuclear localization as described herein for wild-type of the target molecule.

The phrase "pharmaceutically acceptable carrier" refers to an inert substance used as a diluent or vehicle for a drug.

In the context of the present invention, the term "functionally equivalent" means that the subject polypeptides has a biological activity of a reference polypeptide. For example, a functional equivalent of KIF4A would have the microtubule-based motor proteins that generate directional movement along microtubules, like wild-type KIF4A. Assays for determining activity of the motor proteins are well known in the art.

Alternatively, for examples, a functional equivalent of MAPJD associated with HAT complex would have the active to acetylation of histone H4 like wild-type MAPJD associated with HAT complex. Essays for determining such activity are well known in the art.

Further, for example, a functional equivalents of FGFR1OP would have the binding activity to WRNIP and/or ABL1 like wild-type FGFR1OP. Assay for determining the binding activity are well known in the art.

The terms "isolated" and "biologically pure" refer to material that is substantially or essentially free from components which normally accompany it as found in its native state. However, the term "isolated" is not intended to refer to the components present in an electrophoretic gel or other separation medium. An isolated component is free from such separation media and in a form ready for use in another application or already in use in the new application/milieu.

The phrase "conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codons, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicitly described in each disclosed sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" wherein the alteration results in the substitution of an amino acid with a chemically similar amino acid. Conservative substitution tables providing functionally similar amino acids are well known in the art. Such conservatively modified variants are in addition to and do not exclude polymorphic variants, interspecies homolog, and alleles of the invention.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyro sine (Y), Tryptophan (W);
7) Serine (S), Threonine (T); and
8) Cysteine (C), Methionine (M) (*see*, *e.g*., Creighton, *Proteins* (1984)).

In the context of the present invention, a "percentage of sequence identity" is determined by comparing two optimally aligned sequences over a comparison window, wherein the portion of the polynucleotide sequence in the comparison window may comprise additions or deletions (*i.e*., gaps) as compared to the reference sequence (*e.g*., a polypeptide of the invention), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or percent "identity", in the context of two or more nucleic acids or polypeptide sequences, refer to two or more sequences or subsequences that are the same sequences. Two sequences are "substantially identical" if two sequences have a specified percentage of amino acid residues or nucleotides that are the same (*i.e*., 60% identity, optionally 65%, 70%, 75%, 80%, 85%, 90%, or 95% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides in length, or more preferable over a region that is 100 to 500 or 1000 or more nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g.,* by the local homology algorithm of Smith and Waterman (1981) Adv. Appl. Math. 2:482-9, by the homology alignment algorithm of Needleman and Wunsch (1970) J. Mol. Biol. 48:443-53, by the search for similarity method of Pearson and Lipman (1988) Proc. Nat'l. Acad. Sci. USA 85:2444-8, by computerized implementations of these algorithms (GAP, BESTFIT, FASTA, and TFASTA in the Wisconsin Genetics Software Package, Genetics Computer Group, 575 Science Dr., Madison. WI), or by manual alignment and visual inspection (see, *e.g.,* Ausubel et al., Current Protocols in Molecular Biology (1995 supplement)).

Two examples of algorithms that are suitable for determining percent sequence identity and sequence similarity are the BLAST and BLAST 2.0 algorithms, which are described in Altschul et al. (1997) Nuc. Acids. Res. 25:3389-402, and Altschul et al. (1990) J. Mop Biol. 215:403-10, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold *(*Altschul et *al., supra).* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each directions are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4 and a comparison of both strands. For amino acid sequences, the BLASTP program uses as defaults a word length of 3, and expectation (E) of 10, and the BLOSUM62 scoring matrix *(see* Henikoff and Henikoff (1989) Proc. Natl. Acad. Sci. USA 89:10915-9) alignments (B) of 50, expectation (E) of 10, M=5, N=-4, and a comparison of both strands.

The BLAST algorithm also performs a statistical analysis of the similarity between two sequences *(see, e.g.,* Karlin and Altschul (1993) Proc. Natl. Acad Sci. USA 90:5873-7). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "small organic molecules" refers to molecules of a size comparable to those organic molecules generally used in pharmaceuticals. The therm excludes biological macromolecules (*e.g*., proteins, nucleic acids, *etc*.). Preferred small organic molecules range in size up to about 5000 Da, *e.g*., up to 2000 Da, or up to about 1000 Da.

The terms "label" and "detectable label" are used herein to refer to any composition detectable by spectroscopic, photochemical, biochemical, immunochemical, electrical, optical or chemical means. Such labels include biotin for staining with labeled streptavidin conjugate, magnetic beads (*e.g*., DYNABEADS™), fluorescent dyes (*e.g*., fluorescein, Texas red, rhodamine, green fluorescent protein, and the like), radiolabels (*e.g*., ³H, ¹²⁵I, ³⁵S, ¹⁴C, or ³²P), enzymes (*e.g*., horse radish peroxidase, alkaline phosphatase and others commonly used in an ELISA), and calorimetric labels such as colloidal gold or colored glass or plastic (*e.g*., polystyrene, polypropylene, latex, *etc.)* beads. Patents teaching the use of such labels include U.S. Pat. Nos. 3,817,837; 3,850,752; 3,939,350; 3,996,345; 4,277,437; 4,275,149; and 4,366,241. Means of detecting such labels are well known to those of skill in the art. Thus, for example, radiolabels may be detected using photographic film or scintillation counters, fluorescent makers may be detected using a photodetector to detect emitted light Enzymatic labels are typically detected by providing the enzyme with a substrate and detecting, the reaction product produced by the action of the enzyme on the substrate, and calorimetric labels are detected by simply visualizing the colored label.

The term "antibody" as used herein encompasses naturally occurring antibodies as well as non-naturally occurring antibodies, including, for example, single chain antibodies, chimeric, bifunctional and humanized antibodies, as well as antigen-binding fragments thereof, (*e.g*., Fab', F(ab')₂. Fab, Fv and rIgG). *See also,* Pierce Catalog and Handbook, 1994-1995 (Pierce Chemical Co., Rockford, IL). *See also, e.g.,* Kuby, J., Immunology, 3rd Ed., W.H. Freeman & Co., New York (1998). Such non-naturally occurring antibodies can be constructed using solid phase peptide synthesis, can be produced recombinantly or can be obtained, for example, by screening combinatorial libraries consisting of variable heavy chains and variable light chains as described by Huse et al., Science 246:1275-81 (1989), which is incorporated herein by reference. These and other methods of making, for example, chimeric, humanized, CDR--grafted, single chain, and bifunctional antibodies are well known to those skilled in the art (Winter and Harris, Immunol. Today 14:243-6 (1993); Ward et al., Nature 341:544-6 (1989); Harlow and Lane, Antibodies A Laboratory Manual, 1988 511-52; Hilyard et al., Protein Engineering: A practical approach (IRL Press 1992); Borrebaeck, Antibody Engineering, 2d ed. (Oxford University Press 1995); each of which is incorporated herein by reference).

The term "antibody" includes both polyclonal and monoclonal antibodies. The term also includes genetically engineered forms such as chimeric antibodies (*e.g.*, humanized murine antibodies) and heteroconjugate antibodies (*e.g*., bispecific antibodies). The term also refers to recombinant single chain Fv fragments (scFv). The term antibody also includes bivalent or bispecific molecules, diabodies, triabodies, and tetrabodies. Bivalent and bispecific molecules are described in, *e.g*., Kostelny et al. (1992) J Immunol 148:1547-53, Pack and Pluckthun (1992) Biochemistry 31:1579-84, Holliger et al. (1993) Proc Natl Acad Sci USA. 90:6444-8, Gruber et al. (1994) J Immunol 152:5368-74, Zhu et al. (1997) Protein Sci 6:781-8, Hu et al. (1996) Cancer Res. 56:3055-61, Adams et al. (1993) Cancer Res. 53:4026-34, and McCartney, et al, (1995) Protein Eng. 8:301-14*.*

Typically, an antibody has a heavy and light chain. Each heavy and light chain contains a constant region and a variable region, (the regions are also known as "domains"). Light and heavy chain variable regions contain four "framework" regions interrupted by three hyper-variable regions, also called "complementarity-determining regions" or "CDRs". The extent of the framework regions and CDRs has been defined. The sequences of the framework regions of different light or heavy chains are relatively conserved within a species. The framework region of an antibody, that is the combined framework regions of the constituent light and heavy chains, serves to position and align the CDRs in three dimensional spaces.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found.

References to "V_{H}" refer to the variable region of an immunoglobulin heavy chain of an antibody, (including the heavy chain of an Fv, scFv, or Fab. References to "V_{L}" refer to the variable region of an immunoglobulin light chain, including the light chain of an Fv, scFv, dsFv or Fab.

The phrase "single chain Fv" or "scFv" refers to an antibody in which the variable domains of the heavy chain and of the light chain of a traditional two chain antibody have been joined to form one chain. Typically, a linker peptide is inserted between the two chains to allow for proper folding and creation of an active binding site.

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g.,* an enzyme, toxin, hormone, growth factor, drug, *etc*.; or (b) the variable region, or a portion thereof, is altered, replaced or exchanged with a variable region having a different or altered antigen specificity.

A "humanized antibody" is an immunoglobulin molecule that contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al., Nature 321:522-5 (1986); Riechmann et al., Nature 332:323-7 (1988); and Presta, Curr. Op. Struct. Biol. 2:593-6 (1992)). Humanization can be essentially performed following the method of Winter and co-workers (Jones et al., Nature 321:522-5 (1986); Riechmann et al., Nature 332:323-7 (1988); Verhoeyen et al., Science 239:1534-6 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species.

The terms "epitope" and "antigenic determinant" refer to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. *See, e.g.,* Epitope Mapping Protocols in Methods in Molecular Biology, Vol. 66, Glenn E. Morris, Ed (1996).

Other compounds have been developed that target and bind to targets in a manner similar to antibodies. Certain of these "antibody mimics" use non-immunoglobulin protein scaffolds as alternative protein frameworks for the variable regions of antibodies. Thus, the term "antibody mimic" refers to non-antibody binding proteins that use non-immunoglobulin protein scaffolds, including adnectins, avimers, single chain polypeptide binding molecules; and antibody-like binding peptidomimetics, as discussed in more detail below. One of skill will recognize that any method of using antibodies described in this document could also be carried out using antibody mimics.

Ku et al. (Proc. Natl. Acad. Sci. U.S.A. 92(14):6552-6556 (1995)) discloses an alternative to antibodies based on cytochrome b562. Ku et al. (1995) generated a library in which two of the loops of cytochrome b562 were randomized and selected for binding against bovine serum albumin. The individual mutants were found to bind selectively with BSA similarly with anti-BSA antibodies.

Lipovsek et al. (U.S. Patent Nos. 6,818,418 and 7,115,396) discloses an antibody mimic featuring a fibronectin or fibronectin-like protein scaffold and at least one variable loop. Known as Adnectins, these fibronectin-based antibody mimics exhibit many of the same characteristics of natural or engineered antibodies, including high affinity and specificity for any targeted ligand. Any technique for evolving new or improved binding proteins can be used with these antibody mimics.

The structure of these fibronectin-based antibody mimics is similar to the structure of the variable region of the IgG heavy chain. Therefore, these mimics display antigen binding properties similar in nature and affinity to those of native antibodies. Further, these fibronectin-based antibody mimics exhibit certain benefits over antibodies and antibody fragments. For example, these antibody mimics do not rely on disulfide bonds for native fold stability, and are, therefore, stable under conditions which would normally break down antibodies. In addition, since the structure of these fibronectin-based antibody mimics is similar to that of the IgG heavy chain, the process for loop randomization and shuffling can be employed in vitro that is similar to the process of affinity maturation of antibodies in vivo.

Beste et al. (Proc. Natl. Acad. Sci. U.S.A. 96(5):1898-1903 (1999)) discloses an antibody mimic based on a lipocalin scaffold (Anticalin®). Lipocalins are composed of a β-barrel with four hypervariable loops at the terminus of the protein. Beste (1999), subjected the loops to random mutagenesis and selected for binding with, for example fluorescein-Three variants exhibited specific binding with fluorescein, with one variant showing binding similar to that of an anti-fluorescein antibody. Further analysis revealed that all of the randomized positions are variable, indicating that Anticalin® would be suitable to be used as an alternative to antibodies.

Anticalins® are small, single chain peptides, typically between 160 and 180 residues, which provide several advantages over antibodies, including decreased cost of production, increased stability in storage and decreased immunological reaction.

Hamilton et al. (U.S. Patent No. 5,770,380) discloses a synthetic antibody mimic using the rigid, non-peptide organic scaffold of calixarene, attached with multiple variable peptide loops used as binding sites. The peptide loops all project from the same side geometrically from the calixarene, with respect to each other. Because of this geometric confirmation, all of the loops are available for binding, increasing the binding affinity to a ligand. However, in comparison to other antibody mimics, the calixarene-based antibody mimic does not consist exclusively of a peptide, and therefore it is less vulnerable to attack by protease enzymes. Neither does the scaffold consist purely of a peptide, DNA or RNA, meaning this antibody mimic is relatively stable in extreme environmental conditions and has a long life span. Further, since the calixarene-based antibody mimic is relatively small, it is less likely to produce an immunogenic response.

Murali et al. (Cell. Mol. Biol. 49(2):209-216 (2003)) discusses a methodology for reducing antibodies into smaller peptidomimetics, they term "antibody like binding peptidomimetics" (ABiP) which can also be useful as an alternative to antibodies.

Silverman et al. (Nat. Biotechnol. 23: 1556-1561 (2005)) discloses fusion proteins that are single-chain polypeptides comprising multiple domains termed "avimers." Developed from human extracellular receptor domains by in vitro exon shuffling and phage display the avimers are a class of binding proteins somewhat similar to antibodies in their affinities and specificities for various target molecules. The resulting multidomain proteins can comprise multiple independent binding domains that can exhibit improved affinity (in some cases subnanomolar) and specificity compared with single-epitope binding proteins. Additional details concerning methods of construction and use of avimers are disclosed, for example, in U.S. Patent App. Pub. Nos. 20040175756, 20050048512, 20050053973, 20050089932 and 20050221384.

In addition to non-immunoglobulin protein frameworks, antibody properties have also been mimicked in compounds comprising RNA molecules and unnatural oligomers (e.g., protease inhibitors, benzodiazepines, purine derivatives and beta-turn mimics) all of which are suitable for use with the present invention.

As known in the art, aptamers are macromolecules composed of nucleic acid that bind tightly to a specific molecular target. Tuerk and Gold (Science. 249:505-510 (1990)) discloses SELEX (Systematic Evolution of Ligands by Exponential Enrichment) method for selection of aptamers. In the SELEX method, a large library of nucleic acid molecules {e.g., 10¹⁵ different molecules) is produced and/or screened with the target molecule. Isolated aptamers can then be further refined to eliminate any nucleotides that do not contribute to target binding and/or aptamer structure (i.e., aptamers truncated to their core binding domain). See, e.g., Jayasena, 1999, Clin. Chem. 45:1628-1650 for review of aptamer technology.

The terms "polypeptide", "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those Amino acids that are later modified, *e.g.,* hydroxyproline, γ-carboxyglutamate, and O-phosphoserine. Amino acid analogs refers to compounds that have the same basic chemical structure as a naturally occurring amino acid, *e.g.,* an α carbon that is bound to a hydrogen, a carboxyl group, an amino group, and an R group, *e. g.,* homoserine, norleucine, methionine sulfoxide, methionine methyl sulfonium. Such analogs may have modified R groups (*e.g.*, norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be preferred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "recombinant" when used with reference, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, *e.g.,* recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed *in vitro,* in general, by the manipulation of nucleic acid, *e.g.,* using polymerases and endonucleases, in a form not normally found in nature. In this manner, operable linkage of different sequences is achieved. Thus an isolated nucleic acid, in a linear form, or an expression vector formed *in vitro* by lighting DNA molecules that are not normally joined, are both considered recombinant for the purposes of this invention. It is understood that once a recombinant nucleic acid is made and reintroduced into a host cell or organism, it will replicate non-recombinantly, *i.e.,* using the *in vivo* cellular machinery of the host cell rather than *in vitro* manipulations; however, such nucleic acids, once produced recombinantly, although subsequently replicated non-recombinantly, are still considered recombinant for the purposes of the invention. Similarly, a "recombinant protein" is a protein made using recombinant techniques, *i.e.,* through the expression of a recombinant nucleic acid as depicted above.

Unless otherwise defined, all technical and scientific terms used herein have the same meaning as commonly understood by one of ordinary skill in the art to which this invention belongs. In case of conflict, the present specification, including definitions, will control.

### I. Diagnosing cancer:

### I-1. Method for diagnosing cancer or a predisposition for developing cancer

The expression of the KIF4A gene was found to be specifically elevated in patients with cancer. Therefore, the gene identified herein as well as its transcription and translation products find diagnostic utility as a marker for cancer and by measuring the expression of the KIF4A gene in a cell sample, cancer can be diagnosed. Specifically, the present invention provides a method for diagnosing cancer or a predisposition for developing cancer in a subject by determining the expression level of the KIF4A gene in the subject.

Cancers that can be diagnosed by the present method include lung cancer. The present method is particularly suited for diagnosing both or either of SCLCs and NSCLCs.

In the context of the present invention, the term "diagnosing" is intended to encompass predictions and likelihood analysis. The present method is intended to be used clinically in making decisions concerning treatment modalities, including therapeutic intervention, diagnostic criteria such as disease stages, and disease monitoring and surveillance for cancer. According to the present invention, an intermediate result for examining the condition of a subject may be provided. Such intermediate result may be combined with additional information to assist a doctor, nurse, or other practitioner to diagnose that a subject suffers from the disease. Alternatively, the present invention may be used to detect cancerous cells in a subject-derived tissue, and provide a doctor with useful information to diagnose that the subject suffers from the disease.

A subject to be diagnosed by the present method is preferably a mammal. Exemplary mammals include, but are not limited to, *e.g.*, human, non-human primate, mouse, rat, dog, cat, horse, and cow.

It is preferred to collect a biological sample from the subject to be diagnosed to perform the diagnosis. Any biological material can be used as the biological sample for the determination so long as it comprises the objective transcription or translation product of the KIF4A gene. The biological samples include, but are not limited to, bodily tissues and fluids, such as blood, sputum, and urine. Preferably, the biological sample contains a cell population comprising an epithelial cell, more preferably a cancerous epithelial cell or an epithelial cell derived from tissue suspected to be cancerous. Further, if necessary, the cell may be purified from the obtained bodily tissues and fluids, and then used as the biological sample.

According to the present invention, the expression level of the KIF4A gene is determined in the subject-derived biological sample. The expression level can be determined at the transcription (nucleic acid) product level, using methods known in the art. For example, the mRNA of the KIF4A gene may be quantified using probes by hybridization methods (*e.g.*, Northern hybridization). The detection may be carried out on a chip or an array. The use of an array is preferable for detecting the expression level of a plurality of genes (*e.g.*, various cancer specific genes) including the present KIF4A gene. Those skilled in the art can prepare such probes utilizing the sequence information of the KIF4A gene (SEQ ID NO: 52; GenBank Accession No. NM_012310). For example, the cDNA of the KIF4A gene may be used as the probes. If necessary, the probe may be labeled with a suitable label, such as dyes and isotopes, and the expression level of the gene may be detected as the intensity of the hybridized labels.

Furthermore, the transcription product of the KIF4A gene may be quantified using primers by amplification-based detection methods (*e.g.*, RT-PCR). Such primers can also be prepared based on the available sequence information of the gene. For example, the primers (SEQ ID NOs:1 and 2) used in the Example may be employed for the detection by RT-PCR, but the present invention is not restricted thereto.

Specifically, a probe or primer used for the present method hybridizes under stringent, moderately stringent, or low stringent conditions to the mRNA of the KIF4A gene. As used herein, the phrase "stringent hybridization) conditions" refers to conditions under which a probe or primer will hybridize to its target sequence, but to no other sequences. Stringent conditions are sequence-dependent and will be different under different circumstances. Specific hybridization of longer sequences is observed at higher temperatures than shorter sequences. Generally, the temperature of a stringent condition is selected to be about 5°C lower than the thermal melting point (Tₘ) for a specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Topically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes or primers (*e.g.*, 10 to 50 nucleotides) and at least about 60 °C for longer probes or primers. Stringent conditions may also be achieved with the addition of destabilizing agents, such as formamide.

Alternatively, the translation product may be detected for the diagnosis of the present invention. For example, the quantity of the KIF4A protein may be determined. A method for determining the quantity of the protein as the translation product includes immunoassay methods that use an antibody or antibody mimic specifically recognizing the protein. The antibody may be monoclonal or polyclonal. Furthermore, any fragment or modification (*e.g.*, chimeric antibody, scFv, Fab, F(ab')₂, Fv, *etc*.) of the antibody may be used for the detection, so long as the fragment retains the binding ability to the KIF4A protein. Methods to prepare these kinds of antibodies for the detection of proteins are well known in the art, and any method may be employed in the present invention to prepare such antibodies and equivalents thereof.

As another method to detect the expression level of the KIF4A gene based on its translation product, the intensity of staining may be observed via immunohistochemical analysis using an antibody or antibody mimic against the KIF4A protein. Namely, the observation of strong staining indicates increased presence of the protein and at the same time high expression level of the KIF4A gene.

Furthermore, the translation product may be detected based on its biological activity. Specifically, herein, the KIF4A protein was demonstrated to have kinase activity, and to be involved in the migration of cancer cells. Thus, kinase activity of the KIF4A protein may be used as an index of the KIF4A protein existing in the biological sample.

Moreover, in addition to the expression level of the KIF4A gene, the expression level of other cancer-associated genes, for example, genes known to be differentially expressed in SCLCs and NSCLCs, may also be determined to improve the accuracy of the diagnosis.

The expression level of cancer marker gene including the KIF4A gene in a biological sample can be considered to be increased if it increases from the control level of the corresponding cancer marker gene by, for example, 10%, 25%, or 50%; or increases to more than 1.1 fold, more than 1.5 fold, more than 2.0 fold, more than 5.0 fold, more than 10.0 fold, or more.

The control level may be determined at the same time with the test biological sample by using a sample(s) previously collected and stored from a subject/subjects whose disease state (cancerous or non-cancerous) is/are known. Alternatively, the control level may be determined by a statistical method based on the results obtained by analyzing previously determined expression level(s) of the KIF4A gene in samples from subjects whose disease state are known. Furthermore, the control level can be a database of expression patterns from previously tested cells. Moreover, according to an aspect of the present invention, the expression level of the KIF4A gene in a biological sample may be compared to multiple control levels, which control levels are determined from multiple reference samples. It is preferred to use a control level determined from a reference sample derived from a tissue type similar to that of the patient-derived biological sample. Moreover, it is preferred, to use the standard value of the expression levels of the KIF4A gene in a population with a Known disease state. The standard value may be obtained by any method known in the art. For example, a range of mean ± 2 S.D. or mean ± 3 S.D. may be used as standard value.

In the context of the present invention, a control level determined from a biological sample that is known not to be cancerous is called "normal control level". On the other hand, if the control level is determined from a cancerous biological sample, it will be called "cancerous control level".

When the expression level of the KIF4A gene is increased compared to the normal control level or is similar to the cancerous control level, the subject may be diagnosed to be suffering from or at a risk of developing cancer. Furthermore, in case where the expression levels of multiple cancer-related genes are compared, a similarity in the gene expression pattern between the sample and the reference which is cancerous indicates that the subject is suffering from or at a risk of developing cancer.

Difference between the expression levels of a test biological sample and the control level can be normalized to the expression level of control nucleic acids, *e.g*. housekeeping genes, whose expression levels are known not to differ depending on the cancerous or non-cancerous state of the cell. Exemplary control genes include, but are not limited to, β-actin, glyceraldehyde 3-phosphate dehydrogenase, and ribosomal protein PI.

### I-2. Assessing efficacy of cancer treatment

The KIF4A gene differentially expressed between normal and cancerous cells also allow for the course of treatment of cancers to be monitored, and the above-described method for diagnosing cancer can be applied for assessing or testing the efficacy of a treatment on cancer. Specifically, the efficacy of a treatment on cancer can be assessed or tested by determining the expression level of the KIF4A gene in a cell(s) derived from a subject undergoing the treatment. If desired, test cell population are obtained from the subject at various time points, before, during, and/or after the treatment. The expression level of the KIF4A gene can be, for example, determined following the method described above under the item of 'I-1. Method for diagnosing cancer or a predisposition for developing cancer'. For instance, the expression level of the KIF4A gene can be determined by immunoassay using anti-KIF4 antibody or antibody mimic. Specifically, immunohistochemical analysis is preferable method for detecting the expression level of the KIF4A gene. In the context of the present invention, it is preferred that the control level to which the detected expression level is compared is determined from the KIF4A gene expression in a cell(s) not exposed to the treatment of interest.

If the expression level of the KIF4A gene is compared to a control level that is determined from a normal cell or a cell population containing no cancer cell, a similarity in the expression level indicates that the treatment of interest is efficacious and a difference in the expression level indicates less favorable clinical outcome or prognosis of that treatment On the other hand, if the comparison is conducted against a control level that is determined from a cancer cell or a cell population containing a cancer cell(s), a difference in the expression level indicates efficacious treatment, while a similarity in the expression level indicates less favorable clinical outcome or prognosis.

Furthermore, the expression levels of the KIF4A gene before and after a treatment can be compared according to the present method to assess the efficacy of the treatment. Specifically, the expression level detected in a subject-derived biological sample after a treatment (i.e., post-treatment level) is compared to the expression level detected in a biological sample obtained prior to treatment onset from the same subject (*i.e.*, pre-treatment level). A decrease in the post-treatment level compared to the pro-treatment level indicates that the treatment of interest is efficacious while an increase in or similarity of the post-treatment level to the pre-treatment level indicates less favorable clinical outcome or prognosis.

As used herein, the term "efficacious" indicates that the treatment leads to a reduction in the expression of a pathologically up-regulated gene, an increase in the expression of a pathologically down-regulated gene or a decrease in size, prevalence, or metastatic potential of carcinoma in a subject. When a treatment of interest is applied prophylactically, "efficacious" means that the treatment retards or prevents the forming of tumor or retards, prevents, or alleviates at least one clinical symptom of cancer. Assessment of the state of tumor in a subject can be made using standard clinical protocols.

In addition, efficaciousness of a treatment can be determined in association with any known method for diagnosing cancer. Cancers can be diagnosed, for example, by identifying symptomatic anomalies, *e.g.*, weight loss, abdominal pain, back pain, anorexia, nausea, vomiting and generalized malaise, weakness, and jaundice.

### II. Sero diagnosing cancer

By measuring the level of NPTX1 in a subject-derived biological sample, the occurrence of cancer or a predisposition to develop cancer in a subject can be determined. Preferably, cancer is lung cancer. Accordingly, the present invention involves determining (*e.g.*, measuring) the level of NPTX1 in a biological sample. Alternatively, according to the present invention, an intermediate result for examining the condition of a subject may be provided. Such intermediate result may be combined with additional information to assist a doctor, nurse, or other practitioner to diagnose that a subject suffers from the disease. Alternatively, the present invention may be used to detect cancerous cells in a subject-derived tissue, and provide a doctor, with useful information to diagnose that the subject suffers from the disease.. Further, subjects with suspected lung cancer may be screened by the present invention. That is, the present invention provides a method for screening subjects with suspected lung cancer, comprising the steps of:
(a) collecting a blood sample from a subject to be screened;
(b) determining a level of NPTX1 in the blood sample;
(c) comparing the NPTX1 level determined in step (b) with that of a normal control; and
(d) judging that a high NPTX1 level in the blood sample, compared to the normal control, indicates subjects with suspected lung cancer.

Any biological materials may be used as the biological sample for determining the level of NPTX1 so long as either the NPTX1 gene or the NPTX1 protein can be detected in the sample. Preferably, the biological sample comprises blood, serum or other bodily fluids such as sputum. The preferred biological sample is blood or blood derived sample. The blood derived sample includes serum, plasma, or whole blood.

The subject diagnosed for cancer according to the method is preferably a mammal and includes human, non-human primate, mouse, rat, dog, cat, horse and cow.

In one embodiment of the present invention, a gene transcript of the NPTX1 gene (*e.g.*, the NPTX1 protein) is detected to determine the NPTX1 level. The NPTX1 gene can be detected and measured using techniques well Known to one of ordinary skill in the art. The gene transcripts detected by the method include both the transcription and translation products, such as mRNA and proteins. For example, sequences corresponding to NPTX1 gene can be used to construct probes for detecting NPTX1 mRNAs by, *e.g.*, Northern blot hybridization analysis. The hybridization of the probe to a gene transcript in a subject biological sample can be also carried out on a DNA array. As another example; the NPTX1 sequence can be used to construct primers for specifically amplifying the NPTX1 polynucleotide in, *e.g.*, amplification-based detection methods such as reverse-transcription, based polymerase chain reaction (RT-PCR).

In an alternate embodiment, the lever of NPTX1 is determined by measuring the quantity of NPTX1 protein in a biological sample. A method for determining the quantity of the NPTX1 protein in a biological sample includes immunoassay methods. In a preferred embodiment, the immunoassay comprises an ELISA.

The NPTX1 level in the biological sample is then compared with an NPTX1 level associated with a reference sample, such as a normal control sample. The phrase "normal control level" refers to the level of NPTX1 typically found in a biological sample of a population not suffering from cancer. The reference sample is preferably of a similar nature to that of the test sample. For example, if the test sample comprises patient serum, the reference sample should also be serum. The NPTX1 level in the biological samples from control and test subjects may be determined at the same time or, alternatively, the normal control level may be determined by a statistical method based on the results obtained by analyzing the level of NPTX1 in samples previously collected from a control group.

The NPTX1 level may also be used to monitor the course of treatment of cancer. In this method, a test biological sample is provided from a subject undergoing treatment for cancer. Preferably, cancer is lung cancer. Preferably, multiple test biological samples are obtained from the subject at various time points before, during or after the treatment. The level of NPTX1 in the post-treatment sample may then be compared with the level of NPTX1 in the pre-treatment sample or, alternatively, with a reference sample (*e.g.*, a normal control level). For example, if the post-treatment NPTX1 level is lower than the pre-treatment NPTX1 level, one can conclude that the treatment was efficacious. Likewise, if the post-treatment NPTX1 level is similar to the normal control NPTX1 level, one can also conclude that the treatment was efficacious.

An "efficacious" treatment is one that leads to a reduction in the level of NPTX1 or a decrease in size, prevalence or metastatic potential of cancer in a subject. When a treatment is applied prophylactically, "efficacious" means that the treatment retards or prevents occurrence of cancer or alleviates a clinical symptom of cancer. The assessment of cancer can be made using standard clinical protocols. Furthermore, the efficaciousness of a treatment can be determined in association with any known method for diagnosing or treating cancer. For example, cancer is routinely diagnosed histopathologically or by identifying symptomatic anomalies such as chronic cough, hoarseness, coughing up blood, weight loss, loss of appetite, shortness of breath, wheezing, repeated bouts of bronchitis or pneumonia and chest pain.

Moreover, the present method for diagnosing cancer may also be applied for assessing the prognosis of a patient with the cancer by comparing the level of NPTX1 in a patient-derived biological sample with that of a reference sample. Preferably, cancer is lung cancer. Alternatively, the level of NPTX1 in the biological sample may be measured over a spectrum of disease stages to assess the prognosis of the patient An increase in the level of NPTX1 as compared to a normal control level indicates less favorable prognosis. A similarity in the level of NPTX1 as compared to a normal control level indicates a more favorable prognosis of the patient.

In the method of diagnosis of the present invention, the blood concentration of either CEA or proGRP, or both, may be referred to, in addition to the blood concentration of NPTX1, to detect lung cancer. Therefore, the present invention provides methods for diagnosing lung cancer, in which NSCLC is detected when the blood concentration of CEA, in addition to the blood concentration of NPTX1, is higher as compared with healthy individuals. Alternatively, the present invention provides methods for diagnosing lung cancer, in which SCLC is detected when the blood concentration of proGRP, in addition to the blood concentration of NTPTX1, is higher as compared with healthy individuals.

The carcinoembryonic Antigen (CEA) was one of the oncofetal antigens to be applied clinically. It is a complex glycoprotein of molecular weight 20,000 that is associated with the plasma membrane of tumor cells, from which it may be released into the blood.

Although CEA was first identified in colon cancer, an abnormal CEA blood level is specific neither for colon cancer nor for malignancy in general. Elevated CEA levels are found in a variety of cancers other than colonic, including lung, pancreatic, gastric, and breast. As described above, CEA has already been used as serological marker for diagnosing or detecting lung cancer. However, the sensitivity of CEA as a marker for lung cancer, especially NSCLC is somewhat insufficient for detecting lung cancer, completely. Alternatively, it is also well known that gastrin-releasing peptide precursor (proGRP) is a serological tumor marker for SCLC. As described above, proGRP has already been used as serological marker for diagnosing or detecting SCLC. However, the sensitivity of proGRP as a marker for SCLC is somewhat insufficient for detecting SCLC, completely. Accordingly, it is required that the sensitivity of diagnosing lung cancer *e.g.* NSCLC and SCLC would be improved.

In the present invention, novel serological marker for lung cancer NPTX1 is provided. Improvement in the sensitivity of diagnostic or detection method for lung cancer may be achieved by the present invention. Namely, the present invention provides a method for diagnosing lung cancer in a subject comprising the steps of:
(a) collecting a blood sample from a subject to be diagnosed;
(b) determining a level of NPTX1 in the blood sample;
(c) comparing the NPTX1 level determined in step (b) with that of a normal control; and
(d) judging that a high NPTX1 level in the blood sample, compared to the normal control, indicates that the subject suffers from lung cancer.

In preferable embodiments, the diagnostic or detection method of the present invention may further comprises the steps of:
(e) determining a level of either of CEA and proGRP, or both in the blood sample;
(f) comparing the either of CEA and proGRP or both level determined in step (e) with that of a normal control; and
(g) judging that either of high NPTX1 and high CEA levels in the blood sample, compared to the normal control, indicate that the subject suffers from NSCLC, or either of high NPTX1 and high proGRP levels in the blood sample, compared to the normal control, indicate that the subject suffers from SCLC.

By the combination between NPTX1 and CEA and/or proGRP, the sensitivity for detection of lung *i.e*. NSCLC and/or SCLC cancer may be significantly improved. For example, in the group analyzed in the working example mentioned later, sensitivity of CEA for NSCLC is about 41 %. In the meantime, that of combination between CEA and NTPX1 increases to 64 %. In the present invention, "combination of CEA and NPTX1" refers either or both level of CEA and NPTX1 is used as marker. In the preferable embodiments, patient with positive either of CEA and NPTX1 may be judged as suffering from NSCLC. The use of combination of NPTX1 and CEA as serological marker for NSCLC is novel.

Similarly, for example, in the group analyzed in the working example mentioned later, sensitivity of proGRP for SCLC is about 38.5 %. In the meantime, that of combination between proGRP and NPTX1 increases to 76.9 %. In the present invention, "combination of proGRP and NPTX1" refers either or both level of proGRP and NPTX1 is used as marker. In the preferable embodiments, patient with positive either of proGRP and NPTX1 may be judged as suffering from SCLC. The use of combination of NPTX1 and proGRP as serological marker for SCLC is novel.

Therefore, the present invention can greatly improve the sensitivity for detecting NSCLC or SCLC patients, compared to determinations based on results of measuring CEA or proGRP alone. Behind this improvement is the fact that the group of CEA- or proGRP-positive patients and the group of NPTX1-positive patients do not match completely. This fact is further described specifically.

First, among patients who, as a result of CEA or proGRP measurements, were determined to have a lower value than a standard value (i.e. not to have lung cancer), there is actually a certain percentage of patients having lung cancer (i.e. NSCLC or SCLC). Such patients are referred to as CEA- or proGRP-false negative patients. By combining a determination based on CEA or proGRP with a determination based on NPTX1, patients whose NPTX1 value is above the standard value can be found from among the CEA- or proGRP-false-negative patients. That is, from among patients falsely determined to be "negative" due to a low blood concentration of CEA or proGRP, the present invention allows to find patients actually having lung cancer. The sensitivity for detecting lung cancer patients was thus improved by the present invention. Generally, simply combining the results from determination using multiple markers may increase the detection sensitivity, but on the other hand, it often causes a decrease in specificity. However, by determining the best balance between sensitivity and specificity, the present invention has determined a characteristic combination that can increase the detection sensitivity without compromising the specificity.

In the present invention, in order to consider the results of CEA or proGRP measurements at the same time, for examples, the blood concentration of CEA or proGRP may be measured and compared with standard values, in the same way as for the aforementioned comparison between the measured values and standard values of NPTX1. For example, how to measure the blood concentration of CEA or proGRP and compare it to standard values are already known. Moreover, ELISA kits for CEA or proGRP are also commercially available. These methods described in known reports can be used in the method of the present invention for diagnosing or detecting lung cancer.

In the present invention, the standard value of the blood concentration of NPTX1 can be determined statistically. For example, the blood concentration of NPTX1 in healthy individuals can be measured to determine the standard blood concentration of NPTX1 statistically. When a statistically sufficient population can bod gathered, a value in the range of twice or three times the standard deviation (S.D.) from the mean value is often used as the standard value. Therefore, values corresponding to the mean value + 2 x S.D. or mean value + 3 x S.D. may be used as standard values. The standard values set as described theoretically comprise 90% and 99.7% of healthy individuals, respectively.

Alternatively, standard values can also be set based on the actual blood concentration of NPTX1 in lung cancer patients. Generally, standard values set this way minimize the percentage of false positives, and are selected from a range of values satisfying conditions that can maximize detection sensitivity. Herein, the percentage of false positive refers to a percentage, among healthy individuals, of patients whose blood concentration of NPTX1 is judged to be higher than a standard value. On the contrary, the percentage, among healthy individuals, of patients whose blood concentration of NPTX1 is judged to be lower than a standard value indicates specificity. That is, the sum of the false positive percentage and the specificity is always 1. The detection sensitivity refers to the percentage of patients whose blood concentration of NPTX1 is judged to be higher than a Standard value, among all lung cancer patients within a population of individuals for whom the presence of lung cancer has been determined.

Furthermore, in the present invention, the percentage of lung cancer patients among patients whose NPTX1 concentration was judged to be higher than a standard value represents the positive predictive value. On the other hand, the percentage of healthy individuals among patients whose NPTX1 concentration was judged to be lower than a standard value represents the negative predictive value. The relationship between these values is summarized in Table 1. As the relationship shown below indicates, each of the values for sensitivity, specificity, positive predictive value, and negative predictive value, which are indexes for evaluating the diagnostic accuracy for lung cancer, varies depending on the standard value for judging the level of the blood concentration of NPTX1.

**[Table 1]**

| Blood concentration of NPTX1 | Lung cancer patients | Healthy individuals | |
|---|---|---|---|
| High | a: True positive | b: False positive | Positive predictive value a/(a+b) |
| Low | c: False negative | d: True negative | Negative predictive value d/(c+d) |
| | Sensitivity a/(a+c) | Specificity d/(b+d) | |

As already mentioned, a standard value is usually set such that the false positive ratio is low and the sensitivity is high. However, as also apparent from the relationship shown above, there is a trade-off between the false positive ratio and sensitivity. That is, if the standard value is decreased, the detection sensitivity increases. However, since the false positive ratio also increases, it is difficult to satisfy the conditions to have a "low false positive ratio". Considering this situation, for example, values that give the following predicted results may be selected as the preferable standard, values in the present invention.

Standard values for which the false positive ratio is 50% or less (that is, standard values for which the specificity is not less than 50%).

Standard values for which the sensitivity is not less than 20%.

In the present invention, the standard values can be set using an ROC curve. A receiver operating characteristic (ROC) curve is a graph that shows the detection sensitivity on the vertical axis and the false positive ratio (that is, "1 - specificity") on the horizontal axis. In the present invention, an ROC curve can be obtained by plotting the changes in the sensitivity and the false positive ratio, which were obtained after continuously varying then standard value for determining the high/low degree of the blood concentration of NPTX1.

The "standard value" for obtaining the ROC curve is a value temporarily used for the statistical analyses. The "standard value" for obtaining the ROC curve can generally be continuously varied within a range that allows to cover all selectable standard values. For example, the standard value can be varied between the smallest and largest measured NPTX1 values in an analyzed population.

Based on the obtained ROC curve, a preferable standard value to be used in the present invention can be selected from a range that satisfies the above-mentioned conditions. Alternatively, a standard value can be selected based on an ROC curve produced by varying the standard values from a range that comprises most of the measured NPTX1 values,

NPTX1 in the blood can be measured by any method that can quantitate proteins. For example, immunoassay, liquid chromatography, surface plasmon resonance (SPR), mass spectrometry, or such can be applied as methods for quantitating proteins. In mass spectrometry, proteins can be quantitated by using a suitable internal standard. Isotope-labeled NPTX1 and such can be used as the internal standard. The concentration of NPTX1 in the blood can be determined from the peak intensity of NPTX1 in the blood and that of the internal standard. Generally, the matrix-assisted laser desorption/ionization (MALDI) method is used for mass spectrometry of proteins. With an analysis method that uses mass spectrometry or liquid chromatography, NPTX1 can also be analyzed simultaneously with other tumor markers (*e.g.* CEA and/or proGRP).

A preferable method for measuring NPTX1 in the present invention is the immunoassay. The amino acid sequence of NPTX1 is known (GenBank Accession Number NM_002522). The amino acid sequence of NPTX1 is shown in SEQ ID N4: 57, and the nucleotide sequence of the cDNA encoding it is shown in SEQ ID NO: 56. Therefore, those skilled in the art can prepare antibodies by synthesizing necessary immunogens based on the amino acid sequence of NPTX1. The peptide used as immunogen can be easily synthesized using a peptide synthesizer. The synthetic peptide can be used as an immunogen by linking it to a carrier protein.

Keyhole limpet hemocyanin, myoglobin, albumin, and such can be used as the carrier protein. Preferable carrier proteins are KLH, bovine serum albumin, and such. The maleimidobenzoyl-N-hydrosuccinimide ester method (hereinafter abbreviated as the MBS method) and such are generally used to link synthetic peptides to carrier proteins.

Specifically, a cysteine is introduced into the synthetic peptide and the peptide is crosslinked to KLH by MBS using the cysteine's SH group. The cysteine residue may be introduced at the N-terminus or C-terminus of the synthesized peptide.

Alternatively, NPTX1 can be obtained as a genetic recombinant based on the nucleotide sequence of NPTX1 (GenBank Accession Number NM_002522). DNAs comprising the necessary nucleotide sequence can be cloned using mRNAs prepared from NPTX1-expressing tissues. Alternatively, commercially available cDNA libraries can be used as the cloning source. The obtained genetic recombinants of NPTX1, or fragments thereof, can also be used as the immunogen. NPTX1 recombinant expressed in this manner are preferrable as the immunogen for obtaining the antibodies used in the present invention. Commercially available NPTX1 recombinants can also be used as the immunogen.

Immunogens obtained in this manner are mixed with a suitable adjuvant and used to immunize animals. Known adjuvants include Freund's complete adjuvant (FCA) and incomplete adjuvant. The immunization procedure is repeated at appropriate intervals until an increase in the antibody titer is confirmed. There are no particular limitations on the immunized animals in the present invention Specifically, animals commonly used for immunization such as mice, rats, or rabbits can be used.

When obtaining the antibodies as monoclonal antibodies, animals that are advantageous for their production may be used. For Example in mice, many myeloma cell lines for cell fusion are known, and techniques that allow to establish hybridomas with a high probability are already established. Therefore, mice are a desirable immunized animal to obtain monoclonal antibodies.

Furthermore, the immunization treatments are not limited to in vitro treatments. Methods for immunologically sensitizing cultured immunocompetent cells *in vitro* can also be employed. Antibody-producing cells obtained by these methods are transformed and cloned. Methods for transforming antibody-producing cells to obtain monoclonal antibodies are not limited to cell fusion. For example, methods for obtaining clonable transformants by virus injection are known.

Hybridomas that produce the monoclonal antibodies used in the present invention can be screened based on their reactivity to NPTX1. Specifically, antibody-producing cells are first selected by using as an index the binding activity toward NPTX1, or a domain peptide thereof, that was used as the immunogen. Positive clones that are selected by this screening are subcloned as necessary.

The monoclonal antibodies to be used in the present invention can be obtained by culturing the established hybridomas under suitable conditions and collecting the produced antibodies. When the hybridomas are homohybridomas, they can be cultured *in vivo* by inoculating them intraperitoneally to syngeneic animals. In this case, monoclonal antibodies are collected as ascites fluid. When heterohybridomas are used, they can be cultured in vivo using nude mice as a host.

In addition to *in vivo* cultures, hybridomas are also commonly cultured *ex vivo*, in a suitable culture environment. For example, basal media such as RPMI 1640 and DMEM are generally used as the medium for hybridomas. Additives such as animal sera can be added to these media to maintain the antibody-producing ability to a high level. When hybridomas are cultured *ex vivo*, the Monoclonal antibodies can be collected as a culture supernatant. Culture supernatants can be collected by separating from cells after culturing, or by continuously collecting while culturing using a culture apparatus that uses a hollow fiber.

Monoclonal antibodies used in the present invention are prepared from monoclonal antibodies collected as ascites fluid or culture supernatants, by separating immunoglobulin fractions by saturated ammonium sulfate precipitation and further purifying by gel nitration, ion exchange chromatography, or such. In addition, if the monoclonal antibodies are IgGs, purification methods based on affinity chromatography with a protein A or protein G column are effective.

On the other hand, to obtain antibodies used in the present invention as polyclonal antibodies, blood is drawn from animals whose antibody titer increased after immunization, and the serum is separated to obtain an anti-serum. Immune globulins are purified from antisera by known methods to prepare the antibodies used in the present invention. NPTX1-specific antibodies can be prepared by combining immunoaffinity chromatography which uses NPTX1 as a ligand with immunoglobulin purification.

When antibodies against NPTX1 contact NPTX1, the antibodies bind to the antigenic determinant (epitope) that the antibodies recognize through an antigen-antibody reaction. The binding of antibodies to antigens can be detected by various immunoassay principles. Immunoassays can be broadly categorized into heterogeneous analysis methods and homogeneous analysis methods. To maintain the sensitivity and specificity of immunoassays to a high level, the use of monoclonal antibodies is desirable. Methods of the present invention for measuring NPTX1 by various immunoassay formats are specifically explained.

First, methods for measuring NPTX1 using a heterogeneous immunoassay are described. In heterogeneous immunoassays, a mechanism for detecting antibodies that bound to NPTX1 after separating them from those that did not bind to NPTX1 is required.

To facilitate the separation, immobilized reagents are generally used. For example, a solid phase onto which antibodies recognizing NPTX1 have been immobilized is first prepared (immobilized antibodies). NPTX1 is made to bind to these, and secondary antibodies are further reacted thereto.

When the solid phase is separated from the liquid phase and further washed, as necessary, secondary antibodies remain on the solid phase in proportion to the concentration of NPTX1. By Labeling the secondary antibodies, NFTX7 can be quantitated by measuring the signal derived from the label.

Any method may be used to bind the antibodies to the solid phase. For example, antibodies can be physically adsorbed to hydrophobic materials such as polystyrene. Alternatively, antibodies can be chemically bound to a variety of materials having functional groups on their surfaces. Furthermore, antibodies labeled with a binding ligand can be bound to a solid phase by trapping them using a binding partner of the ligand. Combinations of a binding ligand and its binding partner include avidin-biotin and such. The solid phase and antibodies can be conjugated at the same time or before the reaction between the primary antibodies and NPTX1.

Similarly, the secondary antibodies do not need to be directly labeled. That is, they can be indirectly labeled using antibodies against antibodies or using binding reactions such as that of avidin-biotin.

The concentration of NPTX1 in a sample is determined based on the signal intensities obtained using standard samples with known NPTX1 concentrations.

Any antibody can be used as the immobilized antibody and secondary antibody for the heterogeneous immunoassays mentioned above, so long as it is an antibody, or a fragment comprising an antigen-binding site thereof, that recognizes NPTX1. Therefore, it may be a monoclonal antibody, a polyclonal antibody, or a mixture or combination of both. For example, a combination of monoclonal antibodies and polyclonal antibodies is a preferable combination in the present invention. Alternatively, when both antibodies are monoclonal antibodies, combining monoclonal antibodies recognizing different epitopes is preferable.

Since the antigens to be measured are sandwiched by antibodies, such heterogenous immunoassays are called sandwich methods. Since sandwich methods excel in the measurement sensitivity and the reproducibility, they are a preferable measurement principle in the present invention.

The principle of competitive inhibition reactions can also be applied to the heterogeneous immunoassays. Specifically, they are immunoassays based on the phenomenon where NPTX1 in a sample competitively inhibits the binding between NPTX1 with a Known concentration and an antibody. The concentrations of NPTX1 in the sample can be determined by labeling NPTX1 with a known concentration and measuring the amount of NPTX1 that reacted (or did not react) with the antibody.

A competitive reaction system is established when antigens with a known concentration and antigens in a sample are simultaneously reacted to an antibody. Furthermore, analyses by an inhibitory reaction system are possible when antibodies are reacted with antigens in a sample, and antigens with a known concentration are reacted thereafter. In both types of reaction system, reaction systems that excel in the operability can be constructed by setting either one of the antigens with a known concentration used as a reagent component or the antibody as the labeled component, and the other one as the immobilized reagent.

Radioisotopes, fluorescent substances, luminescent substances, substances having an enzymatic activity, macroscopically observable substances, magnetically observable substances, and such are used in these heterogeneous immunoassays. Specific examples of these labeling substances are shown below.

Substances having an enzymatic activity:
peroxidase,
alkaline phosphatase,
urease, catalase,
glucose oxidase,
lactate dehydrogenase, or
amylase, etc.

Fluorescent substances:
fluorescein isothiocyanate,
tetramethylrhodamine isothiocyanate,
substituted rhodamine isothiocyanate, or
dichlorotriazine isothiocyanate, etc.

Radioisotopes:
tritium,
¹²⁵I, or
¹³¹I, etc.

Among these, non-radioactive labels such as enzymes are an advantageous label in terms of safety, operability, sensitivity, and such. Enzymatic labels can be linked to antibodies or to NPTX1 by known methods such as the periodic acid method or maleimide method.

As the solid phase, beads, inner walls of a container, fine particles, porous carriers, magnetic particles, or such are used. Solid phases formed using materials such as polystyrene, polycarbonate, polyvinyltoluene, polypropylene, polyethylene, polyvinyl chloride, nylon, polymethacrylate, latex, gelatin, agarose, glass, metal, ceramic, or such can be used. Solid materials in which functional groups to chemically bind antibodies and such have been introduced onto the surface of the above solid materials are also known. Known binding methods, including chemical binding such as poly-L-lysine or glutaraldehyde treatment and physical adsorption, can be applied for solid phases and antibodies (or antigens).

Although the steps of separating the solid phase from the liquid phase and the washing steps are required in all heterogeneous immunoassays exemplified herein, these steps can easily be performed using the immunochromatography method, which is a variation of the sandwich method.

Specifically, antibodies to be immobilized are immobilized onto porous carriers capable of transporting a sample solution by the capillary phenomenon, then a mixture of a sample comprising NPTX1 and labeled antibodies is deployed therein by this capillary phenomenon. During deployment, NPTX1 reacts with the labeled antibodies, and when it further contacts the immobilized antibodies, it is trapped at that location. The labeled antibodies that did not react with NPTX1 pass through, without being trapped by the immobilized antibodies.

As a result, the presence of NPTX1 can be detected using, as an index, the signals of the labeled antibodies that remain at the location of the immobilized antibodies. If the labeled antibodies are maintained upstream in the porous carrier in advance, all reactions can be initiated and completed by just dripping in the sample solutions and an extremely simple reaction system can be constructed. In the immunochromatography method, labeled components that can be distinguished macroscopically, such as colored particles, can be combined to construct an analytical device that does not even require a special reader.

Furthermore, in the immunochromatography method, the detection sensitivity for NPTX1 can be adjusted. For example, by adjusting the detection sensitivity near the cutoff value described below, the aforementioned labeled components can be detected when the cutoff value is exceeded. By using such a device, whether a subject is positive or negative can be judged very simply. By adopting a constitution that allows a macroscopic distinction of the labels, necessary examination results can be obtained by simply applying blood samples to the device for immunochromatography.

Various methods for adjusting the detection sensitivity of the immunochromatography method are known. For example, a second immobilized antibody for adjusting the detection sensitivity can be placed between the position where samples are applied and the immobilized antibodies (Japanese Patent Application Kokai Publication No. (JP-A) H06-3419S9 (unexamined, published Japanese patent application)). NPTX1 in the sample is trapped by the second immobilized antibody while deploying from the position where the sample was applied to the position of the first immobilized antibody for label detection. After the second immobilized antibody is saturated, NPTX1 can reach the position of the first immobilized antibody located downstream. As a result, when the concentration of NPTX1 comprised in the sample exceeds a predetermined concentration, NPTX1 bound to the labeled antibody is detected at the position of the first immobilized antibody.

Next, homogeneous immunoassays are explained. As opposed to heterogeneous immunological assay methods that require a separation of the reaction solutions as described above, MPTX1 can also be measured using homogeneous analysis methods. Homogeneous analysis methods allow the detection of antigen-antibody reaction products without their separation from the reaction solutions.

A representative homogeneous analysis method is the immunoprecipitation reaction, in which antigenic substances are quantitatively analyzed by examining precipitates produced following an antigen-antibody reaction. Polyclonal antibodies are generally used for the immunoprecipitation reactions. When monoclonal antibodies are applied, multiple types of monoclonal antibodies that bind to different epitopes of NPIX1 are preferably used. The products of precipitation reactions that follow the immunological reactions can be macroscopically observed or can be optically measured for conversion into numerical data.

The immunological particle agglutination reaction, which uses as an index the agglutination by antigens of antibody-sensitized fine particles, is a common homogeneous analysis method. As in the aforementioned immunoprecipitation reaction, polyclonal antibodies or a combination of multiple types of monoclonal antibodies can be used in this method as well. Fine particles can be sensitized with antibodies through sensitization with a mixture of antibodies, or they can be prepared by mixing particles sensitized separately with each antibody. Fine particles obtained in this manner gives matrix-like reaction products upon contact with NPTX1. The reaction products can be detected as particle aggregation. Particle aggregation may be macroscopically observed or can be optically measured for conversion into numerical data.

Immunological analysis methods based on energy transfer and enzyme channeling are known as homogeneous immunoassays. In methods utilizing energy transfer, different optical labels having a donor/acceptor relationship are linked to multiple antibodies that recognize adjacent epitopes on an antigen. When an immunological reaction takes place, the two parts approach and an energy transfer phenomenon occurs, resulting in a signal such as quenching or a change in the fluorescence wavelength. On the other hand, enzyme channeling utilizes labels for multiple antibodies that bind to adjacent epitopes, in which the labels are a combination of enzymes having a relationship such that the reaction product of one enzyme is the substrate of an other. When the two parts approach due to an immunological reaction, the enzyme reactions are promoted; therefore, their binding can be detected as a change in the enzyme reaction rate.

In the present invention, blood for measuring NPTX1 can be prepared from blood drawn from patients. Preferable blood samples are the serum or plasma. Serum or plasma samples can be diluted before the measurements. Alternatively, the whole blood can be measured as a sample and the obtained measured value can be corrected to determine the serum concentration. For example, concentration in whole blood can be corrected to the serum concentration by determining the percentage of corpuscular volume in the same blood sample.

In a preferred embodiment, the immunoassay comprises an ELISA. The present inventors established sandwich ELISA to detect serum NPTX1 in patients with respectable lung cancer.

The NPTX1 level in the blood samples is then compared with an NPTX1 level associated with a reference sample such as a normal control sample. The phrase "normal control level" refers to the level of NPTX1 typically found in a blood sample of a population not suffering from lung cancer. The reference sample is preferably of a similar nature to that of the test sample. For example, if the test samples comprises patient serum, the reference sample should also be serum. The NPTX1 level in the blood samples from control and test subjects may be determined at the same time or, alternatively, the normal control level may be determined by a statistical method based on the results obtained by analyzing the level of NPTX1 in samples previously collected from a control group.

The NPTX1 level may also be used to monitor the course of treatment of lung cancer. In this method, a test blood sample is provided from a subject undergoing treatment for lung cancer. Preferably, multiple test blood samples are obtained from the subject at various time points before, during, or after the treatment. The level of NPTX1 in the post-treatment sample may then be compared with the level of NPTX1 in the pre-treatment sample or, alternatively, with a reference sample (*e.g*., a normal control level). For example, if the post-treatment NPTX1 level is lower than the pre-treatment NPTX1 level, one can conclude that the treatment was efficacious. Likewise, if the post-treatment NPTX1 level is similar to the normal control NPTX1 level, one can also conclude that the treatment was efficacious.

An "efficacious" treatment is one that leads to a reduction in the level of NPTX1 or a decrease in size, prevalence, or metastatic potential of lung cancer in a subject. When a treatment is applied prophylactically, "efficacious" means that the treatment retards or prevents occurrence of lung cancer or alleviates a clinical symptom of lung cancer. The assessment of lung cancer can be made using standard clinical protocols. Furthermore, the efficaciousness of a treatment can be determined in association with any known method for diagnosing or treating lung cancer. For example, lung cancer is routinely diagnosed histopathologically or by identifying symptomatic anomalies.

The diagnosis and detection of lung cancers have been encountering high difficulties. The present invention provides an ELISA for serum NPTX1 is a promising tool to screen lung cancer by combining with other serum makers, *e.g*. CEA and/or proGRP.

Components used to carry out the diagnosis of lung cancer according to the present invention can be combined in advance and supplied as a testing kit. Accordingly, the present invention provides a kit for detecting a lung cancer, comprising:
(i) an immunoassay reagent for determining a level of NPTX1 in a blood sample; and
(ii) a positive control sample for NPTX1.

In the preferable embodiments, the kit of the present invention may further comprise:
(iii) an immunoassay reagent for determining a level of either of CEA and proGRP or both in a blood sample; and
(iv) a positive control sample for CEA and/or proGRP.

The reagents for the immunoassays which constitute a kit of the present invention may comprise reagents necessary for the various immunoassays described above. Specifically, the reagents for the immunoassays comprise an antibody that recognizes the substance to be measured. The antibody can be modified depending on the assay format of the immunoassay. ELISA can be used as a preferable assay format of the present invention. In ELISA, for example, a first antibody immobilized onto a solid phase and a second antibody having a label are generally used.

Therefore, the immunoassay reagents for ELISA can comprise a first antibody immobilized onto a solid phase carrier. Fine particles or the inner walls of a reaction container can be used as the solid phase carrier. Magnetic particles can be used as the fine particles. Alternatively, multi-well plates such as 96-well microplates are often used as the reaction containers. Containers for processing a large number of samples, which are equipped with wells having a smaller volume than in 96-well microplates at a high density, are also known. In the present invention, the inner walls of these reaction containers can be used as the solid phase carriers.

The immunoassay reagents for ELISA may further comprise a second antibody having a label. The second antibody for ELISA may be an antibody onto which an enzyme is directly or indirectly linked. Methods for chemically linking an enzyme to an antibody are known. For example, immunoglobulins can be enzymatically cleaved to obtain fragments comprising the variable regions. By reducing the -SS- bonds comprised in these fragments to -SH groups, bifunctional linkers can be attached. By linking an enzyme to the bifunctional linkers in advance, enzymes can be linked to the antibody fragments.

Alternatively, to indirectly link an enzyme, for example, the avidin-biotin binding can be used. That is, an enzyme can be indirectly linked to an antibody by contacting a biotinylated antibody with an enzyme to which avidin has been attached. In addition, an enzyme can be indirectly linked to a second antibody using a third antibody which is an enzyme-labeled antibody recognizing the second antibody. For example, enzymes such as those exemplified above can be used as the enzymes to label the antibodies.

Kits of the present invention comprise a positive control for NPTX1. A positive control for NPTX1 comprises NPTX1 whose concentration has been determined in advance. Preferable concentrations are, for example, a concentration set as the standard value in a testing method of the present invention. Alternatively, a positive control having a higher concentration can also be combined. The positive control for NPTX1 in the present invention can additionally comprise CEA and/or proGRP whose concentration has been determined in advance. A positive control comprising either CEA or proGRP, or both, and NPTX1 is preferable as the positive control of the present invention.

Therefore, the present invention provides a positive control for detecting lung cancer, which comprises either CEA or proGRP, or both, in addition to NPTX1 at concentrations above a normal value. Alternatively, the present invention relates to the use of a blood sample comprising CEA and/or proGRP and NPTX1 at concentrations above a normal value in the production of a positive control for the detection of lung cancer. It has been known that CEA and proGRP can serve as an index for lung cancer; however, that NPTX1 can serve as an index for lung cancer is a novel finding obtained by the present invention. Therefore, positive controls comprising NPTX1 in addition to CEA or proGRP are novel. The positive controls of the present invention can be prepared by adding CEA and/or proGRP and NPTX1 at concentrations above a standard value to blood samples. For example, sera comprising CEA and/or proGRP and NPTX1 at concentrations above a standard value are preferable as the positive consols of the present invention.

The positive controls in the present invention are preferably in a liquid form. In the present invention, blood samples are used as samples. Therefore, samples used as controls also need to be in a liquid form. Alternatively, by dissolving a dried positive control with a predefined amount of liquid at the time of use, a control that gives the tested concentration can be prepared. By packaging, together with a dried positive control, an amount of liquid necessary to dissolve it, the user can obtain the necessary positive control by just mixing them. NPTX1 used as the positive control can be a naturally-derived protein or it may be a recombinant protein. Similarly, for CEA, a naturally-derived protein can be used. Not only positive controls, but also negative controls can be combined in the kits of the present invention. The positive controls or negative controls are used to verify that the results indicated by the immunoassays are correct.

### III. Antibody

The terms "antibody" as used herein is intended to include immunoglobulins and fragments thereof which are specifically reactive to the designated protein or partial peptide thereof. The present invention provides an antibody that specifically binds to the polypeptide of FGFR1OP (SEQ ID NO: 59) or NPTX1 (SEQ ID NO: 57). In the present invention, the term "specifically" refers to an antibody that distinguishes FGFR1OP or NPTX1. In the preferred embodiment, the present invention provides an antibody which binds to the FGFR1OP specific amino acid sequence from 7 to 173 (SEQ ID NO: 98) of SEQ ID NO: 59or an antibody which binds to the NPTX1 specific amino acid sequence from 20 to 145 (SEQ ID NO: 99) or 297 to 430 (SEQ ID NO: 100) of SEQ ID NO: 57. An antibody can include human antibodies, primatized antibodies, chimeric antibodies, bispecific antibodies, humanized antibodies, antibodies fused to other proteins or radiolabels, and antibody fragments. Furthermore, an antibody herein is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity. An "antibody" indicates all classes (e.g. IgA, IgD, IgE, IgG and IgM). "Antibody fragments" comprise a portion of an intact antibody, generally the antigen binding or variable region of the intact antibody. Examples of antibody fragments include Fab, Fab', F(ab')2, and Fv fragments; linear antibodies; and single chain antibody molecules.

### Production of Antibodies

The subject invention uses antibodies to FGFR1OP or NPTX1. These antibodies will be provided by known methods so long as antigen polypeptide is a sequence selected from the group consisting of SEQ ID NO: 98 (for FGGR1OP), SEQ ID NO: 99 (for NPTX1) and SEQ ID NO: 100 (for NPTX1) (see ***Preparation of antibodies*** in EXAMPLE).

Exemplary techniques for the production of the antibodies used in accordance with the present invention are described.

### III-I. Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOC12, or RN=C=NR, where R and R are different alkyl groups.

Animals are immunized against the antigen, immunogenic conjugates, or derivatives by combining, e.g. 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent.

Conjugates also can be made in recombinant cell culture as protein fusions. Also, aggregating agents such as alum are suitably used to enhance the immune response.

### III-2. Monoclonal antibodies,

Monoclonal antibodies are obtained from a population of substantially homogeneous antibodies, i. e. the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature 256: 495 (1975), or may be made by recombinant DNA methods (U. S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridomas cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Manassas, Virginia, USA. Human myeloma and mouse-human heteromyeloma cell Lines also have been described for the production of human monoclonal antibodies (Kozbor, J. Immunol., 133: 300 1 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The blinding affinity of the monoclonal antibody can, for example, be determined by the 30 Scatchard analysis of Munson et al., Anal. Biochem., 107: 220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPML-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatograph.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese Hamster Ovary (CHO) cells, or myeloma cells that do not otherwise produce immunoglobulin protein, to obtain the synthesis of Monoclonal antibodies in the recombinant host cells. Review articles on recombinant expression in bacteria of DNA encoding the antibody include Skerra et al., Curr. Opinion in Immunol., 5: 256-262 (1993) and Pluckthun, Immunol. Revs., 130: 151-188 (1992).

Another method of generating specific antibodies, or antibody fragments, reactive against an FGFR1OP or NPTX1 is to screen expressions libraries encoding immunoglobulin genes, or portions thereof, expressed in bacteria with a FGFR1 OP or NPTX1 protein or partial peptide thereof. For example, complete Fab fragments, VH regions and Fv regions can be expressed in bacteria using phage expression libraries. See for example, Ward et al., Nature 341: 544-546 (1989); Huse et al., Science 246: 1275-1281 (1989); and McCafferty et al., Nature 348: 552-554 (1990). Screening such libraries with, for example, a FGFR1OP or NPTX1 peptide, can identify immunoglobulin fragments reactive with FGFR1OP or NPTX1 partial peptide. Alternatively, the SCID-hu mouse (available from Genpharm) can be used to produce antibodies or fragments thereof.

In a further embodiment, antibodies or antibody fragments can be isolated from antibody phage libraries generated using the techniques described in McCafferty et al., Nature, 348: 552-554 (1990). Clackson et al., Nature, 352: 624-628 (1991) and Marks et al., J MoL BioL, 222: 581-597 (1991) describe the isolation of murine and human antibodies, respectively, using phage libraries. Subsequent publications describe the production of high affinity (nM range) human antibodies by chain shuffling (Marks et al., BiolTechnology, 10: 779-783 (1992)), as well as combinatorial infection and in vivo recombination as a strategy for constructing very large phage libraries (Waterhouse et al., Nuc. Acids. Res., 21: 2265-2266 (1993)). Thus, these techniques are viable alternatives to traditional Monoclonal antibody hybridoma techniques for isolation of monoclonal antibodies.

The DNA also may be modified, for example, by substituting the coding sequence for human heavy-and light-chain constant domains in place of the homologous murine sequences (U. S. Patent No. 4,816,567; Morrison, et al., Proc. Natl Acad. ScL USA, 81: 6851 (1984)), or by covalently joining to the immunoglobulin coding sequence all or part of the coding sequence for a non-immunoglobulin polypeptide.

Typically, such non-immunoglobulin polypeptides are substituted for the constant domains of an antibody, or they are substituted for the variable domains of one antigencombining site of an antibody to create a chimeric bivalent antibody comprising one antigen-combining site having specificity for an antigen and another antigen-combining site having specificity for a different antigen.

### III-3. Humanized antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321: 522-525 (1986); Reichmann et al., Nature, 332: 323-327 (1988); Verhoeyen et al., Science, 239: 1534-1536 (1988)), by substituting hypervariable region sequences for the corresponding sequences of a human antibody. Accordingly, such "humanized" antibodies are chimeric antibodies (U. S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so called "best-fit" method, the sequence of the variable domain of a rodent antibody is screened against the entire library of known human variable-domain sequences. The human sequence which is closest to that of the rodent is then accepted as the human framework region (FR) for the humanized antibody (Suns et al., J. Immunol., 151:2296 (1993); Chothia et al., J. Mol. Biol, 196: 901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89: 4285 (1992); Presta et al., J. Immunol., 151: 2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three- dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i. e., the analysis of residues that influence the ability of the candidate immunoglobulin to bind its antigen. In this way, FR residues can be selected and combined from the recipient and import sequences so that the desired antibody characteristic, such as increased affinity for the target antigen, is achieved. In general, the hypervariable region residues are directly and most substantially involved in influencing antigen binding.

### III-4. Human antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ line mutant mice will result in the production of human antibodies upon antigen challenge. See, e.g., Jakobovits et al., Proc. Mad. Acad. Sci. USA, 90: 255 1 (1993); Jakobovits et al., Nature, 362: 255-258 (1993); Bruggermann et al., Year in immuno, 7: 33 (1993); and US Patent Nos. 5,591,669,5,589,369 and 5,545,807.

Alternatively, phage display technology (McCafferty et al., Nature 348: 552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats for their review see, e.g., Johnson, Kevin S. and Chiswell, David J., Current Opinion in Structural Biology 3: 564-57 1 (1993). Several sources of V-gene segments can be used for phage display.

Clackson et al., Nature, 352 : 624-628 (1991) isolated a diverse array of anti-oxazolone antibodies from a small random combinatorial library ofV genes derived from the spleens of immunized mice. A repertoire of V genes from unimmunized human donors can be constructed and antibodies to a diverse array of antigens (including self antigens) can be isolated essentially following the techniques described by Marks et al., J. Mol. Biol, 222: 581-597 (1991), or Griffith et al., EMBO J. 12: 725-734 (1993). See, also, US Patent Nos. 5,565,332 and 5,573,905.

Human antibodies may also be generated by in vitro activated B cells (see US Patents 20 5.567,610 and 5,229,275). A preferred means of generating human antibodies using SCID mice is disclosed in commonly-owned, co-pending applications.

### III-5. Antibody fragments

Various techniques have been developed for the production of antibody fragments. In the present invention, antibody fragment may comprise variable region or antigen binding region of the antibody. Traditionally, these fragments were derived via proteolytic digestion of intact antibodies (see, e.g., Morimoto et al., Journal of Biochemical and Biophysical Methods 24: 107-117 (1992) and Brennan et al., Science, 229: 81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F (ab') 2 fragments (Carter et al., Bio/Technology 10: 163-167 (1992)). According to another approach, F (ab') 2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody", e.g., as described in US Patent 5,641,870 for example. Such linear antibody fragments may be monospecific or bispecific.

### III-6. Bispecific antibodies

Bispecific antibodies are antibodies that have binding specificities for at least two different epitopes. Exemplary, an anti-cancer cell marker (e.g. B9838) binding arm may be combined with, an arm which binds to a triggering molecule on a leukocyte such as a T-cell receptor molecule (e.g. CD2 or CD3), or Fc receptors for IgG (FcyR), such as FcyRI (CD64), FcyRII (CD32) and FcyRIH (CD 16) so as to focus cellular defense mechanisms to the cancer cell. Bispecific antibodies may also be used to localize cytotoxic agents to the cancer cell. These antibodies possess a cancer cell marker-binding arm and an arm which binds the cytotoxic agent (e.g. saporin, anti-interferon-a, vinca alkaloid, ricin A chain, methotrexate or radioactive isotope hapten). Bispecific antibodies can be prepared as full length antibodies or antibody fragments (e.g. F (ab) 2 bispecific antibodies).

Methods for making bispecific antibodies are known in the art Traditional production of full length bispecific antibodies is based on the coexpression of two immunoglobulin heavy chain-light chain pairs, where the two chains have different specificities (Millstein et al., Nature, 305: 537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10: 3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121: 210 (1986).

According to another approach described in US Patent No. 5,731,168, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chains) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate "antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patents No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F (ab') 2 fragments. These fragments are reduced in the presence of the dithiol complexing agent sodium arsenite to stabilize vicinal dithiols and prevent intermolecular disulfide formation. The Fab'fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. coli, which can be chemically coupled to form bispecific antibodies. Shalaby et al., J. Exp. Med., 175: 2 17-225 (1992) describe the production of a fully humanized bispecific antibody F (ab') 2 molecule. Each Fab' fragment was separately secreted from E. coli and subjected to directed chemical coupling in vitro to form the bispecific antibody.

Various techniques for making and isolating bispecific antibody fragments directly from recombinant cell culture have also been described. For example, bispecific antibodies have been produced using leucine zippers (Kostelny et al., J. Immunol. 148 (5): 1547-1553 (1992)). The leucine zipper peptides from the Fos and Jun proteins were linked to the Fab'portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90: 6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### III-7. Antibody Conjugates and other modifications

The antibodies used in the methods or included in the articles of manufacture herein are optionally conjugated to cytotoxic or therapeutic agent.

Therapeutic agent herein is included chemotherapeutic agent which is a chemical compound useful in the treatment of cancer. Examples of chemotherapeutic agents include followings and pharmaceutically acceptable salts, acids or derivatives of any of them.

Alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen bustards such as chlorambucil, chlornaphazine, cholophosphamide, estramustine, ifosfamide, mechlorethamine, mechlorethamine oxide hydrochloride, melphalan, novembiehin phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromoinycins, dactinomycin, daunorubicin, detorubicin, 6-diazo-5-oxo-L-norleucine, doxorubicin, epirubicin, esorubicin, idambicin, marcellomycin, mitomycins, mycophenolic acid, nogalamycin, olivomycins, peplomycin, poffiromycin, puromycin, quelamycin, rodorubicin, streptonigrin, streptozocin, tubercidin, ubanimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone ; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfonithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylydrazide; procarbazine; PSK@ razoxane; sizofiran spirogermanium; tenuazonic acid; triaziquone; 2,2', 2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e. g. paclitaxel (TAXOLO, Blistol-Myers Squibb Oncology, Princeton, NJ) and doxetaxel (TAXOTEW, Rh6ne-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine ; novantrone; teniposide; daunomycin; aminopterin; xeloda; ibandronate; CPT-11; topoisomerase inhibitor RFS 2000; difluoromethylornithine (DMFO); retinoic acad; esperamicins; and capecitabine; Also included in this definition are anti-hormonal agents that act to regulate or inhibit hormone action on tumors such as antiestrogens including for example tamoxifen, raloxifene, aromatase inhibiting 4 (5)-imidazoles, 4 hydroxytamoxifen, trioxifene, keoxifene, onapristone, and toremifene (Fareston); and antiandrogens such as flutamide, nilutamide, bicalutamide, leuprolide, and goserelin ; and pharmaceutically acceptable salts, acids or derivatives of any of the above.

Conjugates of an antibody and one or more small molecule toxins, such as a calicheamicin, a maytansine (US Pat No. 5,208,020), a trichothene, and CC 1065 are also contemplated herein. In one preferred embodiment of the invention, the antibody is conjugated to one or more maytansine molecules (e.g. about 1 to about 10 maytansine molecules per antibodies molecule). Maytansine may, for example, be converted to May SS-Me which may be reduced to May-SH3 and reacted with modified antibodies (Chari et al. Cancer Res 52: 127-131 (1992)) to generate a maytansinoid-antibody conjugate.

Alternatively, the antibody may be conjugated to one or more calicheamicin molecules. The calicheamicin family of antibiotics is capable of producing double stranded DNA breaks at sub-picomolar concentrations. Structural analogues of calicheamicin which may be used include, but are not limited to γ¹I, α²I, α³I, N-acetyl-γII. PSAG and θ¹I (Hinman et al. Cancer Res 53: 3336-3342 (1993) and Lode et al, Cancer Research 58: 2925-2928 (1998)).

Enzymatically active toxins and fragment thereof which can be used include diphtheria A chain, nonbinding active fragments of diphtheria, toxin, exotoxin A chain (from Pseudo7nonas aeruginosa), ricin A chain, abrin A chain, modeccin A chain, alpha sarcin, Aleurites fordii proteins, dianthin protein, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates antibody conjugated with a variety of radioactive isotopes. Examples include ²¹¹At, ¹³¹I, ¹²⁵I, ⁹⁰Y, ¹⁸⁶Re, ¹⁸⁸Re, ¹⁵³Sm, ²¹²Bi ³²P and radioactive isotopes of Lu.

Conjugates of the antibody and cytotoxic agent may be made using a variety of bifunctional protein coupling agents such as N-succinimidyl-3- (2-pyriylditliol) propionate (SPDP), suceinimidyl-4- (N-maleimidomethyl) cyclohexane-1-carboxylate, iminothiolane (IT), bifunctional derivatives of imidoesters (such as dimethyl adipimidate HCL), active esters (such as disuccinimidyl substrate), aldehydes (such as glutareldehyde), bis-azido compounds (such as bis (p-azidabenzoyl) bexanediamine), bis-diazonium derivatives (such as bis- (p-diazoniumbenzoyl)-ethylenediamine), diisocyanates (such as tolyene 2,6-dYsocyarvrte); and bis-active fluorine compounds (such as 1, 5-difluoro-2,4-dinitrobenzene). For example, a ricin immunotoxin can be prepared as described in Vitetta et al. Science 238: 1098 (1987). Carbon-14-labeled 1 isothiocyanatobenzyl-3-methyldiethylene triaminepentaacetic acid (MX-DTPA) is an exemplary chelating agent for conjugation of radionucleotide to the antibody. See W094/11026. The linker may be a "cleavable linker" facilitating release of the cytotoxic drug in the cell. For examples, an acid-labile linker, peptidase-sensitive linker, dimethyl linker or disulfide-containing linker (Charm et al. Cancer Research 52: 127-131 (1992)) may be used.

Alternatively, a fusion protein comprising the antibody and cytotoxic agent may be made, e.g. by recombinant techniques or peptide synthesis.

In yet another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

The antibodies of the present invention may also be conjugated with a prodrugs activating enzyme which converts a prodrug (e.g. a peptidyl chemotherapeutic agent, see W081/01145) to an active anti-cancer drug. See, for example, WO 88/07378 and US Pat No. 4,975,278.

The enzyme component of such conjugates includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic5-fluorocytosine into the anti-cancer drug, fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydratecleaving enzymes such as 13-galactosidase and neuraminidase useful for converting glycosylated prodrugs into free drugs; 13-lactamase useful for converting drugs derivatized with 13-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, Nature 328: 457-458 (1987)). Antibody-abzyme conjugates can be prepared as described herein for delivery of the abzyme to a tumor cell population.

The enzymes of this invention can be covalently bound to the antibody by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, e. g., Neuberger et al., Nature, 312: 604-608 (1984)).

Other modifications of the antibody are contemplated herein. For example, the antibody may be linked to one of a variety of nonproteinaceous polymers, e.g. polyethylene glycol, polypropylene glycol, polyoxyalkylenes, or copolymers of polyethylene glycol and polypropylene glycol.

The antibodies disclosed herein may also be formulated as liposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); U. S. Pat. Nos. 4,485,045 and 4, 544,545; and W097/3S731 published October 23,1997. Liposomes with enhanced circulation time are disclosed in U. S. Patent No. 5,013,556.

Particularly useful liposomes can be generated by the reverse phase evaporation method with a lipid composition comprising phosphatidylcholine, cholesterol and PEG derivatized phosphatidylethanolamine (PEG-PE). Liposomes are extruded through filters of defined pore size to yield liposomes with the desired diameter. Fab'fragments of an antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. ANational Cancer Inst. 81(19)1484(1989).

Amino acid sequence modifications of antibodies described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antibody. Any combination, of deletion, insertion, and substitution is made to arrive at the final construct, provided that the final construct possesses the desired characteristics. The amino acid changes also may alter post-translational processes of the anitbody, such as changing the number or position of glycosylation sites.

A useful method for identification of certain residues or regions of the antibody that are preferred locations for mutagenesis is called "alanine scanning mutagenesis" as described by Cunningham and Wells Science, 244:1081-1085 (1989). Here, a residue or group of target residues are identified (e.g., charged residues such as are, asp, his, lys, and flu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N-or C-terminus of the antibody of an enzyme, or a polypeptide which increases the serum half-life of the antibody.

Another type of variant is an amino acid substitution variant These variants have at least one amino acid residue in the antibody molecule replaced by different residue. The sites of greatest interest for substitutional mutagenesis of antibody include the hypervariable regions, but FR alterations are also contemplated

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Naturally occurring residues are divided into groups based on common side-chain properties:
(1) hydrophobic: norleucine, met, ala, val, leu, ile;
(2) neutral hydrophiuic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bonds may be added to the antibody to improve its stability (particularly where the antibody is a fragment such as an Fv fragment).

A particularly preferred type of substitutional variant involves substituting one or more hypervariable region residues of a parent antibody (e.g. a humanized or human antibody). Generally, the resulting variants selected for further development will have improved biological properties relative to The parent antibody from which they are generated. A convenient way for generating such substitutional variants is affinity maturation using phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity) as herein disclosed. In order to identify candidate hypervariable region sites for modification, alanine scanning mutagenesis can be performed to identified hypervariable region residues contributing significantly to antigen binding. Alternatively, or in addition, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody.

Glycosylation of polypepti des is typically either N-linked or O-linked. N-linked refers to the attachment of the carbohydrate moiety to the side chain of an asparagine residue. The tripeptide sequences asparagine-X-serine and asparagine- X-threonine, where X is any Amino acid except proline, are the recognition sequences for enzymatic attachment of the carbohydrate moiety to the asparagine side chain.

Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly seine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more seine or threonine residues to the sequence of the original antibody (for O-linked glycosylation sites).

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to modify the antibodies used in the invention to improve effector function, e.g. so as to enhance antigen-dependent cell-mediated cyotoxicity (ADCC) and/or complement dependent cytotoxicity (CDC) of the antibody. This may be achieved by introducing one or more amino acid substitutions in an Fc region of an antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176: 1191-1195 (1992) and Shopes, B. J linmunol 148 : 2918-2922 (1992).

Homodimeric antibodies with enhanced anti-tumor activity may also be prepared using heterobifunctional cross-linkers as described in Wolff et al. Cancer Research 53: 2560-2565 (1993). Alternatively, an antibody can be engineered which has dual Fc regions and may thereby have enhanced complement lysis and ADCC capabilities. See Stevenson et al. Anti-CancerDrugDesign 3: 2 19-230 (1989).

To increase the serum half life of the antibody, one may incorporate a salvage receptor binding epitope into the antibody (especially an antibody fragment) as described in US Patent 5,739,277, for example. As used herein, the term"salvage receptor binding epitope"refers to an epitope of the Fc region of an IgG molecule (e. g., IgGI, IgG2, IgC3, or IgG4) that is responsible for increasing the in vivo serum half-life of the IgG molecule. Accumulation Ability of Antibody to Tumor Cells In vivo

Some antibodies have high accumulation ability to tumor cells in vivo, but some antibodies don't have this ability. One of reasons of this ability may be the stability of antibody in the body. The accumulation ability to tumor cells is important for utilizing the antibody as the pharmaceutical composition. So, in vivo antibody accumulation is performed in the animal facility in accordance with institutional guidelines. In one embodiment, the mouse (e.g. BALB/cA Jcl-nu mouse) is injected subcutaneously (s.c.) with tumor cells expressing cancer maker (e.g. B9838), in suitable buffer, in the flanks. For biodistribution studies, the mouse with fully established tumors is given radioisotope-labeled antibody via tail vain. The radioactivity for tissues of the mouse is measured.

In the case of increase of the radioactivity for tumor cells in spite of decrease of the radioactivity for the tissues like as blood, liver, kidney, intestine, spleen, pancreas, lung, heart, stomach and muscle decreases as time goes on, the antibody have high accumulation activity.

### IV. Pharmaceutical compositions comprising siRNA

An siRNA against the KIF4A, NPTX1, FGFR1OP and WRNIP1 gene (hereinafter, also referred to, as "KIF4A siRNA", "NPTX1 siRNA", "FGFR1OP siRNA" and "WRNIP1 siRNA") can be used to reduce the expression level of the gene, Herein, the term "siRNA" refers to a double-stranded RNA molecule which prevents translation of a target mRNA. In the context of the present invention, the siRNA comprises a sense nucleic acid sequence and an anti-sense nucleic acid sequence against the up-regulated marker gene, KIF4A, NPTX1, FGFR1OP and WRNIP1. The siRNA is constructed so that it both comprises a sense and complementary antisense sequences of the target gene, *i.e*., a nucleotide comprising a hairpin structure. The siRNA may either be a dsRNA or shRNA.

As used herein, the term "dsRNA" refers to a construct of two RNA molecules comprising complementary sequences to one another and that have annealed together via the complementary sequences to form a double-stranded RNA molecule. The nucleotide sequence of two strands may comprise not only the "sense" or "antisense" RNAs selected from a protein coding sequence of target gene sequence, but also RNA molecule having a nucleotide sequence selected from non-coding region of the target gene.

The term "shRNA", as used herein, refers to an siRNA having a stem-loop structure, comprising a first and second regions complementary to one another, *i.e*., sense and antisense strands. The degree of complementarity and orientation of the regions being sufficient such that base pairing occurs between the regions, the first and second regions being joined by a a loop region, the loop resulting from a lack of base pairing between nucleotides (or nucleotide analogs) within the loop region. The loop region of an shRNA is a single-stranded region intervening between the sense and antisense strands and may also be referred to as "intervening single-strand".

As use herein, the term "siD/R-NA" refers to a double-stranded polynucleotide molecule which is composed of both RNA and DNA, and includes hybrids and chimeras of RNA and DNA and prevents translation of a target mRNA. Herein, a hybrid indicates a molecule wherein a polynucleotide composed of DNA and a polynucleotied composed of RNA hybridize to each other to form the double-stranded molecule; whereas a chimera indicates that one or both of the strands composing the double stranded molecule may contain RNA and DNA. Standard techniques of introducing siD/R-NA into the cell are used. The siD/R-NA includes a CX sense nucleic acid sequence (also referred to as "sense strand"), a CX antisense nucleic acid sequence (also referred to as "antisense strand") or both. The siD/R-NA may be constructed such that a single transcript has both the sense and complementary antisense nucleic acid sequences from the target gene, *e.g*., a hairpin. The siD/R-NA may either be a dsD/R-NA or shD/R-NA.

As used herein, the term "dsD/R-NA" refers to a construct of two molecules comprising complementary sequences to one another and that have annealed together via the complementary sequences to form a double-stranded polynucleotide molecule. The nucleotide sequence of two strands may comprise not only the "sense" or "antisense" polynucleotides sequence selected from a protein coding sequence of target gene sequence, but also polynucleotide having a nucleotide sequence selected from non-coding region of the target gene. One or both of the two molecules constructing the dsD/R-NA are composed of both RNA and DNA (chimeric molecule), or alternatively, one of the molecules is composed of RNA and the other is composed of DNA (hybrid double-strand).

The term "shD/R-NA", as used herein, refers to an siD/R-NA having a stem-loop structure, comprising a first and second regions complementary to one another, *i.e*., sense and antisense strands. The degree of complementarity and orientation of the regions being sufficient such that base pairing occurs between the regions, the first and second regions being joined by a loop region, the loop resulting from a lack of base pairing between nucleotides (or nucleotide analogs) within the loop region. The loop region of an shD/R-NA is a single-stranded region intervening between the sense and antisense strands and may also be referred to as "intervening single-strand".

An siRNA of the KIF4A, NPTX1 and FGFR1 OP gene hybridizes to target mRNA, *i.e*., associates with the normally single-stranded mRNA transcript and thereby interfering with translation of the mRNA, which finally decreases or inhibits production (expression) of the polypeptide encoded by the gene. Thus, an siRNA molecule of the invention can be defined by its ability to specifically hybridize to the mRNA of the KIF4A, NPTX1 and FGFR1OP gene under stringent conditions.

In the context of the present invention, an siRNA is preferably less than 500, 200,100, 50, or 25 nucleotides in length. More preferably an siRNA is 19-25 nucleotides in length. Exemplary target nucleic acid sequences of KIF4A siRNA includes the nucleotide sequences of SEQ ID NO: 32 or 33. The nucleotide "t" in the sequence should be replaced with "u" in RNA or derivatives thereof. Accordingly, for example, the present invention provides double-stranded RNA molecules comprising nucleotide sequence
5'- GGAAGAATTGGTTCTTGAA -3' (SED ID NO: 32) or
5'- GATGTGGCTCAACTCAAAG -3' (SEQ ID NO: 33).

Exemplary target nucleic acid sequences of NPTX1 siRNA includes the nucleotide sequences of SEQ ID NO: 36. The nucleotide "t" in the sequence should be replaced with "u" in RNA or derivatives thereof. Accordingly, for example, the present invention provides double-stranded RNA molecules comprising nucleotide sequence
5'- GGTGAAGAAGAGCCTGCCA -3' (SEQ ID NO: 36).

Exemplary target nucleic acid sequences of FGFR1OP siRNA includes the nucleotide sequences of SEQ ID NO: 37. The nucleotide "t" in the sequence should be replaced with "u" in RNA or derivatives thereof. Accordingly, for example, the present invention provides double-stranded RNA molecules comprising nucleotide sequence
5'- CCTGAAACTAGCACACTGC -3' (SEQ ID NO: 37).

Exemplary target nucleic acid sequences of WRNIP1 siRNA includes the nucleotide sequences of SEQ ID NO: 90. The nucleotide "t" in the sequence should be replaced with "u" in RNA or derivatives thereof. Accordingly, for example, the present invention provides double-stranded RNA molecules comprising nucleotide sequence
5'-CUAGGAAGAUGUUCUGUAAUU-3' (SEQ ID NO: 96) or
5'-CCACUAGGCUGAUGAAGGAUU-3' (SEQ ID NO: 97).

The double-stranded molecules of the invention may contain one or more modified nucleotide and/or non-phosphodiester linkages. Chemical modifications well known in the art are capable of increasing stability, availability, and/or cell uptake of the double-stranded molecule. The skilled person will be aware of other types of chemical modification which may be incorporated into the present molecules (WO03/070744; WO2005/045037). In one embodiment, modifications can be used to provide improved resistance to degradation or improved uptake. Examples of such modifications include phosphorothioate linkages, 2'-O-methyl ribonucleotides (especially on the sense strand of a double-stranded molecule), 2'-deoxy-fluoro ribonucleotides, 2'-deoxy ribonucleotides, "universal base" nucleotides, 5'-C-methyl nucleotides, and inverted deoxyabasic residue incorporation (US20060122137).

In another embodiment, modifications can be used to enhance the stability or to increase targeting efficiency of the double-stranded molecule. Modifications include chemical cross linking between the two complementary strands of a double-stranded molecule, chemical modification of a 3' or 5' terminus of a strand of a double-stranded molecule, sugar modifications, nucleobase modifications and/or backbone modifications, 2-fluoro modified ribonucleotides and 2'-deoxy ribonucleotides (WO2004/029212). In another embodiment, modifications can be used to increased or decreased affinity for the complementary nucleotides in the target mRNA and/or in the complimentary double-stranded molecule strand (WO2005/044976). For example, an unmodified pyrimidine nucleotide can be substituted for a 2-thio, 5-alkynyl, 5-methyl, or 5-propynyl pyrimidine. Additionally, an unmodified purine can be substituted with a 7-deza 7-alkyi, or 7-alkenyi purine. In another embodiment, when the double-stranded molecule is a double-stranded molecule with a 3' overhang, the 3'-terminal nucleotide overhanging nucleotides may be replaced by deoxyribonucleotides (Elbashir SM et al., Genes Dev 2001 Jan 15, 15(2): 188-200). For further details, published documents such as US20060234970 are available. The present invention is not limited to these examples and any known chemical modifications may be employed for the double-stranded molecules of the present invention so long as the resulting molecule retains the ability to inhibit the expression of the target gene.

Furthermore, the double-stranded molecules of the invention may comprise both DNA and RNA, *e.g*., dsD/R-NA or shD/R-NA. Specifically, a hybrid polynucleotide of a DNA strand and an RNA strand or a DNA-RNA chimera polynucleotide shows increased stability. Mixing of DNA and RNA, *i.e*., a hybrid type double-stranded molecule consisting of a DNA strand (polynucleotide) and an RNA strand (polynucleotide), a chimera type double-stranded molecule comprising both DNA and RNA on any or both of the single strands (polynucleotides), or the like may be formed for enhancing stability of the double-stranded molecule. The hybrid of a DNA strand and an RNA strand may be the hybrid in which either the sense strand is DNA and the antisense strand is RNA, or the opposite so long as it has an activity to inhibit expression of the target gene when introduced into a cell expressing the gene. Preferably, the sense strand polynucleotide is DNA and the antisense strand polynucleotide is RNA. Also, the chimera, type double-stranded molecule may be either where both of the sense and antisense strands are composed of DNA and RNA, or where any one of the sense and antisense strands is composed of DNA and RNA so long as it has an activity to inhibit expression of the target gene when introduced into a cell expressing the gene.

In order to enhance stability of the double-stranded molecule, the molecule preferably contains as much DNA as possible, whereas to induce inhibition of the target gene expression, the molecule is required to be RNA within a range to induce sufficient inhibition of the expression. As a preferred example of the chimera type double-stranded molecule, an upstream partial region (*i.e*., a region flanking to the target sequence or complementary sequence thereof within the sense or antisense strands) of the double-stranded molecule is RNA Preferably, the upstream partial region indicates the 5' side (5'-end) of the sense strand and the 3' side (3'-end) of the antisense strand. That is, in preferably embodiments, a region franking to the 3'-end of the antisense strand, or both of a region flanking to the 5'-end of sense strand and a region flanking to 3'-end of antisense strand consists of RNA. For instance, the chimera or hybrid type double-stranded molecule of the present invention comprise following combinations.
(a) sense strand: 5'-[DNA]-3' and
   antisense strand: 3'-(RNA)-[DNA]-5';
(b) sense strand: 5'-(RNA)-[DNA]-3' and
   antisense strand: 3'-(RNA)-[DNA]-5'; or
(c) sense strand: 5'-(RNA)-[DNA]-3' and
   antisense strand; 3'-(RNA)-5'.

The upstream partial region preferably is a domain consisting of 9 to 13 nucleotides counted from the terminus of the target sequence or complementary sequence thereto within the sense or antisense strands of the double-stranded molecules. Moreover, preferred examples of such chimera type double-stranded molecules include those having a strand length of 19 to 21 nucleotides in which at least the upstream half region (5' side region for the sense strand and 3' side region for the antisense strand) of the polynucleotide is RNA and the other half is DNA. In such a chimera type double-stranded molecule, the effect to inhibit expression of the target gene is much higher when the entire antisense strand is RNA (US20050004064).

In the present invention, the double-stranded molecule may form a hairpin, such as a short hairpin RNA, (shRNA) and short hairpin consisting of DNA and RNA (shD/R-NA). The shRNA or shD/R-NA is a sequence of RNA or mixture of RNA and DNA making a tight hairpin turn that can be used to silence gene expression via. RNA interference. The shRNA or shD/R-NA comprises the sense target sequence and the antisense target sequence on a single strand wherein, the sequences are separated by a loop sequence. Generally, the hairpin structure is cleaved by the cellular machinery into dsRNA or dsD/R-NA, which is then bound to the RNA-induced silencing complex (RISC). This complex binds to and cleaves mRNAs which match the target sequence of the dsRNA or dsD/R-NA.

In order to enhance the inhibition activity of the siRNA, nucleotide "u" can be added to the 3'end of the antisense strand of the target sequence. The number of "u"s to be added is at least 2, generally 2 to 10, preferably 2 to 5. The added "u"s form a single strand at the 3'end of the antisense strand of the siRNA.

A loop sequence consisting of an arbitrary nucleotide sequence can be located between the sense and antisense sequence in order to form the hairpin loop structure. Thus, the present invention also provides siRNA having the general formula 5'-[A]-[B]-[A']-3', wherein [A] is a ribonucleotide sequence corresponding to a sequence that specifically hybridizes to an RNA or a cDNA of the KIF4A. NPTX1 and FGFR1OP gene. In preferred embodiments, [A] is a ribonucleotide sequence corresponding to a sequence of the KIF4A, NPTX1 and FGFR1OP gene; [B] is a ribonucleotide sequence consisting of 3 to 23 nucleotides; and [A'] is a ribonucleotide sequence consisting of the complementary sequence of [A]. The region [A] hybridizes to [A'], and then a loop consisting of region [B] is formed. The loop sequence may be preferably 3 to 23 nucleotide in length. The loop sequence, for example, can be selected from a group consisting of following sequences (http://www.ambion.com/techlib/tb/tb_506.html):
CCC, CCACC, or CCACACC: Jacque JM et al., Nature 2002,418:435-8.
UUCG: Lee NS et al., Nature Biotechnology 2002, 20:500-5; Fruscoloni P et al., Proc Natl Acad Sci USA 2003, 100(4):1639-44.
UUCAAGAGA: Dykxhoorn DM et al., Nature Reviews Molecular Cell Biology 2003, 4:457-67.
'UUCAAGAGA ("ttcaagaga" in DNA)' is a particularly suitable loop sequence. Furthermore, loop sequence consisting of 23 nucleotides also provides an active siRNA (Jacque JM et al., Nature 2002, 418:435-8).

Exemplary hairpin siRNA suitable for use in the context of the present invention include, for KIF4A -siRNA,
5'- GGAAGAATTGGTTCTTGAA -[b]- TTCAAGAACCAATTCTTCC -3' (for target sequence of SEQ ID NO: 32); and
5'-GATGTGGCTCAACTCAAAG -[b]-CTTTGAGTTGAGCCACATC-3 ' (for target sequence of SEQ ID NO:33).

Exemplary hairpin siRNA suitable for use in the context of the present invention include, for FGFR1OP -siRNA,
5'- GGTGAAGAAGAGCCTGCCA -[b]-TGGCAGGCTCTTCTTCACC-3' (for target sequence of SEQ ID NO: 36).

Exemplary hairpin siRNA suitable for use in the context of the present invention include, for NPTX1 -siRNA,
5'-CCTGAAACTAGCACACTGC -[b]-GCAGTGTGCTAGTTTCAGG-3' (for target sequence of SEQ ID NO: 37).

Exemplary hairpin siRNA suitable for use in the context of the present invention include, for WRNIP1-siRNA,
5'-CTAGGAAGATGTTCTGTAA-[b]-TTACAGAACATCTTCCTAG -3' (for target sequence of SEQ ID NO: 96); and
5'-CCACTAGGCTGATGAAGGA-[b]-TCCTTCATCAGCCTAGTGG-3' (for target sequence of SEQ ID NO: 97).

The nucleotide sequence of suitable siRNAs can be designed using an siRNA design computer program available from the Ambion website (http://www.ambion.com/techlib/ misc/siRNA_finder.html). The computer program selects nucleotide sequences for siRNA synthesis based on the following protocol.

### Selection of siRNA Target Sites:

1. Beginning with the AUG start codon of the object transcript, scan downstream for AA dinucleotide sequences. Record the occurrence of each AA and the 3' adjacent 19 nucleotides as potential siRNA target sites. Tuschl et al. Genes Cev 1999, 13(24):3191-7 don't recommend against designing siRNA to the 5' and 3' untranslated regions (UTRs) and regions near the start codon (within 75 nucleotides) as these may be richer in regulatory protein binding sites. UTR-binding proteins and/or translation initiation complexes may interfere with binding of the siRNA endonuclease complex.
2. Compare the potential target sites to the human genome database and eliminate from consideration any target sequences with significant homology to other coding sequences. The homology search can be performed using BLAST (Altschul SF et al., Nucleic Acids Res 1997, 25:3389-402; J Mol Biol 1990, 215:403-10.), which can be found on the NCBI server at: www.ncbi.nlm.nih.gov/BLAST/.
3. Select qualifying target sequences for synthesis. At Ambion, preferably several target sequences can be selected along the length of the gene to evaluate.

Standard techniques for introducing siRNA into the cell may be used. For example, an siRNA of KIF4A NPTX1, FGFR1 OP and WRNIP1 can be directly introduced into the cells in a form that is capable of binding to the mRNA transcripts. In these embodiments, the siRNA molecules of the present invention are typically modified as described above for antisense molecules. Other modifications are also possible, for example, cholesterol-conjugated siRNAs have shown improved pharmacological properties (Song et al., Nature Med 2003, 9:347-51).

Alternatively, a DNA encoding the siRNA may be carried in a vector (hereinafter, also referred to as "siRNA vector"). Such vectors may be produced, for example, by cloning the target KIF4A NPTX1, FGFR1OP and WRNIP1 gene sequence into an expression vector having operatively-linked regulatory sequences (*e.g.*, a RNA polymerase III transcription unit from the small nuclear RNA (snRNA) U6 or the human H1 RNA promoter) flanking the sequence in a manner that allows for expression (by transcription of the DNA molecule) of both strands (Lee NS et al., Nature Biotechnology 2002, 20: 500-5). For example, an RNA molecule that is antisense to mRNA of tbe KIF4A, NPTX1, FGFR1OP and WRNIP1 gene is transcribed by a first promoter (*e.g.*, a promoter sequence 3' of the cloned DNA) and an RNA molecule that is the sense strand for the RNA of the KIF4A, NPTX1, FGFR1OP and WRNIP1 gene is transcribed by a second promoter (*e.g.*, a promoter sequence 5' of the cloned DNA). The sense and antisense strands hybridize *in vivo* to generate siRNA constructs for silencing the expression of the KIF4A, NPTX1, FGFR1OP and WRNIP1 gene. Alternatively, the two constructs can be utilized to create the sense and anti-sense strands of a single-stranded siRNA construct. In this case, a construct having secondary structure, *e.g.*, hairpin, is produced as a single transcript that comprises both the sense and complementary antisense sequences of the target gene.

Thus, the present pharmaceutical composition, for treating or preventing cancer comprises either the siRNA or a vector expressing the siRNA *in vivo*.

For introducing the siRNA vector into the cell, transfection-enhancing agent can be used FuGENE6 (Roche diagnostics), Lipofectamine 2000 (Invitrogen), Oligofectamine (Invitrogen), and Nucleofector (Wako pure Chemical) are useful as the transfection-enhancing agent. Therefore, the present pharmaceutical composition may further include such transfection-enhancing agents.

### V. Methods for treating or preventing cancer:

Cancer therapies directed at specific molecular alterations that occur in cancer cells have been validated through clinical development and regulatory approval of anti-tumor pharmaceuticals such as trastuzumab (Herceptin) for the treatment of advanced cancers, imatinib mesylate (Gleevec) for chronic myeloid leukemia, gefitinib (Iressa) for non-small cell lung cancer (NSCLC), and rituximab (anti-CD20 mAb) for B-cell lymphoma and mantle cell lymphoma (Ciardiello F et al., Clin Cancer Res 2001, 7:2958-70, Review; Slamon DJ et al., N Engl J Med 2001, 344:783-92; Rehwald U et al., Blood 2003, 101:420-4; Fang G et al., Blood 2000, 96:2246-53). These drugs are clinically effective and better tolerated than traditional anti-tumor agents because they target only transformed cells. Hence, such drugs not only improve survival and quality of life for cancer patients, but also validate the concept of molecularly targeted cancer therapy. Furthermore, targeted drugs can enhance the efficacy of standard chemotherapy when used in combination with it (Gianni L, Oncology 2002, 63 Suppl 1:47-56; Klejman A et al., Oncogene 2002, 21:5868-76). Therefore, future cancer treatments will probably involves combining conventional drugs with target-specific agents aimed at different characteristics of tumor cells such as angiogenesis and invasiveness.

Accordingly, therapeutics that may be utilized in the context of the present invention include small interfering RNA (siRNA).

Increased levels can be readily detected by quantifying peptide and/or RNA, by obtaining a patient tissue sample (*e.g.*, from biopsy tissue) and assaying it *in vitro* for RNA or peptide levels, structure and/or activity of the expressed peptides (or mRNAs of a gene whose expression is altered). Methods that are well-known within the art include, but are not limited to, immunoassays (*e.g.*, by Western blot analysis, immunoprecipitation followed by sodium dodecyl sulfate (SDS) polyacrylamide gel electrophoresis, immunocytochemistry, *etc.*) and/or hybridization assays to detect expression of mRNAs (*e.g.*, Northern assays, dot blots, *in situ* hybridization, *etc.*).

Prophylactic administration occurs prior to the manifestation of overt clinical symptoms of disease, such that a disease or disorder is prevented or, alternatively, delayed in its progression.

Thus, the present invention provides methods for creating or alleviating a symptom of cancer, or preventing cancer in a subject by decreasing the expression of the KIF4A, NPTX1, FGFR1OP and WRNIP1 gene or the activity of the gene product. The present method is particularly suited for treating or preventing NSCLC and SCLC.

Suitable therapeutics can be administered prophylactically or therapeutically to a subject suffering from or at risk of (or susceptible to) developing cancers. Such subjects can be identified by using standard clinical methods or by detecting an aberrant expression level ("up-regulation" or "over-expression") of the KIF4A, NPTX1, FGFR1OP and WRNIP1 gene or aberrant activity of the gene product.

The dosage and methods for administration vary according to the body-weight, age, sex, symptom, condition of the patient to be treated and the administration method; however, one skilled in the art can routinely select suitable dosage and administration method.

For example, although the dose of an agent that binds to the KIF4A, NPTX1, FGFR1OP and WRNIP1 polypeptide and regulates the activity of the polypeptide depends on the aforementioned various factors, the dose is generally about 0.1 mg to about 100 mg per day, preferably about 1.0 mg to about 50 mg per day and more preferably about 1.0 mg to about 20 mg per day, when administered orally to a normal adult human (60 kg weight).

When administering the agent parenterally, in the form of an injection to a normal adult human (60 kg weight), although there are some differences according to the patient target organ, symptoms and methods for administration, it is convenient to intravenously inject a dose of about 0.01 mg to about 30 mg per day, preferably about 0.1 to about 20 mg per day and more preferably about 0.1 to about 10 mg per day. In the case of other animals, the appropriate dosage amount may be routinely calculated by converting to 60 kg of body-weight.

Similarly, a pharmaceutical composition of the present invention may be used for treating or preventing cancer. Methods well known to those skilled in the art may be used to administer the compositions to patients, for example, as an intraarterial, intravenous, or percutaneous injection or as an intranasal, transbronchial, intramuscular, or oral administration.

For each of the aforementioned conditions, the compositions, *e.g.*, polypeptides and organic compounds, can be administered orally or via injection at a dose ranging from about 0.1 to about 250 mg/kg per day. The dose range for adult humans is generally from about 5 mg to about 17.5 g/day, preferably about 5 mg to about 10 g/day, and most preferably about 100 mg to about 3 g/day. Tablets or other unit dosage forms of presentation provided in discrete units may conveniently contain an amount which is effective at such dosage or as a multiple of the same, for instance, units containing about 5 mg to about 500 mg, usually from about 100 mg to about 500 mg.

The dose employed will depend upon a number of factors, including the age, body weight and sex of the subject, the precise disorder being treated, and its severity. Also the route of administration may vary depending upon the condition and its severity. In any event appropriate and optimum dosages may be routinely calculated by those skilled in the art, taking into consideration the above-mentioned factors.

The dosage of the antisense nucleic acid derivatives of the present invention can be adjusted suitably according to the patient's condition and used in desired amounts. For example, a dose range of 0.1 to 100 mg/kg, preferably 0.1 to 50 mg/kg can be administered.

### VI. Method for assessing the prognosis of cancer:

According to the present invention it was newly discovered that KIF4A, NPTX1 and FGFR1OP expression is significantly associated with poorer prognosis of patients. Thus, the present invention provides a method for determining or assessing the prognosis of a patient with cancer, in particular lung cancer, by detecting the expression level of the KIF4A, NPTX1 or FGFR1OP gene in a biological sample of the patient; comparing the detected expression level to a control level; and determining a increased expression level to the control level as indicative of poor prognosis (poor survival).

Herein, the term "prognosis" refers to a forecast as to the probable outcome of the disease as well as the prospect of recovery from the disease as indicated by the nature and symptoms of the case. Accordingly, a less favorable, negative, poor prognosis is defined by a lower post-treatment survival term or survival rate. Conversely, a positive, favorable, or good prognosis is defined by an elevated post-treatment survival term or survival rate.

The terms "assessing the prognosis" refer to the ability of predicting, forecasting or correlating a given detection or measurement with a future outcome of cancer of the patient (*e.g.*, malignancy, likelihood of curing cancer, survival, and the like). For example, a determination of the expression level of KIF4A, NPTX1 or FGFR1OP over time enables a predicting of an outcome for the patient (*e.g.*, increase or decrease in malignancy, increase or decrease in grade of a cancer, likelihood of curing cancer, survival, and the like).

In the context of the present invention, the phrase "assessing (or determining) the prognosis" is intended to encompass predictions and likelihood analysis of cancer, progression, particularly cancer recurrence, metastatic spread and disease relapse. The present method for assessing prognosis is intended to be used clinically in making decisions concerning treatment modalities, (including therapeutic intervention, diagnostic criteria such as disease staging, and disease monitoring and surveillance for metastasis or recurrence of neoplastic disease.

The patient-derived biological sample used for the method may be any sample derived from the subject to be assessed so long as the KIF4A, NPTX1 or FGFR1OP gene can be detected in the sample. Preferably, the biological sample comprises an lung cell (a cell obtained from the and lung). Furthermore, the biological sample includes bodily fluids such as sputum, blood, serum, or plasma. Moreover, the sample may be cells purified from a tissue. The biological samples may be obtained from a patient at various time points, including before, during, and/or after a treatment.

According to the present invention, it was shown that the higher the expression level of the KIF4A, NPTX1 or FGFR1OP gene measured in the patient-derived biological sample, the poorer the prognosis for post-treatment remission, recovery, and/or survival and the higher the likelihood of poor clinical outcome. Thus, according to the present method, the "control level" used for comparison may be, for example, the expression level of the KIF4A, NPTX1 or FGFR1OP gene detected before any kind of treatment in an individual or a population of individuals who showed good or positive prognosis of cancer, after the treatment, which herein will be referred to as "good prognosis control level". Alternatively, the "control level" may be the expression level of the KIF4A, NPTX1 or FGFR1OP gene detected before, any kind of treatment in an individual or a population of individuals who showed poor or negative prognosis of cancer, after the treatment, which herein will be referred to as "poor prognosis control The "control level" is a single expression pattern derived from a single reference population or from a plurality of expression patterns. Thus, the control level may be determined based on the expression level of the KIF4A, NPTX1 or FGFR1OP gene detected before any kind of treatment in a patient of cancer, or a population of the patients whose disease state (good or poor prognosis) is known. Preferably, cancer is lung cancer. It is preferred, to use the standard value of the expression levels of the KIF4A, NPTX1 and FGFR1OP gene in a patient group with a known disease state. The standard value may be obtained by any method known in the art. For example, a range of mean ± 2 S.D. or mean ± 3 S.D. may be used as standard value.

The control level may be determined at the same time with the test biological sample by using a sample(s) previously collected and stored before any kind of treatment from cancer patient(s) (control or control group) whose disease state (good prognosis or poor prognosis) are known.

Alternatively, the control level may be determined by a statistical method based on the results obtained by analyzing the expression level of the KIF4A, NPTX1 or FGFR1OP gene in samples previously collected and stored from a control group. Furthermore, the control level can be a database of expression patterns from previously tested cells. Moreover, according to an aspect of the present invention, the expression level of the KIF4A, NPTX1 or FGFR1OP gene in a biological sample may be compared to multiple control levels, which control levels are determined from multiple reference samples. It is preferred to use a control level determined from a reference sample derived from a tissue type similar to that of the patient-derived biological sample.

According to the present invention, a similarity in the expression level of the KIF4A, NPTX1 or FGFR1 OP gene to the good prognosis control level indicates a more favorable prognosis of the patient and an increase in the expression level to the good prognosis control level indicates less favorable, poorer prognosis for post-treatment remission, recovery, survival, and/or clinical outcome. On the other hand, a decrease in the expression level of the KIF4A, NPTX1 or FGFR1OP gene to the poor prognosis control level indicates a more favorable prognosis of the patient and a similarity in the expression level to the poor prognosis control level indicates less favorable, poorer prognosis for post-treatment remission, recovery, survival, and/or clinical outcome.

An expression level of the KIF4A, NPTX1 or FGFR1 OP gene in a biological sample can be considered altered when the expression level differs from the control level by more than 1.0, 1.5, 2.0, 5.0, 10.0, or more fold.

The difference in the expression level between the test biological sample and the control level can be normalized to a control, e.g., housekeeping gene. For example, polynucleotides whose expression levels are known not to differ between the cancerous and non-cancerous cells, including those coding for β-actin, glyceraldehyde 3-phosphate dehydrogenase, and ribosomal protein P1, may be used to normalize the expression levels of the KIF4A, NPTX1 or FGFR1OP gene.

The expression level may be determined by detecting the gene transcript in the patient-derived biological sample using techniques well known in the art. The gene transcripts detected by the present method include both the transcription and translation products, such as mRNA and protein.

For instance, the transcription product of the KIF4A, NPTX1 or FGFR1OP gene can be detected by hybridization, *e.g.*, Northern blot hybridization analyses, that use a KIF4A, NPTX1 or FGFR1 OP gene probe to the gene transcript. The detection may be carried out on a chip or an array. The use of an array is preferable for detecting the expression level of a plurality of genes including the KIF4A, NPTX1 or FGFR1OP gene. As another example, amplification-based detection methods, such as reverse-transcription based polymerase chain reaction (RT-PCR) which use primers specific to the KIF4A NPTX1 or FGFR1OP gene may be employed for the detection (*see* Example). The KIF4A, NPTX1 or FGFR1OP gene-specific probe or primers may be designed and prepared using conventional techniques by referring to the whole sequence of the KIF4A, NPTX1 or FGFR1OP gene (SEQ ID NO: 52, 56 and 58, respectively). For example, the primers (SEQ ID NOs: 1 and 2 (KIF4A), 19 and 20 (NPTX1), 17 and 18 (FGFR1OP)) used in the Example may be employed for the detection by RT-PCR but the present invention is not restricted thereto.

Specifically, a probe or primer used for the present method hybridizes under stringent, moderately stringent, or low stringent conditions to the mRNA of the KIF4A, NPTX1 or FGFR1OP gene. As used herein, the phrase "stringent (hybridization) conditions" refers to conditions under which a probe or primer will hybridize to its target sequence, but to no other sequences. Stringent conditions are sequence-dependent and will be different udder different circumstances. Specific hybridization of longer sequences is observed at higher temperatures than shorter sequences. Generally, the temperature of a stringent condition is selected to be about 5°C lower than the thermal melting point (Tm) for a specific sequence at a defined ionic strength and pH. The Tm is the temperature (under defined ionic strength, pH and nucleic acid concentration) at which 50% of the probes complementary to The target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes or primers (*e.g.*, 10 to 50 nucleotides) and at least about 60°C for longer probes or primers. Stringent conditions may also be achieved with the addition of destabilizing agents, such as formamide.

Alternatively, the translation product may be detected for the assessment of the present invention. For example, the quantity of the KIF4A, NPTX1 or FGFR1OP protein may be determined. A method for determining the quantity of the protein as the translation product includes immunoassay methods that use an antibody specifically recognizing the KIF4A, NPTX1 or FGFR1 OP protein. The antibody may be monoclonal or polyclonal, Furthermore, any fragment or modification (*e.g.*, chimeric antibody, scFv, Fab, F(ab')2, Fv, *etc.*) of the antibody may be used for the detection so long as the fragment retains the binding ability to the KIF4A, NPTXI or FGFR1OP protein. Methods to prepare these kinds of antibodies for the detection of proteins are well known in the art, and any method may be employed in the present invention to prepare such antibodies and equivalents thereof.

As another method to detect the expression level of the KIF4A, NPTX1 or FGFR1OP gene based on its translation product, the intensity of staining may be observed via immunohistochemical analysis using an antibody against KIF4A, NPTX1 or FGFR1OP protein. Namely, the observation of strong staining indicates increased presence of the KIF4A, NPTX1 or FGFR1 OP protein and at the same time high expression level of the KIF4A, NPTX1 or FGFR1OP gene.

Furthermore, the KIF4A, NPTX1 or FGFR1OP protein is known to have a cell proliferating activity. Therefore, the expression level of the KIF4A, NPTX1 or FGFR1OP gene can be determined using such cell proliferating activity as an index. For example, cells which express KIF4A, NPTX1 or FGFR1OP are prepared and cultured in the presence of a biological sample, and then by detecting the speed of proliferation, or by measuring the cell cycle or the colony forming ability the cell proliferating activity of the biological sample can be determined.

Moreover, in addition to the expression level of the KIF4A, NPTX1 or FGFR1OP gene, the expression level of other lung cell-associated genes, for example, genes known to be differentially expressed in lung cancer may also be determined to improve the accuracy of the assessment Such other lung cell-associated genes include those described in WHO 2004/031413 and WHO 2005/090603.

The patient to be assessed for the prognosis of cancer according to the method is preferably a mammal and includes human, non-human primate, mouse, rat, dog, cat, horse, and cow.

### VII. A kit for assessing the prognosis of cancer:

The present invention provides a kit for assessing the prognosis of cancer. Preferably, cancer is and lung cancer. Specifically, the kit comprises at least one reagent for detecting the expression of the KIF4A, NPTX1 or FGFR1OP gene in a patient-derived biological sample, which reagent may be selected from the group of:
(a) a reagent for detecting mRNA of the KIF4A, NPTX1 or FGFR1OP gene;
(b) a reagent for detecting the KIF4A, NPTX1 or FGFR1OP protein; and
(c) a reagent for detecting the biological activity of the KIF4A, NPTX1 or FGFR1OP protein.

Suitable reagents for detecting mRNA of the KIF4A, NPTX1 or FGFR1OP gene include nucleic acids that specifically bind to or identify the KIF4A, NPTX1 or FGFR1OP mRNA, such as oligonucleotides which have a complementary sequence to a part of the KIF4A, NPTX1 or FGFR1OP mRNA. These kinds of oligonucleotides are exemplified by primers and probes that are specific to the KIF4A, NPTX1 or FGFR1OP mRNA. These kinds of oligonucleotides may be prepared based on methods well known in the art. If needed, the reagent for detecting the KIF4A, NPTX1 or FGFR1OP mRNA may be immobilized on a solid matrix. Moreover, more than one reagent for detecting the KIF4A NPTX1 or FGFR1OP mRNA may be included in the kit.

On the other hand, suitable reagents for detecting the KIF4A, NPTX1 and FGFR1OP protein include antibodies to the KIF4A, NPTX1 and FGFR1OP protein. The antibody may be monoclonal or polyclonal. Furthermore, any fragment or modification (*e.g.*, chimeric antibody, scFv, Fab, F(ab')2. Fv, *etc.*) of the antibody may be used as the reagent, so long as the fragment retains the binding ability to the KIF4A NPTX1 and FGFR1OP protein. Methods to prepare these kinds of antibodies for the detection of proteins are well known in the art, and any method may be employed in the present invention to prepare such antibodies and equivalents thereof. Furthermore, the antibody may be labeled with signal generating molecules via direct linkage or an indirect labeling technique. Labels and Methods for labeling antibodies and detecting the binding of antibodies to their targets are well known in the art and any labels and methods may be employed for the present invention. Moreover, more than one reagent for detecting the KIF4A, NPTX1 or FGFR1OP protein may be included in the kit.

Furthermore, the biological activity can be determined by, for example, measuring the cell proliferating activity due to the expressed KIF4A, NPTX1 or FGFR1OP protein in the biological sample. For example, the cell is cultured in the presence of a patient-derived biological sample, and then by detecting the speed of proliferation, or by measuring the cell cycle or the colony forming ability the cell proliferating activity of the biological sample can be determined. If needed, the reagent for detecting the KIF4A, NPTX1 or FGFR1 OP mRNA may be immobilized on a solid matrix. Moreover, more than one reagent for detecting the biological activity of the KIF4A, NPTX1 or FGFR1OP protein may be included in the kit.

The kit may comprise more than one of the aforementioned reagents. Furthermore, the kit may comprise a solid matrix and reagent for binding a probe against the KIF4A, NPTX1 or FGFR1OP gene or antibody against the KIF4A, NPTX1 or FGFR1OP protein, a medium and container for culturing cells, positive and negative control reagents, and a secondary antibody for detecting an antibody against the KIF4A, NPTX1 or FGFR1OP protein. For example, tissue samples obtained from patient with good prognosis or poor prognosis may serve as useful control reagents. A kit of the present invention may further include other materials desirable from a commercial and user standpoint, including buffers, diluents, filters, needles, syringes, and package inserts (*e.g.*, written, tape, CD-ROM, *etc.*) with instructions for use. These reagents and such may be comprised in a container with a label. Suitable containers include bottles, vials, and test tubes. The containers may be formed from a variety of materials, such as glass or plastic.

As an embodiment of the present invention, when the reagent is a probe against the KIF4A, NPTX1 and FGFR1OP mRNA, the reagent may be immobilized on a solid matrix, such as a porous strip, to form at least one detection site. The measurement or detention region of the porous strip may include a plurality of sites, each containing a nucleic acid (probe), A test strip may also contain sites for negative and/or positive controls. Alternatively, control sites may be located on a strip separated from the test strip. Optionally, the different detection sites may contain different amounts of immobilized nucleic acids, *i.e.*, a higher amount in the first detection site and lesser amounts in subsequent sites. Upon the addition of test sample, the number of sites displaying a detectable signal provides a quantitative indication of the amount of KIF4A, NPTX1 and FGFR1OP mRNA present in the sample. The detection sites may be configured in any suitably detectable shape and are typically in the shape of a bar or dot spanning the with of a test strip.

The kit of the present invention may further comprise a positive control sample, or KIF4A, NPTX1 and FGFR1OP standard sample. The positive control sample of the present invention may be prepared by collecting KIF4A, NPTX1 and FGFR1OP positive blood samples and then those KIF4A, NPTX1 and FGFR1OP level are assayed. Alternatively, purified KIF4A, NPTX1 or FGFR1OP protein or polynucleotide may be added to KIF4A, NPTX1 or FGFR1OP free serum to form the positive sample or the KIF4A NPTX1 or FGFR1OP standard. In the present invention, purged KDD1 may be recombinant protein, The KIF4A, NPTX1 or FGFR1OP level of the positive controls sample is, for example more than cut off value.

Hereinafter, the present invention is described in more detail with reference to the Examples. However, the following materials, methods and examples only illustrate aspects of the invention and in no way are intended to limit the scope of the present invention. As such, methods and materials similar or equivalent to those described herein can be used in the practice or testing of the present invention.

### VII. Producing and identifying compounds to target molecules mediated disease;

### VIII-1 screening principles:

In view of the evidence provided herein, that some target molecules interacts with partner molecules and the expression levels of some of them are associated with poor prognosis in cancer patients, one aspect of the invention involves identifying test compounds that reduce or prevent the binding between target molecules and partner molecules. In the present invention, the target molecule is selected from the group consisting of KIF4A, MAPJD, and FGFR1OP. Another hand, the partner molecule of the present invention is selected from group consisting of ZNF549, ZNF553, MYC, WRNIP1, and ABL1. Furthermore, combinations of the target molecules and the partner molecules thereof are shown bellow:
KIF4A/ZNF549;
KIF4A/ZNF553;
MAPJD/MYC; and
FGFR1OP/WRNIP1, FGFR1OP/ABL1 or FGFR1OP/WRNIP1/ABL1

Methods for determining target molecule/partner molecule binding include any methods for determining interactions of two proteins. Such assays include, but are not limited to, traditional approaches, such as, cross-linking, co-immunoprecipitation, and co-purification through gradients or chromatographic columns. In addition, protein-protein interactions can be monitored using a yeast-based genetic system described by Fields and co-workers (Fields and Song, Nature 340:245-6 (1989); Chien et al., Proc. Natl. Acad. Sci. USA 88, 9578-82 (1991)) and as disclosed by Chevray and Nathans (Proc. Natl. Acad Sci. USA 89:5789-93 (1992)). Many transcriptional activators, such as yeast GALA, consist of two physically discrete modular domains, one acting as the DNA-binding domain, while the other one functions as the transcription activation domain. The yeast expression system, described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-lacZ reporter gene under control of a GALA-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein protein interactions between two specific proteins using the two-hybrid technique is commercially available from Clontech. This system can also be extended to map protein domains involved in specific protein interactions as well as to pinpoint amino acid residues that are crucial for these interactions.

While the present application refers to "target molecules" and "partner molecule", it is understood that where the interaction of the two is analyzed or manipulated, it is possible to use the binding portions of one or both of the proteins in place of the full-length copies of the proteins. Fragments of target molecule that bind to partner molecule may be readily identified using standard deletion analysis and/or mutagenesis of target molecule to identify fragments that bind to partner molecule. Similar analysis may be used to identify target molecule-binding fragments of partner molecule.

In view of the evidence provided herein that MAPJD associated with HAT complex has activities to acetylate histone H4 and binds E-box, one aspect of the invention involves identifying test compounds that reduce or prevent such activity. The present invention thus provides a method of screening for an agent that modulates acetylation or binding activity of MAPJD associated with HAT complex. The method is practiced by contacting an MAPJD polypeptide or a functional equivalent thereof associated with HAT complex with a substrate to be acetylated under the conditions suitable for acetylation of the substrate, and assaying acetylation level of the substrate. An agent that modulates acetylation activity of the MAPJD associated with HAT complex is thereby identified.

In the present invention, suitable conditions for acetylation of the substrate can be provided *in vivo* or *in vitro*. For instance, the condition for acetylation may be provided by co-transfection of vectors to express elements to form HAT complex Alternatively. MYC expressing cell transfected with MAPJD expressing vector may also be used for the condition for acetylation of the substrate in vivo. Further, MAPJD peptide mixed with element to form HAT complex may be used for the condition for acetylation of the substrate *in vitro*.

In the present invention, preferable substrate to be acetylated is histone H4. As used herein, the term "histone H4" refers to full length histone H4 proteins (*e.g.*, GenBank Accession number: NM_175054) as well as mutants and fragments thereof. For example, fragment peptide comprising acetylation site of histone H4 may also be used as the substrate.

In the present invention, the term "HAT-complex" refers to a complex having histone acetyltransferase activity. In the present invention, preferable "HAT-complex" comprises MYC, MAX (NM_002467), and TRRAP (Figure 11 C), or MYC, MAX, and TIP60 (Frank SR, et al. EMBO Rep. 2003 Jun;4(6):575-80.). Method for preparing such HAT complex is well known (McMahon SB, et al. Mol. Cell. 20, 556-62 (2000); Frank SR. et al. EMBO Rep. 4, 575-80 (2003).

Alternatively, the present invention also provides a method of screening for compounds that are useful for inhibiting the hinging between the proteins of present invention, inhibiting the growth of the lung cancer cells, and treating or preventing lung cancer, the methods comprise the steps of:
(1) contacting a test compound to a MAPJD polypeptide or functional equivalent thereof associated with a HAT complex or MYC and polynucleotide comprising E-box motifs;
(2) detecting a binding between the polypeptide and the polynucleotide; and
(3) selecting a test compound that inhibits the binding.

An agent that interferes binding activity of the MAPJD associated with HAT complex is thereby identified. In the present invention, preferable polynucleotide comprises a E-box motifs (CANNTG) is the 5' flanking region of a gene selected from the group consisting of SBNO1, TGFBRAP1, RIOK1, and RASGEF1A. Generally, at least 2 or more E-box motifs are included in the polynucleotide. In some embodiments, the number of E-box motifs is 3 or more, preferably 4 to 20, alternatively 5 to 15, more preferably 6 to 10. In the present invention, any well known methods to detect a protein-DNA binding may be applied to the present invention. For instance, binding between MAPJD polypeptide or functional equivalent thereof associated with a HAT complex or MYC and polynucleotide comprising E-box motif may be detected by reporter gene assay. Specifically, the method comprises steps of;
(a) contacting a candidate compound with a cell into which a vector, comprising the transcriptional regulatory region of a target gene and a reporter gene that is expressed under the control of the transcriptional regulatory region, has been introduced;
(b) measuring the expression or activity of said reporter gene; and
(c) selecting the candidate compound that reduces the expression or activity level of said reporter gene as compared to the expression or activity level detected in the absence of the candidate compound.

Suitable reporter genes and host cells are well known in the art. A reporter construct suitable for the screening methods of the present invention can be prepared by using the transcriptional regulatory region of the target gens. In the present invention, "target gene" refers to gene regulated by MAPJD associated with HAT complex. When the transcriptional regulatory region of the target gene is known to those skilled in the art, a reporter construct can be prepared by using the previous sequence information. When the transcriptional regulatory region of the target gene remains unidentified, a nucleotide segment containing the transcriptional regulatory region can be isolated from a genome library based on the nucleotide sequence information of the target gene.

In the present invention the binding between MAPJD associated with HAT complex (or MYC) and polynucleotide comprising E-box can be detected *in vitro.* For instance, oligonucleotide comprising E-box may be contacted with MAPJD and HAT complex (or MYC protein), followed by binding product is detected. Thus, the present invention provides a method of screening for compounds that are useful for treating or preventing lung cancer, the methods comprise the steps of:
(1) contacting a test compound to a MAPJD polypeptide associated with a HAT complex or MYC and oligonucleotide comprising E-box motif;
(2) detecting a binding between the polypeptide and the oligonucleotide; and
(3) selecting a test compound that inhibits the binding.

In the present invention, for example, Gel-shift assay or Electrophoretic Mobility Shift Assay (EMSA) can be applied for detection af the binding product, In the present invention, labeled oligonucleotide probe comprising E-box may be used as polynucleotide comprising E-box. Nucleotide sequence of the oligonucleotide can be designed so that the oligonucleotide comprises E-box motif. Specifically, double strand DNA consisting of:
5'-CCCGTCGCACGTGGTGGCCA-3' (SEQ ID NO: 50) and
5'-TGGCCACCACGTGCGACGGG-3' (SEQ ID NO: 51) is used as oligonucleotide probe for EMSA. This nucleotide sequence is selected from 5' franking region of RIOK1 gene which is one of MAPJD's target genes.

In the present invention, the term "functionally equivalent" means that the subject protein or polypeptide has the same or substantially the same acetylation or binding activity as MAPJD associated with HAT complex. In particular, the protein catalyzes the acetylation of a histone H4 protein or a fragment thereof that includes acetylation site. Whether a subject protein has the target activity can be routinely determined by the present invention. Namely, the acetylation activity can be determined by (a) contacting a polypeptide associated with HAT complex with a substrate (*e.g*., a histone H4) under conditions suitable for acetylation of the substrate, and (b) detecting the acetylation level of the substrate.

Methods for preparing proteins that are functional equivalents of a given protein are well known to those skilled in the art and include conventional methods of introducing mutations into the protein. For example, one skilled in the art can prepare proteins functionally equivalent to the human MAPJD protein by introducing an appropriate mutation in the amino acid sequence of the human MAPJD protein using site-directed mutagenesis for example (Hashimoto-Gotoh T. et al. (1995), Gene 152, 271-5; Zoller MJ, and Smith M. (1983), Methods Enzymol. 100, 4687-500; Kramer W. et al. (1984), Nucleic Acids Res. 12, 9441-56; Kramer W, and Fritz HJ. (1987) Methods. Enzymol. 154, 3 50-67; Kunkel, TA (1985), Proc. Natl. Acad. Sci. USA. 82, 488-92). Amino acid mutations can occur in nature, too. A MAPJD polypeptide useful in the context of the present invention includes those proteins having the amino acid sequences of the human MAPJD protein in which one or more amino acids are mutated, provided the resulting mutated proteins are functional equivalents of the human MAPJD protein, more particularly retain the methyltransferase activity of the human MAPJD protein. The number of amino acids to be mutated in such a mutant is generally 20 amino acids or less, typically 10 amino acids or less, preferably 6 amino acids or less, and more preferably 3 amino acids or less. To maintain the acetylation activity, the MYC binding domains preferably conserved in the amino acid sequence of the mutated proteins. Mutated or modified proteins, *i.e*., proteins having amino acid sequences modified by deleting, adding and/or replacing one or more amino acid residues of a certain amino acid sequence, are known to retain the biological activity of the original protein (Mark DF et al., Proc. Natl. Acad. Sci. USA (1984) 81, 5662-6, Zoller MJ. & Smith, M., Nucleic Acids Research (1982) 10, 6487-500, Wang A. et al., Science 224,1431-3, Dalbadie-McFarland G. et al., Proc. Natl. Acid. Sci. USA (1982) 79,6409-13). The amino acid residue to be mutated is preferably mutated into a different amino acid that allows the properties of the amino acid side-chain to be conserved (a process known as conservative amino acid substitution). Examples of properties of amino acid side chains include hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); ahydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Note, the parenthetic letters indicate the one-letter codes of amino acids. An example of a protein in one or more amino acids residues are added to the amino acid sequence of human MAPJD protein (SEQ ID NO: 55 is a fusion protein containing the human MAPJD protein. Fusion proteins include fusions of the human MAPJD protein and other peptides or proteins, and are used in the present invention. Fusion proteins can be made by techniques well known to a person skilled in the art, such as by linking the DNA encoding the human MAPJD protein of the invention with DNA encoding other peptides or proteins, so that the frames match, inserting the fusion DNA into an expression vector and expressing it in a host. There is no restriction as to the peptides or proteins fused to the protein of the present invention. Known peptides that can be used as peptides to be fused to the MAPJD protein include, for example, FLAG (Hopp TP. et al., Biotechnology (1988) 6, 1204-10), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, α-tubulin fragment, B-tag, Protein C fragment, and the like. Examples of proteins that may be fused to a protein of the invention include GST (glutathione-S-transferase), Influenza agglutinin (HA), immunoglobulin constant region, β-galactosidase, MBP (maltose-binding protein), and such. Fusion proteins can be prepared by fusing commercially available DNA, encoding the fusion peptides or proteins discussed above, with the DNA encoding the protein of the present invention and expressing the fused DNA prepared. An alternative method Known in the art to isolate functionally equivalent proteins uses hybridization techniques to identify homologous sequences (Sambrook J. et al., Molecular Cloning 2nd ed. 9.47-9.58, Cold Spring Harbor Lab. Press, 1989). One skilled in the art can readily isolate a DNA having high homology with a whole or part of the *MAPJD* DNA sequence (*e.g*., SEQ ID NO; 54) encoding the human MAPJD protein, and isolate proteins that are functionally equivalent to the human MAPJD protein from the isolated DNA. The proteins used for the present invention include those that are encoded by DNA that hybridize with a whole or part of the RNA sequence encoding the human MAPJD protein and are functional equivalents of the human MAPJD protein. These proteins include mammal homologues corresponding to the protein derived from human or mouse (for example, a protein encoded by a monkey, rat, rabbit and bovine gene). In isolating a cDNA highly homologous to the DNA encoding the human MAPJD protein from animals, it is particularly preferable to use tissues from lung cancers. The condition of hybridization for isolating a DNA encoding a functional equivalent of the human MAPJD protein can be routinely selected by a person skilled in the art. For example, hybridization may be performed by conducting prehybridization at 68°C for 30 mill or longer using "Rapid-hyb buffer" (Amersham LIFE SCIENCE), adding a labeled probe, and warning at 68°C for 1 hour or longer. The following washing step can be conducted, for example, in a low stringent condition. A low stringency condition is, for example, 42°C, 2x SSC, 0.1% SDS, or preferably 50°C, 2x SSC, 0.1% SDS. More preferably, highly stringent conditions are used. In the context of the present invention, a highly stringent condition includes, for example, washing 3 times in 2x SSC, 0.01% SDS at room temperature for 20 min, then washing 3 times in 1x SSC, 0.1% SDS at 37°C for 20 min, and washing twice in 1x SSC, 0.1% SDS at 50°C for 20 min. However, several factors such as temperature and salt concentration can influence the stringency of hybridization and one skilled in the art can suitably select the factors to achieve the requisite stringency. In place of hybridization, a gene amplification method, for example, the polymerase chain reaction (PCR) method, can be utilized to isolate a DNA encoding protein that is functionally equivalent to the human MAPJD protein, using a primer synthesized based on the sequence information of the DNA (SEQ ID NO: 54) encoding the human MAPJD protein (SEQ ID NO: 55). Proteins that are functional equivalents of the human MAPJD protein, encoded by DNA isolated through the above hybridization techniques or by gene amplification techniques, normally have a high homology to the amino acid sequence of the human MAPJD protein. "High homology" (also referred to as "high identity") typically refers to the degree of identity between two optimally aligned sequences (either polypeptide or polynucleotide sequences)..-Typically, high homology or identity refers to homology of 40% or higher, preferably 60% or higher, more preferably 80% or higher, even more preferably 85%, 90%, 95%, 98%, 99%, or higher. The degree of homology or identity between two polypeptide or polynucleotide sequences can be determined by following the algorithm in "Wilbur WJ. and Lipman DJ. Proc.. Natl. Acad. Sci. USA (1983) 80, 726-30". A protein useful in the context of the present invention may have variations in amino acid sequence, molecular weight, isoelectric point, the presence or absence of sugar chains, or form, depending on the cell or host used to produce it or the purification method utilized. Nevertheless, so long as it is a function equivalent of human MAPJD protein (SEQ ID NO: 55), it is useful in the present invention. The proteins useful in the context of the present invention can be prepared as recombinant proteins or natural proteins, by methods well known to those skilled in the art. A recombinant protein can be prepared by inserting a DNA encoding a protein of the present invention (for example, the DNA comprising the nucleotide sequence of SEQ ID NO: 55), into an appropriate expression vector, introducing the vector into an appropriate host cell, obtaining the extract, and purifying the protein by subjecting the extract to chromatography, for example, ion exchange chromatography, reverse phase chromatography, gel filtration, or affinity chromatography utilizing a column to which antibodies against the protein of the present invention is fixed, or by combining more than one of aforementioned columns. In addition, when a protein useful in the context of the present invention is expressed within host cells (for example, animal cells and *E. coli*) as a fusion protein with glutathione-S-transferase protein or as a recombinant protein supplemented with multiple histidines, the expressed recombinant protein can be purified using a glutathione column or nickel column. After purifying the fusion protein, it is also possible to exclude regions other than the objective protein by cutting with thrombin or factor-Xa as required. A natural protein can be isolated by methods known to a person skilled in the art, for example, by contacting an affinity column, in which antibodies binding to the MAPJD protein described below are bound, with the extract of tissues or cells expressing the protein of the present invention. The antibodies can be polyclonal antibodies or monoclonal antibodies. In the present invention, the acetylation activity of a MAPJD polypeptide can be determined by methods known in the art. For example, a MAPJD polypeptide associated with HAT complex and a histone H4 as the substrate can be incubated with a labeled acetyl donor, under suitable assay conditions. Acetylation level of histone may be determined by any method known in the art. For example, acetylation level of histone may be determined by incubating histones and detectably labeled acetyl CoA and subsequently detecting qualitatively or quantitatively, whether the histones are labeled. The acetyl group of the acetyl CoA can be labeled with ³H. Transfer of the label to the histone can be detected, for example, by SDS-PAGE electrophoresis and fluorography. Alternatively, following the reaction, the histone can be separated from the acetyl donor by nitration, and the amount of label retained on the filter quantitated by scintillation counting. Other suitable labels that can be attached to acetyl donors, such as chromogenic and fluorescent labels, and methods of detecting transfer of these labels to histones, are known in the art. Alternatively, the acetylation activity of MAPJD associated with HAT complex can be determined using an unlabeled acetyl donor (e.g. acetyl CoA) and reagents that selectively recognize acetylated histone H4. Antibodies recognizing acetylated histone H4 are commercially available. For instance, Abcom provides some antibodies that specifically recognize histone H4s acetylated at Lys 91, Lys 16, Lys 12, Lys 8, or Lys 5. For example, after incubation of MAPJD associated with HAT complex, substrate to be acetylated and acetyl donor, under conditions suitable for acetylation of the substrate, acetylated substrate can be detected using conventional immunological methods. Any immunological techniques that use an antibody to recognize a acetylated substrate can be used for the detection. Various low-throughput and high-throughput enzyme assay formats are known in the art and can be readily adapted for detection or measuring of the acetylation activity of MAPJD associated with HAT complex. For high-throughput assays, the histone H4 substrate can conveniently be immobilized on a solid support, such as a multiwell plate, slide or chip. Following the reaction, the acetylated product can be detected on the solid support by them methods described above. Alternatively, the acetylation reaction can take place in solution, after which the histone H4 can be immobilized on a solid support, and the acetylated product detected. To facilitate such assays, the solid support can be coated with streptavidin and the histone H4 labeled with biotin, or the solid support can be coated with anti-histone H4 antibodies. The skilled person can determine suitable assay formats depending on the desired throughput capacity of the screen. The present invention also encompasses the use of partial peptides of a protein of the present invention. A partial peptide has an amino acid sequence specific to the MAPJD protein and consists of less than about 400 amino acids, usually less than about 200 and often less than about 100 amino acids, and at least about 7 amino acids, preferably about 8 amino acids or more, and more preferably about 9 amino acids or more. The partial peptide can be used, for example, in the screening for an agent or compound that binds to the MAPJD protein, and the screening for inhibitors of the binding between MAPJD and a co-factor thereof, such as, for example, MYC. The partial peptide containing the MYC binding-domain (i.e. JmjC domain) is preferably used for such screening. A partial peptide useful in the context of the present invention can be produced by genetic engineering, by known methods of peptide synthesis, or by digesting the protein of the invention with an appropriate peptidase. For peptide synthesis, for example, solid phrase synthesis or liquid phase synthesis may be used. In view of the evidence provided herein, that FGFR1OP significantly reduces ABL1-dependent phosphorylation of WRNIP1, one aspect of the invention involves identifying test compounds that modulate, e.g. reduce or prevent, such activity of FGFR1OP. The present invention thus provides a method of screening for an agent that modulates FGFR1OP-mediated inhibition of phosphorylation of WRNIP1 by ABL 1. The method is practiced by contacting an ABL1 polypeptide or a functional equivalent thereof associated with FGFR1OP or a functional equivalent thereof with WRNIP1 or a functional equivalent thereof under the conditions suitable for the interaction thereof, and assaying phosphorylation level of WRNIP1. An agent that modulates phosphorylation of WRNIP1 is thereby identified. In the present invention, suitable conditions for the interaction of FGFR1OP, ABL1 and WRNIP1 can be provided *in vivo* or *in vitro.* For instance, the condition for the interaction may be provided by co-transfection of vectors to express FGFR1OP, ABL1 and WRNIP1. Alternatively, FGFR1OP expressing cell transfected with ABL1 expressing vector and WRNIP1 may also be used for the condition for interaction thereof *in vivo*. Further, ABL1 polypeptide mixed with FGFR1OP polypeptide may be mixed with WNRIP1 as substrate and suitable phosphate doner and incubated in the suitable condition and suitable time for interaction *in vitro*.

As used herein, the term "WRNIP1" refers to full length Werner helicase interacting protein 1 (WRNTP1 alias WHIP) (*e.g*., GenBank Accession No. NM_020135; SEQ ID NO: 93 encoded by SEQ ID NO: 92) as well as mutants and fragments thereof. For examples, fragment peptide comprising phosphorylation site of WRNIP1 may also be used as the substrate.

Moreover the present invention is also based on the discovery that FGFR1OP significantly reduces ABL1-dependent phosphorylation of WRNIP1 and appears to promote cancer cell cycle progression. Accordingly, the present invention provides methods of identifying compounds for preventing or treating lung cancer by identifying compounds that inhibit the reduction of ABL1-dependent phosphorylation of WRNIP1 by FGFR1OP. So that, the present invention provides methods of identifying compounds for preventing or treating lung cancer by identifying compounds that increase a phosphorylation of WRNIP1 by an ABL1 associated with FGFR1OP. Therefore, the present invention also provides a method of screening for compounds that are useful for inhibiting the binging between the proteins of present invention, enhancing the ABL1-mediated phosphorylation of WRNIP1, inhibiting the growth of the lung cancer cells, and treating or preventing lung cancer, the methods comprise the steps of:
(a) contacting a test compound to a FGFR1OP polypeptide or functional equivalent thereof, WRNIP1 polypeptide or functional equivalent thereof and an ABL1 polypeptide or functional equivalent thereof in the suitable condition for phosphorylation of WRNIP1 polypeptide or functional equivalent thereof;
(b) detecting a phosphorylation level of the WRNIP1 polypeptide or functional equivalent thereof, and
(c) selecting the test compound that increase the phosphorylation level detected in step (b) to be phosphorylated as compared to a control phosphorylation level detected in the absence of the test compound as an enhancer; selecting the test compound that decrease the phosphorylation level detected in step (b) to be phosphorylated as compared to a control phosphorylation level detected in the absence of the test compound as an inhibitor.

In the present invention, the term "functionally equivalent" means that the subject protein or polypeptide has the same or substantially the same phosphorylation or binding activity as ABL1. In particular, the protein catalyzes the phosphorylation of a WRNIP1 or a fragment thereof that includes phosphorylation site. Whether a subject protein has the target activity can be routinely determined by the present invention. Namely, the phosphorylation activity can be determined by (a) contacting an ABL1 with a subject protein, under conditions suitable for phosphorylation of the subject protein, and (b) detecting the phosphorylation level of the subject protein. Furthermore, whether a subject protein has the phosphorylation activity can be routinely determined by the present invention. Namely, the kinase activity can be determined by (a) contacting a subject protein with a WRNIP1 under conditions suitable for phosphorylation of the WRNIP1, and (b) detecting the phosphorylation level of the WRNIP1. In addition, the inhibition activity of ABL1-mediated phosphorylation of WRNIP1 can be determined by (a) contacting an ABL1 and WRNIP1 in the presence or absence of the subject protein under conditions suitable for phosphorylation of WRNIP1, and (b) detecting the phosphorylation level of WRNIP1. Furthermore, whether a subject protein has the phosphorylation activity can be routinely determined by the present invention. Namely, the kinase activity can be determined by (a) contacting a subject protein with a WRNIP1 under conditions suitable for phosphorylation of the WRNIP1, and (b) detecting the phosphorylation level of the WRNIP1. If the phosphorylation level of WRNIP1 detected in the presence of the subject protein is higher than that detected in the absence of the subject protein, the subjecte protein has inhibition activity of ABL1-mediated phosphorylation of WRNIP1.

Methods for preparing proteins that are functional equivalents of a given protein are well known to those skilled in the art and include conventional methods of introducing mutations into the protein. For example, one skilled in the art can prepare proteins functionally equivalent to the human ABL1 protein or WRNIP1 protein by introducing an appropriate mutation in the amino acid sequence of the human ABL1 protein or WRNIP1 protein using site-directed mutagenesis, for example (Hashimoto-Gotoh T. et al. (1995), Gene 152, 271-5; Zoller MJ and Smith M. (1983) Methods Enzymol. 100, 468-500; Kramer W, et al. (1984), Nucleic Acids Res. 12, 9441-56; Kramer W and Fritz HJ. (1987) Methods. Enzymol. 154, 350-67; Kunkel TA (1985), Proc. Natl. Acad. Sci. USA. 82, 488-92). Amino acid mutations can occur in nature, too. An ABL1 peptide or a WRNIP1 polypeptide useful in the context of the present invention includes those proteins having the amino acid sequences of the human ABL1 protein or WRNIP1 protein in which one or more Amino acids are mutated, provided the resulting mutated proteins are functional equivalents of the human ABL1 protein or WRNIP1 protein, more particularly retain the phosphorylation activity of WRNIP1 protein by ABL1 protein. The number of amino acids to be mutated in such a mutant is generally 20 amino acids or less, typically 10 amino acids or less, preferably 6 amino acids or less, and more preferably 3 amino acids or less. To maintain the phosphorylation activity, the FGFR1OP binding domains preferably conserved in the amino acid sequence of the mutated proteins.

Mutated or modified proteins, i.e., proteins having amino acid sequences modified by deleting, adding and/or replacing one or more amino acid residues of a certain amino acid sequence, are known to retain the biological activity of the original protein (Mark DF. et al., Proc. Natl. Acad. Sci. USA (1994) 81, 5662-6, Zoller MJ. & Smith M. Nucleic Acids Research (1982) 10, 6497-500, Wang A. et al., Science 224, 1431-3, Dalbadie-McFarland G. et al., Proc. Natl. Acad. Sci. USA (1982) 79, 6409-13).

The amino acid residue to be mutated is preferably mutated into a different amino acid that allows the properties of the amino acid side-chain to be conserved (a process known as conservative amino acid substitution). Examples of properties of amino acid side chains include hydrophobic amino acids (A, I, L, M, F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxyl group containing side-chain (S, T, Y); a sulfur atom containing side-chain (C, M), a carboxylic acid and amide containing side-chain (D, N, E, Q); a base containing side-chain (R, K, H); and an aromatic containing side-chain (H, F, Y, W). Note, the parenthetic letters indicate the one-letter codes of amino acids.

An example of a protein in one or more amino acids residues are added to the amino acid sequence of human ABL1 protein (SEQ ID NO: 93) or WRNIP1 protein (SEQ ID NO: 91) is a fusion protein containing the human ABL1 protein or WRNIP1 protein. Fusion proteins include fusions of the human ABL1 protein or WRNIP1 protein and other peptides or proteins, and are used in the present invention. Fusion proteins can be made by techniques well known to a person skilled in the art, such as by linking the RNA encoding the human ABL1 protein or WRNIP1 protein of the invention with DNA encoding other peptides or proteins, so that the frames match, inserting the fusion DNA into an expression vector and expressing it in a host. There is no restriction as to the peptides or proteins fused to the protein of the present invention.

Known peptides that can be used as peptides to be fused to the ABL1 protein or WRNIP1 protein include, for example, FLAG (Hopp T. P. et al., Biotechnology (1988) 6, 1204-10), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, Ick tag, α-tubulin fragment, B-tag, Protein C fragment, and the like. Examples of proteins that may be fused to a protein of the invention include GST (glutathione-S-transferase), Influenza agglutinin (HA), immunoglobulin constant region, β-galactosidase, MBP (maltose-binding protein), and such.

Fusion proteins can be prepared by fusing commercially available DNA, encoding the fusion peptides or proteins discussed above, with the DNA encoding the protein of the present invention and expressing the fused DNA prepared.

An alternative method known in the art to isolate functionally equivalent proteins uses hybridization techniques to identify homologous sequences (Sambrook J. et al., Molecular Cloning 2nd ed. 9.47-9.58, Cold Spring Harbor Lab. Press, 1999). One skilled in the art can readily isolate a DNA having high homology with a whole or part of the ABL1 RNA or WRNIP1 DNA sequence (*e.g*., SEQ ID NO: 92 or SEQ ID NO: 90) encoding the human ABL1 protein or WRNIP1 protein, and isolate proteins that are functionally equivalent to the human ABL1 protein or WRNIP1 protein from the isolated DNA. The proteins used for the present invention include those that are encoded by DNA that hybridize with a whole or part of the DNA sequence encoding the human ABL1 protein or WRNIP1 protein and are functional equivalents of the human ABL1 protein or WRNIP1 protein. These proteins include mammal homologues corresponding to the protein derived from human or mouse (for example, a protein encoded by a monkey, rat, rabbit and bovine gene). In isolating a cDNA highly homologous to the DNA encoding the human ABL1 protein or WRNIP1 protein from animals, it is particularly preferable to use tissues from lung cancers.

The condition of hybridization for isolating a DNA encoding a functional equivalent of the human ABL1 protein or WRNIP1 protein can be routinely selected by a person skilled in the art. For example, hybridization may be performed by conducting prehybridization at 68°C for 30 mm or longer using "Rapid-hyb buffer" (Amersham LIFE SCIENCE), adding a labeled probe, and warming at 68°C for 1 hour or longer. The following washing step can be conducted, for example, in a low stringent condition. A low stringency condition is, four example, 42°C, 2x SSC, 0.1% SDS, or preferably 50°C, 2x SSC, 0.1% SDS. More preferable, highly stringent conditions are used. In the context of the present invention, a highly stringent condition includes, for example, washing 3 times in 2x SSC, 0.01% SDS at room temperature for 20 min, then washing 3 times in 1x SSC, 0.1 % SDS at 37°C for 20 min, and washing twice in 1x SSC, 0.1 % SDS at 50°C for 20 min. However, several factors such as temperature and salt concentration can influence the stringency of hybridization and one skilled in the art can suitably select the factors to achieve the requisite stringency.

In place of hybridization, a gene amplification method, for example, the polymerise chain reaction (PCR) method, can be utilized to isolate a DNA encoding a protein that is functionally equivalent to the human ABL1 protein or WRNIP1 protein, using a primer synthesized based on the sequence information of the DNA (SEQ ID NO: 92 or SEQ ID NO: 90) encoding the human ABL1 protein or WRNIP1 protein (SEQ ID NO: 93 or SEQ ID NO: 91).

Proteins that are functional equivalents of the human ABL1 protein or WRNIP1 protein, encoded by DNA isolated through the above hybridization techniques or by gene amplification techniques, normally have a high homology to the amino acid sequence of the human ABL1 protein or WRNIP1 protein. "High homology" (also referred to as "high identity") typically refers to the degree of identity between two optimally aligned sequences (either polypeptide or polynucleotide sequences). Typically, high homology or identity refers to homology of 40% or higher, preferable 60% or higher more preferably 80% or higher, even more preferably 85%, 90%, 95%, 98%, 99%, or higher. The degree of homology or identity between two polypeptide or polynucleotide sequences can be determined by following the algorithm in "Wilbur WJ. and Lipman, DJ. Proc. Natl. Acad. Sci. USA (1983) 80, 726-30".

A protein useful in the context of the present invention may have variations in amino acid sequence, molecular weight, isoelectric point, the presence or absence of sugar chains, or form, depending on the cell or host used to produce it or the purification method utilized. Nevertheless, so long as it is a function equivalent of human ABL1 protein or WRNIP1 protein (SEQ ID NO: 93 or SEQ ID NO: 91), it is useful in the present invention.

The proteins useful in the context of the present invention can be prepared as recombinant proteins or natural proteins, by methods well known to those skilled in the art. A recombinant protein can be prepared by inserting a DNA encoding a protein of the present invention (for example, the DNA comprising the nucleotide sequence of SEQ ID NO: 92 or SEQ ID NO: 90), into an appropriate expression vector, introducing the vector into an appropriate host cell, obtaining the extract, and purifying the protein by subjecting the extract to chromatography, for example, ion exchange chromatography, reverse phase chromatography, gel filtration, or affinity chromatography utilizing a column to which antibodies against the protein of the present invention is fixed, or by combining more than one of aforementioned columns.

In addition, when a protein useful in the context of the present invention is expressed within host cells (for example, animal cells and *E. coli*) as a fusion protein with glutathione-S-transferase protein or as a recombinant protein supplemented with multiple histidines, the expressed recombinant protein can be purified using a glutathione column or nickel column.

After purifying the fusion protein, it is also possible to exclude regions other than the objective protein by cutting with thrombin or factor-Xa as required.

A natural protein can be isolated by methods known to a person skilled in the art, for example, by contacting an affinity column, in which antibodies binding to the ABL1 protein or WRNIP1 protein described below are bound, with the extract of tissues or cells expressing the protein of the present invention. The antibodies can be polyclonal antibodies or monoclonal antibodies.

In the present invention, the kinase activity of an ABL1 polypeptide or the phosphorylation of WRNIP1 polypeptide can be determined by methods known in the art. For example, an ABL1 polypeptide and a WRNIP1 polypeptide as the substrate can be incubated with a labeled phosphate donor, under suitable assay conditions. Phosphorylation level of WRNIP1 may be determined by any method known in the art. For example, phosphorylation level of WRNIP1 may be determined by incubating WRNIP1 and detectably labeled phosphate, e.g. γ-³²P, and subsequently detecting qualitatively or quantitatively, whether the WRNIP1 are labeled. Transfer of the label to the WRNIP1 can be detected, for example, by SDS-PAGE electrophoresis and fluorography. Alternatively, following the reaction, the WRNIP1 protein can be separated from the phosphate donor by filtration, and the amount of label retained on the filter quantitated by scintillation counting. Other suitable labels that can be attached to phosphate donors, such as chromogenic and fluorescent labels, and methods of detecting transfer of these labels to WRNIP1; are known in the art.

Alternatively, the kinase activity of ABL1 can be determined using an unlabeled phosphate donor and reagents that selectively recognize phosphorylated WRNIP1. Alternatively, antibodies recognizing phosphorylated WRNIP1 can be used. For example, after incubation of ABL1, WRNIP1 to be phosphorylated and phosphate donor, under conditions suitable for phosphorylation of the WRNIP1, phosphorylated WRNIP1 can be detected using conventional immunological methods. Any immunological techniques that use an antibody to recognize phosphorylated WRNIP1 can be used for the detection.

Furthermore, the inhibition activity of FGFR1OP for the kinase activity of an ABL1 polypeptide or the phosphorylation of WRNIP1 polypeptide can be determined by methods mentioned above. For example an ABL1 polypeptide associated with FGFR1OP and a WRNIP1 polypeptide as the substrate can be incubated with a labeled phosphate donor, under suitable assay conditions. Phosphorylation level of WRNIP1 may be determined by any method known in the art. For example, phosphorylation level of WRNIP1 may be determined by incubating WRNIP1 and detectably labeled phosphate, e.g. γ-³²P, and subsequently detecting qualitatively or quantitatively, whether the WRNIP1 are labeled. Transfer of the label to the WRNIP1 can be detected, for example, by SDS-PAGE electrophoresis and fluorography. Alternatively, following the reaction, the WRNIP1 protein can be separated from the phosphate donor by filtration, and the amount of label retained on the filter quantitated by scintillation counting. Other suitable labels that can be attached to phosphate donors, such as chromogenic and fluorescent labels, and methods of detecting transfer of these labels to WRNIP1, are known in the art.

Various low-throughput and high-throughput enzyme assay formats are known in the art and can be readily adapted for detection or measuring of the ABL1-mediated phosphorylation of WRNIP1. For high-throughput assays, the WRNIP1 can conveniently be immobilized on a solid support, such as a multiwell plate, slide or chip. Following the reaction, the phosphorylated product can be detected on the solid support by the methods described above. Alternatively, the phosphorylation reaction can take place in solution, after which the WRINP1 can be immobilized on a solid support, and the phosphorylated product detected. To facilitate such assays, the solid support can be coated with streptavidin and the WRNIP1 labeled with biotin, or the solid support can be coated with anti-WRNIP1 antibodies. The skilled person can determine suitable assay formats depending on the desired throughput capacity of the screen.

The present invention also encompasses the use of partial peptides of a protein of the present invention. A partial peptide has an amino acid sequence specie to the ABL1 protein and consists of less than about 400 amino acids, usually less than about 200 and often less than about 100 amino acids, and at least about 7 amino acids, preferably about 8 amino acids or more, and more preferably about 9 amino acids or more. The partial peptide can be used, for example, in the screening for an agent or compound that binds to the ABL1 protein, and the screening for inhibitors of the binding between ABL1 and a co-factor thereof, such as, for example, FGFR1OP. The partial peptide containing the FGFR1OP binding-domain is preferably used for such screening.

A partial peptide useful in the context of the present invention can be produced by genetic engineering, by known methods of peptide synthesis, or by digesting the protein of the invention with an appropriate peptidase. For peptide synthesis, for example, solid phase synthesis or liquid phase synthesis may be used.

Any test agent can be used. Examples include, but are not limited to, cell extracts, cell culture supernatant, products of fermenting microorganism, extracts from marine organism, plant extracts, purified or crude proteins, peptides, non-peptide compounds, synthetic micromolecular compounds and natural compounds.

As disclosed herein, any test compounds, including, *e.g*., proteins (including antibodies), muteins, polynucleotides, nucleic acid aptamers, and peptide and nonpeptide small organic molecules, may serve as test compounds of the present invention. Test compounds may be isolated from natural sources, prepared synthetically or recombinantly, or any combination of the same.

For example, peptides may be produced synthetically using solid phase techniques as described in "Solid Phase Peptide Synthesis" by G. Barany and R. B. Merrifield in Peptides, Vol. 2, edited by E. Gross and J. Meienhoffer, Academic Press, New York, N.Y., pp. 100-118 (1980). Similarly, nucleic acids can also be synthesized using the solid phase techniques, as described in Beaucage SL., & Iyer R.P. (1992) Tetrahedron, 48, 2223-311; and Matthes et al., EMBO J., 3:801-5 (1984).

Where modulatory peptides are identified, modifications of peptides of the present invention with various amino acid mimetics or unnatural amino acids are particularly useful in increasing the stability of the peptide *in vivo.* Stability can be assayed in a number of ways. For instance, peptidases and various biological media, such as human plasma and serum, have been used to test stability. *See, e.g.,* Verhoef et al., Eur. J. Drug Metab Pharmacokin. 11:291-302 (1986). Other useful peptide modifications known in the art include glycosylation and acetylation.

Both recombinant and chemical synthesis techniques may be used to produce test compounds of the present invention. For example, a nucleic acid test compound may be produced by insertion into an appropriate vector, which may be expanded when transacted into a competent cell. Alternatively, nucleic acids may be amplified using PCR techniques or expression in suitable hosts (cf. Sambrook et al., Molecular Cloning. A Laboratory Manual 1989, Cold Spring Harbor Laboratory, New York, USA).

Peptides and proteins may also be expressed using recombinant techniques well known in the art, *e.g.*, by transforming suitable host cells with recombinant DNA constructs as described in Morrison DA, J. Bact., 132:349-51 (1977); and Clark-Curtiss & Curtiss, Methods in Enzymology, 101:347-62 (1983).

### VIII-2 Anti- target molecule and anti-partner molecule antibodies;

In some aspects of the present invention, test compounds are anti-target molecule or anti-partner molecule antibodies. In some embodiments, the antibodies are chimeric, including but not limited to, humanized antibodies. In some cases, antibody embodiments of the present invention will bind either target molecule or partner molecule at the interface where one of these proteins associates with the other.

In some embodiments, these antibodies bind target molecule or partner molecule with a Kₐ of at least about 10⁵ mol⁻¹, 10⁶ mol⁻¹ or greater, 10⁷ mol⁻¹ or greater, 10⁸ mol⁻¹ or greater, or 10⁹ mol⁻¹ or greater under physiological conditions. Such antibodies can be purchased from a commercial source, for example, Chemicon, Inc. (Temecula, CA), or can be raised using as an immunogen, such as a substantially purified target molecule or partner molecule protein, *e.g*., a human protein, or an antigenic fragment thereof. Methods of preparing both monoclonal and polyclonal antibodies from provided immunogens are well-known in the art. For purification techniques and methods for identifying antibodies to specific immunogens, *see e.g.,* PCT/US02/07144 (WO/03/077838) incorporated by reference herein in its entirety.

Methods for purifying antibodies using, for example, antibody affinity matrices to form an affinity column are also well known in the art and available commercially (AntibodyShop, Copenhagen, Denmark). Identification of antibodies capable of disrupting target/partner association is performed using the same test assays detailed below for test compounds in general.

### VIII-3 Converting enzymes:

Converting enzymes may act as test compounds of the present invention. In the context of the present invention, converting enzymes are molecular catalysts that perform covalent post-transitional modifications to either target molecule, partner molecule, or both. Converting enzymes of the present invention will covalently modify one or more amino acid residues of target molecule and/or partner molecule in a manner that causes either an allosteric alteration in the structure of the modified protein, or alters the target/partner molecular binding site chemistry or structure of the modified protein in a manner that interferes with binding between target molecule and partner molecule.

Herein, interference with binding between the two molecules refers to a decrease in the Kₐ of binding by at least 25%, 30%, 40%, 50%, 60%, 70% or more relative to the Ka of binding between the proteins measured at 30°C and an ionic strength of 0.1 in the absence of detergents. Exemplary converting enzymes of the invention include kinases, phosphatases, amidases, acetylases, glycosidase and the like.

### VIII-4 Constructing test compound libraries;

Although the construction of test compounds libraries is well known in the art, the present section provides additional guidance in identifying test compounds and construction libraries of such compounds for screening of effective inhibitors of target/partner interaction and/or target molecule-mediated activity such as acetylation or transcriptional activity, *etc*.

### VIII-5 Molecular modeling:

Construction of test compound libraries is facilitated by knowledge of the molecular structure of compounds known to have the properties sought, and/or the molecular structure of the target molecules to be inhibited, *i.e*., target molecules and partner molecules. One approach to preliminary screening of test compounds suitable for further evaluation is computer modeling of the interaction between the test compound and its target. In the present invention, modeling the interaction between target molecules and/or partner molecules provides insight into both the details of the interaction itself, and suggests possible strategies for disrupting the interaction, including potential molecular inhibitors of the interaction.

Computer modeling technology allows visualization of the three-dimensional atomic structure of a selected molecule and the rational design of new compounds that will interact with the molecule. The three-dimensional construct typically depends on data from x-ray crystallographic analysis or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

An example of the molecular modeling system described generally above consists of the CHARMm and QUANTA programs, Polygon Corporation, Waltham, Mass. CHARMm performs the energy minimization and molecular dynamics functions. QUANTA performs the construction, graphic modeling and analysis of molecular structure. QUANTA allows interactive construction, modifications, visualization, and analysis of the behavior of molecules with each other.

A number of articles review computer modeling of drugs interactive with specific proteins, such as Rouvinen, et al., Acta Pharmaceutica Fennica 97, 159-166 (1988); Ripka, New Scientist 54-58. (Jun. 16, 1988); McKinlay and Rossmann, Annu. Rev. Pharmacol. Toxiciol. 29, 111-22 (1989); Perry and Davies, Prog Clin Biol Res. 291:189-93(1989); Lewis and Dean, Proc. R. Soc. Lond. 23 6, 125-40 and 141-62 (1989); and, with respect to a model receptor for nucleic acid components, Askew, et al., J. Am. Chem. Soc. 111, 1082-90 (1989).

Other computer programs that screen and graphically depict chemical are available from companies such as BioDesign, Inc., Pasadena, Calif., Allelix, Inc, Mississauga, Ontario, Canada, and Hypercube, Inc., Cambridge, Ontario. *See, e.g.,* DesJarlais et al. (1998) J. Med. Chem. 31:722-9; Meng et al. (1992) J. Computer Chem. 13:505-24; Menu et al. (1993) Proteins 17:266-78; Shoichet et al. (1993) Science 259:1445-50.

Once a putative inhibitor of the target molecule/partner molecule interaction has been identified, combinatorial chemistry techniques can be employed to construct any number of variants based on the chemical structure of the identified putative inhibitor, as detailed below. The resulting library of putative inhibitors or "test compounds" may be screened using the methods of the present invention to identify test compounds of the library that disrupt the target molecule/partner molecule association.

### VIII-6 Combinatorial chemical synthesis:

Combinatorial libraries of test compounds may be produced as part of a rational drug design program involving knowledge of core structures existing in known inhibitors of the target molecules/partner molecules interaction. This approach allows the library to be maintained at a reasonable size, facilitating high throughput screening. Alternatively, simple, particularly short polymeric molecular libraries may be constructed by simply synthesizing all permutations of the molecular family making up the library. An example of this latter approach would be a library of all peptides six amino acids in length. Such a peptide library could include every 6 amino acid sequence permutation. This type of library is termed a linear combinatorial chemical library.

Preparation of combinatorial chemical libraries is well known to those of skill in the art, and may be generated by either chemical or biological synthesis. Combinatorial chemical libraries include, but are not limited to, peptide libraries *(see, e.g.,* U.S. Patent 5,01.0,175, Furka, Int. J. Pept. Prot, Res. 37:487-:93 (1991) and Houghten et al., Nature 354:84-6 (1991)). Other chemistries for generating chemical diversity libraries can also be used. Such chemistries include, but are not limited to: peptides (*e.g.,* PCT Publication No. WO 91/19735), encoded peptides (*e.g.,* P CT Publication WO 93/20242), random bio-oligomers (*e.g.*, PCT Publication No. WO 92/00091), benzodiazepines (*e.g.*, U.S. Pat. No. 5,288,514), diversomers such as hydantoins, benzodiazepines and dipeptides (DeWitt et al., Proc. Natl. Acad Sci USA 90:6909-13 (1993)), vinylogous polypeptides (Hagihara et al., J Amber. Chem. Soc. 114:6568-70 (1992)), nonpeptidal peptidomimetics with glucose scaffolding (Hirschmann et al., J Amer. Chem. Soc. 114:9217-8 (1992)), analogous organic syntheses of small compound libraries (Chen et al., J. Amer. Chem. Soc, 116-2661 (1994)), oligocarbamates (Cho et al., Science 261:1303-5 (1993)), and/or peptidyl phosphonates (Campbell et al., J. Org. Chem. 59:658 (1994)), nucleic acid libraries (*see* Ausubel, and Sambrook, all *supra*), peptides nucleic acid libraries *(see, e.g.,* U.S. Patent 5,539,083), antibody libraries *(see, e.g.,* Vaughan et al., Natur-e Biotechnology, 14(3):309-14 (1996) and PCT/US96/10287), carbohydrate libraries (*see, e.g,* Liang et al., Science, 274:1520-2 (1996) and U.S. Patent 5,593,853), small organic molecule libraries *(see, e.g.,* benzodiazepines, Gordon EM. Curr Opin Biotechnol. 1995 Dec 1;6(6):624-31.; isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines, 5,288,514, and the like).

### VIII-7 Phage display:

Another approach uses recombinant bacteriophage to produce libraries. Using the "phage method" (Scott and Smith, Science 249:386-90, 1990; Cwirla, et al, Proc. Natl. Acad. Sci., 87:6378-82, 1990; Devlin et al., Science, 249:404-6, 1990), very large libraries can be constructed (*e.g.*, 10⁶ -10⁸ chemical entities). A second approach uses primarily chemical methods, of which the Geyser method (Geysen et al., Mol Immunol. 23:709-15,1986; Geysen et al. J Immunol Methods. 1987 Sep 24;102(2):259-74.) and the method of Fodor et al. (Science 251:767-73, 1991) are examples. Furka et al. (14th International Congress of Biochemistry, Volume #5, Abstract FR:013, 1988; Furka, Int. J. Peptide Proteins Res. 37:487-93, 1991), (US Pat No. 4,631,211) and (US Pat. No. 5,010,175) describe methods to produce a mixture of peptides that can be tested as agonists or antagonists.

Devices for the preparation of combinatorial libraries are commercially available (*see*, *e.g.*, 357 MPS, 390 MRS, Advanced ChemTech, Louisville KY, Symphony, Rainin, Woburn, MA, 433A Applied Biosystems, Foster City, CA, 9050 Plus, Millipore, Bedford, MA). In addition, Numerous combinatorial libraries are themselves commercially available (*see, e.g.*, ComGenex, Princeton, N.J., Tripos, Inc., St. Louis, MO, 3D Pharmaceuticals, Exton, PA, Martek Biosciences, Columbia, MD, *etc*.).

### VIII-8 Screening test compound libraries:

Screening methods of the present invention provide efficient and rapid identification of test compounds that have a high probability of interfering with the target molecules/partner molecules association or with target molecules-mediated activity. Generally, any method that determines the ability of a test compound to interfere with the target molecule/partner molecule association or target molecule-mediated activity is suitable for use with the present invention. For example, competitive and non-competitive inhibition assays in an ELISA' format may be utilized. Control experiments should be performed to determine maximal binding capacity of system (*e.g.*, contacting bound target molecule with partner molecule and determining the amount of partner molecule that binds to target molecule in the examples below).

### VIII-9 Competitive assay format:

Competitive assays may be used for screening test compounds of the present invention. By way of example, a competitive ELISA format may include target molecule (or partner molecule) bound to a solid support The bound target molecule (or partner molecule) would be incubated with partner molecule (or target molecule) and a test compound. After sufficient time to allow the test compound and/or partner molecule (or target molecule) to bind target molecule (or partner molecule), the substrate would be washed to remove unbound material. The amount of partner molecule (or target molecule) bound to target molecule (or partner molecule) is then determined.

This may be accomplished in any of a variety of ways known in the art, for example, by using an partner molecule (or target molecule) species tagged with a detectable label, or by contacting the washed substrate with a labeled anti-partner molecule (or target molecule) antibody. The amount of partner molecule (or target molecule) bound to target molecule (or partner molecule) will be inversely proportional to the ability of the test compound to interfere with the partner molecule/target molecule association. Protein, including but not limited to, antibody, labeling is described in Harlow & Lane, Antibodies, A Laboratory Manual (1988).

In a variation, target molecule (or partner molecule) is labeled with an affinity tag. Labeled target molecule (or partner molecule) is then incubated with a test compound and partner molecule (or target molecule), then immunoprecipitated, The immunoprecipitate is then subjected to Western blotting using an anti-partner molecule (or target molecule) antibody. As with the previous competitive assay format, the amount of partner molecule (or target molecule) found associated with target molecule (or partner molecule) is inversely proportional to the ability of the test compound to interfere with the target/partner association.

### VII-10 Non-competitive assay format;

Non-competitive binding assays may also find utility as an initial screen for test compound libraries constructed in a format that is not readily amenable to screening using competitive assays, such as those described herein. An example of such a library is a phage display library (*See, e.g.,* Barrett, et al. (1992) Anal. Biochem 204,357-64).

Phage libraries find utility in being able to produce quickly working quantities of large numbers of different recombinant peptides. Phage libraries do not lend themselves to competitive assays of the invention, but can be efficiently screened in a non-competitive format to determine which recombinant peptide test compounds bind target molecule or partner molecule. Test compounds identified as binding can then be produced and screened using a competitive assay format. Production and screening of phage and cell display libraries is well-known in the art and discussed in, for example, Ladner et al., WO 88/06630; Fuchs et al. (1991) Biotechnology 9:1369-72; Goward et al. (1993) TIBS 19: 136-40; Charbit et al. (1986) EMBO J 5, 3029-37.; Cull et al. (1992) Proc. Natl. Acad. Sci. U.S.A. 89:1865-9; Cwirla, et al. (1990) Proc. Natl. Acad. Sci. U.S.A. 87, 63 79-82.

An exemplary non-competitive assay would follow an analogous procedure to the one described for the competitive assay, without the addition of one of the components (target molecule or partner molecule). However, as non-competitive formats determine test compound binding to target molecule or partner molecule, the ability of test compound to both target molecule and partner molecule needs to be determined for each candidate. Thus, by way of example, binding of the test compound to immobilized target molecule may be determined by washing away unbound test compound; eluting bound test compound from the support, followed by analysis of the eluate; *e.g.*, by mass spectroscopy, protein determination (Bradford or Lowry assay, or Abs. at 280nm determination.).

Alternatively, the elution step may be eliminated and binding of test compound determined by monitoring changes in the spectroscopic properties of the organic layer at the support surface. Methods for monitoring spectroscopic properties of surfaces include, but are not limited to, absorbance, reflectance, transmittance, birefringence, refractive index, diffraction, surface plasmon resonance, ellipsometry, resonant mirror techniques, grating coupled waveguide techniques and multipolar resonance spectroscopy, all of which are known to those of skill in the art. A labeled test compound may also be used in the assay to eliminate need for an elution step. In this instance, the amount of label associated with them support after washing away unbound material is directly proportional to test compound binding.

A number of well-known robotic systems have been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation Hopkinton, Mass.; Orca, Hewlett Packard, Palo Alto, Calif.), which mimic the manual synthetic operations performed by a chemist. Any of the above devices are suitable for use with the present invention The nature and implementation of modifications to these devices (if any) so that they can operate as discussed herein will be apparent to persons skilled in the relevant art. In addition, numerous combinatorial libraries are themselves commercially available (*see, e.g.,* ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, *etc*.).

### VIII-11 screening converting enzymes:

Test compounds that are converting enzymes may be assayed in a non-competitive format, using co-factors and auxiliary substrates specific for the converting enzyme being assayed. Such co-factors and auxiliary substrates are known to one of skill m the art, given the type of converting enzyme to be investigated.

One exemplary screening procedure for converting enzymes involves first contacting target molecule and/or partner molecule with the converting enzyme in the presence of co-factors and auxiliary substrates necessary to perfom covalent modification of the protein characteristic of the converting enzyme, preferably under physiologic conditions. The modified protein(*s*) is then tested for its ability to bind to its binding partner (*i*.*e.*, binding of target molecule to partner molecule). Binding of the modified protein to its binding partner is then compared to binding of unmodified control pairs to determine if the requisite change in Kₐ noted above has been achieved.

To facilitate the detention of proteins in performing the assay, one or more proteins may be labeled with a detectable label as described above, using techniques well known to those of skill in the art.

### VIII-12 Methods for screens;

The screening embodiments described above are suitable for high through-put determination of test compounds suitable for further investigation.

Alternatively, the test compounds under investigation may be added to proliferating cells and proliferation of the treated cells monitored relative to proliferation of a control population not supplemented with the test compound. Cell lines suitable for screening test compounds will be obvious to one of skill in the art provided with the teachings presented herein. For *in vivo* testing, the test compound may be administered to an accepted animal model.

### VIII-13 Formulating medicaments from identified test compounds:

Accordingly, the present invention includes medicaments and methods useful in preventing or treating cancer, particularly a lung cancer such as non-small cell lung cancer and/or small cell lung cancer. These medicaments and methods comprise at least one test compound of the present invention identified as disruptive to the target molecule/partner molecule interaction or target molecule mediated activity in an amount effective to achieve attenuation or arrest of pathologic cell proliferation. More specifically, a therapeutically effective amount means an amount effective to prevent the development of or to alleviate existing symptoms of the subject being treated.

Individuals to be treated with methods of the present invention may be any individual afflicted with lung cancer, including, *e.g.*, non-small cell lung cancer and small cell lung cancer. Such an individual can be, for example, a vertebrate such as a mammal, including a human, dog, cat, horse, cow, or goat; or any other animal, particularly a commercially important animal or a domesticated animal.

### VIII-14 Determining therapeutic dose range;

Determination of an effective dose range for the medicaments of the present invention is well within the capability of those skilled in the art, especially in light of the detailed disclosure provided herein. The therapeutically effective dose for a test compound can be estimated initially from cell culture assays and/or animal models. For example, a dose can be formulated in animal models to achieve a circulating concentration range that includes the IC₅₀ (the dose where 50% of the cells show the desired effects) as determined in cell culture. Toxicity and therapeutic efficacy of test compounds also can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index (*i.e.*, the ratio between LD₅₀ and ED₅₀). Compounds which exhibit high therapeutic indices may be fused. The data obtained from these cell culture assays and animal studies may be used in formulating a dosage range for use in humans. The dosage of such compounds may lie within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition *See, e.g.,* Fingl *et al.,* (1975), in "The Pharmacological Basis of Therapeutics", Ch. 1 pl. Dosage amount and interval may be adjusted individually to provide plasma levels of the active test compound sufficient to maintain the desired effects.

### VIII-15 Pharmaceutically acceptable excipients;

Medicaments administered to a mammal (*e.g.*, a human) may contain a pharmaceutically-acceptable excipient, or carrier. Suitable excipients and their formulations are described in Remington's Pharmaceutical Sciences, 16th ed., (1980), Mack Publishing Co ., edited by Oslo *et al* For aqueous preparations an appropriate amount of a pharmaceutically-acceptable salt is typically used in the formulations to render the formulation isotonic. Examples of the pharmaceutically-acceptable isotonic excipients include liquids such as saline, Ringer's solution, Hanks's solution, and dextrose solution. Isotonic excipients are particularly important for injectable formulations.

For transmucosal administration, penetrants appropriate to the barrier to be permeated are used in the formulations. Such penetrants are generally known is the art.

Excipients may be used to maintain the correct pH of the formulation. For optimal shelf life, the pH of solutions containing test compounds preferably ranges from about 5 to about 8, and more preferably from about 7 to about 7.5. The formulation may also comprise a lyophilized powder or other optional excipients suitable to the present Invention including sustained release preparations such as semi-permeable matrices of solid hydrophobic polymers, which matrices are in the forum of shaped articles, *e.g.*, films, liposomes or microparticles. It will be apparent to those persons skilled in the art that certain excipients may be more preferable depending upon, for instance, the route of administration, the concentration of test compound being administered, or whether the treatment uses a medicament that includes a protein, a nucleic acid encoding the test compound, or a cell capable of secreting a test compound as the active ingredient.

The pharmaceutical compositions of the present invention may be manufactured in a manner that is itself known, *e.g.*, by means of conventional mixing, dissolving, granulating, dragee-making, levigating, emulsifying, encapsulating, entrapping or lyophilizing processes. Proper formulation is dependent upon the route of administration chosen.

For oral administration, carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by formulating a test compound with a solid dispersible excipient, optionally grinding a resulting mixture and processing the mixture of granules after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Many of the compounds of the present invention may be optionally provided as salts with pharmaceutically compatible counterions. Pharmaceutically compatible salts may be formed with many acids, including but not limited to hydrochloric, sulfuric, acetic, lactic, tartaric, malic, succinic, *etc*, depending upon the application. Salts tend to be more soluble in aqueous or other protonic solvents that are the corresponding free base forms,

In addition to acceptable excipients, formulations of the present invention may include, therapeutic agents other than identified test compounds. For example formulations may include anti-imlammatory agents, pain killers, chemotherapeutics, mucolytics (*e.*g. n-acetylcysteine) and the like. In addition to including other therapeutic agents in the medicament itself, the medicaments of the present invention may also be administered sequentially or concurrently with the one or more other pharmacologic agents. The amounts of medicament and pharmacologic agent depend, for example, on what type of pharmacologic agent(s) is are used, the disease being treated, and the scheduling and routes of administration.

Following administration of a medicament of the invention, the mammal's physiological condition, can be monitored in various ways well known to the skilled practitioner.

### VIII-16 Gene Therapy;

Protein and peptide test compounds identified as disruptors of the target molecule/partner molecule association, or target molecule mediated activity may be therapeutically delivered using gene therapy to patients suffering from lung cancer, *e.g*., non-small cell lung cancer and/or small cell lung cancer. Exemplary test compounds amenable to gene therapy techniques includes, but are not limited to, converting enzymes as well as peptides that directly alter the target molecule/partner molecule association by steric or allosteric interference.

In some aspects, gene therapy embodiments include a nucleic acid sequence encoding a suitable identified test compound of the invention. In some embodiments, the nucleic acid sequence includes those regulatory elements necessary for expression of the test compound in a target cell. The nucleic acid may be equipped to stably insert into the genome of the target cell (*see e.g.,* Thomas KR. and Capecchi MR. (1987) Cell 51:503-12 for a description of homologous recombination cassettes vectors).

Delivery of the nucleic acids into a patient may be either direct in which case the patient is directly exposed to the nucleic acid or nucleic acid-carrying vectors, or indirect, in which case, cells are first transformed with the nucleic acids *in vitro,* then transplanted into the patient These two approaches are known, respectively, as *in vivo* or *ex vivo* gene therapy.

For general reviews of the methods of gene therapy, *see* Goldspiel et al., 1993, Clinical Pharmacy 12:488-505; Wu and Wu, 1991, Biotherapy 3:87-95; Tolstoshev, 1993, Ann. Rev. Pharmacol. Toxicol. 33:573-96; Mulligan, 1993, Science 260:926-32; and Morgan and Anderson, 1993, Ann. Rev. Biochem. 62:191-217). Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression A Laboratory Manual, Stockton Press, NY.

### IX. Screening and Treatment Kits:

The present invention also provides an article of manufacture or kit containing materials for screening for a compound useful in treating or preventing lung cancer. Such an article of manufacture may comprise one or more labeled containers of materials described herein along with instructions for use. Suitable containers include, for example, bottles, vials, and test tubes. The containers may be formed from a variety of materials such as glass or plastic.

In one embodiment, the screening kit comprises: (a) a first polypeptide comprising an partner molecule-binding domain of an target molecule polypeptide; (b) a second polypeptide comprising an target molecule-binding domain of an partner molecule polypeptide, and (c) means (*e.g*., a reagent) to detect the interaction between, the first and second polypeptide.

In some embodiments, the first polypeptide, *i.e*., the polypeptide comprising the partner molecule-binding domain, comprises an target molecules polypeptide. Similarly, in other embodiments, the second polypeptide, *i.e*., the polypeptide comprising the target molecule-binding domain, comprises an partner molecule polypeptide.

In another embodiment, the screening kit may comprise: (a) a cell expressing a target molecule polypeptide or a functional equivalent thereof; and (b) means (*e.g*., a reagent) to detect the acetylation level of the histone H4 or phosphorylation level of the WRNIP1 protein.

The present invention further provides articles of manufacture and kits containing materials useful for treating the pathological conditions described herein are provided. Such an article of manufacture may comprise a container of a medicament as described herein with a label. As noted above, suitable containers include, for example, bottles, vials, and test tubes. The containers may be formed from a variety of materials such as glass or plastic. In the context of the present invention, the container holds a composition having an active agent which is effective for treating a lung cancer. The active agent in the composition may be an identified test compound (*e.g*., antibody, small molecule, *etc*.) capable of disrupting the target molecule/partner molecule association *in vivo*. The label on the container may indicate that the composition is used for treating one or more conditions characterized by abnormal cell proliferation. The label may also indicate directions for administration and monitoring techniques, such as those described herein.

In addition to the container described above, a treatment kit of the present invention may optionally comprise a second container housing a pharmaceutically-acceptable diluent It may further include other materials desirable from a commercial and user standpoint including other buffer, diluents, filter, needles, syringes, and package inserts with instructions for use.

The compositions may, if desired, be presented in a pack or dispenser device which may contain one or more unit dosage forms containing the active ingredient The pack may, for examples, comprise metal or plastic foil, such as a blister pack. The pack or dispenser device may be accompanied by instructions for administration. Compositions comprising a compound of the invention formulated in a compatible Pharmaceutical carrier may also be prepared, placed in an appropriate container, and labeled for treatment, of an indicated condition.

### EXAMPLE

### Materials and Methods

### Lung-cancer Cell Lines and Tissue Samples.

The human lung-cancer ceil lines used in this example were as follows: non small-cell lung cancer (NSCLCs) A427, A549, LC74, LC319, PC-3, PC-9, PC-14, NCI-H1666, NCI-H1781, NCI-H596, NCI-H647, NCI-H1373, NCI-1703, SW1573, NCI-H1373, EBC-1, RERF-LC-AI, SK-LU-1, SK-MES-1, LU61, NCI-H226, NCI-H520, LX-1 and NCI-H2170; and small-cell lung cancers (NSCLC) DMS-114, DMS-273, SBC-3, and SBC-5. All cells were grown in monolayer in appropriate medium supplemented with 10% fetal calf serum (FCS) and were maintained at 37°C in atmospheres of humidified air with 5% CO₂. Human small airway epithelial cells (SAEC) were grown in optimized medium (SAGM) purchased from Cambrex Bio Science Inc. (Walkersville, MD). Primary lung-cancer tissue samples had been obtained with informed consent as described previously (Kikuchi T, et al. Oncogene 22: 2192-205,2003.).

For KIF4A_{:} a total of 357 NSCLCs and adjacent normal lung-tissue samples for immunostaining on tissue microarray were also obtained from patients who underwent surgery at Saitama Cancer Center (Saitama, Japan). 29 SCLC samples obtained from Hiroshima University (Hiroshima, Japan) and Saitama Cancer Center were also used in the example.

For MAPJD, primary NSCLC samples, of which 22 were classified as adenocarcinomas (ADCs), 14 as squamous-cell carcinomas (SCCs),and once as adenosquamous carcinomas (ASC), had been obtained earlier with informed consent from 37 patients. A total of 300 formalin-fixed primary NSCLCs and adjacent normal lung tissue samples used for immunostaining on tissue microarrays had been obtained with informed consent from patients undergoing surgery This study and the use of all clinical materials mentioned were approved by individual institutional Ethical Committees.

For NPTX1, a total of 374 formalin-fixed samples of primary NSCLCs including 238 ADCs, 95 SCCs, 28 LCCs, 13 ASCs, and 3 LCNEC, and adjacent normal lung tissue, had been obtained earlier along with clinicopathological data from patients who had undergone' surgery at Saitama Cancer Center (Saitama, Japan). 13 SCLCs were obtained from individuals who underwent autopsy. The histological classification of the tumor specimens was based on WHO criteria (Travis WD). Surgical samples were selected for the study on the basis of the following criteria: (1) patients were newly diagnosed and previously untreated; (2) their tumors were pathologically diagnosed as lung cancers; (3) survived for > 3 months after surgery; (4) did not die of causes other than lung cancer within 5 years after surgery; and (5) were followed for > 3 years after surgery (for patients who remained alive).

For FGFROP1, A total of 419 formalin-fixed samples of primary NSCLCs including 263 ADCs, 115 SCCs, 28 LCCs, 13 adenosquamous carcinomas (ASCs) and adjacent normal lung tissues, had been obtained earlier along with clinicopathological data from patients undergoing surgery. NSCLC specimen and five tissues (heart, liver, lung, kidney, and testis) from post-mortem materials (2 individuals with NSCLC) bad been obtained earlier with informed consent The histological classification of the tumor specimens was performed by the WHO criteria (Travis WD, et al. Berlin: Springer, 1999.).

Serum samples were obtained with informed consent from 90 healthy individuals as controls (79 males and 11 females; median age 48.39 ± 7.34 SD, range 41.1-55.73). The healthy individuals showed no abnormalities in complete blood cell counts, C-reactive proteins (CRP), erythrocyte sedimentation rates, liver function tests, renal function tests, urinalyses, fecal examinations, chest X-rays, or electrocardiograms. Serum samples were also obtained with informed consent from 152 lung-cancer patients (116 males and 36 females; median age 64.15 ± 10.98 SD, range 53.17-75.13). Samples were selected for the study on the basis of the following criteria: (1) patients were newly diagnosed and previously untreated and (2) their tumors were pathologically diagnosed as lung cancers (stages I - IV). These 152 cases included 70 ADCs, 30 SCCs, and 52 SCLCs. Clinicopathological records were fully documented. Serum was obtained at the time of diagnosis and stored at -80°C. Disease staging in all 152 cases was supported by a computed tomography (CT) scan of the chest and abdomen, bone scintigraphy, and magnetic resonance imaging (MRI) of the head.

This study and the use of all clinical materials were approved by individual institutional ethical committees.

### Semi-Quantitative RL-PCR.

Total RNA was extracted from cultured cells using Trizol reagent (Life Technologies) According to the manufacturer's protocol. Extracted RNAs were treated with DNase I (Nippon Gene) and reversely-transcribed using oligo (dT) primer and Superscript II. Semi-quantitative RT-PCR experiments were carried out with the following synthesized KIF4A-Specific primers, ZNF549-specific primers, ZNF553-specific primers or with beta-actin (ACTB)-specific primers as an internal control:
KIF4A, 5'-CAAAAACCAGCTTCITCTCTGG-3' (SEQ ID NO: 1) and
   5'-CAGGAAAGATCACAACCTCATTC-3' (SEQ ID NO: 2);
2NF549, 5'-GCCCACiTTA-kTGOGTTTTGA-5' (SEQ ID NO: 3) and
   5'-CACGCCTGGCTAATTTTTGT-3' (SEQ ID NO: 4);
ZNT553, 5'-GAGGGAGAAGGAAGGGAAGA-3' (SEQ ID NO: 5) and
   5'-AATGCCTACTGTGTGCTAGGC-3' (SEQ ID NO: 6);
MAPJD, 5'-AGGAGAAGTTGGAGGTGGAAA-3' (SEQ ID NO: 7) and
   5'-CAGATGAAAGATCCAAATTCCAA-3' (SEQ ID NO: 8);
SBNO1 5'-CTGACAGTGCATGTCTTTGG-3' (SEQ ID NO: 9) and
   5'-TTCTGCAGCACACATTAGGA-3' (SEQ ID NO: 10);
TGFBRAP1 (transforming growth factor, beta receptor associated protein 1),
   5'-AGAGTATCACACCCACTTAGCTG-3' (SEQ ID NO: 11) and
   5'-GACAGGTGAACTCTTGTATGTTTCTG-3'(SEQ ID NO. 12);
R1OK1 (RIO kinase 1 (yeast)),
   5'-GAAGACAGCCAAGACGAAAA-3 (SEQ ID NO: 13)' and
   5'-TCCTCTGTCAACACCAGACA-3' (SEQ ID NO: 14);
RASGEF1A (RasGEF domain family, member 1A),
   5'-TTTCCCATGTCTGACTTCGT-3' (SEQ ID NO: 15) and
5'-CAATGTCTTCAGGCTCTTCC-3'(S EQ ID NO: 16);
   FGFR1OP, 5'-CTGCTGGTACGTGTGATCTTTG-3' (SEQ ID NO: 17) and
5'-ACCTTAATGGTCTAACAAACCTTCC-3' (SEQ ID NO: 18);
   NPTX1, 5'-TAACCTTGATAGAAGAACCTTGG-3' (SEQ ID NO: 19) and
5'-GCAAATGAGACAAAATTGGGAC-3' (SEQ ID NO: 20);
   ACTB, 5'-GAGGTGATAGCATTGCTTTCG-3' (SEQ ID NO: 21) and
5'-CAAGTCAGTGTACAGGTAAGC-3' (SEQ ID NO: 22).

PCR reactions were optimized for the number of cycles to ensure product intensity within the logarithmic phase of amplification.

### Detection of fusion transcripts by RT-PCK.

FGFR1-FGFR1OP fusions transcripts were detected, as previously reported (Popovici C, et al. Blood 1999;93:13 81-9.), for the 23 human lung-cancer cell lines. The wild type FGFR1OP and FGFR1 were amplified using FGFR1OP-specific sense 5'-CATTCTCCACCAAAGTCACCA-3' (SEQ ID NO: 23) and FGFR1OP-specific antisense 5'-CCCGCTTGTCTTCTTCT-3' (SEQ ID NO: 24) primers (PCR product size is 101 bp.),

FGFR1-specific sense 5'-ATCATCTATTGCACAGGGGCC-3' (SEQ ID NO: 25) and FGFR1-specific antisense 5'-CATACTCAGAGACCCCTGCTAGC-3' (SEQ ID NO: 26) primers (PCR product of 258 bp), respectively. FGFR1OP-FGFR1 fusion transcript was amplified with FGFR1OP-specific sense and FGFR1-specific antisense primers (PCR product size, 162 bp). FGFR1-FGFR1OP fusion transcript was amplified with FGFR1-specific sense and FGFR1OP-specific antisense primers (PCR product size, 197 bp).

### Northern-blot Analysis.

Human multiple-tissue blots (23 normal tissues including heart, brain, placenta, lung, liver, skeletal muscle, kidney, pancreas spleen, thymus, prostate, testis, ovary, small intestine, colon, peripheral blood leukocyte, stomach, thyroid, spinal cord, lymph node, trachea, adrenal gland and bone marrow; BD Biosciences Clontech) were hybridized with a ³²P-labeled PCR product of *KlF4A, MAPJD, NPTX1 and FGFPOP1.* The cDNA probe of KIF4A or NTPTX1 was prepared by RT-PCR using the primers described above and the partial-length cDNA of MAPJD or FGFR1OP was prepared by RT-PCR using primers
5'-CTGGAAACAAGGCAGTAGTGATT-3' (SEQ ID NO: 27) and
5'-GTACACTGAAGCCTGAAGGTGAT-3' (SEQ ID NO: 28) for MAPJD;
5'-TAATAGTACCAGCCATCGCTCAG-3' (SEQ ID NO: 94) and
5'-ATCCTACGGCTTTATTGACACCT-3' (SEQ ID NO: 95) for FGFR1OP.

Pre-hybridization, hybridization, and washing were performed according to the supplier's recommendations. The blots were autoradiographed at room temperature for 30 hours with intensifying BAS screens (BIO-RAD).

### Preparation or antibodies.

For detection of MAPJD, rabbit antibodies specific for MAPJD were raised by immunizing rabbits with recombinant human MAPJD protein, and purified using standard protocols.

For detection of FGFR1OP, rabbit antibodies specific for extracellular portion of FGFR1OP were raised by immunizing rabbits with 6-histidine fused human FGFR1OP protein (codons 7-173 (SEQ ID NO: 98); GenBank Accession No. NM_007045), and purified with standard protocols using affinity columns (Affi-gel 10; Bio-Rad Laboratories, Hercules, CA, USA) conjugates with the 6-hisfidine fused protein. On western blots the present inventors confirmed that the antibody was specific to FGFR10P, using lysates from NSCLC, tissues and cell lines as well as normal lung tissues.

For detection of NPTX1, rabbit antibodies specific for NPTX1 (BB017) were raised by immunizing rabbits with GST-fused human NPTX1 protein (codons 20-145 (SEQ ID NO: 99) and 297-430 (SEQ ID NO: 100)), and purified with standard protocols using a standard protocol. Mouse monoclonal antibodies specific for human NPTX1 (mAb-75-1) were raised by DNA immunization methods using gene-gun to transfect plasmid expressing human NPTX1 protein to mice skin cells. On western blots, it was confirmed that these antibodies were specific to NPTX1, using lysates from NSCLC tissues as well as normal lung tissues.

### Western-blotting.

Cells were lysed in lysis buffer; 50 mM Tris-HCl (pH 8.0), 150 mM NaCl, 0.5% NP-40, 0.5% deoxycholate-Na, 0.1% SDS, plus protease inhibitor (Protease Inhibitor Cocktail Set III; Calbiochem Darmstadt). The present inventors used an ECL western-blotting analysis system (GE Healthcare Bio-sciences), as previously described (Kato T, et al. Cancer Res. 2005;65(13):5638-46.; Furukawa C, et al. Cancer Res. 2005;65(16):7102-10.).

For detection of KIF4A, a commercially available goat polyclonal anti-KIF4A antibody was purchased from abcam Inc. (Catalog No. ab3815) and was proved to be Specific to human KIF4A, by western-blot analysis using lysates of lung-cancer cell lines *(see* Fig. 1B).

For detection of MAPJD, blots were incubated with a rabbit anti-MAPJD polygonal antibody, anti-myc antibody (9E10; Santa Cruz Biotechnology, Inc., Santa Cruz, CA) and anti-Flag antibody (M2; Santa Cruz Biotechnology, Inc.). A sheep anti-mouse IgG-horseradish peroxidase (HRP) antibody (GE Healthcare Bio-sciences) or a goat anti-rabbit IgG-HRP antibody (GE Healthcare Bio-sciences) was served as the secondary antibodies for the experiments.

For detection of NPTX1, blots were incubated incubated with a mouse monoclonal anti-human NPTX1 antibody (mAb-75-1). A goat anti-mouse IgG-HRP antibody (Amersham Biosciences) was served as the secondary antibodies for these experiments.

For detection, blots were incubated with a rabbit polyclonal anfi-FGFR1OP antibody, a rabbit polygonal WRNIP1 antibody (abcam), a rabbit polyclonal ABL 1 antibody (Santa Cruz), a mouse Monoclonal beta-actin (ACTB) antibody (SIGMA) or a mouse monoclonal anti-c-Myc antibody (Santa Cruz). Antigen-antibody complexes were detected using secondary antibodies conjugated to horseradish peroxidase (GE Healthcare Bio-sciences). Protein bands were visualized by ECL Western Blotting Detection Reagents (GE Healthcare Bio-sciences), as previously described (Kato *T, et al. supra*.; Suzuki C, *et al. supra.).*

### Immunocytochemistry.

Cultured cells were washed twice with PBS(-), fixed in 4% formaldehyde solution for 30 min at 37°C, and rendered permeable by treatment for 3 minutes with PBS(-) containing 0.1% Triton X-100. Cells were covered with CAS-BLOCK (ZYMED) for 7 minutes to block non-specific binding prior to the primary antibody reaction. Then the cells were incubated with polyclonal antibody to human KIF4A protein (abcam), a rabbit antibody to human MAPJD, a mouse monoclonal anti-human NPTX1 antibody (mAb-75-1), a rabbit polyclonal anti-FGFR1OP antibody, a rabbit polyclonal anti-WRNIP1 (abcam), a rabbit polyclonal ABL1 antibody (Santa Cruz Biotechnology, Inc.) or rabbit polyclonal anti-alpha-tubulin (TUBA) (SIGMA).

The immune complexes were stained with a donkey anti-goat secondary antibody conjugated to Alexa488 (Molecular Probes), a goat anti-rabbit secondary antibody conjugated to FITC (Cappel, Durham, NC) or rhodamine (Cappel), an anti-mouse secondary antibody conjugated to Alexa Fluor 488 (Molecular Probes) or an anti-rabbit secondary antibody conjugated to Alexa Fluor 594 (Molecular Probes), respectively, and viewed with a laser confocal microscope (TCS SP2 AOBS: Leica Microsystems). DNA was stained with 4',6-diamidino-2-phenylindole (DAPI). Images were viewed and assessed using a confocal microscope at wavelengths of 488, 594 nm (TCS SP2 AOBS: Leica Microsystems).

### Immunohistochemistry and Tissue-microarray Analysis.

To investigate the significance of KIF4A, MAPJD, NPTX1 or FGFROP1 over-expression in clinical lung cancers, the present inventors stained tissue sections using ENVISION+ Kit/HRP (DakoCytomation, Glostrup, Denmark). Anti-KIF4A antibody (abcam), rabbit antibody to human MAPJD, mouse monoclonal anti-human NPTX1 antibody (mAb-75-1) or a rabbit polyclonal anti-human FGFR1OP antibody was added after blocking of endogenous peroxidase and proteins, and teach section was incubated with HRP-labeled anti-goat IgG, HRP-labelad anti-rabbit IgG or HRP-labeled anti-mouse IgG as the secondary antibody. Substrate-chromogen was added and the specimens were counterstained with hematoxylin. Tumor-tissue microarrays were constructed as published previously, using formalin-fixed NSCLCs (Chin SF, et al. Mol Pathol 2003; 56(5): 275-9., Callagy G, et al. Diagn. Mol. Pathol 2003; 12(1):27-34., J Pathol 2005;205:388-96.). Tissue areas for sampling were selected based on visual alignment with the corresponding HE-stained sections on slides. Three, four, or five tissue cores (diameter 0.6 mm; height 3 - 4 mm) taken from donor-tumor blocks were placed into recipient paraffin blocks using a tissue microarrayer (Beecher Instruments). A core of normal tissue was punched from each case. Five-µm sections of the resulting microarray block were used for immunohistochemical analysis.

The staining pattern of KIF4A was assessed semi-quantitatively by three independent investigators without prior knowledge of the clinicopathological data, each of whom recorded staining intensity as absent (scored as 0) or positive (1+). Lung-cancers were scored as positive only if all reviewers defined them as such.

The staining pattern of MAPJD was assessed semi-quantitatively as absent or positive by three independent investigators without prior knowledge of the clinical follow-up data. Cases with fewer than 20% of nuclear MAPJD-stained tumor cells were judged as MAPJD-negative. Cases were accepted as positive only if reviewers independently defined them as such.

The staining pattern of NPTX1 was assessed semi-quantitatively by three independent investigators without prior knowledge of clinicopathological data. The intensity of NPTX1 staining was evaluated using following criteria: absent (score 0), weakly positive (score 1), or strongly positive (score 2) without prior knowledge of clinicopathological data.

The staining pattern of FGFR1OP was assessed semi-quantitatively by three independent investigators without prior knowledge of clinicopathological data. The intensity of FGFR1OP staining was evaluated using following criteria: strong positive (2+), dark brown staining in more than 50% of tumor cells completely obscuring cytoplasm; weak positive (1+), any lesser degree of brown staining appreciable in tumor cell cytoplasm; absent (scored as 0), no appreciable staining in tumor cells. Cases were accepted only as strongly positive if reviewers independently defined them as such.

### Statistical analysis.

Statistical analyses were performed using the StatView statistical program (SaS, Gary, NC, USA). The present inventors used contingency tables to analyze the relationship between KIF4A, NPTX1 or FGFROP1 expression and clinicopathological variables in NSCLC patients. Tumor-specific survival curves were calculated from the date of surgery to the time of death related to NSCLC, or to the last follow-up observation. Kaplan-Meier curves were calculated for each relevant variable and for KTF4A, NPTX1 or FGFR1OP expression; differences in survival times among patient subgroups were analyzed using the log-rank test Univariate and multivariate analyses were performed with the Cox's proportional-hazard regression model to determine associations between clinicopathological variables and cancer-related mortality. First, the present inventors analyzed associations between death and possible prognostic factors including age, gender, histological type, pT-classification, and pN-classification, taking into consideration one factor at a time. Second, multivariate Cox's analysis was applied on backward (stepwise) procedures that always forced KIF4A, NPTX1 or FGFROP1 expression into the model, along with any and all variables that satisfied an entry level of a *P* value less than 0.05. As the model continued to add factors, independent factors did not exceed an exit level of *P <* 0.05.

### ELISA.

ELISA plates (Nunc Maxisorp Bioscience, Inc., Naperville, IL) were incubated with 100 µl/well of the mouse anti-NPTX1 (mAb-75-1) at 4 µg/ml for 2 hours at room temperature and three washes were done in 1% bovine serum albumin (BSA) in PBS with 0.05% Tween (PBST) at room temperature. Following blocking with 5% BSA in PBS for 2 hour at 4°C over night and the palates were washed three times. That plates were further incubated overnight at 4°C with 100 µl/well of the diluted sera (5-fold dilution) by in 1% BSA in PBS (Reagent Diluent). The plates were then incubated with biotin-coojugated rabbit polyclonal anti-NPTX1 antibody (BB017), which was biotinylated by 0.01 µg/ml of biotin labeling kit-NH2 (Dojindo Labolatories, Kumamoto, Japan), for 2 hours at room temperature, followed by three washes in PBST. The wells were developed using. Streptavidin-Horseradish Peroxidase (SAv-HRP) Conjugate at room temperature for 20 minutes. After three times wash 100 µl per well color solution was added to the wells and allowed to room temperature for 20 minutes. The reaction was stopped by adding 100 µl of 2 N sulfuric acid. Color intensity was determined by a photometer at a wavelength of 450 nm, with a reference wavelength of 570 nm.

A standard NPTX1 proteins purified by pleural effusion from a SCLC patient where added onto each plate. The index value was defined by the following formula: index value = [(ODsample - ODNC) / (ODPC - ODNC)] x 100. The negative controls revealed the nonspecific background noise of the system, which was subtracted from all values on the plate. Levels of proGRP or CEA in serum were measured by ELISA with a commercially available enzyme test kit, according to the same protocol as above. Differences in the levels of NPTX1 and proGRP/CEA between tumor groups and a healthy control group were analyzed by Mann-Whitney U tests. The levels of NPTX1 and proGRP/CEA were additionally evaluated by receiver-operating characteristic curve analysis to determine cutoff levels with optimal diagnostic accuracy and likelihood ratios. The correlation coefficients for these two markers were calculated with Pearson's correlation coefficient. Significance was defined as P < 0.05.

### DNA Interference Assay.

The present inventors had previously established a vector-based RNA interference (RNAi) system, psiH1BX3.0 that was designed to synthesize siRNAs in mammalian cells (Suzuki C et al, Cancer Res 2003;63:7038-41., Cancer Res. 2005; 65, 11314-25.; Kato T, et al. Cancer Res. 2005;65(13):5638-46., Furukawa C, et al. Cancer Res. 2005;65(16):7102-10.). 10 µg of siRNA-expression vector was tansfected using 30 µl of Lipofectamine 2000 (Invitrogen) into lung-cancer cell line, (NSCLC cell line) A549 for MAPJD and NPTX1, LC319 for MAPJD and FGFROP1; (SCLC cell line) SBC-5 forKIF4A. NPTX1 and FGFROP1.

The transfected cells were cultured for seven days in the presence of appropriate concentrations of geneticin (G418), and the number of colonies was counted by Giemsa staining, and viability of cells was evaluated by MTT assay at 7 days after the treatment; biefly, cell-counting kit-8 solution (DOJINDO) was added to each dish at a concentration of 1/10 volume, and the plates were incubated at 37°C for additional 2 hours. Absorbance was then measured at 490 nm, and at 630 nm as a reference, with a Microplate Reader 550 (BIO-RAD). To confirm suppression of *KIF4A, MAPJD, NPTX1 or FGFROP1* mRNA expression, semi-quantitative RT-PCR experiments were carried out with the following synthesized *KIF4A*-specific primers, MAPJD-specific primers, NPTX1 specific primers or FGFROP1-specific primers according to the standard protocol.

The target sequences of the synthetic oligonucleotides for RNAi were as follows:
control 1 (Luciferase/LUC: *Photinus pyralis* luciferase gene), 5'-CGTACGCGGAATACTTCGA-3' (SEQ ID NO: 29);
control 2 (Scramble/SCR: *chloroplast Euglena gracilis* gene coding for 5S and 16S rRNAs), 5'-GCGCGCTTTGTAGGATTCG-3' (SEQ ID NO: 30);
(EGFP: enhanced green fluorescent protein (GFP) gene, a mutant of Aequorea victoria GFP), 5'-GAAGCAGCACGACTTCTTC-3' (SEQ ID NO: 31);
siRNA-*KIF4A*-#1, 5'-GGAAGAATTGGTTCTTGAA-3' (SEQ ID NO: 32);
siRNA-*KIF4A*-#2, 5'-GATGTGGCTCAACTCAAAG-3' (SEQ ID NO: 33);
siRNA-MAPJD-1 (si-MAPJD-1) 5'-GCAGCTGCGAAGTGTTGTA-3' (SEQ ID NO: 34);
siRNA-MAPJD-2 (si-MAPJD-2) 5'-GATACGAAAGCAGCTGCGA-3' (SEQ ID NO: 35);
NPTX1 siRNA-2 (NPTX1 si-2), 5'-GGTGAAGAAGAGCCTGCCA-3' (SEQ ID NO: 36);
siRNA-FGFR1OP-1 (si-1), 5'-CCTGAAACTAGCACACTGC-3' (SEQ ID NO: 37);
WRNIP1-si#1, 5'-CUAGGAAGAUGUUCUGUAAUU-3' (SEQ ID NO: 96) (Dermacon Cat No. D-010072-02);
WRNIP1-si#2, 5'-CCACUAGGCUGAUGAAGGAUU-3' (SEQ ID NO: 97) (Darmacon Cat No. D-010072-03).

To validate RNAi system, individual control siRNAs were tested by semi-quantitative RT-PCR to confirm the decrease in expression of the corresponding target genes that had been transiently transfected to COS-7 cells. Down-regulation of *FGFR1OP* expression by functional siRNA, but not by controls, was also confirmed in the cell lines used for this assay.

### Flow Cytometry.

Cells were plated at densities of 5x10⁵ cells/100-mm dish, transfected with siRNA-expression vectors, and cultured in the presence of appropriate concentrations of genetic. Cells were trypsinized five days after transfection, collected in PBS, and fixed in 70% cold ethanol for 30 min. After treatment with 100 µg/ml RNase (Sigma-Aldrich Co., St. Louis, MO), the cells were stained with 50 µg/ml propidium iodide (Sigma-Aldrich Co.) in PBS. Flow cytometry was performed on a Becton Dickinson FACScan and analyzed by ModFit software (Verity Software House, Inc., Topsham, ME). The cells selected from at least 20,000 ungated cells were analyzed for DNA content Cells were also prepared using Annexin V-FITC Apoptosis Detection Kit (Bio Vision, Inc., Mountain View, CA) for Annexin V-binding assay, according to the supplier's protocol.

### KIF4A, MAPJD or FGFR1OP-expressing MH3 T3 Transfectants.

KIF4A-expressing stable transfectants and MAPJD-expressing stable transfectants were established according to a standard protocol. The entire coding region of KIF4A, MAPJD or FGFR1OP was amplified by RT-PCR. The products were cloned into appropriate sites of a pcDNA3.1-myc/His A(+) vector (Invitrogen) that contained c-myc-His-epitope sequences (LDEESILKQE-HHHHHH) at the C-terminal of the KIF4A protein, the NIAPJD protein or FGF1OP protein. Using FuGENE 6 Transfection Reagent (Roche Diagnostics, Basel, Switzerland) according to the manufacturer's instructions, the present investors transfected COS-7 cells and/or NIH3T3 cells, which do not express endogenous KIF4A, MAPJD or FGF1OP, with plasmids expressing either MAPJD (pcDNA3.1-MAPJD-myc/His), KIF4A (pcDNA3.1-KIF4A-myc/His), FGF1OP (pCDNA3.1/myc-His-FGFR1OP) or mock plasmids (pcDNA3.1). Transfected cells were cultured in DMEM or RPMI containing 10% FCS and geneticin (0.4 mg/ml) for 14 days; then 50 individual colonies were trypsinized and screened for stable transfectants by a limiting-dilution assay. Expression of MAPJD, KIF4A or FGFR1OP was determined in each clone by RT-PCR, western blotting, and immunostaining.

### Cell-growth Assay.

NIH3T3 or COS-7 transfectants that stably expressed MAPJD, KIF4A or mock were seeded onto 6-well plates (5x10⁴ cells/well), and maintained in appropriate medium containing 10% PCS and 0.4 mg/ml geneticin for 24, 48, 72, and 96 hours. At each time point, cell proliferation was evaluated by the MTT assay. All experiments were done in triplicate. Absorbance was then measured at 490 nm, and at 630 nm as a reference, with a Microplate Reader 550 (BIO-RAD).

### Matrigel Invasion Assay.

To achieve FLAG-tagged KIF4A, the present inventors cloned the entire coding sequence into the appropriate site of p3xFLAG-CMV-10 plasmid vector (SIGMA). COS-7 and NIH3T3 cells transfected either with plasmids expressing KIF4A or with mock plasmids were grown to near confluence in DMEM containing 10% FCS. The cells were harvested by trypsinization, washed in DMEM without addition of serum or proteinase inhibitor, and suspended in DMEM at 5x10⁵ cells/ml. Before preparing the cell suspension, the dried layer of Matrigel matrix (Becton Dickinson Labware) was rehydrated with DMEM for 2. hours at room temperature. DMEM (0.75 ml) containing 10% FCS was added to each lower chamber in 24-well Matrigel invasion chambers, and 0.5 ml (2.5x10⁵ cells) of cell suspension were added to each insert of the upper chamber. The plates of inserts were incubated for 22 hours at 37°C. After incubation, the chambers were processed; cells invading through the Matrigel were fixed and stained by Giemsa as directed by the supplier (Becton Dickinson Labware).'

COS-7 cells transfected either with plasmids expressing FGFR1OP (pCDNA3.1/myc-His-FGFR1OP) or with mock plasmids were grown to near confluence in DMEM containing 10% FCS. The cells were harvested by trypsinization, washed in DMEM without addition of serum or proteinase inhibitor, and suspended in DMEM at concentration of 1x10⁵ cells/ml. Before preparing the cell suspension, the dried layer of Matrigel matrix (Becton Dickinson Labware) was dehydrated with DMEM for 2 hours at room temperature. DMEM (0.75 ml) containing 10% FCS was added to each lower chamber in 24-well Matrigel invasion chambers, and 0.5 ml (5 x 10⁴ cells) of cell suspension was added to each insert of the upper chamber. The plates of inserts were incubated for 22 hours at 37°C. After incubation the chambers were processed; cells invading through the Matrigel were fixed and stained by Giemsa as directed by the supplier (Becton Dickinson Labware).

### Identification of KIF4A-associated Proteins.

Cell extracts from SCLC cell line DMS273 were precleared by incubation at 4°C for 1 hour with 40 µl of protein G-agarose beads, in final volumes of 2 ml of inmunoprecipitation buffer (0-5% NP40, 50 mM Tris-HCl, 150 mM NaCl) in the presence of proteinase inhibitor. After centrifugation at 10,000 rpm for 1 minute at 4°C, the supernatants were incubated at 4°C with a goat polyclonal and-KIF4A antibody (abcam) and normal goat IgG (R&D) for 3 hours, respectively. Then the supernatants were incubated at 4°C with protein G-agarose beads for 1 hour. After the beads were collected from each sample by centrifugation at 5,000 rpm for 2 minutes and washed six times With 1 ml of immunoprecipitation buffer, they were resuspended in 30 µl of Laemmli sample buffer and boiled for 5 minutes before the proteins were loaded on 7.5% SDS-PAGE gels (Bio-Rad). After electrophoresis, the gels were stained with silver. Protein bands found specifically in extracts treated with anti-KIF4A antibody were excised to serve for analysis by matrix-assisted laser desorption/ionization time of flight mass spectrometry (MALDI-TOF-MS; AXIMA-CFR plus, SHIMADZU BIOTECH, Kyoto, Japan).

### Identification of Downstream Genes of MAPJD by cDNA Microarray.

LC319 cells were transfected with either siRNA against MAPJD (si-MAPJD-2) or Luciferase (control siRNA). mRNAs were extracted 12, 18, and 24 hours after the transfection, labeled with Cy5 or Cy3 dye, and subjected to co-hybridization onto cDNA microarray slides containing 23,040 genes. After normalization of the data, genes with signals higher than the cut-off value were analyzed further. Genes whose intensity was significantly decreased in accordance with the reduction of MAPJD expression, were initially selected using SOM cluster analysis (Kohonen T. Proceedings of the IEEE 78, 1464-80 (1990).). Validation of candidate downstream genes of MAPJD was performed with semi-quantitative RT-PCR experiments of the same mRNAs from LC319 cells used for microarray hybridization, with gene-specific primers described above.

### Reporter Gene Assay.

The fragment of each downstream gene was amplified by PCR using the following
primers: 5'-CGACGCGTCGCTCTA-ACCATTCATCAGCTC-3' (SEQ ID NO: 38) and 5'-CCGCTCGAGCGGACTAATTCCACTCTCAC-3' (SEQ ID NO: 39) for SBNO1,
5'-CGACGCGTCGAGACATTCTGACCATAGCACC-3' (SEQ ID NO: 40) and
5'-CCGCTCGAGCGGATCATGTCTACTGGCTGATC-3' (SEQ ID NO: 41) for TGFBRAP1,
5'-CGACGCGTCGTTCCTACAATGTCTTCAGTC-3' (SEQ ID NO: 42) and
5'-CCGCTCGAGCGGTTCAGAAGCCAACAGTGGC-3' (SEQ ID NO: 43) for RIOKI,
5'-CGACGCGTCGCAAGACCTTCACCATGTGG-3' (SEQ ID NO: 44) and
5'-CCGCTCGAGCGGAGACAACGGACGTCTGCG-3' (SEQ ID NO: 45) for RASGEF1A, and cloned into the pGL3 basic vector. Luciferase assays were performed using a Dual-Luciferase Reporter Assay System according to the manufacture's instructions (Promega, Madison, WI).

### Immunoprecipitation Assays.

Cell extracts from lung-cancer cell line LC319, transfected with plasmids expressing MAPJD or c-MYC (p3xFLAG-MAPJD or MYC) or mock vector (control), were pre-cleared by incubation at 4°C for 1 hour with 100 µl of protein G-agarose beads, in final volumes of 2 ml of immunoprecipitation buffer (0.5% NP-40, 50 mM Tris-HCl, 150 mM NaCl) in the presence of proteinase inhibitor. Immunoprecipitatíon and additional western-blotting were performed as previously described (Kato T, et al. Cancer Res. 2005; 65: 5638-46.).

Cell extracts from lung-cancer cell line LC319 were pre-cleared by incubation at 4°C for 1 hour with 100 µl of protein G-agarose beads in a final volume of 2 ml of immunoprecipitation buffer (0.5% NP-40, 50 mM Tris-HCl, 150 mM NaCl) in the presence of proteinase inhibitor. After centrifugation at 1000 rpm for 5 min at 4°C, the supernatant was incubated at 4°C with anti-FGFR1OP polyclonal antibody or normal rabbit IgG for 2 hours. The beads were then collected by centrifugation at 5000 rpm for 2 min and washed six times with 1 ml of each immunoprecipitation buffer. The washed beads were resuspended in 50 µl of Laemmli sample buffer and boiled for 5 min, and the proteins were separated by 5 - .. 20% SDS PAGE gels (BIO RAD). After electrophoresis, the gels were stained with silver. Protein bands specifically found in extracts immunoprecipitated with anti-FGFR1OP polyclonal antibody were excised and served for matrix-assisted laser desorption/ionization-time of flight mass spectrometry (MALDI-TOF-MS) analysis (AXIMA-CFR plus, SHIMADZU BIOTECH).

### Chromatin Immunoprecipitation (ChIP) Assay.

Cells were cross-linked in 1% formaldehyde for 10 min. The fixed chromatin samples were subjected to immunoprecipitation using ChIP assay kit according to the manufacturer's instructions (UPSTATE, Charlottesville, VA). The sets of primers used for CHIP assay are
5'-CGACGCGTCGTCTGTTCTGAGCTTCCATAC-3' (SEQ ID NO: 46) and
5'-CCGCTCGAGCGGACTAATTCCACTCTCAC-3' (SEQ ID NO: 39) for SBNO1,
5'-CGACGCGTCGGAAAGTCTCACTTCCAATGG-3' (SEQ ID NO: 47) and
5'-CCGCTCGAGCGGATCATGTCTACTGGCTGATC-3' (SEQ ID NO: 41) for TGFBRAP1,
5'-CGACGCGTCGATAGATGTTCCAGAGACATTC-3' (SEQ ID NO: 48) and
5'-CCGCTCGAGCGGTTCAGAAGCCAACAGTGGC-3' (SEQ ID NO: 43) for RIOK1,
5'-CGACGCGTCGCACTGAAGTAATCATGGCAAC-3' (SEQ ID NO: 49) and
5'-CCGCTCGAGCGGAGACAACGGACGTCTGCG-3' (SEQ ID NO: 45) for RASGEF1A.

### Electrophoretic Mobility Shift Assay (EMSA).

MAPJD and MYC proteins were purified by immunoprecipitation of cell extracts from LC319 cells transfected with the plasmids expressing myc-tagged MAPJD or MYC protein. EMSA was performed by incubating MAPJD or MYC with ³²P-labeled oligonucleotide using a standard protocol. The sequences of oligonucleotides for the double-stranded DNA probe were:
5'-CCCGTCGCACGTGGTGGCCA-3' (SEQ ID NO: 50) and
5'-TGGCCACCACGTGCGACGGG-3' (SEQ ID NO: 5 1).

### In vitro kinase assay and in vivo phosphorylation.

For in vitro kinase assays, full-length human recombinant His-tagged ABL1 (Invitrogen) was incubated with the anti-c-myc immunoprecipitates from COS-7 cells transfected with expression plasmids for c-myc-tagged FGFR1OP (pCDNA3.1/myc-His-FGFRIOP) or expression plasmids for c-myc-tagged WRNIP1 (pCDNA3.1/myc-His-WRNIP1) in kinase assay buffer (50 mM Tris, pH7.4, 10 mM MgCl₂, 2 mM dithiothreitol, 1 mM NaF, 0.2 mM ATP) for 60 min at 30 °C. The reactions were stopped by addition of Laemmli sample buffer and heating at 95 °C for 5 mm. Proteins were resolved by SDS-PAGE and then western-blot or [γ-³²P]ATP incorporation analysis. For *in vivo* phosphorylation, COS-7 cells were co-transfected with expression plasmids for c-myc-tagged WRNIP1 and expression plasmids for Flag-tagged ABL1 (pCAGGS/Flag- ABL1). Cells were lysed with RIPA buffer containing Protease Inhibitor and phosphatase inhibitor (1 mM sodium fluoride, 1 mM sodium orthovanadate, 2 mM imidazole, 4 mM sodium tartrate) and followed by SDS-PAGE and immunoblotting to detect phosphotyrosine.

### BrdU-incorporation assay.

Lung-cancer A549 cells transfected with plasmids designed to express ABL1 (pCDNA3.1/myc-His-ABL1), FGFR1OP (pCDNA3.1/myc-His-FGFR1OP), or WRNIP1 (pCDNA3.1/myc-His-WRNIP1), or mock plasmids (pCDNA3.1/myc-His), were cultured for 48 hours. BrdU (5-bromodeoxyuridine) solution was then added in culture medium, and the cells were incubated for 8 hours and fixed; incorporated BrdU was measured using a commercially available kit (Cell Proliferation ELISA, BrdU; Roche Diagnostics, Basel, Switzerland).

### Results

### Expression in Lung Cancers and Normal Tissues,

### KIF4A

Using a cDNA microarray to screen for elements that were highly transactivated in a large proportion of lung cancers, the present inventors identified *KIF4A* gene (GenBank AccessionNo. NM_012310; SEQ ID NO: 53 encoded by SEQ ID NO: 52.) as a good candidate. This gene showed a 5-fold or higher level of expression in the great majority of SCLC cases and in about 40% of NSCLCs the present inventors examined. Subsequently the present inventors confirmed its transactivation by semi-quantitative RT-PCR experiment in 8 of 8 SCLC cases and transactivated in 5 of 10 NSCLC cases (2 of ADCs and 3 of 5 SCCs; Fig. 1A). The present inventors confirmed over-expression of KIF4A protein on western blots using anti-KIF4A antibody in 10 of 12 lung-cancer cell lines (Fig. 1B). Northern blot analysis using *KIF4A* cDNA as a probe identified a 5.0-kb transcript observed exclusively and abundantly in testis among the 23 normal human tissues examined (Fig. 1C). To determine the subcellular localization of endogenous KIF4A in lung cancer cells, the present inventors performed immunocytochemical analysis using anti-KIF4A polyclonal antibodies; KIF4A protein was localized in the cytoplasm and nucleus of DMS273 cells (Fig. 1D). Furthermore the present inventors compared KIF4A protein expression level in normal tissues with lung cancers using anti-KIF4A polyclonal antibodies by immunohistochemistry. KIF4A expressed abundantly in testis and lung cancers, but not in 4 normal organ tissues (liver heart, kidney, lung) (Fig. 1E). In testis or lung cancers, KIF4A was mainly stained at cytoplasm and/or nucleous of primary spermatocytes or cancer cells.

### MAPJD

The present inventors previously screened 23,040 genes on a cDNA macroarray to detect transcripts indicating 5-fold or higher expression in cancer cells than in normal control cells in more than half of 37 NSCLCs analyzed. Among the up-regulated genes, the present inventors identified the C14orf169 (later termed to MAPJD (GenBank Accession NO. NM_024644; SEQ ID NO: 55 encoded by SEQ ID NO; 54), myc-associated protein with JmjC domain, due to the evidences shown below) transcript and confirmed its over-expression in 9 of 11 representative NSCLC cases by semi-quantitative RT-PCR experiments (Fig. 1A). The present inventors also observed high levels of MAPJD expression in 25 of the 26 lung-cancer cell lines the present inventors examined, whereas only very weak PCR product was detectable in cells derived from normal airway epithelia (SAEC and BEAS2B) (Fig. 1A). The present inventors then performed immunocytochemical analysis using anti-MAPJD antibody to examine the subcellular localization of MAPJD in lung-cancer cells. Endogenous MAPJD was detected in nucleolus and diffusely in nucleoplasm (representative data of LC319 was shown in Fig. 1D). Immunohistochemical analysis with anti-MAPJD polyclonal antibody using tissue microarrays consisting of 300 NSCLCs revealed positive staining of the nucleus in 80% of ADCs (132 of 164 cases examined), 81% of SCCs (85 of 105 cases), 62% of large-cell carcinomas (LCCs) (13 of 21 cases), and 90% of bronchioloalveolar-cell carcinomas (BACs) (9 of 10 cases), while no staining was observed in any of their adjacent normal lung tissues (Fig. 1E). Northern blotting using MAPJD cDNA as a probe identified a very weak 2.5-kb band ubiquitously in 73 normal human tissues examined and additional semi-quantitative RT-PCR experiments using the same primers detected MAPJD transcript in lung-cancer cells, much more abundantly than normal tissues examined (heart, liver, lung, bone marrow, testis, ovary, and placenta; data not shown).

### NPTX1

To search for novel target molecules for development of therapeutic agents and/or diagnostic markers for NSCLC, the present inventors first screened genes that showed more than a 5-fold higher level of expression in cancer cells than in normal cells, in half or more of the certain histological type(s) of lung cancer samples analyzed by cDNA microarray (Kikuchi T, et al. Oncogene 2003;22:2192-205.). Among 27,648 genes screened, the present inventors identified the *NPTX1* transcript (GenBank Accession No. NM_02522, SEQ ID NO: 57 encoded by SEQ ID NO: 56.) indicating 5-fold or higher expression in cancer cells than in normal lung cells (control) in 68% of the lung ADC samples and in 80% of SCLCs examined, and confirmed its transactivation by semi-quantitative RT-PCR experiments in 7 of 15 additional long-cancer tissues and in 17 of 23 lung-cancer, cell lines (Fig. 1A). The present inventors performed immunofluorescence analysis to examine the subcellular localization of endogenous NPTX1 in lung-cancer cell lines, A549 and SBC-5. NPTX1 was detected at cytoplasm of tumor cells with granular appearance (Fig. 1D). Since the NPTX1 is thought to be secreted (Schlimgen AK, et al. Neuron. 1995 Mar;14(3):519-26.), the present inventors applied ELISA method to examine its presence in the culture media of lung-cancer cell lines. High levels of NPTX1 protein were detected in media of COS-7 cell transiently transfected with NPTX1-expression vector and A549 cell cultures, but not in the medium of SBC-5 cells (Fig. 1D). The amounts of detectable NPTX1 in the culture media accorded well with the expression levels of NPTX1 detected with RT-PCR. Northern-blot analysis using human cDNA as a probe detected a transcript of 5.1-kb highly expressed in brain and adrenal gland; no expression was observed in any other vital organs including lung, heart, liver, and kidney (Fig. 1C). The present inventors also examined expression of NPTX1 protein with anti-NPTX1 antibody on four normal tissues (liver, heart, kidney, and lung) and SCLC tissues, and found that positive NPTX1 staining appeared in lung tumor tissues, while NPTX1 was hardly detectable in the four normal tissues examined (Fig. 1E).

### FGFR1OP

To search for novel target molecules for development of therapeutic agents and/or diagnostic markers for NSCLC, the present inventors first screened genes that showed more than a 5-fold higher level of expression in cancer cells than in normal cells, in half or more of the lung cancers analyzed by cDNA microarray (Kikuchi T, et al. Oncogene 2003;22:2192-205.). The present inventors next compared the expression profile data in NSCLC with those in 31 normal human tissues previously obtained by this inventor's group (27 adult and 4 fetal organs) (Ochi K, et al. J Hum Genet 2003;48:177-82.; Saito-Hisaminato A, et al. DNA Res 2002;9:35-45.) and selected candidate genes as therapeutic targets, which are specifically expressed in NSCLCs, but not highly in normal tissues. The present inventors found that the *FGFR1OP* transcript (GenBank Accession No. NM_007045, SEQ ID NO: 5 9 encoded by SEQ ID NO: 58.) indicating 5-fold or higher expression in cancer cells than in normal lung cells (control) in 84% of the NSCLC samples examined, and confirmed its transactivation by semi-quantitative RT-PCR experiments in 9 of 14 additional lung-cancer tissues. In addition, the present inventors observed up-regulation of FGFR1OP in 17 of 19 lung-cancer cell lines (NSCLC and SCLC samples) (Fig. 1A). Northern blot analysis using an FGFR1OP cDNA fragment as the probe identified a transcript of about 1.8-kb that was highly and exclusively expressed in testis among 23 normal human tissues examined (Fig. 1C). Predicting the expression of chimeric transcripts over the breakpoint, also, the present inventors performed RT-PCR analyses in various lung cancer cell lines using FGFR1OP and FGFR1 specific primers previous reported (Popovici C, et al. Blood 1999;93:1381-9.; Guasch G, et al. Mol Cell Biol 2001;21:8129-42., Blood 2004;103:309-12.). Both FGFR1OP and FGFR1 transcripts were detected in all lung cancer cell lines examined, but FGFR1OP-FGFR1 and FGFR1-FGFR1OP reciprocal transcripts were not detected at all (data not shown). The present inventors subsequently generated rabbit polyclonal antibody specific to human FGFR1OP and confirmed by western-blot analysis an expression of FGFR1OP protein in 9 cancer cell lines of lung and lung cancer tissues, in which the FGFR1OP transcript had been detected at a high level (Fig. 1B). The present inventers performed immunofluorescence analysis to examine the subcellular localization of endogenous FGFR1OP in lung-cancer cell line NCI-H520, SBC5 and LC319, and found that FGFR1OP was located at cytoplasm and/or nucleus of tumor cells with granular appearance as well as centrosome (Fig. 1D). LC319 cells, synchronized using aphidicolin, were harvested for flow cytometric and immunofluorescence analyses at various time-points after release from the cell-cycle arrest. Before removal of aphidicolin (0 hours), FGFR1 OP was observed in nucleus and cytoplasm with granular appearance. At 1.5 hours after release when most cells were in S phase, FGFR1OP was stained in nucleus and cytoplasm as well as centrosome. Interestingly, FGFR1OP was detected mainly in perinucleus as well as in nucleus at 4 hours when the cells started to enter G2/M phase. At 9 hours when most cells were in G2/M phase, FGFR1 OP was localized mainly in centrosome and cytoplasm (Fig. ID). FGFR1OP protein levels were not changed during cell cycle progression, as detected by western-blot analysis (data not shown). The present inventors next examined expression of FGFR1 or protein with the anti-FGFR1OP antibody on five normal tissues (heart, liver, lung, kidney, and testis) and NSCLC tissues, and found that positive staining was observed only in cytoplasm and/or nucleus of primary lung cancer and testis (Fig. 1E).

### Association of lover-expression with Poor Prognosis.

### KIF4A

Using tissue microarrays prepared from 357 NSCLCs and 29 SCLCs, the present inventors performed immunohistochemical analysis with anti-KIF4A polyclonal antibodies and found positive staining in 127 of NSCLC cases (36%), and 66% of SCLCs (19/29) (Fig. 2A), while no staining was observed in any of normal lung tissues examined. Of these, KIP4A-positive NSCLC cases, 68 were ADCs (30% of 223); 37 were SCCs (39% of 94 cases); 14 were LCCs (52% of 27 cases); 8 were adenosquamous cell carcinomas (ASC; 62% of 13).

The present inventors then tried to correlate expression of this protein in surgically treated NSCLCs with various clinicopathologic variables. The sample size of SCLCs treated with identical protocol was too small to be evaluated further. Statistical analysis revealed that gender (higher in male; *P* = 0.0287 by chi-square test) and histology (higher in non-ADCs; *P* = 0.0097 by chi-square test) were significantly associated with the KIF4A positivity (the details are shown in Table 2).

Kaplan-Meier method indicated significant association between positive staining in NSCLCs and survival rate (P = 0.0005 by the log-rank test; Fig. 2B). By univariate analysis, histology (ADCs vs non-ADCs), pT (T1 versus T2-4), pN (N0 versus N1-2), age (≦65 vs > 65), gender (female vs male), and KIF4A expression (absent versus positive) were all significantly related to poor tumor-specific survival among NSCLC patients (Table 3). Furthermore, multivariate analysis using the Cox propoitional-hazard model indicated that pT stage, pN stage, age, and KIF4A staining were an independent prognostic factor for NSCLC (Table 3).

**Table 2: Association between KIF4A-positivity in NSCLC tissues and patients' characteristics (n=357)**

| | | Total | KIF4A positive | KIF4A absent | Chi-square | *P*-value positive vs absent |
|---|---|---|---|---|---|---|
| | | N=357 | N=127 | N=230 | | |
| Gender | | | | | | |
| | Female | 107 | 29 | 78 | 4.784 | 0.0287 |
| | Male | 250 | 98 | 152 | | |

| Age (years) | | | | | | |
|---|---|---|---|---|---|---|
| | ≦ 65 | 185 | 71 | 114 | 1.317 | NS |
| | > 65 | 172 | 56 | 116 | | |

| Histological type | | | | | | |
|---|---|---|---|---|---|---|
| | ADC | 223 | 69 | 155 | 6.692 | 0.0097 |
| | non-ADC | 134 | 59 | 75 | | |

| pT factor | | | | | | |
|---|---|---|---|---|---|---|
| | T1 | 113 | 36 | 77 | 0.996 | NS |
| | T2+T3+T4 | 244 | 91 | 153 | | |

| pN factor | | | | | | |
|---|---|---|---|---|---|---|
| | N0 | 212 | 75 | 137 | 0.009 | NS |
| | N1+N2 | 145 | 52 | 93 | | |

| | | | | | | |
|---|---|---|---|---|---|---|
| ADC, adenocarcinoma non-ADC, squamous-cell carcinoma plus large-cell carcinoma plus adenosquamous-cell carcinoma NS, no significance | | | | | | |

**Table 3. Cox's proportional hazards model analysis of prognostic factors in patients with NSCLCs**

| Variables | | Hazards ratio | 95% CI | Unfavorable/Favorable | *P*-value |
|---|---|---|---|---|---|
| Univariate analysis | | | | | |
| | KIF4A | 1.690 | 1.254-2.276 | Positive/Negative | 0.0006 |
| | Age (years) | 1.544 | 1.152-2.069 | > 65/65 ≦ | 0.0036 |
| | Gender | 1.690 | 1.203-2.372 | Male/Female | 0.0025 |
| | pT factor | 2.708 | 1.857-3.931 | T2+T3+T4/T1 | <0.0001 |
| | pN factor | 2.369 | 1.769-3.171 | N1+N2/N0 | <0.0001 |
| | Histological type | 1.407 | 1.050-1.884 | non-ADC/ADC | 0.0222 |

| Multivariate analysis | | | | | |
|---|---|---|---|---|---|
| | KIF4A | 1.659 | 1.223-2.251 | Positive/Negative | 0.0011 |
| | Age (years) | 1.754 | 1.300-2.365 | > 65/65 ≦ | 0.0002 |
| | Gender | 1.386 | 0.954-2.014 | Male/Female | 0.0868 |
| | pT factor | 2.054 | 1391-3.034 | T2+T3+T4/T1 | 0.0003 |
| | pN factor | 2.445 | 1.808-3.307 | N1+N2/NO | <0.0001 |
| | Histological type | 0.985 | 0.713-1.361 | non-ADC/ADC | NS |

### NPTX1

To verify the biological and clinicopathological significance of NPTX1, the present inventors also examined the expression of NPTX1 protein by means of tissue microarrays containing primary lung cancers tissues from unselected 374 patients who underwent surgical resection. NPTX1 localized at the plasma membrane as well as in the cytoplasm of tumor cells, but was hardly detectable in surrounding normal tissues (Figs. 2C). Positive staining was observed in 93 (39.1 %) of 238 ADC cases examined, 26 (27.4 %) of 95 SCCs, 12 (42.9%) of 28 LCCs, 8 (61.5%) of 13 ASCs, and 9 (69.2%) of 13 SCLCs, while no staining was observed in any of the normal portions of the same tissues. The present inventors next evaluated the association between NPTX1 status and clinicopathological variables among surgically resected NSCLCs. In this study, pT classification (pT2-4 versus pT1; P < 0.0001 by Fisher's exact test) and pN classification (pN1-2 versus pN0; P = 0.0040 by Fisher's exact test) were significantly associated with the NPTX1 status (Table 4).

The median survival time of patients with absent/weak NPTX1-staining (scored 0, 1+) was significantly longer than that of NSCLC patients with strong NPTX1-staining (scored 2+) (P < 0.0001 by log-rank test; Fig. 2D). The present inventors also used univariate analysis to evaluate associations between patient prognosis and other factors including age (65 ≧ versus <65), gender (male versus female), histological classification (non-ADC versus ADC), pT classification (pT2-4 versus pT1), pN classification (pN1-2 versus pN0), and NPTX1 status (scored 2+ versus scored 0, 1+) (Table 5). Among those parameters, strong NPTX1-positivity (P < 0.001), male gender (P = 0.0010), non-adenocarcinoma histologic type (P = 0.0081), advanced pT stage (P < 0.0001), and advanced pN stage (P < 0.0001) were significantly associated with poor prognosis. In multivariate analysis of prognostic factors, strong NPTX1 expression (P < 0.0001) as well as pT factor (P = 0.0008) and pN factor (P < 0.0001) were significant and independent unfavorable prognostic factors (Table 5).

**Table 4. Association between NPTX1-positivity in NSCLC tissues and patients characteristics (n=374)**

| | | Total | NPTX1 strong positive | NPTX1 weak positive | NPTX1 absent | *P*-value strong/weak vs absent |
|---|---|---|---|---|---|---|
| | | n = 374 | n = 139 | n = 71 | n = 164 | |
| Gender | | | | | | |
| | Male | 259 | 104 | 47 | 108 | NS |
| | Female | 115 | 35 | 24 | 56 | |

| Age (years) | | | | | | |
|---|---|---|---|---|---|---|
| | <65 | 188 | 72 | 35 | 81 | NS |
| | ≧ 65 | 186 | 67 | 36 | 83 | |

| Histological type | | | | | | |
|---|---|---|---|---|---|---|
| | ADC | 238 | 93 | 38 | 107 | NS |
| | SCC | 95 | 26 | 24 | 45 | |
| | Others | 41 | 20 | 9 | 12 | |

| pT factor | | | | | | |
|---|---|---|---|---|---|---|
| | T1 | 125 | 27 | 26 | 72 | <0.0001** |
| | T2+T3+T4 | 249 | 107 | 45 | 92 | |

| pN factor | | | | | | |
|---|---|---|---|---|---|---|
| | N0 | 229 | 72 | 44 | 113 | 0.0040** |
| | N1+N2 | 145 | 67 | 27 | 51 | |

| | | | | | | |
|---|---|---|---|---|---|---|
| ADC, adenocarcinoma; SCC, squamous-cell carcinoma Others, large-cell carcinoma (LCC) plus adenosquamous-cell carcinomas (ASC) *ADC versus non-ADC ***P* < 0.05 (Fisher's exact test) NS, no significance | | | | | | |

**Table 5. Cox's proportional hazards model analysis of prognostic factors in patients with NSCLCs**

| Variables | | Hazards ratio | 95% CI | Unfavorable/Favorable | *P*-value |
|---|---|---|---|---|---|
| Univariate analysis | | | | | |
| | NPTX1 | 2.224 | 1.672-2.958 | Strong(+) / Weak(+) or (-) | <0.0001* |
| | Age (years) | 1.329 | 0.998-1.770 | 65≧/<65 | NS |
| | Gender | 1.750 | 1.256-2.440 | Male/Female | 0.001* |
| | Histological type | 1.474 | 1.106-1.965 | non-ADC/ADC¹ | 0.0081* |
| | pT factor | 2.667 | 1.860-3.822 | T2+T3+T4/T1 | <0.0001* |
| | pN factor | 2.565 | 1.928-3.414 | N1+N2/N0 | <0.0001* |
| | NPTX1 | 1.898 | 1.412-2.552 | Strong(+)/Weak(+)or(-) | <0.0001* |
| | Gender | 1.331 | 0.922-1.921 | Male/Female | NS |
| | Histological type | 1.248 | 0.907-1.717 | non-ADC/ADC¹ | NS |
| | pT factor | 1.910 | 1.309-2.789 | T2+T3+T4/T1 | 0.0008* |
| | pN factor | 2.236 | 1.674-2.986 | N1+N2/N0 | <0.0001* |

| | | | | | |
|---|---|---|---|---|---|
| ¹ADC, adenocarcinoma **P* < 0.05 NS, no significance | | | | | |

### FGFR1OP

To verify the biological and clinicopathological significance of FGFR1OP, the present inventors also examine the expression of FGFR1OP proteins by means of tissue microarrays containing primary lung-cancer tissues from patients who underwent curative surgical resection (Fig, 2E). Positive staining (Scored 2+, 1+) was observed in 229 (87%) of 263 ADC cases examined, 114 (99%) of 115 SCCs, 24 (86%) of 28 LCCs and 12 (92%) of 13 ASCs, while no staining was observed in any of the normal portions of the same tissues. The present inventors classified a pattern of FGFR1OP expression on the tissue array ranging from absent (scored as 0) to weak/strong positive (scored as 1+ ∼ 2+). Strong FGFR1OP expression (scored 2+) was observed in 127 (49%) of 263 ADC cases examined, 62 (54%) of 115 SCCs, 12 (43%) of 28 LCCs and 10 (77%) of 13 ASCs.

The present inventors next evaluated the association between FGFR1OP status and clinicopathological variables among surgically resected lung cancers, and found that lymph node metastasis were significantly associated with the FGFR1OP status (pN1-2 versus pN0; P = 0.0048 by Fisher's exact test) (Table 6).

The median survival time of absent / weak FGFR1OP-staining (scored 0, 1+) patients was significantly longer than that of moderately / strong FGFR1OP-staining patients (scored 2+) among NSCLC patients (P < 0.0001 by log-rank test; Fig. 2F). The present inventors also used univariate analysis to evaluate associations between patient prognosis and other factors including
age (*65* ≧ versus <65),
gender (male versus female),
histological classification (other histological types versus adenocarcinoma), pT classification (pT3+4 versus pT1+2) and pN classification (pN₁₋₂ versus pN₀), smoking history (current and former smoker versus never-smoker), and FGFR1OP status (scored 2+ versus scored 0,1+) (Table 7).

Among those parameters, strong FGFR1OP expression (P < 0.0001), elderly (P = 0.0018), male gender (P = 0.0021), non-adenocarcinoma histologic type (P = 0.011), advanced pT stage (P < 0.0001) and advanced pN stage (P < 0.0001) were significantly associated with poor prognosis. In multivariate analysis of prognostic factors, strong FGFR1OP expression as well as elderly (P < 0.0001), advanced pT stage (P = 0.001), and advanced pN stage (*P* < 0.0001) were significant and independent unfavorable prognostic factors (Table 7).

**Table 6. Association between FGFR1OP-positivity in NSCLC tissues and patients' characteristics (n=402)**

| | | Total | FGFR1OP strong positive | FGFR1OP weak/absent | P-value strong positive vs weak/absent |
|---|---|---|---|---|---|
| | | n=419 | n=211 | n=208 | |
| Gender | | | | | |
| | Male | 289 | 154 | 135 | NS |
| | Female | 130 | 57 | 73 | |

| Age (years) | | | | | |
|---|---|---|---|---|---|
| | <65 | 206 | 109 | 97 | NS |
| | ≧65 | 213 | 102 | 111 | |

| Histological type | | | | | |
|---|---|---|---|---|---|
| | ADC | 263 | 127 | 136 | NS* |
| | SCC | 115 | 62 | 53 | |
| | Others | 41 | 22 | 19 | |

| pT factor | | | | | |
|---|---|---|---|---|---|
| | T1+T2 | 302 | 144 | 158 | NS |
| | T3+T4 | 117 | 67 | 50 | |

| pN factor | | | | | |
|---|---|---|---|---|---|
| | N0 | 259 | 116 | 143 | 0.0048** |
| | Nl+N2 | 160 | 95 | 65 | |

| Smoking history | | | | | |
|---|---|---|---|---|---|
| | Never smoker | 131 | 66 | 65 | NS |
| | Smoker | 288 | 145 | 143 | |

| | | | | | |
|---|---|---|---|---|---|
| ADC, adenocarcinoma; SCC, squamous-cell carcinoma Others, large-cell carcinoma (LCC) plus adenosquamous-cell carcinoma (ASC) *ADC vs on-ADC (SCC and Others) ***P* < 0.05 (Fisher's exact test) NS, no significance | | | | | |

**Table 7. Cox's proportional hazards model analysis of prognostic factors in patients with NSCLCs**

| Variables | | Hazards ratio | 95% CI | Unfavorable/Favorable | *P*-value |
|---|---|---|---|---|---|
| Univariate analysis | | | | | |
| | FGFR1OP | 2.292 | 1.708-3.075 | Strong(+)/Weak(+)or(-) | <0.0001* |
| | Age (years) | 1.574 | 1.183-2.095 | 65≧/<65 | 0.0018* |
| | Gender | 1.666 | 1.203-2.308 | Male/Female | 0.0021* |
| | Histological type | 1.442 | 1.087-1.912 | non-ADC/ADC¹ | 0.011* |
| | pT factor | 1.850 | 1.379-2.482 | T3+T4/T1+T2 | <0.0001* |
| | pN factor | 2.316 | 1.749-3.068 | N1+N2/N0 | <0.0001* |

| Multivariate analysis | | | | | |
|---|---|---|---|---|---|
| | FGFR1OP | 1.962 | 1.456-2.643 | Strong(+)/Weak(+) or (-) | <0.0001* |
| | Age (years) | 1.911 | 1.428-2.558 | 65≧/<65 | <0.0001* |
| | Gender | 1.368 | 0.954-1.963 | Male/Female | 0.0886 |
| | Histological type | 1.101 | 0.805-1.507 | non-ADC/ADC¹ | 0.5459 |
| | pT factor | 1.652 | 1.225-2.228 | T3+T4/T1+T2 | 0.001* |
| | pN factor | 2.194 | 1.637-2.939 | N1+N2/N0 | <0.0001* |

| | | | | | |
|---|---|---|---|---|---|
| ¹ADC, adenocarcinoma **P* < 0.05 | | | | | |

### Serum Levels of NPTX1 in Lung Cancer Patients.

Because the *in vitro* findings had suggested a possibility for development of a novel tumor maker for lung cancer, the present inventors investigated whether the NPTX1 is secreted into sera of patients with lung cancer. ELISA experiments detected NPTX1 in serologic samples from lung cancer patients and also from normal individuals; serum levels of NPTX1 in lung cancer patients were 135.0 ± 104.0 U/ml (mean ± ISD) and those in healthy individuals were 67.9 ± 48.6 U/ml (The difference was significant with P-value of < 0.001 by Mann-Whitney U test; Fig. 3). When classified according to histologic type, the serum levels of NPTX1 were 142.1 ± 85.8 U/ml in lung ADC patients, 113.1 ± 92.5U/ml in lung SCC patients, and 138.0 ± 131.9 U/ml in SCLC patients; the differences among the three histologic types were not significant.

### Combination Use of NPTX1 and proGRP/CEA as Tumor Markers.

To evaluate the feasibility of using serum HPTX1 level as a tumor detection biomarker, the present inventors also measured by ELISA serum levels of CEA for NSCLC and proGRP for SCLC patients, two conventional tumor markers for these histological types of lung cancer, in the same patients and controls. Cutoff levels in this assay determined by ROC analyses were set to result in optimal diagnostic accuracy and likelihood ratios for NPTX1, CEA, and proGRP, *i.e*., 140 U/ml for NPTX1 (with a sensitivity of 37% for NSCLC and 38.5% for SCLC, and a specificity of 93.3%), 2.5 ng/ml for CEA (with a sensitivity of 41% and a specificity of 93.3% for NSCLC) and 46 pg/ml for proGRP (with a sensitivity of 69.2% and a specificity of 98.9% for SCLC). Measuring both NPTX1 and CEA in serum can improve overall sensitivity for detection of NSCLC to 64%. False-positive rates for either of the two tumor markers among normal volunteers (control group) amounted to 12.2%. On the other hand, measuring both NPTX1 and proGRP in serum can improve overall sensitivity for detection of SCLC to 76.9%. False-positive results for either of the two tumor markers among normal volunteers (control group) amounted to 7.7%.

### Inhibition of Growth of Lung Cancer Cells by Small Interfering RNA

### KIF4A

To assess whether KIF4A is essential for growth or survival of lung cancer cells, the present inventors constructed plasmids to express siRNAs against KIF4A (si-KIF4As) as well as control plasmids (siRNAs for Luciferase and Scramble) and transfected them into SBC-5 cells. The mRNA levels in cells transfected with si*-KTF4*#1 or -#2 were significantly decreased in comparison with cells transfected with either control siRNAs. The present inventors observed significant decreases in the number of colonies formed and in the numbers of viable cells measured by MTT assay (representative data of SBC-5 was shown in Figs. 4A-C).

### MAPJD

To assess whether MAPJD is essential for growth or survival of lung-cancer cells, the present inventors designed and constructed plasmids to express siRNA against MAPJD (si-MAPJD-1 and -2) and two control siRNAs (for Luciferase (LUC), or Scramble (SCR)), and transfected each of them into LC319 and A549 cells. The amount of MAPJD transcript in the lung-cancer cells transacted with si-MAPJD-1 or -2 was significantly decreased in comparison with those transacted with either of the two control siRNAs (Fig. 5A, left upper panels); transfection of si-MAPJD-1 or -2 also resulted in significant decreases in colony numbers and cell viability measured by colony-formation and MTT assays (P = 1.1x10⁻⁷ (si-2); unpaired t-test) (Fig. 5A, left lower and right panels). To clarify the mechanisms of this phenotype further, the present inventors performed flow cytometrical analysis using LC319 cells that had been transfected with si-MAPJD-2, and found that the sub-G1 proportion of cells treated with si-MAPJD-2 was significantly higher than those treated with control siRNA (LUC) (Fig. 5B). Moreover, the present inventors confirmed increased number of apoptotic cells transfected with si-MAPJD-2 in comparison to those transfected with LUC using Annexin V-binding assay (Fig. 5C).

### NPTX1

To assess whether up-regulation of NPTX1 plays a role in growth or survival of lung-cancer cells, the present inventors designed and constructed plasmids to express siRNA against NPTX1 (si-NPTX1-2), along with two different control plasmids (siRNAs for Luciferase (LUC), and Scramble (SCR)), and transfected them into A549 and SBC-5 cells to suppress expression of endogenous NPTX1. The amount of NPTX1 in the cells transfected with si-NPTX1-2 was significantly less than in cells transfected with any of the two control siRNAs (Fig. 6, upper panels). In accord with its suppressive effect on protein levels, transected si-NPTX1-2 caused significant decreases in colony numbers and cell viability measured by colony-formation and MTT assays, but no such effects were observed by two controls (Fig. 6, middle and lower panels).

### FGFR1OP

To assess whether up-regulation of FGFR1OP plays a role in growth or survival of lung-cancer cells, the present inventors designed and constructed plasmids to express siRNA against FGFR1OP (si-1), along with three different control plasmids (siRNAs for Luciferase (LUC), and Scramble (SC)), and transfected them into LC319 and SBC-5 cells to suppress expression of endogenous FGFR1OP (Figs. 7A, B). The amount of FGFR1OP transcript in the cells transfected with si-1 was significantly less than in cells transfected with any of the three control siRNAs (Figs. 7A, B, upper panels). Cell viability and colony numbers measured by MTT and colony-formation assays were reduced significantly in cells transected with si-1 in comparison with cells transfected with the three control siRNAs or ineffective siRNA (six-1) (Figs. 7A, B, middle and lower panels).

### WRNIP1

To further assess whether expression of WRNIP1 plays a role in growth of lung-cancer cells, the present inventors then examined the biological significance of the WRNIP1 function in pulmonary carcinogenesis using siRNAs against WRNIP1 (si-WRNIP1-#1 and - #2). Treatment of LC319 cells with siRNA oligonucleotides against WRNIP1 (si-WRNIP1-#1 or -#2) suppressed expression of the endogenous WRNIP1 in comparison to the control siRNAs (Fig. 15, upper panels). In accordance with the reduced expression of WHNIP1, LC319 cells showed significant decreases in cell viability and numbers of colonies (Fig. 15, middle and lower panels). These results strongly supported the possibility that WRNIP1 might also play a significant role in growth/survival of lung cancer cells.

### Cellular Growth and Invasive Effect in Mammalian Cells.

### KIF4A

To determined the effect of KIF4A on growth and transformation of mammalian cells, the present inventors carried out *in vitro* assays using COS-7 cells that transiently expressed KIF4A (COS-7-KIF4A). Growth of the COS-7-KIF4A cells was promoted in comparison with the empty vector controls (Fig. 4D), as determined by the MTT assay. There was also a remarkable tendency of COS-7-KIF4A cells to form larger colonies than empty-vector clones did.

As the immunohistochemical analysis on tissue microarray had indicated that lung-cancer patents with KIF4A positive tumors showed shorter cancer-specific survival period than patients whose tumors were negative for KIF4A, the present inventors performed Matrigel invasion assays to determine whether KIF4A might play a role in cellular invasive ability. Invasion of COS-7-KIF4A cells or NIH3T3-KIF4A cells through Matrigel was significantly enhanced, compared to the control cells transfected with mock plasmids, thus Independently suggesting that KIF4A could also contribute to the highly malignant phenotype of lung-cancer cells (Figs. 4E-G).

### MAPJD

To further verify a potential role of MAPJD in tumorigenesis, the present inventors established NIH3T3 cells that stably expressed exogenous MAPJD. The growth rate of NIH3T3-derivative cells that stably expressed MAPJD was much higher than that of cells transfected with mock plasmid in a MAPJD-dose-dependent manner (Fig. 5D).

### FGFROP1

To further confirm the effects of FGFR1OP on growth of mammalian cells, the present inventors carried out in vitro assays using COS-7 cells that transiently expressed FGFR1OP (FGFR1OP/COS-7). Growth of the FGFR1OP/COS-7 cells was promoted in comparison with the empty vector controls (Fig. 7C), as determined by the MTT assay, thus independently suggest that FGFR1OP is essential for growth of mammalian cells.

As the immunohistochemical analysis on tissue microarray had indicated that lung cancer patients with FGFR1OP positive tumors showed shorter cancer-specific survival period than those with FGFR1OP-negative tumors, the preset inventors examined a possible role of FGFR1OP in cellular migration and invasion using Cell migration and Matrigel invasion assays, using COS-7 cells. As shown in Fig. 7, transfection of FGFR1OP cDNA into the cell line significantly enhanced its migration (Fig. 7D) as well as invasive activity through Matrigel (Fig. 7E), compared to cells transected with mock vector.

### Identification of Proteins Interacting with KIF4A.

To elucidate the function of KIF4A in lung cancer cells, the present inventors looked for protein(s) that would interact with KIF4A. Lysates of DMS273 cells were extracted and immunoprecipitated with anti-KIF4A polygonal antibody. Protein complexes were stained with SilverQuest (Invitrogen) on SDS-PAGE gels. Two bands around 75-kDa, which were seen in immunoprecipitates of cell lysates with anti-KIF4A antibody, were extracted and their peptide sequences were determined by MALDI-TOF mass spectrometric analysis (Fig. 8A). This procedure identified ZNF549 (GenBank Accession No. NM_153263, SEQ ID NO: 87 encoding by SEQ ID NO: 86.) and ZNF553 (GenBank Accession No. NM_152652, SEQ ID NO: 89 encoded by SEQ ID NO: 88.) as candidates that interact with KIF4A in lung-cancer cells.

Semi-quantitative RT-PCR analysis revealed that these two genes were over-expressed in lung cancer samples compared with normal lung (data not shown). Immunocytochemical analysis using DMS273 or SBC-5 cells transfected with Flag/HA-tagged-ZNF549 (pCAGCSn 3FH-ZNF549) or Flag/HA-tagged-ZNF553 (pCAGGSn 3FH-ZNF553) revealed co-localization of endogenous KIF4A and these two proteins mainly at nucleus (representative data of SBC-5 was shown in Figs. 8B, C), suggesting that KIF4A-ZNF549 and/or KIF4A-ZNF553 complexes may play an important role in lung carcinogenesis.

### Identification of MAPJD Target Genes.

Since MAPJD localized at nucleolus as well as nucleoplasm, and includes a JmjC domain which is suggested to play a role in the transcriptional regulation, the present inventors first attempted to identity downstream genes specifically regulated by MAPJD in cancer cells. siRNA-MAPJD-2 or siRNA-LUC (control siRNA) was transfected into LC319 cells in which MAPJD was expressed at a high level, and alterations in gene expression at various time-points were monitored using a cDNA microarray consisting of 23,040 genes. Among hundreds of genes that had been down-regulated by this approach, the present inventors selected 53 genes whose expression were significantly decreased in accordance with the reduction of MAPJD expression by performing the self-organizing map (SOM) clustering analysis (Kohonen T. Proceeding of the IEEE 78, 1464-80 (1990).).

Semi-quantitative RT-PCR analysis confirmed time-dependent reduction of these 53 candidate transcripts in LC319 cells transfected with si-MAPJD-2, but not with control siRNA for LUC (data not shown). The present inventors also evaluated the transactivation of these genes in accordance with introduction of exogenous MAPJD expression in lung-cancer cell lines (data not shown), and finally selected four top candidate MAPJD-target genes, SBNO1 (GenBank Accession NO: AK001563, SEQ ID NO: 61 encoded by SEQ ID NO: 60.), TGFBRAP1 (GenBank Accession NO: NM_004257, SEQ ID NO: 63 encoded by SEQ ID NO: 62.), RIOK1 (GenBank Accession NO: NM_031480, SEQ ID NO: 65 encoded by SEQ ID NO: 64.), and RASGEF1A (GenBank Accession NO: NM_145313, SEQ ID NO: 67 encoded by SEQ ID NO: 66.), which were the most significantly induced by exogenous MAPJD expressions.

To examine the possible promoter-specific transactivation of these target genes by MAPJD, the present inventors co-transfected into LC319 cells reporter plasmids containing a 1-kb upstream region of the putative transcription start site of each of the four genes fused to a luciferase reporter gene, and MAPJD expressing plasmids. MAPJD transfected cells displayed higher luciferase activity than mock-transfected cells (Fig. 9A).

### Interaction of MAPJD with MYC and their Transcriptional Regulation.

The reporter gene assay suggested that MAPJD could act as a transcription factor or regulate gene expressions together with some transcription factor(s). Hence, the present inventors searched for candidate consensus binding-motif of transcription factors common to regulatory regions of these four candidate MAPJD-target genes, and found that all of the four candidate genes contained several copies of E-box (CANNTG) in their possible transcriptional regulatory regions. Since MYC (GenBank Accession No. NM_002467) is known to be one of the proteins binding to the E-box, the present inventors then examined by immunoprecipitation assay the association of exogenously expressed MAPJD or MYC with endogenous ones in LC319 cells (Fig. 9B). To examine the association of MAPJD and/or MYC with promoter sites of the four genes, the present inventors performed ChIP assay with anti-MAPJD antibody or anti-MYC antibodies using extracts of LC319 cells. Genomic segments corresponding to the 1-kb upstream region containing the putative transcription start sites were confirmed to be associated with both MAPJD and MYC proteins (Fig. 9C). To investigate transcriptional activity of MAPJD and MYC on the MAPJD-target genes, the present inventors performed luciferase assay. LC319 cells transfected with MYC or MAPJD induced higher luciferase activity than mock transfected cells, indicating that MAPJD and MYC are responsible for the transactivation of the four genes (Fig. 9D). Moreover, co-transfection of both MAPJD and MYC expression plasmids further increased luciferase activity of reporter plasmids (Fig. 9D). These data suggest that MAPJD and MYC could form a complex and synergistically regulate the transcriptional activity of the candidate MAPJD-target genes.

The present inventors subsequently focussed on the E-box motifs in the possible promoter region of the RIOK1 gene, which has 8 E-box motifs from -1504 to +35 (Fig. 10A). ChIP assay with anti-MAPJD and anti-MYC antibodies using nuclear extracts of LC319 cells detected that only the genomic segment-7 containing an E-box motif seemed to be most significantly associated with endogenous MAPJD, while MYC almost equally bound to all of the 7 segments (data not shown). These data suggest that MAPJD was possibly associated with the E-box of segment-7 in the RIOK1 gene.

The present inventors also performed an EMSA using a double-stranded oligonucleotide probe that corresponds to the possible binding sequence and myc-tagged MAPJD protein purified by immunoprecipitation. A shifted band was detected in the presence of MAPJD, while no shifted-band was observed after cold competitive oligonucleotides were added, supporting the specific interaction between the oligonucleotide probe and MAPJD (Fig. 10B, left panel). The band was super-shifted after the addition of anti-MAPJD antibody, but not that of normal rabbit IgG, thus independently confirming the specific interaction (Fig. 10C, left panel). MYC also interacted with this probe specifically (Fig. 10B, C, right panels).

These data and the fact that LC319 cells highly express endogenous MYC and MAPJD raise the following hypotheses about the interaction between MAPJD-MYC complex and the E-box site; 1) MAPJD could firstly recognize and bind to this E-box site and then, like many other transcription factors or enhancer proteins, change nucleosomal structure of this site in the mechanism like that has been demonstrated in transactivation by acetylation of histone H4. MAPJD might subsequently recruit MYC to this site and enhance transcription of target genes; or 2) MAPJD might form a heterodimer with MYC as is the case of MAX. MYC-MAPJD heterodimers might hind to DNA and recruit chromatin modifying factors. Although the precise mechanism is unclear, MAPJD could effectively help MYC-mediated transactivation system by binding to E-box motif and/or recruiting chromatin-modifying factors to this site.

### Enhancement of MYC-related HAT Complex Recruitment to the Target Genes and Histone H4 Acetylation by MAPJD.

MYC is known to activate gene transcription by binding to E-boxes in regulatory regions of its target genes and by recruiting protein complexes containing the TRRAP protein together with GCN5/PCAF or TIP60 histone acetyltransferases (HATs) that preferentially acetylate histone H3 and H4 (Nesbit CE, et al. 1999. Oncogene 18:3004-16.; Blackwell TK, et al. 1999. Science 250: 1149-51.; Blackwood EM, and RN Eisenman 1991. Science. 251:1211-7.; McMahon SB, et al. Mol. Cell. Viol. 20, 556-62 (2000).; Frank SR et al. EMBO Rep. 4, 575-80 (2003).). Therefore, the present inventors investigated whether MAPJD had functional effects on recruitment of the MYC-regulated HAT complex to the four MAPJD-target genes and/or their HAT activity.

The present inventors first examined possible interaction of FLAG-MAPJD with endogenous MYC and other proteins that were included in the MYC-HAT complex. Immunoprecipitation with antibodies specific to endogenous MYC (sc-764; Santa Cruz Biotechnology, Inc.), TRRAP (sc-11411), or TIP60 (sc-5725) followed by immunoblotting with anti-FLAG antibody detected the interaction of exogenous MAPJD protein with endogenous MYC, TRRAP, and TIP60 in LC319 cells (Fig. 11A). Next, to investigate the level of the recruitment of the HAT complexes to the promoter region of the four MAPJD-target genes, and that of their histone acetylation, the present inventors performed ChIP assay with antibodies to a MYC cofactor TRRAP and tri-acetylated histone H4 using LC319 cells transfected with MAPJD expression vectors. The present inventors observed the enhanced association of TRRAP with individual gene regulatory regions, and the increase in tri-acetylated histone H4 (AcH4) levels in the cells transfected with MAPJD expression plasmid (Fig. 11B). The data suggest that MAPJD might induce the recruitment of HAT complex to chromatin and enhance histone acetylation and consequently prompt the expression of the MAPJD-target genes.

### Identification of WRNIP1 and ABL1 as a novel molecule interacting with FGFR1OP.

To elucidate the function of FGFR1OP in carcinogenesis, the present inventors attempted to identify proteins that would interact with FGFR1OP in lung-cancer cells. Cell extracts from LC319 cells were immunoprecipitated with anti-FGFR1OP antibody or rabbit IgG (negative control). Following separation by SDS-PAGE, protein complexes were silver-stained. Protein band, which was seen in immunoprecipitates by anti-FGFR1OP antibody, but not in those by rabbit IgG, was excised, trypsin-digested, and subjected to mass spectrometry analyses. Peptides from protein band matched sequences in Werner helicase interacting protein 1 (WRNIP1 alias WHIP) and Abelson murine leukemia viral oncogene homolog 1 (ABL1 alias c-Abl) (Fig. 12A right panel). The present inventors confirmed the cognate interaction between FGFR1OP and WRNIP1 (GenBank Accession No. NM_020135, SEQ ID NO: 91 encoded by SEQ ID NO: 90.) or ABL1 (GenBank Accession No. NM_007313, SEQ ID NO: 93 encoded by SEQ ID NO: 92.), by immunoprecipitation experiments in LC319 cells (Fig. 12A left panel). To examine the subcellular localization of endogenous FGFR1OP and WRNIP1, the present inventors performed immunocytochemical analysis using anti-FGFR1OP or WRNIP1 polyclonal antibodies; both FGFR1OP and WRNIP1 were detected as diffuse spotty signals over the nucleus (Fig. 12B).

### DNA damage response of FGFR1OP, WRNP1 and ABL1.

The present inventers identified DNA damage response proteins, WRNIP1 and ABL1, interacting with FGFR1OP. When DNA is damaged in cells, many DNA damage-signaling proteins are recruited to the damaged loci and form discrete nuclear foci. The order and timing of these events are thought to be critical for checkpoint response and DNA repair. Therefore, the present inventers analyzed the accumulation of FGFR1OP, WRNIP1 and ABL1 protein level in lung cancer cell line, A549 cells, at indicated time points after the exposure to UV radiation (Fig. 12C). In A549 cells, DNA damaged increase in the amounts of WRNIP1 was detectable, however, the expression levels of FGFR1OP and ABL1 remained unchanged regardless of DNA damage (Fig. 12C). Next, the present inventers examined the location of the endogenous FGFR1OP (Fig. 12D), WRNIP1 (Fig. 12E) and ABL1 (Fig. 12F) at 24 hours after the exposure to UV radiation. It was observed the formation of nuclear foci by FGFR1OP and WRNIP1 in response to DNA damage (Fig. 12). While ABL1 localized in the cytoplasm of A549 cells with untreated UV radiation, the population of ABL1 was found in the nucleus by a significant ABL1 translocation after then exposure to UV radiation (Fig. 12). These results indicated that FGFR1OP is likely to be a DNA damage related protein by interaction with ABL1 and WRNIP1, of course their relationship should be further elucidated. ABL1 is targeted to FGFR1OP or WRNIP1 in the nucleus in the response to DNA damage.

### WRNLP1 (a novel molecule interacting with FGFR1OP) is tyrosine phosphorylated by ABL1 in vitro and in vivo.

To determine whether FGFR1OP or WRNIP1 is potential as a substrate for ABL1, c-myc tagged FGFR1OP (immunoprecipitates) or c-myc-tagged WRNIP1 (immunoprecipitates) was incubated with recombinant ABL1 (Figs. 13A and 13B). Results of the western-blot analysis showed that WRNIP1 was tyrosine-phosphorylated by ABL1 tyrosine kinase *in vitro* (Fig. 13A, right panels) and that total WRNIP1 protein levels were not affected by the treatment, whereas there was no detectable phosphorylation of FGFR1OP (Fig. 13A, left panels). To validate the phosphorylation of WRNIP1, the present inventers performed kinase assay by incubating c-myc tagged FGFR1OP (immunoprecipitates) with recombinant ABL1 in the presence of [γ-³²P]ATP. In the same result as western-blot, analysis of the products by autoradiography showed that ABL1 phosphorylated WRNIP1 (Fig. 13B). To examined whether WRNIP1 is phosphorylated by ABL1 *in vivo*, COS-7 cells were co-transfected with expression plasmids for c-myc-tagged WRNIP1 and expression plasmids for Flag-tagged ABL1. Results from western-blot showed similar amounts of WRNIP1 protein in the different co-transfected cells (Fig. 13C, lower panel). Analysis of anti-c-myc immunoprecipitates (WRNIP1) with anti-phosphotyrosine antibody demonstrated the tyrosine phosphorylation of WRNIP1 by ABL1 (Fig. 13C, upper panel). Therefore, WRNIP1 is an *in vivo* substrate of ABL1.

### Inhibition of ABL1-dependent phosphorylation of WRNP1 by FGFR1OP.

To elucidate the function of the interaction between FGFR1OP and WRNIP1/ABL1 in lung carcinogenesis, the present inventors examined the subcellular localization of these proteins in lung cancer cells, A549. Immunocytochemical analysis using anti-FGFR1OP and anti-WRNIP1/ABL1 antibodies demonstrated that endogenous FGFR1OP was co-localized with endogenous WRNIP1 and ABL1 mainly in perinucleus as well as in nucleus at S-G2/M phase (Fig. 14A).

To determine whether FGFR1OP could affect phosphorylation of WRNIP1 by ABL1, *in vitro* kinase assay was performed by incubating c-myc tagged WRNIP1 with His-tagged ABL1 in the absence or presence of recombinant GST-tagged FGFR1OP. As shown in Figure 14B, FGFR1OP significantly inhibited the ABL1 kinase activity on WRNIP1 in a dose-dependent manner. The effect of FGFR1OP over-expression on ABL1-induced cell cycle arrest was examined. The present inventors measured the BrdU incorporation ability of A549 cells that were over-expressed either ABL1, or both ABL1 and FGFR1OP, and found that ABL1-induced cell cycle arrest was recovered by over-expression of FGFR1OP (Fig. 14C). These results suggested that FGFR1OP might block phosphorylation of WRMP1 by ABL1 and play a significant role in pulmonary carcinogenesis.

### Discussion

### KIF4A

Several molecular-targeting drugs have been developed and proved their efficacy in cancer therapy, however patients showing good response are very limited (Ranson M, et al. J Clin Oncol 2002;20:2240-50.). Therefore, further development of molecular-targeting drugs for cancer is urgently awaited.

The present inventors have screened the therapeutic target molecules by the following strategy: (I) Identifying up-regulate genes in lung cancer by genome-wide cDNA microarray system (Kikuchi T, et al. Oncogene 22: 2192-205, 2003.; Kakiuchi S, et al. Mol Cancer Res 1: 485-99, 2003.), (II) Verifying the candidate genes for its no or low level of expression in normal tissues by northern-blotting (Saito-Hisaminato A, et al. DNA Res. 2002 Apr 30;9(2):35-45.; Ochi K, et al. J Hum Genet 2003;48(4):177-82.), (III) Validating clinicopathological significance of their over-expression by means of tissue microarray containing hundreds of archived lung-cancer samples (Suzuki C, et al. Cancer Res. 2005; 65, 11314-25.; Ishikawa N, et al. Clin Cancer Res. 2004;10(24):8363-70., Cancer Res. 2005; 65, 9176-84.; Kato T, et al. Cancer Res. 2005;65(13):5638-46.; Furukawa C, et al. Cancer Res. 2005;65(16):7102-10.), (IV) Verifying whether the target gene is essential for growth or the survival of cancer cells by RNAi assay (Suzuki C, et al. Cancer Res. 2005; 65, 11314-25., Cancer Res 2003;63:7038-41.; Kato T, et al. Cancer Res. 2005;65(13):5638-46.; Furukawa C, et al. Cancer Res. 2005;65(16):7102-10.). Using this approach the present inventors have shown here that KIF4A is frequently over-expressed in clinical lung-cancer samples, and cell lines, and that those gene products play indispensable roles in the growth and progression of lung-cancer cells.

Kinesins constitute a superfamily of microtubule-based motor proteins with 45 members in mice and humans that represent diverse functions, including the transport of vesicles, organelles, chromosomes, protein complexes, and mRNA (Lawrence CJ, et al. J Cell Biol. 2004 Oct 11;167(1):19-22.; Miki H, et al. Trends Cell Biol. 2005 Sep;15(9):467-76.; Hirokawa N. et al. Science. 1998 Jan 23;279(5350):519-26.). The inhibition of the mitotic kinesin Eg5 by small molecules such as monastrol is being evaluated as an approach to develop a novel class of antiproliferative drugs for the treatment of malignant tumors (Muller C, et al. Cancer Chemother Pharmacol. 2006 May 16; [Epub ahead of print]; Koller E, et al. Cancer Res. 2006 Feb 15;66(4):2059-66.).

The KIF4 subfamily consists of KIF4A, KIF4B, KIF21A and KIF21B (Lawrence CJ, et al. J Cell Biol. 2004 Oct 11;167(1):19-22.). KIF4A plays essential roles in regulating anaphase spindle dynamics and the completion of cytokinesis (Zhu C and Jiang W. et al. Proc Nat Acad Sci U S A. 102: 343-8, 2005.). KIF4A is a novel component of the chromosome condensation and segregation machinery functioning in multiple steps of mitotic division. In MRC-5 human fetal lung fibroblast cells, KIF4A was prominently localized at nucleus during interphase, but from prophase to telophase KIF4A was present on chromosome arms. In addition, the protein accumulated in the mid-zone and formed the cytokinetic ring until cytokinesis (Mazumdar M, et al. J Cell Biol. 2004 Aug 30;166(5):613-20. Epub 2004 Aug 23.). KIF4 is also reported to be related to neuronal survival (Midorikawa R, et al. Cell. 2006 Apr 21;125(2):371-83.).

Treatment of NSCLC cells with specific siRNA to reduce expression of *KIF4A* resulted in growth suppression. The present inventors also found other evidence supporting the significance of this pathway in carcinogenesis; *e.g*., the expression of KIF4A also resulted in the significant promotion of the cell growth and invasion in *in vitro* assays. Moreover, clinicopathological evidence obtained through the tissue-microarray experiments demonstrated that NSCLC patients with tumors expressing KIF4A showed shorter cancer-specific survival periods than those with negative KIF4A expression. The results obtained by *in vitro* and *in vivo* assays show that over-expressed KIF4A is an important growth factor and is associated with cancer cell growth and invasion, inducing a highly malignant phenotype of lung-cancer cells. In this study, the present inventors also documented interaction of KIF4A with two zinc finger proteins ZNF549 and 2NF553 and their co-localization at cytoplasm and nucleus in cancer cells. The combined evidence suggests that KIF4A could transport some important nuclear proteins including transcription factors from cytoplasm to nucleus, to promote specific gene transcription in human cancer cells, and then enhance the growth and/or survival process in cancer cells.

### MAPJD

Molecular-targeted drugs are expected to be highly specific to malignant cells, and have minimal adverse effects due to their well-defined mechanism of action. Toward identification of appropriate molecular targets for development of such drugs, the present inventors combined genome-wide expression analysis for electing genes that were over-expressed in lung-cancer cells with high-throughput screening of loss-of-function effects by means of The RNAi technique (Kikuchi T, et al. Oncogene 2003;22:2192-205., Int J Oncol 2006;28,799-805.; Kakiuchi S, et al. Mol Cancer Res 2003;1:485-99., Hum Mol Genet 2004;13:3029-43.; Suzuki C, et al. Cancer Res 2003;63:7038-41., Cancer Res. 2005; 65: 11314-25.; Ishikawa N, et al. Clin Cancer Res 2004;10:8363-70., Cancer Res. 2005;65(20):9176-84.; Kato T, et al. Cancer Res. 2005; 65: 5638-46.; Furukawa C, et al. Cancer Res. 2005;65(16):7102-10.).

Using this systematic approach the present inventors found MAPJD to be frequently over-expressed in clinical NSCLC samples as well as cell lines, and showed that over-expression of this gene product plays an indispensable role in the growth of lung-cancer cells. The human MAPJD encodes a 641 amino-acid protein with a JmjC domain at the amino-acid sequence of 209-465. Since JmjC domain was likely to be found together with DNA- or chromatin-binding domains the JmjC domain-containing proteins are supposed to be good candidates for enzymatic components that regulate chromatin remodeling and/or gene expressions (Eilbracht J, et al. Mol Biol Cell. 2004 Apr;15(4):1816-32.).

Many studies have demonstrated that cell cycle progression rates are closely related to MYC levels (Nesbit CE, et al. 1999. Oncogene 18:3004-16.). Modest increase in the levels of MYC was supposed to be an initiating event in the various forms of human cancer (Nesbit CE, *et al. supra.).* MYC regulates the transcription of downstream target genes by recruiting the acetyltransferase complexes to the target genes. In this system, it was suggested that MYC may work through the modification of HAT activities, in concert with other chromatin remodeling enzymes, some of which may not be identified yet, and that different combinations of HATs and chromatin remodeling factors might be applied depending on various situations (Nesbit CE, *et al. supra.;* Blackwell TK, et al. 1999. Science 250:1149-51.; Blackwood EM, and RN. Eisenman. 1991. Science. 251:1211-7.; McMahon SB, et al. Mol Cell Biol 20, 556-62 (2000).; Frank SR, et al. EMBO Rep. 4, 5 75-80 (2003).).

The present inventors here demonstrated that MAPJD transactivated a set of genes possibly related to lung cancer cell proliferation, by interacting with a MYC-TRRAP-TIP60 transcriptional complex, and inducing histone acetylation (Fig. 11C). The present inventors identified 4 downstream target genes of MAPJD, all of which have been supposed to be involved in oncogenic signalings. SBNO1 is a downstream component of the Notch signaling pathway that is known to function in the oncogenic process, and is required during embryogenesis and oncogenesis in Drosophila (Coyle-Thompson CA, et al. Development 1993; 119:377-95.; Radtke F, et al. Nat Rev Cancer 2003;10:756-67.). TGFBRAP1 binds to TGFB receptors that are reportedly related to tumorigenesis (Charng MJ, et al. J Biol Chem 1998;273:9365-8.; Wurthner JU, et al. J Biol Chem 2001;276:19495-502.). RIOK1 is reported to be over-expressed in colon cancers (Line A, et al. Can Immunol Immunotherapy 2002;51:574-82.). RASGEF1A contain a RasGEF domain, which might play an important role in the Ras-signaling pathway. The data presented here indicate that MAPJD is one of the key regulators to selectively activate the transcription of several genes, along with MYC oncogene.

MAPJD was previously reported as a dual location protein in the nucleolus and in a special type of synchronously replicating chromatin, however, its physiological function remains unclear (Eilbracht J, et al. Mol Biol Cell. 2004 Apr;15(4):1816-32.). The present inventors confirmed that MAPJD localized at nucleolus and nucleoplasm in cancer cells. The nucleolus is known to function in ribosome biogenesis, a complicated process that includes the transcription of rRNA genes, the processing and modification of these transcripts, and their assembly with both ribosomal proteins as well as non-ribosomal ones to guide the formation of preribosomal particles (Scheer U, et al. Curr Opin Cell Biol 1999; 11:385-90.; Zhao J, et al. Mol Cell 2003;11:405-13.).

However, it was recently reported that nucleolus also harbors diverse functions that involved the assembly of various other ribonucleoprotein particles, the modification of small RNAs, the control of the cell cycle, the sequestration of regulatory molecules, and nuclear export process (Olson MO, et al. Int Rev Cytol 2002;219:199-266.; Gerbi SA, et al. Curr Opin Cell Biol 2003;15:318-25.). MAPJD plays an important role on oncogenesis, by interacting with several nuclear proteins as well as MYC.

### NPTX1

The present inventors have screened the therapeutic target molecules by the following strategy: (I) Identifying up-regulate genes in lung cancer by genome-wide cDNA microarray system (Kikuchi T, et al. Oncogene 2003;22:2192-205.; Kakiuchi S, et al. Mol Cancer Res 2003;1:485-99., Hum Mol Genet 2004;13:3029-43.), (II) Verifying the candidate genes for its no or low level of expression in normal tissues by northern-blotting (Saito-Hisaminato A, et al. DNA Res. 2002 Apr 30;9(2):35-45.; Ochi K, et al. J Hum Genet 2003;48(4):177-82.), (III) Validating clinicopathological significance of their over-expression by means of tissue microarray containing hundreds of archived lung-cancer samples (Suzuki C, et al. Cancer Res. 2005; 65, 11314-25.; Ishikawa N, et al. 2004 Dec 15;10(24):8363-70., Cancer Res. 2005; 65, 9176-84.; Kato T, et al. Cancer Res. 2005;65(13):5638-46.; Furukawa C, et al. Cancer Res. 2005; 65, 7102-10.), (IV) Verifying whether the target gene is essential for growth or the survival of cancer cells by RNAi assay (Suzuki C, et al. Cancer Res 2003;63:7038-41., Cancer Res. 2005; 65, 11314-25.; Kato T, et al. Cancer Res. 2005;65(13):5638-46.; Furukawa C, et al. Cancer Res. 2005; 65, 7102-10.).

Using this approach the present inventors have shown here that NPTX1 is frequently over-expressed in clinical lung-cancer samples, and cell lines, and that those gene products play indispensable roles in the growth and progression of lung-cancer cells.

NPTX1 encodes a 430 amino acids protein of a subfamily of "long pentraxin", with an amino-terminal coiled-coil domains and a carboxy-terminal pentraxin domain (Goodman AR, et al. Cytokine Growth Factor Rev. 1996 Aug;7(2):191-202.; Dodds DC, et al. 1997 Aug 22;272(34):21488-94.). The pentraxin domain on neuronal pentraxins is similar to the mammalian C-reactive protein and serum amyloid protein (Goodman *et al. supra.).* NPTX1 shares these structural features with NPTX2, and both NPTX1 and NPTX2 are secreted glycoproteins expressed exclusively in the CNS (Schlimgen AK, et al. Neuron. 1995 Mar;14(3):519-26.; Goodman AR, *et al. supra.;* Dodds DC, *et al.* 1 *supra.).*

NPTX1 and NPTX2 have been reported to form a heterocomplex and play some roles in activity-dependent synoptic plasticity (Xu DS). Complex formation is dependent on their distinct N-terminal coiled-coil domains, while their closely homologous C-terminal pentraxin domains mediate association with AMPA-type glutamate receptors (O'Brien RJ et al., Neuron. 1999 Jun;23(2):309-23., O'Brien R, et al. J Neurosci. 2002 Jun 1;22(11):4487-98.). Several observations have suggested that the overexpression of NPTX1 in cerebullar granule neurons undergoing apoptosis (DeGregorio-Rocasolano N), (Enguita M). Both the c-Jun NH2-terminal kinase (JNK) and glycogen synthase kinase 3 (GSK3) signaling pathways are the main routes by which potassium deprivation activates apoptotic cell death.

In fact, NPTX1 is induced in hypoxic-ischemic brain injury and that antisense oligonucleotides directed against NPTX1 mRNA prevent hypoxia- and AMPA-induced neuronal death (Hossain MA). It was also found that NPTX1 co-localizes with AMPA glutamate receptor subunit (GluR1) clusters and that hypoxia induces a time-dependent increase in NPTX1-GluR1 interactions. These results suggest a novel mechanism by which NPTX1 might accentuate excitoxicity through interactions with GluR1 and potentially other glutamate receptor subtypes. In spite of the known cadence that NPTX1 in accord with its functional partnership indispensable for neuron homeostasis, the expression pattern of NPTX1 was not necessarily concordant with that of NPTX2, NPTXR and AMPA-type receptors (GluR1-4) (data not shown) by semi-quantitative RT-PCR analysis, suggesting that there might be another pathway involving NPTX1 in lung carcinogenesis. Additional studies to identify molecular mechanism of NPTX1 in lung carcinogenesis may contribute should yield new understanding of the signaling pathway mediated by NPTX1 expression.

The present inventors demonstrated that NPTX1 protein was over-expressed in 37.2 % of surgically resected specimens from NSCLC patients, while NPTX1 protein was over-expressed in 69.2 % of that from SCLC patients. Treatment of NSCLC cells with specific siRNA to reduce expression of NPTX1 resulted in growth suppression. In addition, clinicopathological evidence obtained through tissue-microarray experiments demonstrated that NSCLC patients with tumors expressing NPTX1 showed shorter cancer-specific survival periods than those with negative NPTX1 expression. The results obtained by in vitro and in vivo assays show that over-expressed NPTX1 is an important growth factors and is associated with cancer cell growth, inducing a highly malignant phenotype of lung-cancer cells.

To demonstrate the feasibility of applying NPTX1 as the diagnostic tool, the present inventors compared serum levels of NPTX1 with those of CEA or ProGRP, a conventional diagnostic markers for NSCLCs and SCLCs, in terms of sensitivity and specificity for diagnosis. An assay combining both markers (NPTX1+CEA or NPTX1+ProGRP) increased the sensitivity such that about 69.2 - 76.9% of the patients with lung cancer were diagnosed as positive while 7.7 - 12.2% of healthy volunteers were falsely diagnosed as positive. The data presented here sufficiently demonstrate a clinical application of NPTX1 itself as a serological/histochemical marker for lung cancers.

### FGFROP1

Molecular-targeted therapies are expected to be highly specific to malignant cells, with minimal adverse reactions due to their well-defined mechanisms of action. Equally desirable prospects are minimally invasive and highly sensitive and specific new diagnostic methods that would adapt readily to clinical settings. As an approach to that goal, the present inventors have undertaken a strategy that combines screening of candidate molecules by genome-wide expression analysis with high-throughput screening of loss-of-function effects, using the RNAi technique (Suzuki C, et al. Cancer Res 2003;63:7038-41., Cancer Res 2005;65:11314-25.; Ishikawa N, et al. Clin Cancer Res 2004;10:8363-70., 2005 Cancer Res 2005;65:9176-9184; Kato T, et al. Cancer Res 2005;65:5638-46.; Furukawa C, et al. Cancer Res 2005;65:7102-10.).

In addition, the present inventors have been using the tissue-microarray method to analyze hundreds of archived clinical samples for validation of potential target proteins (Suzuki C, et al. Cancer Res 2005;65:11314-25.; Ishikawa N, et al. Clin Cancer Res 2004;10:8363-70., 2005 Cancer Res 2005;65:9176-9184; Kato T, et al. Cancer Res 2005;65:5638-46.; Furukawa C, et al, Cancer Res 2005;65:7102-10.). Using this combined approach, the present inventors have shown here that FGFR1OP is frequently over-expressed in clinical lung-cancer samples, and cell lines, and that the gene product plays indispensably roles in the growth and progression of lung-cancer cells.

FGFR1OP protein encodes 399 amino acids with a LisH domain. It is suggested that LisH motifs contribute to the regulation of microtubule dynamics, either by mediating dimerization, or else by binding cytoplasmic dynein heavy chain or microtubules directly (Sapir T, et al. Eur J Biochem 1999;265:181-8.; Cabana A. et al. Proc Natl Acad Sci U S A 2001;98:6429-34.). A t(6;8)(q27;p11) chromosomal translocation, fusing FGFR1OP gene and the FGFR1 gene, has been found in cases of myeloproliferative disorder (Popovici C, et al. Blood 1999;93:1381-9.; Guasch G, et al. Mol Cell Biol 2001;21:8129-42., Blood 2004;103:309-12.). The resulting chimeric protein contains the N-terminal leucine-rich region of this encoded protein fused to the catalytic domain of FGFR1. LisH domain FGFR1OP-FGFR1 fusion kinase targets the centrosome, activates signaling pathways at this organelle, and sustains continuous entry in the cell cycle (Delaval B, et al. Cancer Res 2005;65:7231-40.).

However, it is possible that FGFR1OP merely contributes an activating dimerization domain to the FGF-receptor kinase. In the present invention, no expression of chimeric FGFR1OP-FGFR1 was detectable (data not shown), and the expression pattern of FGFR was not concordant with that of FGFR1OP (data not shown), indicating that FGFR1OP-FGFR1 fashion kinase could not play a major role(s) in lung carcinogenesis.

The subcellular localization of FGFR1 OP fusion kinase proteins has been studied however, often in transfected cells with high levels of expression or in hematopoietic cells of MPD mouse model; FGFR1OP proteins are found predominantly in the centrosome (Andersen JS, et al. Nature 2003:426:570-4.; Yan X, et al. Mol Biol Cell 2006; 17:634-44.; Delaval B, et al. Cancer Res 2005;65:7231-40.). In the experiments, FGFR1OP localized not only in the centrosome (Fig. 1D), but also the nucleus and cytoplasm of lung cancer cells. In this study, the present inventors demonstrated that FGFR1OP was highly expressed in a great majority (90%) of surgically resected NSCLC specimens. Treatment ofNSCLC cells with specific siRNA to reduce expression of FGFR1OP resulted in growth suppression. The present inventors also found other evidence supporting the significance of this pathway in carcinogenesis; *e.g*., the expression of FGFR1OP also resulted in the significant promotion of the cell growth in *in vitro* assays. The LIS1 protein containing LisH domain as well as FGFR1OP associated dynein and dynactin. These proteins are enriched at the leading cell edge during healing of wounded NIH3T3 cell monolayers and subsequent cell migration. Inhibition of these proteins interfere not only with reorientation of the microtubule network, but also with persistent directed cell migration as well (Dujardin DL, et al. J Cell Biol. 2003 Dec 22;163(6):1205-11.). Similarly, our treatment of NSCLC cells with specific siRNA to reduce expression of FGFR1OP resulted in interfere with re-orientation of the microtubule network (data not shown).

Moreover, clinicopathological evidence obtained through the tissue-microarray experiments demonstrated that NSCLC patients with tumors strongly expressing FGFR1OP showed shorter cancer-specific survival periods than those with negative/weak FGFR1OP expression. This is the first study to show prognostic value of FGFR1OP expression in human cancers. Although the precise mechanism of FGFR1OP in lung carcinogenesis is unknown, the results obtained by in vitro and in vivo assays strongly suggested that over-expressed FGFR1OP is likely to be an important growth factor, inducing a highly malignant phenotype of lung-cancer cells. In the previous application, the present inventors found the over-expression of FGFR1OP in more than half if bladder cancer, cervical cancer, prostate cancer, renal cell cancer and osteosrcoma (data not shown). This suggest that over-expression of FGFR1OP might play a significant role in progression of various types of cancer as well.

The present inventors further found WRNIP1 and ABL1 as a novel molecule interacting with FGFR1OP, Interestingly, immunocytochemical analyses revealed co-localization of FGFR1OP with its interacting proteins, WRNIP1 and ABL1, mainly in perinucleus as well as in nucleus of NSCLC cells at S-G2/M phases (Fig. 1D), The results may suggest the significant role(s) of FGFR1OP through interaction with WRNIP1/ABL1 at this phase during cancer cell cycle progression. WRNIP1 is known to interact with the N-terminal portion of WRN. Sgs1 and Mgs1, the homologue of WRN and WRNIP1, have been identifies in budding yeast, respectively (Gangloff S, et al. Mol Cell Biol 1994;14:8391-8.; Hishida T, et al. Proc Natl Acad Sci USA 2001;98:8283-9.).

Previously, dismptants of the Sgs1 have shown an accelerated aging phenotype, and disruptants of the Mgs1 in wild-type cells and Sgs1 disruptants have resulted in slightly accelerated aging and enhancement of the premature aging phenotype of Sgs1 disruptants, respectively (Kawabe Yi, et al. J Biol Chem 2001;276:20364-9.). In yeast, Mgs1 has a tight functional interaction with DNA polymerase δ. A null Mgs1 mutations partially alleviates growth defects of pol31 mutant yeast, which bear a mutation in the second subunit of DNA polymerase δ (Brauzei D, et al. Mol Genet Genomics 2002;268:371-86.). These reports indicate that Mgs1 interact with the DNA replication machinery to modulate the function of DNA polymerase δ (POLD) during DNA replication or replication-associated repair.

Recently it was shown that human WRNIP1 forms homo-oligomeric complexes that physically interact with POLD and stimulate its DNA synthesis activity, mainly by increasing the frequency of initiation (Tsurimoto T, et al. Genes Cells 2005 Jan; 10:13-22.). The present inventors hypothesize that association of FGFR1OP with WRNIP1 may regulate DNA polymerase and activation of DNA synthetases, and subsequent up-regulation of tumor cell growth.

On the other hand, the ubiquitously expressed ABL1 is non-receptor tyrosine kinase distributed in the nucleus and cytoplasm (Wen ST, et al. EMBO J. 1996 Apr 1;15(7):1583-95.; Taagepera S, et al. Proc Natl Acad Sci U S A. 1998 Jun 23;95(13):7457-62.). Nuclear ABL1 is activated by genotoxic stress, including DNA double strand breaks and cross-links, inducts apoptosis (Kharbanda et al., 1995; Yuan et al., 1997). Activation of nuclear ABL1 by DNA damage contributes to apoptosis by mechanism that partly depend on p53, p73 and Rad9 (Kharbanda S, et al. Nature. 1995 Aug 31;376(6343):785-8.; Gong JG, et al. Nature. 1999 Jun 24;399(6738):806-9.; Yoshida K, et al. Mol Cell Biol. 2002 May;22(10):3292-300.; Yuan ZM, et al. Nature. 1999 Jun 24;399(6738):814-7., Pros Natl Acad Sci U S A. 1997 Feb 18;94(4):1437-40.). In addition cellular responses to DNA damage involve interaction of ABL1 with DNA repair proteins, including Rad51, Rad52, BRCA1, and a UV-damaged DNA binding protein (Cong F, et al. J Biol Chem. 2002 Sep 20;277(38):34870-8. Epub 2002 Jul 9.; Foray N, et al. Mol Cell Biol. 2002 Jun;22(12):4020-32.; Kitao H & Yuan ZM. J Biol Chem. 2002 Dec 13;277(50):48944-8. Epub 2002 Oct 11.; Yuan ZM, et al. J Biol Chem. 1998 Feb 13;273(7):3799-802.). Also, tyrosine phosphorylation by ABL1 plays important regulatory roles in the DNA damage response, previous reports have been shown that phosphorylation of Rad51 by ABL1 inhibits its strand exchange activity (Yuan ZM, et al. J Biol Chem. 1998 Feb 13;273(7):3799-802.), and that BRCA1 is phosphorylated by ABL1 in an ATM dependent manner (Foray N, et al. Mol Cell Biol. 2002 Ju;22(12):4020-32). Thus, ABL1 appears to function in coordinating recombinational DNA repair with the induction of apoptosis. Recent study, also, has identified that WRN is phosphorylated by ABL1, its tyrosine phosphorylation inhibits of both WRN exonuclease and helicase activities. Previous reports, WRN, WRNIP1 and POLD may form a ternary complex, function to regulate POLD-mediated DNA synthesis when the replication for complex is stalled by DNA damage or structural stress (Kawabe Yi, et al. J Biol Chem. 2001 Jun 8;276(23):20364-9. Epub 2001 Apr 11.; Tsurimoto T, et al. Genes Cells. 2005 Jan;10(1):13-22.). While WRNIP1 can be serine-and tyrosine-phosphorylated (Beausoleil SA, et al. Proc Natl Acad Sci U S A. 2004 Aug 17;101(33):12130-5. Epub 2004 Aug 9.; Foray N, et al. Mol Cell Biol. 2002 Jun;22(12):4020-32.; Kitao H & Yuan ZM. J Biol Chem. 2002 Dec 13;277(50):48944-8. Epub 2002 Oct 11.; Yuan ZM, et al. J Biol Chem. 1998 Feb 13;273(7):3799-802.), the kinase which phosphorylates WRNIP1 directly has not been reported. In present invention, it have provided the first evidence that WRNIP1 tyrosine is phosphorylated by ABL1 in vivo and *in vitro.*

In summary, activation of KIF4A has a specific functional role for growth and/or malignant phenotype of cancer cells. These data allow for the design of new anti-cancer drugs to specifically target the oncogenic activity of KIF4A and/or the KIF4A-interacting protein complex that is essential for cancer cell growth. Thus, the present invention provides a new therapeutic and diagnostic strategy for treatment of lung-cancer patients.

In summary, the present inventors demonstrated that MAPJD is involved in the gene transcription as a member of a MYC transcriptional complex. Since MAPJD is essential for promotion of growth of lung cancer, it is a novel therapeutic target for development of anti-cancer drugs.

In conclusion, the present inventors have identified NP1X1 as targets for development of diagnostic and prognostic makers as well as novel therapeutic drugs for lung cancer.

In summary, the present inventors have shown that over-expressed FGFR1OP is an essential contributor to a growth-promoting pathway and to aggressive features of NSCLCs. The data reported here allow for the design biomarkers and anti-cancer drugs specific for lung cancer, targeting the FGFR1OP molecule. Furthermore, it was indicated that over-expressed FGFR1OP significantly reduced ABL1-dependent phosphorylation of WRNIP1 and resulted in the cell cycle progression of lung tumors, and that FGFR1OP could be an essential contributor to aggressive features of lung cancers.

### Industrial Applicability

The gene expression analysis of small-cell lung carcinomas (SCLCs) and non-small cell lung cancers (NSCLCs) described herein, obtained through a combination of laser-capture dissection and genome-wide cDNA microarray, has identified specific genes as targets for lung cancer prevention and therapy. Based on the expression of a subset of these differentially expressed genes, the present invention provides molecular diagnostic markers for identifying and detecting lung cancer.

The methods described herein are also user in the identification of additional molecular targets for prevention, diagnosis and treatment of lung cancer. The data reported herein add to a comprehensive understanding of lung cancer, facilitate development of novel diagnostic strategies, and provide clues for identification of molecular targets for therapeutic drugs and preventative agents. Such information contributes to a more profound understanding of lung tumorigenesis, and provides indicators for developing novel strategies for diagnosis, treatment, and ultimately prevention of lung cancer.

Furthermore, the methods described herein are also useful in diagnosis of lung cancer including small-cell lung carcinomas (SCLCs) and non-small cell lung cancers (NSCLCs) as well as prediction of the poor prognosis of the patients with these diseases. Moreover, the present invention also provides useful candidates for development of therapeutic approaches for cancer including lung cancers.

As demonstrated herein, KIF4-A, MAPJD, and FGFR1OP interacts with ZNF553, MYC, and WRNIP1 respectively, and the inhibition, of the interaction leads to the inhibition of cell proliferation of lung cancer cells. Thus, agents that inhibit the binding of KIF4A/ZNF549, KIF4A/ZNF553, MAPJD/MYC, FGFR1OP/WRNIP1, or FGFR1OP/ABL1 and prevent its activity find therapeutic utility as anti-cancer agents, particularly anti-cancer agents for the treatment of lung cancers. Furthermore, the present invention reveals that MAPJD associated with HAT complex has activity to acetylate histone H4. MAPJD associated with HAT complex also binds to E-box which are found in the 5' flanking region of SBNO1, TGFBRAP1, RIOK1, and RASGEF1A gene. Accordingly, agents that inhibit the acetylation activity or the binding of MAPJD associated with HAT complex and prevent its activity find therapeutic utility as anti-cancer agents, particularly anti-cancer agents for the treatment of lung cancers. Furthermore, the present invention reveals that ABL1 associated with FGFR1OP has activity to phosphorylate WRNIP1. Accordingly, agents that inhibit the phosphorylation activity of ABL1 associated with FGFR1OP or the binding of FGFR1OP/ABL1or FGFR1OP/WRNIP1, and prevent its activity find therapeutics utility as anti-cancer agents, particularly anti-cancer agents for the treatment of lung cancers.

Unless otherwise defined, all technical and scientific terms used herein have the same meaning as commonly understood by one of ordinary skill in the art to which this invention belongs. In case of conflict, the present specification, including definitions, will control.

All publications, databases, Genbank sequence, patent applications, patents and other references mentioned herein are incorporated by reference in their entirety.

While the invention has been described in detail and with reference to specific embodiments thereof, it will be apparent to one skilled in the art that various changes and modifications can be made therein without departing from the spirit and scope of the invention.

Furthermore, the present invention relates to the following items:
1. A method for diagnosing lung cancer or a predisposition for developing lung cancer in a subject, comprising the step of determining the expression level of the *KIF4A* gene in a subject-derived biological sample, wherein an increase in said expression level as compared to a normal control level of said gene indicates that said subject suffers from or is at a risk of developing lung cancer.
2. The method of item 1, wherein said expression level is at least 10% greater than the normal control level.
3. The method of item 1, wherein said expression level is determined by any of the methods selected from the group consisting of:
   (a) detecting mRNA of the *KIF4A* gene;
   (b) detecting a protein encoded by the KIF4A gene, and
   (c) detecting a biological activity of the protein encoded by the *KIF4A* gene.
4. The method of item I, wherein said subject-derived biological sample comprises an epithelial cell.
5. The method of item 1, wherein said subject-derived biological sample comprises a cancer cell.
6. The method of item 1, wherein said subject-derived biological sample comprises a cancerous epithelial cell.
7. The method of item I, wherein said cancer is SCLCs and NSCLCs.
8. A kit comprising a detection reagent which binds to the transcription or translation product of the *KIF4A* gene.
9. A method for diagnosing lung cancer in a subject, comprising the steps of:
   (a) collecting a blood sample from a subject to be diagnosed;
   (b) determining a level of *NPTX1* in the blood sample;
   (c) comparing the *NPTX1* level determined in step (b) with that of a normal control; and
   (d) judging that a high *NPTX1* level in the blood sample, compared to the normal control, indicates that the subject suffers from cancer.
10. The method of item 9, wherein the cancer is SCLCs and NSCLCs.
11. The method of item 9, wherein the blood sample is selected from the group consisting of whole blood, serum, and plasma.
12. The method of item 9, wherein the *NPTX1* level is determined by detecting the *NPTX1* protein in the serum.
13. The method of item 9, wherein the *NPTX1* protein is detected by immunoassay.
14. The method of item 3, wherein the immunoassay is an ELISA.
15. The method of item 13, wherein the immunoassay is sandwich method which uses an anti- *NPTX1* polyclonal antibody as a detection antibody.
16. The method of item 9, wherein the method further comprises the step of
   (e) determining an either of level of CEA and an level of proGRP, or both in the blood sample;
   (f) comparing either of CEA level and proGRP level, or both level determined in step (b) with that of a normal control; and
   (g) judging that a high CEA level in the blood sample, compared to the normal control, indicates that the subject suffers from NSCLC, and a high proGRP level in the blood sample, compared to the normal controls, indicates that the subject suffers from SCLC.
17. A kit for detecting a lung cancer, wherein the kit comprises:
   (a) an immunoassay reagent for determining a level of *NPTX1* in a blood sample; and
   (b) a positive control sample for *NPTX1.*
18. The kit of item 17, wherein the lung cancer is SCLCs and NSCLCs.
19. The kit of item 17, wherein the positive control sample is positive for *NPTX1.*
20. The kit of item 17, wherein the positive control sample is liquid form.
21. A double-stranded molecule comprising a sense strand and an antisense strand, wherein the sense strand comprises a ribonucleotide sequence corresponding to a target sequence selected from the group consisting of SEQ ID NOs: 32, 33, 36, and 37, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the *KIF4A*, *NPTX1 or FGFR1OP* gene, inhibits expression of said gene.
22. A vector encoding the double-stranded molecule of item 21.
23. The vector of item 22, wherein the vector encodes a transcript having a secondary structure and comprises the sense strand and the antisense strand.
24. The vector of item 23, wherein the transcript further comprises a single-stranded ribonucleotide sequence linking said sense strand and said antisense strand.
25. A vector comprising a polynucleotide comprising a combination of a sense strand nucleic acid and an antisense strand nucleic acid, wherein said sense strand nucleic acid comprises nucleotide sequence of SEQ ID NOs: 32, 33,36, and 37, and said antisense strand nucleic acid consists of a sequence complementary to the sense strand.
26. The vector of item 25, wherein said polynucleotide has the general formula 5'-[A]-[B]-[A']-3' formula
   wherein [A] is a nucleotide sequence of SEQ ID NOs: 32, 33, 36, and 37; [B] is a nucleotide sequence consisting of 3 to 23 nucleotides; and [A'] is a nucleotide sequence complementary to [A].
27. A pharmaceutical composition for treating or preventing lung cancer comprising a pharmaceutically effective amount of a small interfering RNA (siRNA) that inhibits expression of *KIF4A, NPTX1, FGFR1OP,* or *WRNIP1* as an active ingredient, and a pharmaceutically acceptable carrier.
28. The pharmaceutical composition of item 27, wherein the siRNA comprises a nucleotide sequence selected from the group consisting of SEQ ID NOs: 32, 33, 36, 37, 96 and 97 as the target sequence.
29. The composition of item 36, wherein the siRNA has the general formula

   5'-[A]-[B]-[A']-3'

   wherein [A] is a ribonucleotide sequence corresponding to a nucleotide sequence of SEQ ID NOs: 32,33,36,37,96 and 97; [B] is a ribonucleotide sequence consisting of 3 to 23 nucleotides; and [A'] is a ribonucleotide sequence complementary to [A].
30. The composition of any one of items 27 to 29, wherein the lung cancer is SCLCs and NSCLCs.
31. A method for assessing the prognosis of a patient with cancer, which method comprises the steps of:
   (a) detecting the expression level of *KIF4A, NPTX1* or *FGFR1OP* gene in a patient-derived biological sample;
   (b) comparing the detected expression level to a control level; and
   (c) determining the prognosis of the patient based on the comparison of (b).
32. The method of item 31, wherein the control level is a good prognosis control level and an increase of the expression level compared to the control level is determined as poor prognosis.
33. The method of item 32, wherein the increase is at least 10% greater than said control level.
34. The method of item 33, wherein said method comprises determining the expression level of other cancer-associated genes.
35. The method of item 34, wherein said expression level is determined by any one method selected atom the group consisting of
   (a) detecting mRNA of the *KIF4A, NPTX1* or *FGFR1OP* gene;
   (b) detecting the KIF4A, NPTX1 or FGFR1OP protein; and
   (c) detecting the biological activity of the KIF4A, NPTX1 or FGFR1OP protein.
36. The method of item 35, wherein said expression level is determined by detecting hybridization of a probe to a gene transcript of the *KIF4A, NPTX1* or *FGFR1OP* gene.
37. The method of item 36, wherein the hybridization step is carried out on a DNA array,
38. The method of item 31, wherein said expression level is determined by detecting the binding of an antibody against the KIF4A. NPTX1 or FGFR1OP protein as the expression level of the *KF4A, NPTZX1* or *FGFR1OP* gene.
39. The method of item 31, wherein said biological sample comprises sputum or blood.
40. The method of item 31, wherein the cancer is lung cancer.
41. A kit for assessing the prognosis of a patient with cancer which comprise a reagent selected from the group consisting of:
   (a) a reagent for detecting mRNA of the *KIF4A, NPTX1 or FGFR1OP* gene;
   (b) a reagent for detecting the KIF4A, NPTX1 or FGFR1OP protein; and
   (c) a reagent for detecting the biological activity of the KIF4A, NPTX1 or FGFR1OP protein.
42. The kit of item 41, wherein the reagent is an antibody against the KIF4A, NPTX1 and FGFR1 OP protein.
43. The kit of item 41, wherein the cancer is lung cancer.
44. A methods of screening for an inhibitor of a binding, the methods comprise the steps of:
   (1) contacting a polypeptide comprising an partner molecule-binding domain of a target molecule with a polypeptide comprising an target molecule-binding domain of a partner molecule in the presence of a test compound;
   (2) detecting binding between the peptides; and
   (3) selecting a test compound that inhibits the binding;
   wherein a combination of the target molecule and the partner molecule thereof is selected from the group consisting of KIF4A/ZNF549, KIF4A/ZNF553, MAPJD/MYC, FGFR1OP/WRNIP1, FGFR1OP/ABL1, and FGFR1OP/WRNIP/ABL1.
*45.* A methods of screening for compounds that are useful for treating or preventing lung cancer, the methods comprise the steps of:
   (1) contacting a polypeptide comprising an partner molecule-binding domain of a target molecule with a polypeptide comprising an target molecule-binding domain of a partner molecule in the presence of a test compound;
   (2) detecting binding between the peptides; and
   (3) selecting a test compound that inhibits the binding;
   wherein a combination of the target molecule and the partner molecule thereof is selected from the group consisting of KIF4A/ZNF549, KIF4A/ZNF553. MAPJD/MYC, FGFR1OP/WRNIP1, FGFR1OP/ABL1, and FGFR1OP/WRNIP1/ABL1.
46. The method of item 44 or 45, wherein the polypeptide comprising the partner molecule-binding domain comprises any one peptide selected from the group consisting of KIF4A polypeptide, MAPJD polypeptide, and FGFR1OP polypeptide.
47. The method of item 44 or 45, wherein the polypeptide composing the target molecule-binding domain comprises any one peptide selected from the group consisting of ZNF549 polypeptide, ZNF553 polypeptide. MYC polypeptide. WRNIP1 polypeptides, and FGFR1OP polypeptide.
48. A kit for screening for an inhibitor of a binding, the kit comprises:
   (a) a polypeptide comprising an partner molecule-binding domain of a target molecule;
   (b) a polypeptide comprising an target molecule-binding domain of a partner molecule,; and
   (c) reagent to detect the interaction between the polypeptides,
   wherein a combination of the target molecule and the partner molecule thereof is selected from the group consisting of KIF4A/ZNF549; KIF4A/ZNF5S3, MAPJD/MYC, FGFR1OP/WRNIP1, FGFR1OP/ABL1, and FGFR1OP/WRNIP1/ABL1.
49. A kit for screening for a compound useful in treating or preventing lung cancer, the kit comprises:
   (a) a polypeptide comprising an partner molecule-binding domain of a target molecules ;
   (b) a polypeptide comprising an target molecule-binding domain of a partner molecule; and
   (c) reagent to detect the interaction between the polypeptides,
   wherein a combination of the target molecule and the partner molecule thereof is selected from the group consisting of KIF4A/ZNF549, KIF4A/ZNF553, MAPJD/MYC, FGFR1OP/WRNIP1, FGFR1OP/ABL1. and FGFR1OP/WRNIP1/ABL1.
50. The kit of item 48 or 49, wherein the polypeptide comprising the partner molecule-binding domain comprises any one peptide selected from the group consisting of KIF4A polypeptide, MAPJD polypeptide, and FGFR1OP polypeptide.
51. The kit of item 48 or 49, wherein the polypeptide comprising the target molecule-binding domain comprises any one peptide selected from the group consisting of ZNF549 polypeptide, ZNF553 polypeptide, MYC polypeptide, WRNIP1 polypeptide, and ABL1 polypeptide.
52. A methods of screening for a modulator of a MAPJD/HAT complex-mediated acetylation, the methods comprise the steps of:
   (1) contacting a test compound to a MAPJD polypeptide associated with a HAT complex and a substrate that is acetylated by the polypeptide under the condition capable of acetylation of the substrate;
   (2) detecting a acetylation level of the substrate; and
   (3) selecting the test compound that decreases the acetylation level of the substrate to be acetylated as compared to a control acetylation level detected in the absence of the test compound as an inhibitor; selecting the test compound that increases the acetylation level of the substrate to be acetylated as compared to a control acetylation level detected in the absence of the test compound as an enhancer.
53. A methods of screening for compounds that are useful for treating or preventing lung cancer, the methods comprise the steps of:
   (1) contacting a test compound to a MAPJD polypeptide associated with a HAT complex and a substrate that is acetylated by the polypeptide under the condition capable of acetylation of the substrate;
   (2) detecting a acetylation level of the substrate; and
   (3) selecting the test compound that decreases the acetylation level of the substrate to be acetylated as compared to a control acetylation level detected in the absence of the test compound.
54. The method of item 52 or 53, wherein the substrate to be acetylated is histone H4.
55. The method of item 52 or 53, wherein the MAPJD polypeptide and the HAT complex are expressed in a living cell.
56. A kit for screening for a modulator of a MAPJD/HAT complex-mediated acetylation, wherein the kit comprises:
   (a) a cell expressing an MAPJD polypeptide and a HAT complex, and
   (b) reagent to detect the acetylation level of histone H4.
57. A kit for screening for a compound useful in treating or preventing lung cancer, wherein the kit comprises:
   (a) a cell expressing an MAPJD polypeptide and a HAT complex, and
   (b) reagent to detect the acetylation level of histone H4,
58. A Methods of screening for an inhibitor for a binding between E-box motif and a complex selected from the group consisting of MAPJD/HAT complex and MAPJD/MYC complex, the methods comprise the steps of:
   (1) contacting a test compound to a MAPJD polypeptide or functional equivalent thereof associated with a HAT complex or MYC and polynucleotide comprising E-box motif;
   (2) detecting a binding between the polypeptide and the polynucleotide; and
   (3) selecting a test compound that inhibits the binding. -
59. A methods of screening for compounds that are useful for treating or preventing lung cancer, the methods comprise the steps of:
   (1) contacting a test compound to a MAPJD polypeptide or functional equivalent thereof associated with a HAT complex or MYC and polynucleotide comprising E-box motif;
   (2) detecting a binding between the polypeptide and the polynucleotide; and
   (3) selecting a test compound that inhibits the binding.
60. The method of item 58 or 59, wherein the polynucleotide is a vector comprising the transcriptional regulatory region and an reporter gene that is expressed under the control of the transcriptional regulatory region, wherein the transcriptional regulatory region comprises the E-box motif, and wherein the binding is detected by measuring the expression level or activity of said reporter gene.
61. The method of item 58 or 59, wherein the polynucleotide comprises the nucleotide sequence selected from the 5' flanking region of a gene selected from the group consisting of SBNO1, TGFBRAP1, RIOK1, and RASGEF1A.
62. A kit for screening for an inhibitor for a binding between E-box motif and a complex selected from the group consisting of MAPJD/HAT complex and MAPJD/MYC complex, wherein the kit comprises:
   (a) a cell expressing an MAPJD polypeptide or functional equivalent thereof and a HAT complex or MYC, wherein the cell is transfected with a vector comprising the transcriptional regulatory region and an reporter gene that is expressed under the control of the transcriptional regulatory region, wherein the transcriptional regulatory region comprises the E-box motif, and
   (b) reagent to detect the expression level or the activity of the reporter gene.
63. A kit for screening for a compound useful in treating or preventing lung cancer, wherein the kit comprises:
   (a) a cell expressing an MAPJD polypeptide or functional equivalent thereof and a HAT complex or MYC, wherein the cell is transfected with a vector comprising the transcriptional regulatory region and an reporter gene that is expressed under the control of the transcriptional regulatory region, wherein the transcriptional regulatory region comprises the E-box motif, and
   (b) reagent to detect the expression level or the activity of the reporter gene.
64. A methods of screening for a modulator of ABL1-mediated phosphorylation of WRNYP1, the methods comprise the steps of:
   (a) contacting a test compound to a FGFR1OP polypeptide or functional equivalent thereof, WRNIP1 polypeptide or functional equivalent thereof and an ABL1 polypeptide or functional equivalent thereof in the suitable condition for phosphorylation of WRNIP1 polypeptide;
   (b) detecting a phosphorylation level of the WRNIP1 polypeptide or functional equivalent thereof; and
   (c) selecting the test compound that increases the phosphorylation level detected in step (b) to be phosphorylated as compared to a control phosphorylation level detected in the absence of the test compound as an enhancer; selecting the test compound that decreases the phosphorylation level detected in step (b) to be phosphorylated as compared to a control phosphorylation level detected in the absence of the test compound as an inhibitor.
65. A methods of screening for compounds that are useful for treating or preventing lung cancer, the methods comprise the steps of:
   (a) contacting a test compound to a FGFR1OP polypeptide or functional equivalent thereof, WRNIP1 polypeptide or functional equivalent thereof and an ABL1 polypeptide or functional equivalent thereof in the suitable condition for phosphorylation of WRNIP1 polypeptide;
   (b) detecting a phosphorylation level of the WRNIP1 polypeptide or functional equivalent thereof; and
   (c) selecting the test compound that increases the phosphorylation level detected in step (b) to be phosphorylated as compared to a control phosphorylation level detected in the absence of the test compound.
66. A methods of screening for a modulator of ABL1-mediated phosphorylation of WRNIP1, the methods comprise the steps of:
   (a) contacting a test compound to a cell expressing an FGFR1OP polypeptide or functional equivalent thereof, a WRNIP1 polypeptide or functional equivalent thereof and a ABL1 polypeptide or functional equivalent thereof,
   (b) detecting a phosphorylation level of the WRNIP1 polypeptide or functional equivalent thereof, and
   (c) selecting the test compound that increases the phosphorylation level detected in step (b) to be phosphorylated as compared to a control phosphorylation level detected in the absence of the test compound as an enhancer; selecting the test compound that decreases the phosphorylation level detected in step (b) to be phosphorylated as compared to a control phosphorylation level detected in the absence of the test compound as an inhibitor.
67. A methods of screening for compounds that are useful for treating or preventing lung cancer, the methods comprise the steps of:
   (a) contacting a test compound to a cell expressing an FGFR1OP polypeptide or functional equivalent thereof, a WRNIP1 polypeptide or functional equivalent thereof and a ABL1 polypeptide or functional equivalent thereof;
   (b) detecting a phosphorylation level of the WRNIP1 polypeptide or functional equivalent thereof; and
   (c) selecting the test compound that increases the phosphorylation level detected in step (b) to be phosphorylated as compared to a control phosphorylation level detected in the absence of the test compound.
68. A kit for screening for a modulator of ABL1-mediated phosphorylation of WRNIP1, wherein the kit comprises:
   (a) a cell expressing an FGFR1OP polypeptide or functional equivalent thereof, a WRNIP1 polypeptide or functional equivalent thereof and a ABL1 polypeptide or functional equivalent thereof, and
   (b) reagent to detect the phosphorylation level of the WRNIP1 polypeptide or functional equivalent thereof.
69. A kit for screening for a compound useful in treating or preventing lung cancer, wherein the kit comprises:
   (a) a cell expressing an FGFR1OP polypeptide or functional equivalent thereof, a WRNIP1 polypeptide or functional equivalent thereof and a ABL1 polypeptide or functional equivalent thereof, and
   (b) reagent to detect the phosphorylation level of the WRNIP1 polypeptide or functional equivalent thereof.
70. A method of treating or preventing lung cancer in a subject comprising administering to said subject an agent or compound selected from group consisting of:
   (a) agent that inhibits a binding between a target molecule and partner molecule, wherein a combination of the target molecule and the partner molecule thereof is selected from the group consisting of KIF4A/ZNF549, KIF4A/ZNF553, MAPJD/MYC, FGFR1OP/WRNIP1, and FGFR1OP/ABL1,
   (b) agent that inhibits an acetylation of histone H4 by MAPJD polypeptide associated with a HAT complex,
   (c) agent that inhibits a binding between a MAPJD polypeptide associated with a HAT complex or MYC and polynucleotide comprising E-box motif, and
   (d) agent that enhances a phosphorylation of WRBIP1 polypeptide by ABL1 polypeptide.
71. A composition for treating or preventing lung cancer, said composition comprising as an active ingredient a pharmaceutically effective amount of a compound selected by a method of any one of items 52, 58, 62, and 66, and a pharmaceutically acceptable carrier.
72. Use of agent or compound selected from group consisting of:
   (a) agent that inhibits a binding between a target molecule and partner molecule, wherein a combination of the target molecule and the partner molecule thereof is selected from the group consisting of KIF4A/ZNF549, KIF4A/ZNF553, MAPJD/MYC, FGFR1OP/WRNIP1, and FGFR1OP/ABL1,
   (b) agent that inhibits an acetylation of histone H4 by MAPJD polypeptide associated with a HAT complex.
   (c) agent that inhibits a binding between a MAPJD polypeptide associated with a HAT complex or MYC and polynucleotide comprising E-box motif, and
   (d) agent that enhances a phosphorylation of WRBIP1 polypeptide by ABL1 polypeptide.
73. An antibody produced by a method comprising the steps of,
   (a) immunizing an animal by an antigen peptide selected from the group consisting of SEQ ID NO: 98 (codons 7-173) of SEQ ID NO: 5 9 (FGFR1OP), (codons 20-145) of SEQ ID NO: 57 (NPTX1) or SEQ ID NO: 100 (codons 297-430) of SEQ ID NO: 57 (NPTX1); and
   (b) selected an antibody that specifically binds to the antigen peptide of step (a).

## Claims

1. A method for diagnosing lung cancer in a subject, comprising the steps of:
(a) collecting a blood sample from a subject to be diagnosed;
(b) determining a level of *NPTX1* in the blood sample;
(c) comparing the *NPTX1* level determined in step (b) with that of a normal control; and
(d) judging that a high *NPTX1* level in the blood sample, compared to the normal control, indicates that the subject suffers from cancer.

2. The method of claim 1, wherein the cancer is SCLCs or NSCLCs.

3. The method of claim 1 or 2, wherein the blood sample is selected from the group consisting of whole blood, serum, and plasma.

4. The method of any one of claims 1 to 3, wherein the *NPTX1* level is determined by detecting the *NPTX1* protein in the serum.

5. The method of claim 4, wherein the *NPTX1* protein is detected by immunoassay.

6. The method of claim 5, wherein the immunoassay is an ELISA.

7. The method of claim 5, wherein the immunoassay is sandwich method which uses an anti- *NPTX1* polyclonal antibody as a detection antibody.

8. The method of any one of claims 1 to 7, wherein the method further comprises the step of
(e) determining an either of level of CEA and an level of proGRP, or both in the blood sample;
(f) comparing either of CEA level and proGRP level, or both level determined in step (b) with that of a normal control; and
(g) judging that a high CEA level in the blood sample, compared to the normal control, indicates that the subject suffers from NSCLC, and a high proGRP level in the blood sample, compared to the normal control, indicates that the subject suffers from SCLC.

9. A kit for detecting a lung cancer, wherein the kit comprises:
(a) an immunoassay reagent for determining a level of *NPTX1* in a blood sample; and
(b) a positive control sample for *NPTX1.*

10. The kit of claim 9, wherein the lung cancer is SCLCs or NSCLCs.

11. The kit of claim 9 or 10, wherein the positive control sample is positive for *NPTX1.*

12. The kit of any one of claims 9 to 11, wherein the positive control sample is liquid form.


SEQUENCE LISTING

<110> ONCOTHERAPY SCIENCE, INC.

<120> Prognostic Markers and Therapeutic Targets for Lung Cancer

<130> R1301 EP S3

<140> EP 07 80 6302.1
<141> 2007-08-23

<150> US 60/840,376
<151> 2006-08-25

<150> US 60/894,801
<151> 2007-03-14

<160> 101

<170> PatentIn version 3.4

<210> 1
<211> 22
<212> DNA
<213> Artificial

<220>
<221> Source
<223> /note="Description of Artificial Sequence: An artificially synthesized
primer sequence for RT-PCR"

<400> 1
caaaaaccag cttcttctct gg 22

<210> 2
<211> 23
<212> DNA
<213> Artificial

<220>
<221> Source

<223>  /note="Description of Artificial Sequence: An artificially synthesized
primer sequence for RT-PCR"

<400>  2
caggaaagat cacaacctca ttc                                              23

<210>  3
<211>  20
<212>  DNA
<213>  Artificial

<220>
<221>  Source
<223>  /note="Description of Artificial Sequence: An artificially synthesized
primer sequence for RT PCR"

<400>  3
gcccagttaa tgggttttga                                                  20

<210>  4
<211>  20
<212>  DNA
<213>  Artificial

<220>
<221>  Source
<223>  /note="Description of Artificial Sequence: An artificially synthesized
primer sequence for RT-PCR"

<400>  4
cacgcctggc taatttttgt                                                  20

<210>  5
<211>  20
<212>  DNA
<213>  Artificial

<220>

<221>  Source
<223>  /note="Description of Artificial Sequence: An artificially synthesized
primer sequence for RT-PCR"

<400>  5
gagggagaag gaagggaaga                                                    20

<210>  6
<211>  21
<212>  DNA
<213>  Artificial

<220>
<221>  Source
<223>  /note="Description of Artificial Sequence: An artificially synthesized
primer sequence for RT-PCR"

<400>  6
aatgcctact gtg[?]gctagg c                                                21

<210>  7
<211>  21
<212>  DNA
<213>  Artificial

<220>
<221>  Source
<223>  /note="Description of Artificial Sequence: An artificially synthesized
primer sequence for RT-PCR"

<400>  7
aggagaagtt ggaggtggaa a                                                  21

<210>  8
<211>  23
<212>  DNA

<213>  Artificial

<220>
<221>  Source
<223>   /note="Description of Artificial Sequence: An artificially synthesized
primer sequence for RT-PCR"

<400>  8
cagatgaaag atccaaattc caa                                                23

<210>  9
<211>  20
<212>  DNA
<213>  Artificial

<220>
<221>  Source
<223>   /note="Description of Artificial Sequence: An artificially synthesized
primer sequence for RT-PCR"

<400>  9
ctgacagtgc atgtctttgg                                                    20

<210>  10
<211>  20
<212>  DNA
<213>  Artificial

<220>
<221>  Source
<223>   /note="Description of Artificial Sequence: An artificially synthesized
primer sequence for RT-PCR"

<400>  10
ttctgcagca cacattagga                                                    20

<210>  11
<211>  23
<212>  DNA

<213>  Artificial

<220>
<221>  Source
<223>  /note="Description of Artificial Sequence: An artificially synthesized
primer sequence for RT-PCR"

<400>  11
agagtatcac acccacttag ctg                                                23

<210>  12
<211>  26
<212>  DNA
<213>  Artificial

<220>
<221>  Source
<223>  /note="Description of Artificial Sequence: An artificially synthesized
primer sequence for RT-PCR"

<400>  12
gacaggtgaa ctcttgtatg tttctg                                             26

<210>  13
<211>  20
<212>  DNA
<213>  Artificial

<220>
<221>  Source
<223>  /note="Description of Artificial Sequence: An artificially synthesized
primer sequence for RT-PCR"

<400>  13
gaagacagcc aagacgaaaa                                                    20

<210>  14
<211>  20
<212>  DNA

<213>  Artificial

<220>
<221>  Source
<223>  /note="Description of Artificial Sequence: An artificially synthesized
primer sequence for RT-PCR"

<400>  14
tcctctgtca acaccagaca                                                    20

<210>  15
<211>  20
<212>  DNA
<213>  Artificial

<220>
<221>  Source
<223>  /note="Description of Artificial Sequence: An artificially synthesized
primer sequence for RT-PCR"

<400>  15
tttcccatgt ctgacttcgt                                                    20

<210>  16
<211>  20
<212>  DNA
<213>  Artificial

<220>
<221>  Source
<223>  /note="Description of Artificial Sequence: An artificially synthesized
primer sequence for RT-PCR"

<400>  16
caatgtcttc aggctcttcc                                                    20

<210>  17
<211>  22
<212>  DNA

<213>  Artificial

<220>
<221>  Source
<223>  /note="Description of Artificial Sequence: An artificially synthesized
primer sequence for RT-PCR"

<400>  17
ctgctggtac gtgtgatctt tg                                                22

<210>  18
<211>  25
<212>  DNA
<213>  Artificial

<220>
<221>  Source
<223>  /note="Description of Artificial Sequence: An artificially synthesized
primer sequence for RT-PCR"

<400>  18
accttaatgg tctaacaaac cttcc                                             25

<210>  19
<211>  23
<212>  DNA
<213>  Artificial

<220>
<221>  Source
<223>  /note="Description of Artificial Sequence: An artificially synthesized
primer sequence for RT-PCR"

<400>  19
taaccttgat agaagaacct tgg                                               23

<210>  20
<211>  22
<212>  DNA

<213>  Artificial

<220>
<221>  Source
<223>   /note="Description  of  Artificial  Sequence:  An  artificially  synthesized
primer sequence for RT-PCR"

<400>  20
gcaaatgaga caaaattggg ac                                                22

<210>  21
<211>  21
<212>  DNA
<213>  Artificial

<220>
<221>  Source
<223>   /note="Description  of  Artificial  Sequence:  An  artificially  synthesized
primer sequence for RT-PCR"

<400>  21
gaggtgatag cattgctttc g                                                 21

<210>  22
<211>  21
<212>  DNA
<213>  Artificial

<220>
<221>  Source
<223>   /note="Description  of  Artificial  Sequence:  An  artificially  synthesized
primer sequence for RT-PCR"

<400>  22
caagtcagtg tacaggtaag c                                                 21

<210>  23
<211>  21
<212>  DNA

<213>  Artificial

<220>
<221>  Source
<223>  /note="Description of Artificial Sequence: An artificially synthesized
primer for PCR"

<400>  23
cattctccac caaagtcacc a                                                21

<210>  24
<211>  17
<212>  DNA
<213>  Artificial

<220>
<221>  Source
<223>  /note="Description of Artificial Sequence: An artificially synthesized
primer for PCR"

<400>  24
cccgcttgtc ttcttct                                                     17

<210>  25
<211>  21
<212>  DNA
<213>  Artificial

<220>
<221>  Source
<223>  /note="Description of Artificial Sequence: An artificially synthesized
primer for PCR"

<400>  25
atcatctatt gcacaggggc c                                                21

<210>  26
<211>  23
<212>  DNA

<213>  Artificial

<220>
<221>  Source
<223>   /note="Description  of  Artificial  Sequence:  An  artificially  synthesized
primer for PCR"

<400>  26
catactcaga gacccctgct agc                                                23

<210>  27
<211>  23
<212>  DNA
<213>  Artificial

<220>
<221>  Source
<223>   /note="Description  of  Artificial  Sequence:  An  artificially  synthesized
primer for PCR"

<400>  27
ctggaaacaa ggcagtagtg att                                                23

<210>  28
<211>  23
<212>  DNA
<213>  Artificial

<220>
<221>  Source
<223>   /note="Description  of  Artificial  Sequence:  An  artificially  synthesized
primer for PCR"

<400>  28
gtacactgaa gcctgaaggt gat                                                23

<210>  29
<211>  19
<212>  DNA

<213>  Artificial

<220>
<221>  Source
<223>  /note="Description of Artificial Sequence: A target sequence for siRNA"

<400>  29
cgtacgcgga atacttcga                                                    19

<210>  30
<211>  19
<212>  DNA
<213>  Artificial

<220>
<221>  Source
<223>  /note="Description of Artificial Sequence: A target sequence for siRNA"

<400>  30
gcgcgctttg taggattcg                                                    19

<210>  31
<211>  19
<212>  DNA
<213>  Artificial

<220>
<221>  Source
<223>  /note="Description of Artificial Sequence: A target sequence for siRNA"

<400>  31
gaagcagcac gacttcttc                                                    19

<210>  32
<211>  19
<212>  DNA
<213>  Artificial

<220>

<221>  Source
<223>  /note="Description of Artificial Sequence: A target sequence for siRNA"

<400>  32
ggaagaattg gttcttgaa                                                    19

<210>  33
<211>  19
<212>  DNA
<213>  Artificial

<220>
<221>  Source
<223>  /note="Description of Artificial Sequence: A target sequence for siRNA"

<400>  33
gatgtggctc aactcaaag                                                    19

<210>  34
<211>  19
<212>  DNA
<213>  Artificial

<220>
<221>  Source
<223>  /note="Description of Artificial Sequence: A target sequence for siRNA"

<400>  34
gcagctgcga agtgttgta                                                    19

<210>  35
<211>  19
<212>  DNA
<213>  Artificial

<220>
<221>  Source
<223>  /note="Description of Artificial Sequence: A target sequence for siRNA"

<400> 35
gatacgaaag cagctgcga 19

<210> 36
<211> 19
<212> DNA
<213> Artificial

<220>
<221> Source
<223> /note="Description of Artificial Sequence: A target sequence for siRNA"

<400> 36
ggtgaagaag agcctgcca 19

<210> 37
<211> 19
<212> DNA
<213> Artificial

<220>
<221> Source
<223> /note="Description of Artificial Sequence: A target sequence for siRNA"

<400> 37
cctgaaacta gcacactgc 19

<210> 38
<211> 30
<212> DNA
<213> Artificial

<220>
<221> Source
<223> /note="Description of Artificial Sequence: An artificially synthesized
primer for PCR"

<400> 38
cgacgcgtcg ctctaaccat tcatcagctc 30

<210> 39
<211> 29
<212> DNA
<213> Artificial

<220>
<221> Source
<223> /note="Description of Artificial Sequence: An artificially synthesized
primer for PCR"

<400> 39
ccgctcgagc ggactaattc cactctcac 29

<210> 40
<211> 31
<212> DNA
<213> Artificial

<220>
<221> Source
<223> /note="Description of Artificial Sequence: An artificially synthesized
primer for PCR"

<400> 40
cgacgcgtcg agacattctg accatagcac c 31

<210> 41
<211> 32
<212> DNA
<213> Artificial

<220>
<221> Source
<223> /note="Description of Artificial Sequence: An artificially synthesized
primer for ChIP assay"

<400> 41
ccgctcgagc ggatcatgtc tactggctga tc 32

<210> 42
<211> 30
<212> DNA
<213> Artificial

<220>
<221> Source
<223> /note="Description of Artificial Sequence: An artificially synthesized
primer for ChIP assay"

<400> 42
cgacgcgtcg ttcctacaat gtcttcagtc 30

<210> 43
<211> 31
<212> DNA
<213> Artificial

<220>
<221> Source
<223> /note="Description of Artificial Sequence: An artificially synthesized
primer for ChIP assay"

<400> 43
ccgctcgagc ggttcagaag ccaacagtgg c 31

<210> 44
<211> 29
<212> DNA
<213> Artificial

<220>
<221> Source
<223> /note="Description of Artificial Sequence: An artificially synthesized
primer for ChIP assay"

<400> 44
cgacgcgtcg caagaccttc accatgtgg 29

<210> 45
<211> 30
<212> DNA
<213> Artificial

<220>
<221> Source
<223> /note="Description of Artificial Sequence: An artificially synthesized
primer for ChIP assay"

<400> 45
ccgctcgagc ggagacaacg gacgtctgcg 30

<210> 46
<211> 30
<212> DNA
<213> Artificial

<220>
<221> Source
<223> /note="Description of Artificial Sequence: An artificially synthesized
primer for ChIP assay"

<400> 46
cgacgcgtcg tctgttctga gcttccatac 30

<210> 47
<211> 30
<212> DNA
<213> Artificial

<220>
<221> Source
<223> /note="Description of Artificial Sequence: An artificially synthesized
primer for ChIP assay"

<400> 47
cgacgcgtcg gaaagtctca cttccaatgg 30

<210> 48
<211> 31
<212> DNA
<213> Artificial

<220>
<221> Source
<223> /note="Description of Artificial Sequence: An artificially synthesized
primer for ChIP assay"

<400> 48
cgacgcgtcg atagatgttc cagagacatt c 31

<210> 49
<211> 31
<212> DNA
<213> Artificial

<220>
<221> Source
<223> /note="Description of Artificial Sequence: An artificially synthesized
primer for ChIP assay"

<400> 49
cgacgcgtcg cactgaagta atcatggcaa c 31

<210> 50
<211> 20
<212> DNA
<213> Artificial

<220>
<221> Source
<223> /note="Description of Artificial Sequence: Artificially Synthesized
Sequence for EMSA"

<400> 50
cccgtcgcac gtggtggcca 20

<210> 51
<211> 20
<212> DNA
<213> Artificial

<220>
<221> Source
<223> /note="Description of Artificial Sequence: Artificially Synthesized
Sequence for EMSA"

<400> 51
tggccaccac gtgcgacggg 20

<210> 52
<211> 4348
<212> DNA
<213> Homo sapiens

<220>
<221> CDS
<222> (61)..(3756)

<400> 52
gggaggccca gggagaacgg ggaagggaca tttagtttga gacggtgctg agataggatc 60

atg aag gaa gag gtg aag gga att cct gta aga gtg gcg ctg cgt tgt 108
Met Lys Glu Glu Val Lys Gly Ile Pro Val Arg Val Ala Leu Arg Cys
1               5                   10                  15

cgc cct ctg gtc ccc aaa gag att agc gag ggc tgc cag atg tgc ctt 156
Arg Pro Leu Val Pro Lys Glu Ile Ser Glu Gly Cys Gln Met Cys Leu
            20                  25                  30

tcc ttc gtg ccc gga gag cct cag gtg gtg gtt ggt aca gat aaa tcc 204
Ser Phe Val Pro Gly Glu Pro Gln Val Val Val Gly Thr Asp Lys Ser
        35                  40                  45

ttc acc tac gat ttt gta ttt gat ccc tct act gaa cag gaa gaa gtc 252

Phe Thr Tyr Asp Phe Val Phe Asp Pro Ser Thr Glu Gln Glu Glu Val
    50                  55                  60

ttc aat aca gca gta gcg cca ctc ata aaa ggt gta ttt aaa gga tat 300
Phe Asn Thr Ala Val Ala Pro Leu Ile Lys Gly Val Phe Lys Gly Tyr
65                  70                  75                  80

aat gca acg gtc ctg gcc tat ggg cag act ggc tct gga aaa acc tat 348
Asn Ala Thr Val Leu Ala Tyr Gly Gln Thr Gly Ser Gly Lys Thr Tyr
                85                  90                  95

tca atg gga ggt gca tat act gca gag caa gag aat gaa cca aca gtt 396
Ser Met Gly Gly Ala Tyr Thr Ala Glu Gln Glu Asn Glu Pro Thr Val
            100                 105                 110

ggg gtt att cct agg gta ata caa ctg ctc ttc aaa gaa att gat aaa 444
Gly Val Ile Pro Arg Val Ile Gln Leu Leu Phe Lys Glu Ile Asp Lys
        115                 120                 125

aag agt gac ttt gaa ttt act ctg aaa gtg tct tac tta gag att tac 492
Lys Ser Asp Phe Glu Phe Thr Leu Lys Val Ser Tyr Leu Glu Ile Tyr
    130                 135                 140

aat gaa gaa att ttg gat ctt cta tgt cca tct cgt gag aaa gct caa 540
Asn Glu Glu Ile Leu Asp Leu Leu Cys Pro Ser Arg Glu Lys Ala Gln
145                 150                 155                 160

ata aat ata cga gag gat cct aag gaa ggc ata aag att gtg gga ctc 588
Ile Asn Ile Arg Glu Asp Pro Lys Glu Gly Ile Lys Ile Val Gly Leu
                165                 170                 175

act gag aag act gtt ttg gtt gcc ttg gat act gtt tcc tgt ttg gaa 636
Thr Glu Lys Thr Val Leu Val Ala Leu Asp Thr Val Ser Cys Leu Glu
            180                 185                 190

cag ggc aac aac tct agg act gtg gcc tcc acg gct atg aac tcc cag 684
Gln Gly Asn Asn Ser Arg Thr Val Ala Ser Thr Ala Met Asn Ser Gln
        195                 200                 205

tcg tcc cga tct cat gcc atc ttt aca atc tcc tta gag caa gga aag 732
Ser Ser Arg Ser His Ala Ile Phe Thr Ile Ser Leu Glu Gln Gly Lys
    210                 215                 220

aaa agt gac aag aat agc agc ttt cgc tcc aag ctg cat ctt gta gac 780
Lys Ser Asp Lys Asn Ser Ser Phe Arg Ser Lys Leu His Leu Val Asp
225                 230                 235                 240

ctc gct gga tca gaa aga cag aag aaa acc aag gct gaa ggg gat cgt 828
Leu Ala Gly Ser Glu Arg Gln Lys Lys Thr Lys Ala Glu Gly Asp Arg
                245                 250                 255

cta aaa gag ggt att aat att aac cga ggc ctc cta tgc ttg gga aat 876
Leu Lys Glu Gly Ile Asn Ile Asn Arg Gly Leu Leu Cys Leu Gly Asn
            260                 265                 270

gta atc agt gct ctt gga gat gac aaa aag ggt ggc ttt gcg ccc tac 924
Val Ile Ser Ala Leu Gly Asp Asp Lys Lys Gly Gly Phe Ala Pro Tyr
        275                 280                 285

aga gat tcc aag ttg act cga ctg ctt caa gat tct cta gga ggt aat 972
Arg Asp Ser Lys Leu Thr Arg Leu Leu Gln Asp Ser Leu Gly Gly Asn
    290                 295                 300

agc cat act ctt atg ata gcc tgt gtg agt cct gct gac tcc aat cta 1020
Ser His Thr Leu Met Ile Ala Cys Val Ser Pro Ala Asp Ser Asn Leu
305                 310                 315                 320

gag gaa aca tta aat acc ctt cgc tat gct gac aga gca aga aaa atc 1068
Glu Glu Thr Leu Asn Thr Leu Arg Tyr Ala Asp Arg Ala Arg Lys Ile
                325                 330                 335

aag aac aaa cct att gtt aat att gat ccc cag aca gct gaa ctt aat 1116
Lys Asn Lys Pro Ile Val Asn Ile Asp Pro Gln Thr Ala Glu Leu Asn
            340                 345                 350

cat cta aag caa cag gta caa cag cta caa gtc ttg ttg cta cag gcc 1164
His Leu Lys Gln Gln Val Gln Gln Leu Gln Val Leu Leu Leu Gln Ala
        355                 360                 365

cat gga ggt acc ctg cct gga tct ata act gtg gaa cca tca gag aat 1212
His Gly Gly Thr Leu Pro Gly Ser Ile Thr Val Glu Pro Ser Glu Asn
    370                 375                 380

cta caa tcc ctg atg gag aag aat cag tcc ctg gta gag gag aat gaa 1260

Leu Gln Ser Leu Met Glu Lys Asn Gln Ser Leu Val Glu Glu Asn Glu
385                 390                 395                 400

aaa tta agt cgt ggt ctg agc gag gca gct ggt cag aca gcc cag atg     1308
Lys Leu Ser Arg Gly Leu Ser Glu Ala Ala Gly Gln Thr Ala Gln Met
                405                 410                 415

ttg gag agg atc att tgg aca gag caa gcg aat gaa aaa atg aac gcc     1356
Leu Glu Arg Ile Ile Trp Thr Glu Gln Ala Asn Glu Lys Met Asn Ala
            420                 425                 430

aag cta gaa gag ctc agg cag cat gcg gcc tgc aaa ctg gat ctt caa     1404
Lys Leu Glu Glu Leu Arg Gln His Ala Ala Cys Lys Leu Asp Leu Gln
        435                 440                 445

aag cta gtg gag act ttg gaa gac cag gaa ttg aaa gaa aat gta gag     1452
Lys Leu Val Glu Thr Leu Glu Asp Gln Glu Leu Lys Glu Asn Val Glu
    450                 455                 460

ata att tgt aac ctg cag caa ttg att acc cag tta tcg gat gaa act     1500
Ile Ile Cys Asn Leu Gln Gln Leu Ile Thr Gln Leu Ser Asp Glu Thr
465                 470                 475                 480

gtt gct tgc atg gct gca gcc att gat act gcg gtg gag caa gaa gcc     1548
Val Ala Cys Met Ala Ala Ala Ile Asp Thr Ala Val Glu Gln Glu Ala
                485                 490                 495

caa gta gaa acc agt cca gag acg agc agg tct tct gac gct ttt acc     1596
Gln Val Glu Thr Ser Pro Glu Thr Ser Arg Ser Ser Asp Ala Phe Thr
            500                 505                 510

act cag cat gct ctc cgt caa gcg cag atg tct aag gag ctg gtt gag     1644
Thr Gln His Ala Leu Arg Gln Ala Gln Met Ser Lys Glu Leu Val Glu
        515                 520                 525

ttg aat aaa gcg ctt gca ctg aaa gag gcc ctg gct agg aag atg act     1692
Leu Asn Lys Ala Leu Ala Leu Lys Glu Ala Leu Ala Arg Lys Met Thr
    530                 535                 540

cag aat gac agc caa ctg cag cct att cag tac caa tac cag gat aac     1740
Gln Asn Asp Ser Gln Leu Gln Pro Ile Gln Tyr Gln Tyr Gln Asp Asn
545                 550                 555                 560

ata aaa gag cca gaa tta gaa gtc atc aat ctg caa aag gaa aag gaa 1788
Ile Lys Glu Pro Glu Leu Glu Val Ile Asn Leu Gln Lys Glu Lys Glu
                565                 570                 575

gaa ttg gtt ctt gaa ctt cag aca gca aag aag gat gcc aac caa gcc 1836
Glu Leu Val Leu Glu Leu Gln Thr Ala Lys Lys Asp Ala Asn Gln Ala
            580                 585                 590

aag ttg agt gag cgc cgc cgc aaa cgt ctc cag gag ctg gag ggt caa 1884
Lys Leu Ser Glu Arg Arg Arg Lys Arg Leu Gln Glu Leu Glu Gly Gln
        595                 600                 605

att gct gat ctg aag aag aaa ctg aat gag cag tcc aaa ctt ctg aaa 1932
Ile Ala Asp Leu Lys Lys Lys Leu Asn Glu Gln Ser Lys Leu Leu Lys
    610                 615                 620

cta aag gaa tcc aca gag cgt act gtc tcc aaa ctg aac cag gag ata 1980
Leu Lys Glu Ser Thr Glu Arg Thr Val Ser Lys Leu Asn Gln Glu Ile
625                 630                 635                 640

cgg atg atg aaa aac cag cgg gta cag tta atg cgt caa atg aaa gaa 2028
Arg Met Met Lys Asn Gln Arg Val Gln Leu Met Arg Gln Met Lys Glu
                645                 650                 655

gat gct gag aag ttt aga cag tgg aag cag aaa aga gac aaa gaa gta 2076
Asp Ala Glu Lys Phe Arg Gln Trp Lys Gln Lys Arg Asp Lys Glu Val
            660                 665                 670

ata cag tta aaa gaa cga gac cgt aag agg caa tat gag ctg ctg aaa 2124
Ile Gln Leu Lys Glu Arg Asp Arg Lys Arg Gln Tyr Glu Leu Leu Lys
        675                 680                 685

ctt gaa aga aac ttc cag aaa caa tcc aat gtg ctc aga cgt aaa acg 2172
Leu Glu Arg Asn Phe Gln Lys Gln Ser Asn Val Leu Arg Arg Lys Thr
    690                 695                 700

gag gag gca gca gct gcc aac aag cgt ctc aag gat gct ctc cag aaa 2220
Glu Glu Ala Ala Ala Ala Asn Lys Arg Leu Lys Asp Ala Leu Gln Lys
705                 710                 715                 720

caa cgg gag gtt gca gat aag cgg aaa gag act cag agc cgt gga atg 2268

Gln Arg Glu Val Ala Asp Lys Arg Lys Glu Thr Gln Ser Arg Gly Met
                725                 730                 735

gaa ggc act gca gct cga gtg aag aat tgg ctt gga aac gaa att gag 2316
Glu Gly Thr Ala Ala Arg Val Lys Asn Trp Leu Gly Asn Glu Ile Glu
            740                 745                 750

gtt atg gtc agt act gag gaa gcc aaa cgc cat ctg aat gac ctc ctt 2364
Val Met Val Ser Thr Glu Glu Ala Lys Arg His Leu Asn Asp Leu Leu
        755                 760                 765

gaa gat aga aag atc ctg gct caa gat gtg gct caa ctc aaa gaa aaa 2412
Glu Asp Arg Lys Ile Leu Ala Gln Asp Val Ala Gln Leu Lys Glu Lys
    770                 775                 780

aag gaa tct ggg gag aat cca cct cct aaa ctc cgg agg cgt aca ttc 2460
Lys Glu Ser Gly Glu Asn Pro Pro Pro Lys Leu Arg Arg Arg Thr Phe
785                 790                 795                 800

tcc ctt act gaa gtg cgt ggt caa gtt tcg gag tca gaa gat tct att 2508
Ser Leu Thr Glu Val Arg Gly Gln Val Ser Glu Ser Glu Asp Ser Ile
                805                 810                 815

aca aag cag att gaa agc cta gag act gaa atg gaa ttc agg agt gct 2556
Thr Lys Gln Ile Glu Ser Leu Glu Thr Glu Met Glu Phe Arg Ser Ala
            820                 825                 830

cag att gct gac cta cag cag aag ctg ctg gat gca gaa agt gaa gac 2604
Gln Ile Ala Asp Leu Gln Gln Lys Leu Leu Asp Ala Glu Ser Glu Asp
        835                 840                 845

aga cca aaa caa cgc tgg gag aat att gcc acc att ctg gaa gcc aag 2652
Arg Pro Lys Gln Arg Trp Glu Asn Ile Ala Thr Ile Leu Glu Ala Lys
    850                 855                 860

tgt gcc ctg aaa tat ttg att gga gag ctg gtc tcc tcc aaa ata cag 2700
Cys Ala Leu Lys Tyr Leu Ile Gly Glu Leu Val Ser Ser Lys Ile Gln
865                 870                 875                 880

gtc agc aaa ctt gaa agc agc ctg aaa cag agc aag acc agc tgt gct 2748
Val Ser Lys Leu Glu Ser Ser Leu Lys Gln Ser Lys Thr Ser Cys Ala
                885                 890                 895

gac atg cag aag atg ctg ttt gag gaa cga aat cat ttt gcc gag ata 2796
Asp Met Gln Lys Met Leu Phe Glu Glu Arg Asn His Phe Ala Glu Ile
            900                 905                 910

gag aca gag tta caa gct gag ctg gtc aga atg gag caa cag cac caa 2844
Glu Thr Glu Leu Gln Ala Glu Leu Val Arg Met Glu Gln Gln His Gln
        915                 920                 925

gag aag gtg ctg tac ctt ctc agc cag ctg cag caa agc caa atg gca 2892
Glu Lys Val Leu Tyr Leu Leu Ser Gln Leu Gln Gln Ser Gln Met Ala
    930                 935                 940

gag aag cag tta gag gaa tca gtc agt gaa aag gaa cag cag ctg ctg 2940
Glu Lys Gln Leu Glu Glu Ser Val Ser Glu Lys Glu Gln Gln Leu Leu
945                 950                 955                 960

agc aca ctg aag tgt cag gat gaa gaa ctt gag aaa atg cga gaa gtg 2988
Ser Thr Leu Lys Cys Gln Asp Glu Glu Leu Glu Lys Met Arg Glu Val
                965                 970                 975

tgt gag caa aat cag cag ctt ctc cga gag aat gaa atc atc aag cag 3036
Cys Glu Gln Asn Gln Gln Leu Leu Arg Glu Asn Glu Ile Ile Lys Gln
            980                 985                 990

aaa ctg acc ctc ctc cag gta gcc agc aga cag aaa cat ctt cct aag 3084
Lys Leu Thr Leu Leu Gln Val Ala Ser Arg Gln Lys His Leu Pro Lys
        995                 1000                1005

gat acc ctt cta tct cca gac tct tct ttt gaa tat gtc cag cct 3129
Asp Thr Leu Leu Ser Pro Asp Ser Ser Phe Glu Tyr Val Gln Pro
    1010                1015                1020

aag cca aaa cct tct cgt gtt aaa gaa aag ttc ctg gag caa agc 3174
Lys Pro Lys Pro Ser Arg Val Lys Glu Lys Phe Leu Glu Gln Ser
    1025                1030                1035

atg gac atc gag gat cta aaa tat tgt tca gag cat tct gtg aat 3219
Met Asp Ile Glu Asp Leu Lys Tyr Cys Ser Glu His Ser Val Asn
    1040                1045                1050

gag cat gag gat ggt gat ggt gat gat gat gag ggg gat gac gag 3264

Glu His Glu Asp Gly Asp Gly Asp Asp Asp Glu Gly Asp Asp Glu
    1055                1060                1065

gaa tgg aag cca aca aaa tta gtt aat gtg tcc agg aag aac atc 3309
Glu Trp Lys Pro Thr Lys Leu Val Asn Val Ser Arg Lys Asn Ile
    1070                1075                1080

caa ggg tgt tcc tgc aag ggc tgg tgt gga aac aag caa tgt ggg 3354
Gln Gly Cys Ser Cys Lys Gly Trp Cys Gly Asn Lys Gln Cys Gly
    1085                1090                1095

tgc agg aag caa aag tca gac tgt ggt gtg gac tgt tgc tgt gac 3399
Cys Arg Lys Gln Lys Ser Asp Cys Gly Val Asp Cys Cys Cys Asp
    1100                1105                1110

ccc aca aag tgt cgg aac cgc cag caa ggc aag gat agc ttg ggc 3444
Pro Thr Lys Cys Arg Asn Arg Gln Gln Gly Lys Asp Ser Leu Gly
    1115                1120                1125

act gtt gaa cgg acc cag gat tca gaa agc tcc ttc aaa ctg gag 3489
Thr Val Glu Arg Thr Gln Asp Ser Glu Ser Ser Phe Lys Leu Glu
    1130                1135                1140

gat cct acc gag gtg acc cca gga ttg agc ttc ttt aat ccc gtc 3534
Asp Pro Thr Glu Val Thr Pro Gly Leu Ser Phe Phe Asn Pro Val
    1145                1150                1155

tgt gcc acc ccc aat agc aag atc ctg aaa gag atg tgc gat gtg 3579
Cys Ala Thr Pro Asn Ser Lys Ile Leu Lys Glu Met Cys Asp Val
    1160                1165                1170

gag cag gtg ctg tca aag aag act ccc cca gct ccc tcc cct ttt 3624
Glu Gln Val Leu Ser Lys Lys Thr Pro Pro Ala Pro Ser Pro Phe
    1175                1180                1185

gac ctc cca gag ttg aaa cat gta gca aca gaa tac caa gaa aac 3669
Asp Leu Pro Glu Leu Lys His Val Ala Thr Glu Tyr Gln Glu Asn
    1190                1195                1200

aag gct cca ggg aag aaa aag aaa cgg gct ctg gcc agc aac acc 3714
Lys Ala Pro Gly Lys Lys Lys Lys Arg Ala Leu Ala Ser Asn Thr
    1205                1210                1215

agc ttc ttc tct ggc tgc tcc cct atc gaa gaa gag gcc cac 3756
Ser Phe Phe Ser Gly Cys Ser Pro Ile Glu Glu Glu Ala His
    1220                1225                1230

tgaagttgga gtcatcatct ctacccccag tctggcttgg gagatgcttt caggttgcag 3816

ccagaagggg ttttttaaat gacttctctg gatttcaggt ttcttgctgt tgaaaaaagg 3876

aacaaagcgt tactgaaaag aaggtaacct ttgttggatg tgggccttag cctccaggtc 3936

cagactacta ctctatgttc tccagaaggg tgctaagtca cctactgaag agagaaccaa 3996

ctgactttcc tattgactca tcaggaacca gtcctcagtc tggtcaagtt gtttcttatt 4056

tgtgagcagt tcaggctatc tcctgatggg gatgaggcca aggctttctt atcttttggt 4116

tgtctctgct taatggagga gcctggccta ggatggaggc ctggcttaga tctttcattc 4176

cacctcagga atgaggttgt gatctttcct gtcctgaccc tctctgaatt atgtttcaat 4236

agtactcttg attgtctgcc atgttgttga agcaaatgaa ttatttttaa atgttaagta 4296

agtaaataaa ccttagcccg tctttttttt tttttttttt tttttttttt tt 4348

<210> 53
<211> 1232
<212> PRT
<213> Homo sapiens

<400> 53

Met Lys Glu Glu Val Lys Gly Ile Pro Val Arg Val Ala Leu Arg Cys
1               5                   10                  15

Arg Pro Leu Val Pro Lys Glu Ile Ser Glu Gly Cys Gln Met Cys Leu
            20                  25                  30

Ser Phe Val Pro Gly Glu Pro Gln Val Val Val Gly Thr Asp Lys Ser

        35                  40                  45

Phe Thr Tyr Asp Phe Val Phe Asp Pro Ser Thr Glu Gln Glu Glu Val
    50                  55                  60

Phe Asn Thr Ala Val Ala Pro Leu Ile Lys Gly Val Phe Lys Gly Tyr
65                  70                  75                  80

Asn Ala Thr Val Leu Ala Tyr Gly Gln Thr Gly Ser Gly Lys Thr Tyr
                85                  90                  95

Ser Met Gly Gly Ala Tyr Thr Ala Glu Gln Glu Asn Glu Pro Thr Val
            100                 105                 110

Gly Val Ile Pro Arg Val Ile Gln Leu Leu Phe Lys Glu Ile Asp Lys
        115                 120                 125

Lys Ser Asp Phe Glu Phe Thr Leu Lys Val Ser Tyr Leu Glu Ile Tyr
    130                 135                 140

Asn Glu Glu Ile Leu Asp Leu Leu Cys Pro Ser Arg Glu Lys Ala Gln
145                 150                 155                 160

Ile Asn Ile Arg Glu Asp Pro Lys Glu Gly Ile Lys Ile Val Gly Leu
                165                 170                 175

Thr Glu Lys Thr Val Leu Val Ala Leu Asp Thr Val Ser Cys Leu Glu
            180                 185                 190

Gln Gly Asn Asn Ser Arg Thr Val Ala Ser Thr Ala Met Asn Ser Gln
        195                 200                 205

Ser Ser Arg Ser His Ala Ile Phe Thr Ile Ser Leu Glu Gln Gly Lys
    210                 215                 220

Lys Ser Asp Lys Asn Ser Ser Phe Arg Ser Lys Leu His Leu Val Asp
225                 230                 235                 240

Leu Ala Gly Ser Glu Arg Gln Lys Lys Thr Lys Ala Glu Gly Asp Arg
                245                 250                 255

Leu Lys Glu Gly Ile Asn Ile Asn Arg Gly Leu Leu Cys Leu Gly Asn
            260                 265                 270

Val Ile Ser Ala Leu Gly Asp Asp Lys Lys Gly Gly Phe Ala Pro Tyr
        275                 280                 285

Arg Asp Ser Lys Leu Thr Arg Leu Leu Gln Asp Ser Leu Gly Gly Asn
    290                 295                 300

Ser His Thr Leu Met Ile Ala Cys Val Ser Pro Ala Asp Ser Asn Leu
305                 310                 315                 320

Glu Glu Thr Leu Asn Thr Leu Arg Tyr Ala Asp Arg Ala Arg Lys Ile
                325                 330                 335

Lys Asn Lys Pro Ile Val Asn Ile Asp Pro Gln Thr Ala Glu Leu Asn
            340                 345                 350

His Leu Lys Gln Gln Val Gln Gln Leu Gln Val Leu Leu Leu Gln Ala
        355                 360                 365

His Gly Gly Thr Leu Pro Gly Ser Ile Thr Val Glu Pro Ser Glu Asn

370                 375                 380

Leu Gln Ser Leu Met Glu Lys Asn Gln Ser Leu Val Glu Glu Asn Glu
385                 390                 395                 400

Lys Leu Ser Arg Gly Leu Ser Glu Ala Ala Gly Gln Thr Ala Gln Met
                405                 410                 415

Leu Glu Arg Ile Ile Trp Thr Glu Gln Ala Asn Glu Lys Met Asn Ala
            420                 425                 430

Lys Leu Glu Glu Leu Arg Gln His Ala Ala Cys Lys Leu Asp Leu Gln
        435                 440                 445

Lys Leu Val Glu Thr Leu Glu Asp Gln Glu Leu Lys Glu Asn Val Glu
    450                 455                 460

Ile Ile Cys Asn Leu Gln Gln Leu Ile Thr Gln Leu Ser Asp Glu Thr
465                 470                 475                 480

Val Ala Cys Met Ala Ala Ala Ile Asp Thr Ala Val Glu Gln Glu Ala
                485                 490                 495

Gln Val Glu Thr Ser Pro Glu Thr Ser Arg Ser Ser Asp Ala Phe Thr
            500                 505                 510

Thr Gln His Ala Leu Arg Gln Ala Gln Met Ser Lys Glu Leu Val Glu
        515                 520                 525

Leu Asn Lys Ala Leu Ala Leu Lys Glu Ala Leu Ala Arg Lys Met Thr
    530                 535                 540

Gln Asn Asp Ser Gln Leu Gln Pro Ile Gln Tyr Gln Tyr Gln Asp Asn
545                 550                 555                 560

Ile Lys Glu Pro Glu Leu Glu Val Ile Asn Leu Gln Lys Glu Lys Glu
                565                 570                 575

Glu Leu Val Leu Glu Leu Gln Thr Ala Lys Lys Asp Ala Asn Gln Ala
            580                 585                 590

Lys Leu Ser Glu Arg Arg Arg Lys Arg Leu Gln Glu Leu Glu Gly Gln
        595                 600                 605

Ile Ala Asp Leu Lys Lys Lys Leu Asn Glu Gln Ser Lys Leu Leu Lys
    610                 615                 620

Leu Lys Glu Ser Thr Glu Arg Thr Val Ser Lys Leu Asn Gln Glu Ile
625                 630                 635                 640

Arg Met Met Lys Asn Gln Arg Val Gln Leu Met Arg Gln Met Lys Glu
                645                 650                 655

Asp Ala Glu Lys Phe Arg Gln Trp Lys Gln Lys Arg Asp Lys Glu Val
            660                 665                 670

Ile Gln Leu Lys Glu Arg Asp Arg Lys Arg Gln Tyr Glu Leu Leu Lys
        675                 680                 685

Leu Glu Arg Asn Phe Gln Lys Gln Ser Asn Val Leu Arg Arg Lys Thr
    690                 695                 700

Glu Glu Ala Ala Ala Ala Asn Lys Arg Leu Lys Asp Ala Leu Gln Lys

705                 710                 715                 720

Gln Arg Glu Val Ala Asp Lys Arg Lys Glu Thr Gln Ser Arg Gly Met
                725                 730                 735

Glu Gly Thr Ala Ala Arg Val Lys Asn Trp Leu Gly Asn Glu Ile Glu
            740                 745                 750

Val Met Val Ser Thr Glu Glu Ala Lys Arg His Leu Asn Asp Leu Leu
        755                 760                 765

Glu Asp Arg Lys Ile Leu Ala Gln Asp Val Ala Gln Leu Lys Glu Lys
    770                 775                 780

Lys Glu Ser Gly Glu Asn Pro Pro Pro Lys Leu Arg Arg Arg Thr Phe
785                 790                 795                 800

Ser Leu Thr Glu Val Arg Gly Gln Val Ser Glu Ser Glu Asp Ser Ile
                805                 810                 815

Thr Lys Gln Ile Glu Ser Leu Glu Thr Glu Met Glu Phe Arg Ser Ala
            820                 825                 830

Gln Ile Ala Asp Leu Gln Gln Lys Leu Leu Asp Ala Glu Ser Glu Asp
        835                 840                 845

Arg Pro Lys Gln Arg Trp Glu Asn Ile Ala Thr Ile Leu Glu Ala Lys
    850                 855                 860

Cys Ala Leu Lys Tyr Leu Ile Gly Glu Leu Val Ser Ser Lys Ile Gln
865                 870                 875                 880

Val Ser Lys Leu Glu Ser Ser Leu Lys Gln Ser Lys Thr Ser Cys Ala
                885                 890                 895

Asp Met Gln Lys Met Leu Phe Glu Glu Arg Asn His Phe Ala Glu Ile
            900                 905                 910

Glu Thr Glu Leu Gln Ala Glu Leu Val Arg Met Glu Gln Gln His Gln
        915                 920                 925

Glu Lys Val Leu Tyr Leu Leu Ser Gln Leu Gln Gln Ser Gln Met Ala
    930                 935                 940

Glu Lys Gln Leu Glu Glu Ser Val Ser Glu Lys Glu Gln Gln Leu Leu
945                 950                 955                 960

Ser Thr Leu Lys Cys Gln Asp Glu Glu Leu Glu Lys Met Arg Glu Val
                965                 970                 975

Cys Glu Gln Asn Gln Gln Leu Leu Arg Glu Asn Glu Ile Ile Lys Gln
            980                 985                 990

Lys Leu Thr Leu Leu Gln Val Ala Ser Arg Gln Lys His Leu Pro Lys
        995                 1000                1005

Asp Thr Leu Leu Ser Pro Asp Ser Ser Phe Glu Tyr Val Gln Pro
    1010                1015                1020

Lys Pro Lys Pro Ser Arg Val Lys Glu Lys Phe Leu Glu Gln Ser
    1025                1030                1035

Met Asp Ile Glu Asp Leu Lys Tyr Cys Ser Glu His Ser Val Asn

1040                 1045                 1050

Glu His  Glu Asp Gly Asp Gly  Asp Asp Asp Glu Gly  Asp Asp Glu
    1055                 1060                 1065

Glu Trp  Lys Pro Thr Lys Leu  Val Asn Val Ser Arg  Lys Asn Ile
    1070                 1075                 1080

Gln Gly  Cys Ser Cys Lys Gly  Trp Cys Gly Asn Lys  Gln Cys Gly
    1085                 1090                 1095

Cys Arg  Lys Gln Lys Ser Asp  Cys Gly Val Asp Cys  Cys Cys Asp
    1100                 1105                 1110

Pro Thr  Lys Cys Arg Asn Arg  Gln Gln Gly Lys Asp  Ser Leu Gly
    1115                 1120                 1125

Thr Val  Glu Arg Thr Gln Asp  Ser Glu Ser Ser Phe  Lys Leu Glu
    1130                 1135                 1140

Asp Pro  Thr Glu Val Thr Pro  Gly Leu Ser Phe Phe  Asn Pro Val
    1145                 1150                 1155

Cys Ala  Thr Pro Asn Ser Lys  Ile Leu Lys Glu Met  Cys Asp Val
    1160                 1165                 1170

Glu Gln  Val Leu Ser Lys Lys  Thr Pro Pro Ala Pro  Ser Pro Phe
    1175                 1180                 1185

Asp Leu  Pro Glu Leu Lys His  Val Ala Thr Glu Tyr  Gln Glu Asn
    1190                 1195                 1200

Lys Ala Pro Gly Lys Lys Lys Lys Arg Ala Leu Ala Ser Asn Thr
    1205                1210                1215

Ser Phe Phe Ser Gly Cys Ser Pro Ile Glu Glu Glu Ala His
    1220                1225                1230

<210> 54
<211> 1232
<212> PRT
<213> Homo sapiens

<400> 54

Met Lys Glu Glu Val Lys Gly Ile Pro Val Arg Val Ala Leu Arg Cys
1               5                   10                  15

Arg Pro Leu Val Pro Lys Glu Ile Ser Glu Gly Cys Gln Met Cys Leu
            20                  25                  30

Ser Phe Val Pro Gly Glu Pro Gln Val Val Val Gly Thr Asp Lys Ser
        35                  40                  45

Phe Thr Tyr Asp Phe Val Phe Asp Pro Ser Thr Glu Gln Glu Glu Val
    50                  55                  60

Phe Asn Thr Ala Val Ala Pro Leu Ile Lys Gly Val Phe Lys Gly Tyr
65                  70                  75                  80

Asn Ala Thr Val Leu Ala Tyr Gly Gln Thr Gly Ser Gly Lys Thr Tyr
                85                  90                  95

Ser Met Gly Gly Ala Tyr Thr Ala Glu Gln Glu Asn Glu Pro Thr Val
            100                 105                 110

Gly Val Ile Pro Arg Val Ile Gln Leu Leu Phe Lys Glu Ile Asp Lys
        115                 120                 125

Lys Ser Asp Phe Glu Phe Thr Leu Lys Val Ser Tyr Leu Glu Ile Tyr
    130                 135                 140

Asn Glu Glu Ile Leu Asp Leu Leu Cys Pro Ser Arg Glu Lys Ala Gln
145                 150                 155                 160

Ile Asn Ile Arg Glu Asp Pro Lys Glu Gly Ile Lys Ile Val Gly Leu
                165                 170                 175

Thr Glu Lys Thr Val Leu Val Ala Leu Asp Thr Val Ser Cys Leu Glu
            180                 185                 190

Gln Gly Asn Asn Ser Arg Thr Val Ala Ser Thr Ala Met Asn Ser Gln
        195                 200                 205

Ser Ser Arg Ser His Ala Ile Phe Thr Ile Ser Leu Glu Gln Gly Lys
    210                 215                 220

Lys Ser Asp Lys Asn Ser Ser Phe Arg Ser Lys Leu His Leu Val Asp
225                 230                 235                 240

Leu Ala Gly Ser Glu Arg Gln Lys Lys Thr Lys Ala Glu Gly Asp Arg
                245                 250                 255

Leu Lys Glu Gly Ile Asn Ile Asn Arg Gly Leu Leu Cys Leu Gly Asn
            260                 265                 270

Val Ile Ser Ala Leu Gly Asp Asp Lys Lys Gly Gly Phe Ala Pro Tyr
        275                 280                 285
Arg Asp Ser Lys Leu Thr Arg Leu Leu Gln Asp Ser Leu Gly Gly Asn
    290                 295                 300
Ser His Thr Leu Met Ile Ala Cys Val Ser Pro Ala Asp Ser Asn Leu
305                 310                 315                 320
Glu Glu Thr Leu Asn Thr Leu Arg Tyr Ala Asp Arg Ala Arg Lys Ile
                325                 330                 335
Lys Asn Lys Pro Ile Val Asn Ile Asp Pro Gln Thr Ala Glu Leu Asn
            340                 345                 350
His Leu Lys Gln Gln Val Gln Gln Leu Gln Val Leu Leu Leu Gln Ala
        355                 360                 365
His Gly Gly Thr Leu Pro Gly Ser Ile Thr Val Glu Pro Ser Glu Asn
    370                 375                 380
Leu Gln Ser Leu Met Glu Lys Asn Gln Ser Leu Val Glu Glu Asn Glu
385                 390                 395                 400
Lys Leu Ser Arg Gly Leu Ser Glu Ala Ala Gly Gln Thr Ala Gln Met
                405                 410                 415
Leu Glu Arg Ile Ile Trp Thr Glu Gln Ala Asn Glu Lys Met Asn Ala
            420                 425                 430
Lys Leu Glu Glu Leu Arg Gln His Ala Ala Cys Lys Leu Asp Leu Gln
        435                 440                 445

Lys Leu Val Glu Thr Leu Glu Asp Gln Glu Leu Lys Glu Asn Val Glu
    450                 455                 460

Ile Ile Cys Asn Leu Gln Gln Leu Ile Thr Gln Leu Ser Asp Glu Thr
465                 470                 475                 480

Val Ala Cys Met Ala Ala Ala Ile Asp Thr Ala Val Glu Gln Glu Ala
                485                 490                 495

Gln Val Glu Thr Ser Pro Glu Thr Ser Arg Ser Ser Asp Ala Phe Thr
            500                 505                 510

Thr Gln His Ala Leu Arg Gln Ala Gln Met Ser Lys Glu Leu Val Glu
        515                 520                 525

Leu Asn Lys Ala Leu Ala Leu Lys Glu Ala Leu Ala Arg Lys Met Thr
    530                 535                 540

Gln Asn Asp Ser Gln Leu Gln Pro Ile Gln Tyr Gln Tyr Gln Asp Asn
545                 550                 555                 560

Ile Lys Glu Pro Glu Leu Glu Val Ile Asn Leu Gln Lys Glu Lys Glu
                565                 570                 575

Glu Leu Val Leu Glu Leu Gln Thr Ala Lys Lys Asp Ala Asn Gln Ala
            580                 585                 590

Lys Leu Ser Glu Arg Arg Arg Lys Arg Leu Gln Glu Leu Glu Gly Gln
        595                 600                 605

Ile Ala Asp Leu Lys Lys Lys Leu Asn Glu Gln Ser Lys Leu Leu Lys
    610                 615                 620

Leu Lys Glu Ser Thr Glu Arg Thr Val Ser Lys Leu Asn Gln Glu Ile
625                 630                 635                 640

Arg Met Met Lys Asn Gln Arg Val Gln Leu Met Arg Gln Met Lys Glu
                645                 650                 655

Asp Ala Glu Lys Phe Arg Gln Trp Lys Gln Lys Arg Asp Lys Glu Val
            660                 665                 670

Ile Gln Leu Lys Glu Arg Asp Arg Lys Arg Gln Tyr Glu Leu Leu Lys
        675                 680                 685

Leu Glu Arg Asn Phe Gln Lys Gln Ser Asn Val Leu Arg Arg Lys Thr
    690                 695                 700

Glu Glu Ala Ala Ala Ala Asn Lys Arg Leu Lys Asp Ala Leu Gln Lys
705                 710                 715                 720

Gln Arg Glu Val Ala Asp Lys Arg Lys Glu Thr Gln Ser Arg Gly Met
                725                 730                 735

Glu Gly Thr Ala Ala Arg Val Lys Asn Trp Leu Gly Asn Glu Ile Glu
            740                 745                 750

Val Met Val Ser Thr Glu Glu Ala Lys Arg His Leu Asn Asp Leu Leu
        755                 760                 765

Glu Asp Arg Lys Ile Leu Ala Gln Asp Val Ala Gln Leu Lys Glu Lys
    770                 775                 780

Lys Glu Ser Gly Glu Asn Pro Pro Pro Lys Leu Arg Arg Arg Thr Phe
785                 790                 795                 800

Ser Leu Thr Glu Val Arg Gly Gln Val Ser Glu Ser Glu Asp Ser Ile
                805                 810                 815

Thr Lys Gln Ile Glu Ser Leu Glu Thr Glu Met Glu Phe Arg Ser Ala
            820                 825                 830

Gln Ile Ala Asp Leu Gln Gln Lys Leu Leu Asp Ala Glu Ser Glu Asp
        835                 840                 845

Arg Pro Lys Gln Arg Trp Glu Asn Ile Ala Thr Ile Leu Glu Ala Lys
    850                 855                 860

Cys Ala Leu Lys Tyr Leu Ile Gly Glu Leu Val Ser Ser Lys Ile Gln
865                 870                 875                 880

Val Ser Lys Leu Glu Ser Ser Leu Lys Gln Ser Lys Thr Ser Cys Ala
                885                 890                 895

Asp Met Gln Lys Met Leu Phe Glu Glu Arg Asn His Phe Ala Glu Ile
            900                 905                 910

Glu Thr Glu Leu Gln Ala Glu Leu Val Arg Met Glu Gln Gln His Gln
        915                 920                 925

Glu Lys Val Leu Tyr Leu Leu Ser Gln Leu Gln Gln Ser Gln Met Ala
    930                 935                 940

Glu Lys Gln Leu Glu Glu Ser Val Ser Glu Lys Glu Gln Gln Leu Leu
945                 950                 955                 960

Ser Thr Leu Lys Cys Gln Asp Glu Glu Leu Glu Lys Met Arg Glu Val
                965                 970                 975

Cys Glu Gln Asn Gln Gln Leu Leu Arg Glu Asn Glu Ile Ile Lys Gln
            980                 985                 990

Lys Leu Thr Leu Leu Gln Val Ala Ser Arg Gln Lys His Leu Pro Lys
        995                 1000                1005

Asp Thr Leu Leu Ser Pro Asp Ser Ser Phe Glu Tyr Val Gln Pro
    1010                1015                1020

Lys Pro Lys Pro Ser Arg Val Lys Glu Lys Phe Leu Glu Gln Ser
    1025                1030                1035

Met Asp Ile Glu Asp Leu Lys Tyr Cys Ser Glu His Ser Val Asn
    1040                1045                1050

Glu His Glu Asp Gly Asp Gly Asp Asp Asp Glu Gly Asp Asp Glu
    1055                1060                1065

Glu Trp Lys Pro Thr Lys Leu Val Asn Val Ser Arg Lys Asn Ile
    1070                1075                1080

Gln Gly Cys Ser Cys Lys Gly Trp Cys Gly Asn Lys Gln Cys Gly
    1085                1090                1095

Cys Arg Lys Gln Lys Ser Asp Cys Gly Val Asp Cys Cys Cys Asp
    1100                1105                1110

Pro Thr Lys Cys Arg Asn Arg Gln Gln Gly Lys Asp Ser Leu Gly
    1115                1120                1125

Thr Val Glu Arg Thr Gln Asp Ser Glu Ser Ser Phe Lys Leu Glu
    1130                1135                1140

Asp Pro Thr Glu Val Thr Pro Gly Leu Ser Phe Phe Asn Pro Val
    1145                1150                1155

Cys Ala Thr Pro Asn Ser Lys Ile Leu Lys Glu Met Cys Asp Val
    1160                1165                1170

Glu Gln Val Leu Ser Lys Lys Thr Pro Pro Ala Pro Ser Pro Phe
    1175                1180                1185

Asp Leu Pro Glu Leu Lys His Val Ala Thr Glu Tyr Gln Glu Asn
    1190                1195                1200

Lys Ala Pro Gly Lys Lys Lys Lys Arg Ala Leu Ala Ser Asn Thr
    1205                1210                1215

Ser Phe Phe Ser Gly Cys Ser Pro Ile Glu Glu Glu Ala His
    1220                1225                1230

<210> 55
<211> 2469
<212> DNA
<213> Homo sapiens

<400> 55
cgctttagct tcggccccgg ccggccgggc ggggaagact ggtgtggtct ggccatggat 60

gggctccagg ccagtgcagg gccgttgagg cgcgggcggc cgaggcgccg gcgcaagccc 120

cagccacaca gcgggtcggt cctggccctg cccttgaggt ccaggaagat acgaaagcag 180

ctgcgaagtg ttgtatcccg catggcagcg ctgaggacgc agacgctgcc tagcgagaac 240

tcggaggaat cgagggtgga gtcgacggcc gacgacctgg gggacgcgct acccggtggg 300

gcggcggtgg cggccgtccc ggacgcagcc cggcgagagc catacggcca cctggggccc 360

gcagagctgc tggaggcctc gcccgccgcg cgctccctgc agaccccgtc ggcgcgcctg 420

gtgcccgctt ccgcgccgcc cgcgcgcctg gtggaggtgc ccgccgcgcc ggtccgggtg 480

gtggagacct cggccctgct gtgcaccgcg caacacttag cggccgtcca gtcgtccggg 540

gcccctgcga cggcgtcggg gccgcaggtg gataacacgg gtggggagcc ggcctgggac 600

tccccgctgc ggcgcgtctt ggccgagctg aaccgcatcc ccagcagccg gcggcgagcg 660

gcccgcctct ttgagtggct catcgcgccc atgccgccag atcacttcta ccggcgccta 720

tgggagcgcg aggcggtgct ggtgcggcgg caggaccaca cctactacca gggacttttc 780

tctaccgctg acctggattc gatgctgcgc aacgaggagg tgcagttcgg ccagcatttg 840

gacgccgctc gctacatcaa cggacgacgc gagaccctga acccacccgg ccgcgcgctg 900

cccgccgccg cgtggtccct gtaccaggcc ggctgctccc tgcgtctcct ctgtccgcag 960

gctttctcta ctactgtgtg gcagtttttg gctgtgcttc aagagcagtt tggaagcatg 1020

gcaggctcca acgtttacct cacgccccct aactcgcagg gctttgcccc ccactacgac 1080

gacatcgagg ccttcgtgct gcagctggaa ggtaggaaac tctggcgtgt ataccgaccc 1140

cgagccccaa ccgaggaact ggctctgaca tccagcccca acttcagtca ggacgacctc 1200

ggtgagccgg tgctgcagac cgtgctggaa cctggagatt tgctgtattt tcctcggggc 1260

ttcattcacc aagctgaatg ccaggatgga gtccactctc tgcacctcac cttgtccacg 1320

taccagcgca atacctgggg tgacttctta gaggccatac tgcctctggc agtgcaggct 1380

gcaatggaag aaaatgtgga gtttcggagg ggtctgcccc gagacttcat ggattacatg 1440

ggggcccagc attcagattc taaggatccg cgaagaaccg ctttcatgga gaaggtgcgg 1500

gtcttggttg cccgcctggg acactttgct cctgttgatg ctgtggccga ccagcgagcc 1560

aaagacttca ttcacgattc tctgccccct gttttgactg atagggagag ggcactaagt 1620

gtttacgggc ttccaattcg ctgggaggct ggagaacctg taaacgtggg ggcccagttg 1680

acaacagaaa cagaagtcca tatgcttcag gatgggatag ctcggctggt gggtgagggg 1740

ggccatttgt ttctctatta cacagtggaa aactcccgtg tgtatcatct ggaagaaccc 1800

aagtgcttgg aaatataccc ccagcaagct gatgccatgg aactgttgct tggttcttat 1860

ccagagtttg tgagagtggg ggacctgccc tgtgacagtg tggaggacca gctgtccttg 1920

gcaaccacgt tgtatgataa ggggctgctg ctcactaaga tgcctctagc cctaaattag 1980

tttcttgttg attgctggaa acaaggcagt agtgattctc cgctgccact gctacctttt 2040

tttttttttt ttttccttaa actcacgttc ttaccttgat aagcatcagt gtgctcacat 2100

ttacctttat cactgcttca gtgtcacaaa cctcggaagg tcttctagga agaaccatct 2160

catctaggta caaaaggaaa aggagaagtt ggaggtggaa aaaaaaccct tgatccgtga 2220

tcatttcaga gcaccaactt catcaccttc aggcttcagt gtactgggta acactgacca 2280

tgtcgttctg cttgagacag atattagatt ttttttggaa tttggatctt tcatctgagt 2340

tctttttcat gggcgggtcg gggtcagtat cctgtttgtt attgttaaat ttgtatgaac 2400

cttagaaaag ttattaaagt gccaaagaat gaaaaaaaaa aaaaaaaaaa aaaaaaaaaa 2460

aaaaaaaaa 2469

<210> 56

<211>  5441
<212>  DNA
<213>  Homo sapiens

<220>
<221>  CDS
<222>  (159)..(1457)

<400>  56
agtgggggcc tgatagcgcg gcggtgtgga ccgcgcggcc gaagagcgcg gcgcccagag      60

cgcgggccgc tcgcggagcc acagcccgag ccgggtccca gccggagccg agccccagcc     120

gagccgagcc gggcccggag cgcccggtgc ccgcagcc atg ccg gcc ggc cgc gcc     176
                                          Met Pro Ala Gly Arg Ala
                                          1               5

gcg cgc acc tgt gcg ctg ctc gcc ctc tgc ctc ctg ggc gcc ggg gcc       224
Ala Arg Thr Cys Ala Leu Leu Ala Leu Cys Leu Leu Gly Ala Gly Ala
            10                  15                  20

cag gat ttc ggg ccg acg cgc ttc atc tgc acc tcg gtg ccc gtg gac       272
Gln Asp Phe Gly Pro Thr Arg Phe Ile Cys Thr Ser Val Pro Val Asp
        25                  30                  35

gcc gac atg tgc gcc gcg tcc gtg gcc gcc ggc ggc gcc gag gag ctc       320
Ala Asp Met Cys Ala Ala Ser Val Ala Ala Gly Gly Ala Glu Glu Leu
    40                  45                  50

cgg agc agc gtg ctg cag ctc cgc gag acg gtg ctg cag cag aag gag       368
Arg Ser Ser Val Leu Gln Leu Arg Glu Thr Val Leu Gln Gln Lys Glu
55                  60                  65                  70

acc atc ctg agc cag aag gag acc atc cgc gag ctg acc gcc aag ctg       416
Thr Ile Leu Ser Gln Lys Glu Thr Ile Arg Glu Leu Thr Ala Lys Leu
                75                  80                  85

ggc cgc tgc gag agc cag agc acg ctg gac ccc gga gcc ggc gag gcc       464
Gly Arg Cys Glu Ser Gln Ser Thr Leu Asp Pro Gly Ala Gly Glu Ala
            90                  95                  100

cgg gcg ggc ggc ggc cgc aag cag ccc ggc tcg ggc aag aac acc atg     512
Arg Ala Gly Gly Gly Arg Lys Gln Pro Gly Ser Gly Lys Asn Thr Met
        105                 110                 115

ggc gac ctg tcc cgg aca ccg gcc gcc gag acg ctc agc caa ctc ggg     560
Gly Asp Leu Ser Arg Thr Pro Ala Ala Glu Thr Leu Ser Gln Leu Gly
    120                 125                 130

caa act ttg caa tcg ctc aaa acc cgc ctg gag aac ctc gag cag tac     608
Gln Thr Leu Gln Ser Leu Lys Thr Arg Leu Glu Asn Leu Glu Gln Tyr
135                 140                 145                 150

agc cgc ctc aat tcc tcc agc cag acc aac agc ctc aag gat ctg ctg     656
Ser Arg Leu Asn Ser Ser Ser Gln Thr Asn Ser Leu Lys Asp Leu Leu
                155                 160                 165

cag agc aag atc gat gag ctg gag agg cag gtg ctg tcc cgg gtg aac     704
Gln Ser Lys Ile Asp Glu Leu Glu Arg Gln Val Leu Ser Arg Val Asn
            170                 175                 180

acc ctg gag gag ggc aag ggg ggc ccc agg aac gac acc gag gag agg     752
Thr Leu Glu Glu Gly Lys Gly Gly Pro Arg Asn Asp Thr Glu Glu Arg
        185                 190                 195

gtc aag atc gag acc gcc ctg acc tcc ctg cac cag cgg atc agc gag     800
Val Lys Ile Glu Thr Ala Leu Thr Ser Leu His Gln Arg Ile Ser Glu
    200                 205                 210

ctc gag aaa ggt cag aaa gac aac cgc cct gga gac aag ttc cag ctc     848
Leu Glu Lys Gly Gln Lys Asp Asn Arg Pro Gly Asp Lys Phe Gln Leu
215                 220                 225                 230

aca ttc cca ctg cgg acc aac tat atg tat gcc aag gtg aag aag agc     896
Thr Phe Pro Leu Arg Thr Asn Tyr Met Tyr Ala Lys Val Lys Lys Ser
                235                 240                 245

ctg cca gag atg tac gcc ttc act gtc tgc atg tgg ctc aag tcc agc     944
Leu Pro Glu Met Tyr Ala Phe Thr Val Cys Met Trp Leu Lys Ser Ser
            250                 255                 260

gcc acg cca ggt gtg ggc acg ccc ttc tcc tac gct gtg ccc ggc cag     992
Ala Thr Pro Gly Val Gly Thr Pro Phe Ser Tyr Ala Val Pro Gly Gln

        265                 270                 275

gcc aac gag ctg gtc ctc att gag tgg ggc aac aac ccc atg gag atc 1040
Ala Asn Glu Leu Val Leu Ile Glu Trp Gly Asn Asn Pro Met Glu Ile
    280                 285                 290

ctc atc aat gac aag gtg gcc aag ttg cct ttt gtc atc aat gat ggc 1088
Leu Ile Asn Asp Lys Val Ala Lys Leu Pro Phe Val Ile Asn Asp Gly
295                 300                 305                 310

aag tgg cac cac atc tgt gtc acc tgg acc acc cgg gac ggg gtc tgg 1136
Lys Trp His His Ile Cys Val Thr Trp Thr Thr Arg Asp Gly Val Trp
                315                 320                 325

gag gcc tac cag gat ggc acg cag ggt ggc agt ggc gag aac ttg gcg 1184
Glu Ala Tyr Gln Asp Gly Thr Gln Gly Gly Ser Gly Glu Asn Leu Ala
            330                 335                 340

ccc tat cac ccc atc aag ccc cag ggc gtg ctg gtg ctg ggc cag gag 1232
Pro Tyr His Pro Ile Lys Pro Gln Gly Val Leu Val Leu Gly Gln Glu
        345                 350                 355

cag gac act ctg ggt ggt ggg ttt gat gcc acc cag gca ttt gtg ggt 1280
Gln Asp Thr Leu Gly Gly Gly Phe Asp Ala Thr Gln Ala Phe Val Gly
    360                 365                 370

gag ctg gcc cac ttc aac atc tgg gac cgc aag ctg acc ccc ggg gag 1328
Glu Leu Ala His Phe Asn Ile Trp Asp Arg Lys Leu Thr Pro Gly Glu
375                 380                 385                 390

gtg tac aac ctg gcc acc tgc agc acc aag gct ctg tcc ggc aat gtc 1376
Val Tyr Asn Leu Ala Thr Cys Ser Thr Lys Ala Leu Ser Gly Asn Val
                395                 400                 405

atc gcc tgg gct gaa tcc cac atc gag atc tac gga ggg gcc acc aag 1424
Ile Ala Trp Ala Glu Ser His Ile Glu Ile Tyr Gly Gly Ala Thr Lys
            410                 415                 420

tgg acc ttc gag gcc tgt cgc cag atc aac tga gcacggcagg ccaggctgag 1477
Trp Thr Phe Glu Ala Cys Arg Gln Ile Asn
        425                 430

cccgcccgcc ctcgccccct gcttgtgcgg cgatgatctg ttttgtgcgt ctcttctctc 1537

ccttttcccc aggaatgaac cgaggccgtc gcccctgcac acgcacacgc acacagcctg 1597

gttttgtcct catgcacacg aagcagcccc tgctcccatc tgtccctgag gaagccccac 1657

ttctctgtag gagcccggac tctctcaggc atgccccatt cacagctgaa gtgggtgctg 1717

caacgtcttg aacaaggcag aagttggtga gaggatctgt gtgtgcgtgt ctacatgtgt 1777

gtgtctacgt gtgtgcgtgc gtggctgggg gaggcctttt ctttgaggac gtacctcatt 1837

tccttctttc ttctggcttt ggaaaaatct catgatgaaa attcatattt gccaactttg 1897

ttagctgcgt gcgtgctttg gggttggtgc aacctcagta cacgcatttg tctttgtttg 1957

caaacctttc tcagagcgac atatctttat attgatgtaa taaatgtctt ttagtggttt 2017

gtcaaaggcc gggggcgggg gctctctaca gagaattttt attttgtaat agaagtgaac 2077

tgtctctgaa gggtgaaggc aggccgtcct gggatggtac cctgtgctct cccgtggagg 2137

agaggggatg gctgaggaca ctggccctta ccccagggcc agacagcatc catccctgct 2197

gtttgcatct gagagcagca tggggcctgg gaggtcggcc tgtgtgccca gctcagctag 2257

ctctgcccca ggacggccct gccctcgacc ttcccacctc ctcagatcct gcaaggctgg 2317

ggtctgcccc tcccttctca cctctggagc tgtgctgcac tgcttcagcc cagagggccc 2377

tgagagagga gcgtgccacc cacagcccgg gaagccgggc cccagcaccc ctctcctttg 2437

gcctccggca gtgcagacca gaggggacct tttaaggaaa gaagccgtgt ttcgatgaag 2497

acctggccac atggggccac tgggacttca acccagccca tcggtgggaa ggtccttttt 2557

gggggccttt gacagccata tccctcccag cacaccaggc gccaggtgag ctggttcaga 2617

cccctccagg ggtactccag agacctcacg tgtggagcca ggcctggcca gggcaggggc 2677

ctgaaaccca ctcctccatc tcatggggct cacggcctac agcagcccac aagctgccac 2737

tggccggcga cactgacacc tgagcagtgt ccagaacctt tttgcctttt tttgttcccc 2797

gtgaaaagca acatggacat ttccttctag tccttccaag gaggggagag aagtgtatgt 2857

gcatttgtgt gtgtgtgtgt gtgttgtgtg tgtgtgtgcg ctaagtgaga aagagagcag 2917

gctcgggagg ccctgcccag ggtaggagga gcttcctgct ttgcaccatc tggtggtcgc 2977

acgccctgag ggcaccccga ctctgtctcc aggagtctca tcagcaaacc gctgacaagt 3037

ctttctagaa attctactgc actgcctggc tcagctgcag ctgcagacat ttctgcagga 3097

ggagcaggtg tttctgtctt ctgttccttc tagggccacc tgtcccctta aacacaggtc 3157

cacgttgtgt caagaaccta gtgcatctgt gtgtgtctgt cagtgtctct gtgtcagtgt 3217

tcttgtgggt gtctgcacgg tacccggccg ccgttctgca atgcatcact cccgcagagg 3277

ggggtgcaga tcaggcgccg tgctgcgcgt tgttgttcaa cagtggcttt ttcttagata 3337

atcgtgcttc ctcagcgccc gtcgggttgt ggcatccttg gatctgcagg gatcttctcc 3397

gtttgcatgt tcctcggggt ggcgtgttcc ttgctccctg ggtccgacat gtgttcccgc 3457

acctgcatgg actgccccgg ttctgtgttg tgtgccgagt gccgcccagt gttctgtgac 3517

cacccgtgta gctactgaaa atggctgggt aagcaagtca agggtgttgg aggaggtcaa 3577

gagagagctc agtttccctc tccccctccc caaacacacc aagaagcatt tttaacgtgt 3637

aggttgagaa caagcctaaa ggattcccac agctgggagc cagcaagaga gcttggagtc 3697

gcctctctag accagatcta gccccaccct cactccagcc atctcggagc ccttgtgtag 3757

gcaacgcccg gtgcgggctg tgtggggtgc tcccctgcca gcacctccgg ccagccccgc 3817

ccctgccgat ctactggacc gcagaccacc ttctgccccc gtgggccagg tgggagctgt 3877

ccgttcagga ccatgagcca tcctctgccc tgactagcga ggggcagagc acaccccagt 3937

gcttacgcct ccacccctgc agcctcctgg cccgctcacc ttcctcaccc ctcctctgac 3997

ccacccatgg tgccagggcc gaagctgacc tttagctccc tcctgcccct tgctagggtc 4057

tgagccaagc ccctcgactc cctcactgtg ttgacacttg gcactttgct ggccccgaga 4117

aaggtcgatg acacagccgc aaatctaatc cacgtagttc ccatttactc cttaatctga 4177

ttgatgttcc ctcttgcact gaataataca tgcctctctc aggtaagcca ttttataaaa 4237

caagaagata aaaagcactg ttgaggcagt gtttgctttt gccgagctgg tgtccgacag 4297

ctccctgggt gtccggggtg ggagagctgt tgacagaagc tctccgggcc ctcaggggct 4357

tagatcccac ttgagtcgta agccttcttg cttttgataa cacagtatta tttctcttac 4417

tgtagaagaa aaagtttatt accaaacaag agtattttta tgaaagaaaa ggacaaacct 4477

ataaattaac tcaacctata tctcccttga aaatactttc aggctccacc aaaacgtaga 4537

actgaaagca tgtattttgg aagaaagaga tacattttgt atgctttctt ttccttttgt 4597

agattcccag tttattttct aagactgcaa agatcacttt gtcaccagcc ctgggacctg 4657

agaccaaggg ggtgtcttgt gggcagtgag ggggtgagga gaggctggca tgaggttcag 4717

tcattccagt gagctccaaa gaggggccac ctgttctcaa aagcatgttg gggaccagga 4777

ggtaaaactg gccatttatg gtgaacctgt gtcttggagc tgacttacta agtggaatga 4837

gccgaggatt tgaatatcag ttctaacctt gatagaagaa ccttgggtta catgtggttc 4897

acattaagag gatagaatcc tttggaatct tatggcaacc aaatgtggct tgacgaagtc 4957

gtggtttcat ctcttaaaca cagtgtgtaa atttattcaa ctaacgatgg gaaatgtatt 5017

acttctgtac acagtggact gaagtgcaat ttgttgaaag ggaacaagtc attgaagaga 5077

aaaaaaaaaa gcccaatact tagagtccca attttgtctc atttgccaaa aaaaaaaaaa 5137

aaaaaaaaaa gcaaaccccc tatggttgat attgttataa tgtatatact gtataatatg 5197

aaagagaatc gatgtatctc actttttcat tatttgctaa ccaaagctgt acatttttca 5257

tatgatctgc agccttttgg gtatcaaatg ggtcaaaacc atgggacctg ccacctccca 5317

tcagcaattc tggaaatgca ctatttctac tggtattctt gctttttttt tttttttcat 5377

tttcttgctg aaatgacatg aattgttgag tttattttta cccagtaaag agtggagaaa 5437

gact 5441

<210> 57
<211> 432
<212> PRT
<213> Homo sapiens

<400> 57

Met Pro Ala Gly Arg Ala Ala Arg Thr Cys Ala Leu Leu Ala Leu Cys
1               5                   10                  15

Leu Leu Gly Ala Gly Ala Gln Asp Phe Gly Pro Thr Arg Phe Ile Cys
            20                  25                  30

Thr Ser Val Pro Val Asp Ala Asp Met Cys Ala Ala Ser Val Ala Ala
        35                  40                  45

Gly Gly Ala Glu Glu Leu Arg Ser Ser Val Leu Gln Leu Arg Glu Thr
    50                  55                  60

Val Leu Gln Gln Lys Glu Thr Ile Leu Ser Gln Lys Glu Thr Ile Arg
65                  70                  75                  80

Glu Leu Thr Ala Lys Leu Gly Arg Cys Glu Ser Gln Ser Thr Leu Asp
                85                  90                  95

Pro Gly Ala Gly Glu Ala Arg Ala Gly Gly Gly Arg Lys Gln Pro Gly
            100                 105                 110

Ser Gly Lys Asn Thr Met Gly Asp Leu Ser Arg Thr Pro Ala Ala Glu
        115                 120                 125

Thr Leu Ser Gln Leu Gly Gln Thr Leu Gln Ser Leu Lys Thr Arg Leu
    130                 135                 140

Glu Asn Leu Glu Gln Tyr Ser Arg Leu Asn Ser Ser Ser Gln Thr Asn
145                 150                 155                 160

Ser Leu Lys Asp Leu Leu Gln Ser Lys Ile Asp Glu Leu Glu Arg Gln
                165                 170                 175

Val Leu Ser Arg Val Asn Thr Leu Glu Glu Gly Lys Gly Gly Pro Arg
            180                 185                 190

Asn Asp Thr Glu Glu Arg Val Lys Ile Glu Thr Ala Leu Thr Ser Leu
        195                 200                 205

His Gln Arg Ile Ser Glu Leu Glu Lys Gly Gln Lys Asp Asn Arg Pro
    210                 215                 220

Gly Asp Lys Phe Gln Leu Thr Phe Pro Leu Arg Thr Asn Tyr Met Tyr
225                 230                 235                 240

Ala Lys Val Lys Lys Ser Leu Pro Glu Met Tyr Ala Phe Thr Val Cys
                245                 250                 255

Met Trp Leu Lys Ser Ser Ala Thr Pro Gly Val Gly Thr Pro Phe Ser
            260                 265                 270

Tyr Ala Val Pro Gly Gln Ala Asn Glu Leu Val Leu Ile Glu Trp Gly
        275                 280                 285

Asn Asn Pro Met Glu Ile Leu Ile Asn Asp Lys Val Ala Lys Leu Pro
    290                 295                 300

Phe Val Ile Asn Asp Gly Lys Trp His His Ile Cys Val Thr Trp Thr
305                 310                 315                 320

Thr Arg Asp Gly Val Trp Glu Ala Tyr Gln Asp Gly Thr Gln Gly Gly
                325                 330                 335

Ser Gly Glu Asn Leu Ala Pro Tyr His Pro Ile Lys Pro Gln Gly Val
            340                 345                 350

Leu Val Leu Gly Gln Glu Gln Asp Thr Leu Gly Gly Gly Phe Asp Ala
        355                 360                 365

Thr Gln Ala Phe Val Gly Glu Leu Ala His Phe Asn Ile Trp Asp Arg
    370                 375                 380

Lys Leu Thr Pro Gly Glu Val Tyr Asn Leu Ala Thr Cys Ser Thr Lys
385                 390                 395                 400

Ala Leu Ser Gly Asn Val Ile Ala Trp Ala Glu Ser His Ile Glu Ile
                405                 410                 415

Tyr Gly Gly Ala Thr Lys Trp Thr Phe Glu Ala Cys Arg Gln Ile Asn
            420                 425                 430

<210>  58
<211>  1902

<212>  DNA
<213>  Homo sapiens

<220>
<221>  CDS
<222>  (86)..(1285)

<400>  58
cggccgcgta gcccgcgcgc ggagcgtacc ctgctgcggc cgttggccgt tagcgcggct      60

tcggcggttg tcttggagaa gcaag atg gcg gcg acg gcg gcc gca gtg gtg      112
                            Met Ala Ala Thr Ala Ala Ala Val Val
                            1               5

gcc gag gag gac acg gag ctg cgg gac ctg ctg gtg cag acg ctg gag      160
Ala Glu Glu Asp Thr Glu Leu Arg Asp Leu Leu Val Gln Thr Leu Glu
10                  15                  20                  25

aac agc ggg gtc ctg aac cgc atc aag gct gaa ctc cga gca gct gtg      208
Asn Ser Gly Val Leu Asn Arg Ile Lys Ala Glu Leu Arg Ala Ala Val
                30                  35                  40

ttt tta gca cta gag gag caa gaa aaa gta gag aac aaa act cct tta      256
Phe Leu Ala Leu Glu Glu Gln Glu Lys Val Glu Asn Lys Thr Pro Leu
            45                  50                  55

gtt aat gag agc ctg aaa aag ttt tta aat acc aaa gac ggt cgt tta      304
Val Asn Glu Ser Leu Lys Lys Phe Leu Asn Thr Lys Asp Gly Arg Leu
        60                  65                  70

gtg gct agt ctt gtt gca gaa ttt ctt cag ttt ttt aac ctt gac ttt      352
Val Ala Ser Leu Val Ala Glu Phe Leu Gln Phe Phe Asn Leu Asp Phe
    75                  80                  85

act ttg gct gtt ttt caa cct gaa act agc aca ctg caa ggt ctc gaa      400
Thr Leu Ala Val Phe Gln Pro Glu Thr Ser Thr Leu Gln Gly Leu Glu
90                  95                  100                 105

ggt cga gag aat tta gcc cga gat tta ggt ata att gaa gca gaa ggt      448
Gly Arg Glu Asn Leu Ala Arg Asp Leu Gly Ile Ile Glu Ala Glu Gly
                110                 115                 120

act gtg ggt gga ccc tta tta tta gaa gtg atc agg cgc tgt caa cag 496
Thr Val Gly Gly Pro Leu Leu Leu Glu Val Ile Arg Arg Cys Gln Gln
            125                 130                 135

aaa gaa aaa ggg cca acc act ggg gaa ggt gca ctt gat cta tct gat 544
Lys Glu Lys Gly Pro Thr Thr Gly Glu Gly Ala Leu Asp Leu Ser Asp
        140                 145                 150

gta cat tct cca cca aag tca cca gag gga aaa aca agt gca cag aca 592
Val His Ser Pro Pro Lys Ser Pro Glu Gly Lys Thr Ser Ala Gln Thr
    155                 160                 165

aca cca agt aag ata cca agg tat aaa gga caa ggt aag aag aag aca 640
Thr Pro Ser Lys Ile Pro Arg Tyr Lys Gly Gln Gly Lys Lys Lys Thr
170                 175                 180                 185

agc ggg cag aag gct ggt gac aag aag gcc aat gat gag gcc aat cag 688
Ser Gly Gln Lys Ala Gly Asp Lys Lys Ala Asn Asp Glu Ala Asn Gln
                190                 195                 200

agt gat aca agt gtc tcc ttg tca gaa ccc aag agc aaa agc agc ctt 736
Ser Asp Thr Ser Val Ser Leu Ser Glu Pro Lys Ser Lys Ser Ser Leu
            205                 210                 215

cac tta ctg tcc cat gaa aca aaa att gga tct ttt cta agc aac aga 784
His Leu Leu Ser His Glu Thr Lys Ile Gly Ser Phe Leu Ser Asn Arg
        220                 225                 230

act tta gat ggc aaa gac aaa gct ggc ctt tgt cca gat gaa gat gat 832
Thr Leu Asp Gly Lys Asp Lys Ala Gly Leu Cys Pro Asp Glu Asp Asp
    235                 240                 245

atg gaa gga gat tct ttc ttt gat gat ccc att cct aag cca gag aaa 880
Met Glu Gly Asp Ser Phe Phe Asp Asp Pro Ile Pro Lys Pro Glu Lys
250                 255                 260                 265

act tac ggt ttg agg aag gaa cct agg aag caa gca gga agt ctg gcc 928
Thr Tyr Gly Leu Arg Lys Glu Pro Arg Lys Gln Ala Gly Ser Leu Ala
                270                 275                 280

tcg ctc tcg gat gca ccc ccc tta aaa agt gga ctc agc tcc ctg gcg 976

Ser Leu Ser Asp Ala Pro Pro Leu Lys Ser Gly Leu Ser Ser Leu Ala
            285                 290                 295

gga gcc cct tct tta aaa gac tct gag agt aaa agg gga aat aca gtt 1024
Gly Ala Pro Ser Leu Lys Asp Ser Glu Ser Lys Arg Gly Asn Thr Val
        300                 305                 310

ttg aaa gat ctg aaa ttg atc agt gat aaa att gga tca ctt gga tta 1072
Leu Lys Asp Leu Lys Leu Ile Ser Asp Lys Ile Gly Ser Leu Gly Leu
    315                 320                 325

gga act gga gaa gat gat gac tat gtt gat gat ttt aat agt acc agc 1120
Gly Thr Gly Glu Asp Asp Asp Tyr Val Asp Asp Phe Asn Ser Thr Ser
330                 335                 340                 345

cat cgc tca gag aaa agt gag ata agt att ggt gaa gag ata gaa gaa 1168
His Arg Ser Glu Lys Ser Glu Ile Ser Ile Gly Glu Glu Ile Glu Glu
                350                 355                 360

gac ctt tct gtg gaa ata gat gac atc aat acc agt gat aag ctt gat 1216
Asp Leu Ser Val Glu Ile Asp Asp Ile Asn Thr Ser Asp Lys Leu Asp
            365                 370                 375

gac ctc aca caa gat ctg act gta tcc cag ctc agt gat gtt gcg gat 1264
Asp Leu Thr Gln Asp Leu Thr Val Ser Gln Leu Ser Asp Val Ala Asp
        380                 385                 390

tat ctg gaa gat gtt gca tag acacgaagaa ggaagtattc taattaacaa 1315
Tyr Leu Glu Asp Val Ala
    395

ggacagagga ctgaccggtt ccattttttt tttttccaga caatcactca gctggaatgt 1375

ctgctctcta ttggtgcctt gcatttcaaa aacactgcag atatttttta aaagtaattt 1435

tcattttact aaacaaaata cttcctattt gagcccatgt gtggaagatt taatattctt 1495

aatttaactg tacatttctt tatggaaatt gattatctac actcagtttc attacaggga 1555

aggaacccat gaaaacatca gtgttaagag catgatgaaa ggtgtcaata aagccgtagg 1615

atcgcgcaac cctttgtgtg tgtggctgct ggtacgtgtg atctttgaaa accttggctt 1675

tagccctctg gaatcagagc ttacccacca tagtatattt tgatattagg tggttctaca 1735

catagttggc aaaatgactt ggtaaatttg taatgctgaa gtatattagt ataagttaaa 1795

tttgatgtgt caactttatt ttgtatttcc ttccatttgg aaggtttgtt agaccattaa 1855

ggttatatta aagtactctt gtgtgtgcta aaaaaaaaaa aaaaaaa 1902

<210> 59
<211> 399
<212> PRT
<213> Homo sapiens

<400> 59

Met Ala Ala Thr Ala Ala Ala Val Val Ala Glu Glu Asp Thr Glu Leu
1               5                   10                  15

Arg Asp Leu Leu Val Gln Thr Leu Glu Asn Ser Gly Val Leu Asn Arg
            20                  25                  30

Ile Lys Ala Glu Leu Arg Ala Ala Val Phe Leu Ala Leu Glu Glu Gln
        35                  40                  45

Glu Lys Val Glu Asn Lys Thr Pro Leu Val Asn Glu Ser Leu Lys Lys
    50                  55                  60

Phe Leu Asn Thr Lys Asp Gly Arg Leu Val Ala Ser Leu Val Ala Glu
65                  70                  75                  80

Phe Leu Gln Phe Phe Asn Leu Asp Phe Thr Leu Ala Val Phe Gln Pro
                85                  90                  95

Glu Thr Ser Thr Leu Gln Gly Leu Glu Gly Arg Glu Asn Leu Ala Arg

            100                 105                 110

Asp Leu Gly Ile Ile Glu Ala Glu Gly Thr Val Gly Gly Pro Leu Leu
        115                 120                 125

Leu Glu Val Ile Arg Arg Cys Gln Gln Lys Glu Lys Gly Pro Thr Thr
    130                 135                 140

Gly Glu Gly Ala Leu Asp Leu Ser Asp Val His Ser Pro Pro Lys Ser
145                 150                 155                 160

Pro Glu Gly Lys Thr Ser Ala Gln Thr Thr Pro Ser Lys Ile Pro Arg
                165                 170                 175

Tyr Lys Gly Gln Gly Lys Lys Lys Thr Ser Gly Gln Lys Ala Gly Asp
            180                 185                 190

Lys Lys Ala Asn Asp Glu Ala Asn Gln Ser Asp Thr Ser Val Ser Leu
        195                 200                 205

Ser Glu Pro Lys Ser Lys Ser Ser Leu His Leu Leu Ser His Glu Thr
    210                 215                 220

Lys Ile Gly Ser Phe Leu Ser Asn Arg Thr Leu Asp Gly Lys Asp Lys
225                 230                 235                 240

Ala Gly Leu Cys Pro Asp Glu Asp Asp Met Glu Gly Asp Ser Phe Phe
                245                 250                 255

Asp Asp Pro Ile Pro Lys Pro Glu Lys Thr Tyr Gly Leu Arg Lys Glu
            260                 265                 270

Pro Arg Lys Gln Ala Gly Ser Leu Ala Ser Leu Ser Asp Ala Pro Pro
        275                 280                 285

Leu Lys Ser Gly Leu Ser Ser Leu Ala Gly Ala Pro Ser Leu Lys Asp
    290                 295                 300

Ser Glu Ser Lys Arg Gly Asn Thr Val Leu Lys Asp Leu Lys Leu Ile
305                 310                 315                 320

Ser Asp Lys Ile Gly Ser Leu Gly Leu Gly Thr Gly Glu Asp Asp Asp
                325                 330                 335

Tyr Val Asp Asp Phe Asn Ser Thr Ser His Arg Ser Glu Lys Ser Glu
            340                 345                 350

Ile Ser Ile Gly Glu Glu Ile Glu Glu Asp Leu Ser Val Glu Ile Asp
        355                 360                 365

Asp Ile Asn Thr Ser Asp Lys Leu Asp Asp Leu Thr Gln Asp Leu Thr
    370                 375                 380

Val Ser Gln Leu Ser Asp Val Ala Asp Tyr Leu Glu Asp Val Ala
385                 390                 395

<210>  60
<211>  2298
<212>  DNA
<213>  Homo sapiens

<220>
<221>  CDS
<222>  (80)..(925)

<400> 60
aatagaagat cgctcgggaa ttcttactct cgataaagat tataacaaca taggaaaatt 60

cttaaataga attttaggc atg gag gtg cat cag cag aat gcg tta ttt cag 112
Met Glu Val His Gln Gln Asn Ala Leu Phe Gln
1               5                   10

tat ttt gcg gac aca ctt act gca gtt gtt caa aat gcc aaa aaa aat 160
Tyr Phe Ala Asp Thr Leu Thr Ala Val Val Gln Asn Ala Lys Lys Asn
            15                  20                  25

gga aga tat gat atg gga atc tta gat ctt ggt tct gga gat gaa aaa 208
Gly Arg Tyr Asp Met Gly Ile Leu Asp Leu Gly Ser Gly Asp Glu Lys
        30                  35                  40

gtg cgg aaa agt gat gtt aaa aag ttt ctg act cca gga tat tca acc 256
Val Arg Lys Ser Asp Val Lys Lys Phe Leu Thr Pro Gly Tyr Ser Thr
    45                  50                  55

tct ggc cac gta gaa tta tac aca att agt gta gag agg gga atg tca 304
Ser Gly His Val Glu Leu Tyr Thr Ile Ser Val Glu Arg Gly Met Ser
60                  65                  70                  75

tgg gag gaa gct acc aag att tgg gct gag ctg aca gga cca gac gat 352
Trp Glu Glu Ala Thr Lys Ile Trp Ala Glu Leu Thr Gly Pro Asp Asp
                80                  85                  90

ggc ttt tac ttg tca ttg caa ata agg aac aac aag aaa act gcc atc 400
Gly Phe Tyr Leu Ser Leu Gln Ile Arg Asn Asn Lys Lys Thr Ala Ile
            95                  100                 105

tta gtt aaa gaa gtg aat cct aaa aag aaa ctt ttc tta gtt tat cga 448
Leu Val Lys Glu Val Asn Pro Lys Lys Lys Leu Phe Leu Val Tyr Arg
        110                 115                 120

cca aat act ggg aag cag ctc aaa tta gaa att tat gct gat cta aaa 496
Pro Asn Thr Gly Lys Gln Leu Lys Leu Glu Ile Tyr Ala Asp Leu Lys
    125                 130                 135

aag aaa tat aag aag gtc gtc tca gat gat gcc ctg atg cac tgg tta 544
Lys Lys Tyr Lys Lys Val Val Ser Asp Asp Ala Leu Met His Trp Leu

140                 145                 150                 155

gat cag tat aat tca tct gca gat act tgt act cat gct tat tgg cgc      592
Asp Gln Tyr Asn Ser Ser Ala Asp Thr Cys Thr His Ala Tyr Trp Arg
                160                 165                 170

ggc aat tgc aaa aaa gca agc ttg ggg cta gtt tgt gaa ata ggt ctt      640
Gly Asn Cys Lys Lys Ala Ser Leu Gly Leu Val Cys Glu Ile Gly Leu
            175                 180                 185

cgt tgc cgt aca tat tat gta tta tgt ggt tca gtg ctg agt gtc tgg      688
Arg Cys Arg Thr Tyr Tyr Val Leu Cys Gly Ser Val Leu Ser Val Trp
        190                 195                 200

aca aaa gtt gag ggt gtt cta gca tct gtc agt ggc aca aac gtg aag      736
Thr Lys Val Glu Gly Val Leu Ala Ser Val Ser Gly Thr Asn Val Lys
    205                 210                 215

atg cag atc gtg cgg cta aga acg gaa gat ggg caa cgg att gta ggt      784
Met Gln Ile Val Arg Leu Arg Thr Glu Asp Gly Gln Arg Ile Val Gly
220                 225                 230                 235

ttg atc att ccg gca aat tgt gtg tct cct ctt gta aat ctc cta tca      832
Leu Ile Ile Pro Ala Asn Cys Val Ser Pro Leu Val Asn Leu Leu Ser
                240                 245                 250

act tca gac cag tct caa cag ctt gcg gtc caa cag aaa cag cta tgg      880
Thr Ser Asp Gln Ser Gln Gln Leu Ala Val Gln Gln Lys Gln Leu Trp
            255                 260                 265

caa cag cat cac cct cag agc atc acc aac ttg agc aac gca tga          925
Gln Gln His His Pro Gln Ser Ile Thr Asn Leu Ser Asn Ala
        270                 275                 280

agaacagaca ggtttcaaca tggatggatc tgaaatgctg ttgaagcata tcatttgcat    985

aaaaatcagg gacagtttcc aaagaattat atattttttt cagttgtgct ctctagttag   1045

tttttttggg agtaaggaca aacctggaat agatagcaaa actgaaaatc agcagtgctg   1105

atggtggtac atatgtcttt cctttagctt ctcccctgat aattcccatc tgcttttact   1165

tcgggtgagc agagggggat gtgtgtgtgc gtgtgtgtca gtctgtttgt gagtgtgtta 1225

aaggctacag accacagttg gtttaaaatg cttggaactt cccaaactgg ctttacttta 1285

tgtttataca gtgctcaggg ttaacgcagt acatccatgc cattgctgtg ggaggtatcc 1345

ccggatgcat gtgttttgag tctataaata tagaaaatat atattggttt ctttttccaa 1405

cttaataggt ttattaaagc atgaaa[?]gaa aggttgcata tcatgcattc aggttatttt 1465

ctaatttttg ttctgacagt gcatgtcttt ggaagcatgc tgaaacaaga ttaacacagg 1525

agtcgagtaa cagagagaaa catttgttag atgtacagca ttggttattg catttttata 1585

gtgtttatac ctgggtattg cttcaaaccc tgcagacccc tccttcccct tctccctgcc 1645

ctgggtttct ggtcaaggta atgaatacat acatttttct gtgataaaac tcttaaaagt 1705

taattttaat gtattaatag tattcctcat gtgtgctgca gaaatggcta tgagcctctt 1765

aaatttacat ttgcaactta aaggtagttt tagaaggaag tacaaattgg ctttcatctt 1825

gcaaacaatc gttttttact tcattatctt aatttgcttt gtcactcata aaaaggaaac 1885

catacctgag ttgtagacaa [?]gaggaaaca cttgaggctt ctgctgtgtg ttcttttgtt 1945

attgttgtta ttgttgttac tcagtaactt gaatattgtt taatgtgttg taagacgtag 2005

agtttatctc aagctgttaa aaatggtaat gtacaaatgt gaatagacac ttatctatat 2065

aatatgggta agttttgttt cgcctataat agatgtttat aaaaacaagt gaggggacag 2125

ttggtctttt tatcttttct ttcttttttct ttcttttctt tttttctttt ttttcttttt 2185

tttttttttt gcttccacag gttgcactat tgaaaaatcg agattgtata aacctggtaa 2245

aaagctgcaa gatgccaaaa tcttgtagat gtcaaataaa aagttattat act 2298

<210> 61
<211> 281
<212> PRT

<213> Homo sapiens

<400> 61

Met Glu Val His Gln Gln Asn Ala Leu Phe Gln Tyr Phe Ala Asp Thr
1               5                   10                  15

Leu Thr Ala Val Val Gln Asn Ala Lys Lys Asn Gly Arg Tyr Asp Met
            20                  25                  30

Gly Ile Leu Asp Leu Gly Ser Gly Asp Glu Lys Val Arg Lys Ser Asp
        35                  40                  45

Val Lys Lys Phe Leu Thr Pro Gly Tyr Ser Thr Ser Gly His Val Glu
    50                  55                  60

Leu Tyr Thr Ile Ser Val Glu Arg Gly Met Ser Trp Glu Glu Ala Thr
65                  70                  75                  80

Lys Ile Trp Ala Glu Leu Thr Gly Pro Asp Asp Gly Phe Tyr Leu Ser
                85                  90                  95

Leu Gln Ile Arg Asn Asn Lys Lys Thr Ala Ile Leu Val Lys Glu Val
            100                 105                 110

Asn Pro Lys Lys Lys Leu Phe Leu Val Tyr Arg Pro Asn Thr Gly Lys
        115                 120                 125

Gln Leu Lys Leu Glu Ile Tyr Ala Asp Leu Lys Lys Lys Tyr Lys Lys
    130                 135                 140

Val Val Ser Asp Asp Ala Leu Met His Trp Leu Asp Gln Tyr Asn Ser
145                 150                 155                 160

Ser Ala Asp Thr Cys Thr His Ala Tyr Trp Arg Gly Asn Cys Lys Lys
                165                 170                 175

Ala Ser Leu Gly Leu Val Cys Glu Ile Gly Leu Arg Cys Arg Thr Tyr
            180                 185                 190

Tyr Val Leu Cys Gly Ser Val Leu Ser Val Trp Thr Lys Val Glu Gly
        195                 200                 205

Val Leu Ala Ser Val Ser Gly Thr Asn Val Lys Met Gln Ile Val Arg
    210                 215                 220

Leu Arg Thr Glu Asp Gly Gln Arg Ile Val Gly Leu Ile Ile Pro Ala
225                 230                 235                 240

Asn Cys Val Ser Pro Leu Val Asn Leu Leu Ser Thr Ser Asp Gln Ser
                245                 250                 255

Gln Gln Leu Ala Val Gln Gln Lys Gln Leu Trp Gln Gln His His Pro
            260                 265                 270

Gln Ser Ile Thr Asn Leu Ser Asn Ala
        275                 280

<210>  62
<211>  2960
<212>  DNA
<213>  Homo sapiens

<220>
<221>  CDS

<222>  (66)..(2648)

<400>  62
cggggcccca ggccgcgggg cggcgcggga cggcgggcgc cggcgccgca gatcagccag      60

tagac atg atg agc atc aaa gcc ttt acg ctt gtc tct gct gtg gag cgg      110
      Met Met Ser Ile Lys Ala Phe Thr Leu Val Ser Ala Val Glu Arg
      1               5                   10                  15

gag ctg ctg atg ggc gac aag gag cgc gtc aac ata gag tgc gtg gag      158
Glu Leu Leu Met Gly Asp Lys Glu Arg Val Asn Ile Glu Cys Val Glu
                20                  25                  30

tgc tgc ggc agg gac ctc tac gtg ggc acc aac gac tgc ttc gtc tac      206
Cys Cys Gly Arg Asp Leu Tyr Val Gly Thr Asn Asp Cys Phe Val Tyr
            35                  40                  45

cac ttc ctg ttg gag gag agg cca gtg cct gct ggg cca gcc acg ttc      254
His Phe Leu Leu Glu Glu Arg Pro Val Pro Ala Gly Pro Ala Thr Phe
        50                  55                  60

act gcc acc aaa cag ctg cag aga cac ttg ggc ttc aag aag ccc gtg      302
Thr Ala Thr Lys Gln Leu Gln Arg His Leu Gly Phe Lys Lys Pro Val
    65                  70                  75

aac gag ctg cgc gcg gcc tca gca ctc aac agg ctg ctg gtg ctg tgt      350
Asn Glu Leu Arg Ala Ala Ser Ala Leu Asn Arg Leu Leu Val Leu Cys
80                  85                  90                  95

gac aac tcc atc agc ctg gtc aac atg ctg aac ctc gag cca gtg cct      398
Asp Asn Ser Ile Ser Leu Val Asn Met Leu Asn Leu Glu Pro Val Pro
                100                 105                 110

tcg ggg gcc cgc atc aag ggg gca gcc acg ttt gca ctg aac gag aac      446
Ser Gly Ala Arg Ile Lys Gly Ala Ala Thr Phe Ala Leu Asn Glu Asn
            115                 120                 125

cct gtg agt ggg gac ccc ttc tgt gta gaa gtt tgc atc atc tct gtc      494
Pro Val Ser Gly Asp Pro Phe Cys Val Glu Val Cys Ile Ile Ser Val
        130                 135                 140

aaa cgc aga acc atc cag atg ttt ctg gtg tac gag gac cgg gtg cag      542

Lys Arg Arg Thr Ile Gln Met Phe Leu Val Tyr Glu Asp Arg Val Gln
    145                 150                 155

atc gtc aag gag gtg tcg act gcc gag cag ccc ctc gct gtg gct gtg 590
Ile Val Lys Glu Val Ser Thr Ala Glu Gln Pro Leu Ala Val Ala Val
160                 165                 170                 175

gac ggc cac ttc ctg tgt ctg gct ctg acc act cag tac atc atc cac 638
Asp Gly His Phe Leu Cys Leu Ala Leu Thr Thr Gln Tyr Ile Ile His
                180                 185                 190

aat tac agc aca ggc gtc tcc cag gac ctt ttt ccc tac tgc agt gag 686
Asn Tyr Ser Thr Gly Val Ser Gln Asp Leu Phe Pro Tyr Cys Ser Glu
            195                 200                 205

gag agg ccg ccg atc gtc aag agg ata ggg aga cag gag ttc ctg ctg 734
Glu Arg Pro Pro Ile Val Lys Arg Ile Gly Arg Gln Glu Phe Leu Leu
        210                 215                 220

gcg ggc ccc gga ggg ctg ggc atg ttt gcc aca gtc gca ggg ata tcc 782
Ala Gly Pro Gly Gly Leu Gly Met Phe Ala Thr Val Ala Gly Ile Ser
    225                 230                 235

cag cgt gcc ccc gtg cac tgg tcg gag aat gtg att ggg gcg gct gtg 830
Gln Arg Ala Pro Val His Trp Ser Glu Asn Val Ile Gly Ala Ala Val
240                 245                 250                 255

tcc ttt cca tac gtc ata gcg ctc gat gac gaa ttc atc aca gtc cac 878
Ser Phe Pro Tyr Val Ile Ala Leu Asp Asp Glu Phe Ile Thr Val His
                260                 265                 270

agc atg ttg gat cag caa cag aag cag acg ctg ccc ttt aag gag ggc 926
Ser Met Leu Asp Gln Gln Gln Lys Gln Thr Leu Pro Phe Lys Glu Gly
            275                 280                 285

cat atc cta cag gac ttt gaa gga aga gtg atc gtt gcc aca agt aaa 974
His Ile Leu Gln Asp Phe Glu Gly Arg Val Ile Val Ala Thr Ser Lys
        290                 295                 300

gga gtt tac atc ttg gtt cca tta cct ttg gaa aaa caa ata cag gat 1022
Gly Val Tyr Ile Leu Val Pro Leu Pro Leu Glu Lys Gln Ile Gln Asp
    305                 310                 315

ctt cta gca agc cgc aga gta gaa gag gct ttg gtt tta gca aaa gga 1070
Leu Leu Ala Ser Arg Arg Val Glu Glu Ala Leu Val Leu Ala Lys Gly
320                 325                 330                 335

gcc cgg agg aac att cca aag gaa aaa ttt cag gta atg tac aga agg 1118
Ala Arg Arg Asn Ile Pro Lys Glu Lys Phe Gln Val Met Tyr Arg Arg
                340                 345                 350

att ctg cag cag gcg gga ttt ata cag ttt gca caa ctt cag ttc ctg 1166
Ile Leu Gln Gln Ala Gly Phe Ile Gln Phe Ala Gln Leu Gln Phe Leu
            355                 360                 365

gaa gct aaa gag ctc ttc aga agc ggc cag ctt gat gtc cgg gag ctg 1214
Glu Ala Lys Glu Leu Phe Arg Ser Gly Gln Leu Asp Val Arg Glu Leu
        370                 375                 380

atc tct ctc tac ccc ttc ctg ttg ccc acc tcc tcc tcc ttc acc cgg 1262
Ile Ser Leu Tyr Pro Phe Leu Leu Pro Thr Ser Ser Ser Phe Thr Arg
    385                 390                 395

tcc cac cct cct ctt cat gag tac gca gac ctg aac cag ctg acc cag 1310
Ser His Pro Pro Leu His Glu Tyr Ala Asp Leu Asn Gln Leu Thr Gln
400                 405                 410                 415

ggg gac cag gag aag atg gcc aag tgc aaa cgc ttc ctc atg agc tac 1358
Gly Asp Gln Glu Lys Met Ala Lys Cys Lys Arg Phe Leu Met Ser Tyr
                420                 425                 430

ctg aac gag gtc cgc agc aca gag gta gca aat ggc tac aag gag gac 1406
Leu Asn Glu Val Arg Ser Thr Glu Val Ala Asn Gly Tyr Lys Glu Asp
            435                 440                 445

atc gac aca gcc ttg ctc aaa ctg tat gca gag gct gac cac gac agc 1454
Ile Asp Thr Ala Leu Leu Lys Leu Tyr Ala Glu Ala Asp His Asp Ser
        450                 455                 460

ctg ctg gac ctc ctg gtc act gag aac ttc tgt ctt ctg acg gac agt 1502
Leu Leu Asp Leu Leu Val Thr Glu Asn Phe Cys Leu Leu Thr Asp Ser
    465                 470                 475

gct gcc tgg cta gag aag cac aaa aag tat ttt gca ctt gga ctg ctc 1550

Ala Ala Trp Leu Glu Lys His Lys Lys Tyr Phe Ala Leu Gly Leu Leu
480                 485                 490                 495

tat cat tat aat aac caa gat gct gct gca gtt cag ttg tgg gtg aac      1598
Tyr His Tyr Asn Asn Gln Asp Ala Ala Ala Val Gln Leu Trp Val Asn
                500                 505                 510

att gtg aat ggc gat gtc cag gac tcc aca cgc tca gac ctg tat gaa      1646
Ile Val Asn Gly Asp Val Gln Asp Ser Thr Arg Ser Asp Leu Tyr Glu
            515                 520                 525

tac atc gtg gat ttt ctt acc tac tgc tta gac gag gaa cta gtg tgg      1694
Tyr Ile Val Asp Phe Leu Thr Tyr Cys Leu Asp Glu Glu Leu Val Trp
        530                 535                 540

gcc tat gct gat tgg gtc ctg cag aaa agt gaa gag gtc gga gtt cag      1742
Ala Tyr Ala Asp Trp Val Leu Gln Lys Ser Glu Glu Val Gly Val Gln
    545                 550                 555

gtt ttc acc aag aga cct ttg gat gaa cag cag aag aac agt ttt aat      1790
Val Phe Thr Lys Arg Pro Leu Asp Glu Gln Gln Lys Asn Ser Phe Asn
560                 565                 570                 575

cca gac gac att atc aat tgc ctt aaa aaa tac cct aaa gcc ctt gtg      1838
Pro Asp Asp Ile Ile Asn Cys Leu Lys Lys Tyr Pro Lys Ala Leu Val
                580                 585                 590

aag tat ctg gaa cat ctt gtg ata gac aag aga ctg cag aaa gaa gag      1886
Lys Tyr Leu Glu His Leu Val Ile Asp Lys Arg Leu Gln Lys Glu Glu
            595                 600                 605

tat cac acc cac tta gct gtg ctg tac ctg gaa gag gtg ctg ctg cag      1934
Tyr His Thr His Leu Ala Val Leu Tyr Leu Glu Glu Val Leu Leu Gln
        610                 615                 620

agg gcc tcc gcc agt ggc aag ggt gca gag gcc acc gag acg cag gcc      1982
Arg Ala Ser Ala Ser Gly Lys Gly Ala Glu Ala Thr Glu Thr Gln Ala
    625                 630                 635

aag ctg cgg cgg ctg ctc cag aaa tct gat tta tac cga gtc cac ttt      2030
Lys Leu Arg Arg Leu Leu Gln Lys Ser Asp Leu Tyr Arg Val His Phe
640                 645                 650                 655

ctt ctc gag agg ctg cag gga gct ggc ctg ccc atg gag agc gcc atc 2078
Leu Leu Glu Arg Leu Gln Gly Ala Gly Leu Pro Met Glu Ser Ala Ile
                660                 665                 670

ctg cac ggg aag ctg ggc gag cat gag aag gcg ctg cat atc ctg gtg 2126
Leu His Gly Lys Leu Gly Glu His Glu Lys Ala Leu His Ile Leu Val
            675                 680                 685

cac gag ctg cag gac ttt gca gcg gcc gag gac tac tgc ctg tgg tgc 2174
His Glu Leu Gln Asp Phe Ala Ala Ala Glu Asp Tyr Cys Leu Trp Cys
        690                 695                 700

tcc gag ggc cga gac cca ccc cac cgc cag caa ctc ttt cac acg ctg 2222
Ser Glu Gly Arg Asp Pro Pro His Arg Gln Gln Leu Phe His Thr Leu
    705                 710                 715

ctg gcc atc tac ctg cat gct ggc ccc act gcc cac gag ctg gcc gtg 2270
Leu Ala Ile Tyr Leu His Ala Gly Pro Thr Ala His Glu Leu Ala Val
720                 725                 730                 735

gct gcc gtg gac ctg ctg aac cgc cac gcc acc gaa ttt gat gca gcc 2318
Ala Ala Val Asp Leu Leu Asn Arg His Ala Thr Glu Phe Asp Ala Ala
                740                 745                 750

cag gtg ctg cag atg ctg cct gac acc tgg tca gtg cag ctc ctc tgc 2366
Gln Val Leu Gln Met Leu Pro Asp Thr Trp Ser Val Gln Leu Leu Cys
            755                 760                 765

cca ttc ctg atg ggg gcc atg agg gac agc atc cat gcc agg agg acc 2414
Pro Phe Leu Met Gly Ala Met Arg Asp Ser Ile His Ala Arg Arg Thr
        770                 775                 780

atg cag gtg gct ctc ggc ctg gcc agg tcc gaa aac tta atc tac acc 2462
Met Gln Val Ala Leu Gly Leu Ala Arg Ser Glu Asn Leu Ile Tyr Thr
    785                 790                 795

tac gat aag atg aag ttg aaa gga agc tca atc caa ctc tca gac aaa 2510
Tyr Asp Lys Met Lys Leu Lys Gly Ser Ser Ile Gln Leu Ser Asp Lys
800                 805                 810                 815

aag ctt tgt cag ata tgc caa aat ccc ttt tgt gag cct gtg ttt gtt 2558

Lys Leu Cys Gln Ile Cys Gln Asn Pro Phe Cys Glu Pro Val Phe Val
                820                 825                 830

aga tac cca aat ggt ggt ctt gtg cac acc cac tgt gcc gcc agc aga 2606
Arg Tyr Pro Asn Gly Gly Leu Val His Thr His Cys Ala Ala Ser Arg
            835                 840                 845

cac aca aac ccc agc tca tcc agt cct ggc act cgg act tga 2648
His Thr Asn Pro Ser Ser Ser Ser Pro Gly Thr Arg Thr
        850                 855                 860

aaagcttggc ccaagggtgc gaggggaact ccgagttctt tctgagcctg ctggacatga 2708

agagcagatg accaccatgc tctgtgtcag ccaggacgaa gggagacatc tgggtgccag 2768

ggagccttcc gtccatacgc aacaaggact cctcgctgct gaccaggctc tatgaatgta 2828

catggaatga ccaggctcta tgaatgtaca tggaaaaccc ctaaaaatcc ccaaaacaga 2888

aacatacaag agttcacctg tccaggtaca tagtaatcca agacagaggt ctactttgaa 2948

aaaaaaaaaa aa 2960

<210> 63
<211> 860
<212> PRT
<213> Homo sapiens

<400> 63

Met Met Ser Ile Lys Ala Phe Thr Leu Val Ser Ala Val Glu Arg Glu
1               5                   10                  15

Leu Leu Met Gly Asp Lys Glu Arg Val Asn Ile Glu Cys Val Glu Cys
            20                  25                  30

Cys Gly Arg Asp Leu Tyr Val Gly Thr Asn Asp Cys Phe Val Tyr His
        35                  40                  45

Phe Leu Leu Glu Glu Arg Pro Val Pro Ala Gly Pro Ala Thr Phe Thr
    50                  55                  60

Ala Thr Lys Gln Leu Gln Arg His Leu Gly Phe Lys Lys Pro Val Asn
65                  70                  75                  80

Glu Leu Arg Ala Ala Ser Ala Leu Asn Arg Leu Leu Val Leu Cys Asp
                85                  90                  95

Asn Ser Ile Ser Leu Val Asn Met Leu Asn Leu Glu Pro Val Pro Ser
            100                 105                 110

Gly Ala Arg Ile Lys Gly Ala Ala Thr Phe Ala Leu Asn Glu Asn Pro
        115                 120                 125

Val Ser Gly Asp Pro Phe Cys Val Glu Val Cys Ile Ile Ser Val Lys
    130                 135                 140

Arg Arg Thr Ile Gln Met Phe Leu Val Tyr Glu Asp Arg Val Gln Ile
145                 150                 155                 160

Val Lys Glu Val Ser Thr Ala Glu Gln Pro Leu Ala Val Ala Val Asp
                165                 170                 175

Gly His Phe Leu Cys Leu Ala Leu Thr Thr Gln Tyr Ile Ile His Asn
            180                 185                 190

Tyr Ser Thr Gly Val Ser Gln Asp Leu Phe Pro Tyr Cys Ser Glu Glu
        195                 200                 205

Arg Pro Pro Ile Val Lys Arg Ile Gly Arg Gln Glu Phe Leu Leu Ala

    210                 215                 220

Gly Pro Gly Gly Leu Gly Met Phe Ala Thr Val Ala Gly Ile Ser Gln
225                 230                 235                 240

Arg Ala Pro Val His Trp Ser Glu Asn Val Ile Gly Ala Ala Val Ser
                245                 250                 255

Phe Pro Tyr Val Ile Ala Leu Asp Asp Glu Phe Ile Thr Val His Ser
            260                 265                 270

Met Leu Asp Gln Gln Gln Lys Gln Thr Leu Pro Phe Lys Glu Gly His
        275                 280                 285

Ile Leu Gln Asp Phe Glu Gly Arg Val Ile Val Ala Thr Ser Lys Gly
    290                 295                 300

Val Tyr Ile Leu Val Pro Leu Pro Leu Glu Lys Gln Ile Gln Asp Leu
305                 310                 315                 320

Leu Ala Ser Arg Arg Val Glu Glu Ala Leu Val Leu Ala Lys Gly Ala
                325                 330                 335

Arg Arg Asn Ile Pro Lys Glu Lys Phe Gln Val Met Tyr Arg Arg Ile
            340                 345                 350

Leu Gln Gln Ala Gly Phe Ile Gln Phe Ala Gln Leu Gln Phe Leu Glu
        355                 360                 365

Ala Lys Glu Leu Phe Arg Ser Gly Gln Leu Asp Val Arg Glu Leu Ile
    370                 375                 380

Ser Leu Tyr Pro Phe Leu Leu Pro Thr Ser Ser Ser Phe Thr Arg Ser
385                 390                 395                 400

His Pro Pro Leu His Glu Tyr Ala Asp Leu Asn Gln Leu Thr Gln Gly
                405                 410                 415

Asp Gln Glu Lys Met Ala Lys Cys Lys Arg Phe Leu Met Ser Tyr Leu
            420                 425                 430

Asn Glu Val Arg Ser Thr Glu Val Ala Asn Gly Tyr Lys Glu Asp Ile
        435                 440                 445

Asp Thr Ala Leu Leu Lys Leu Tyr Ala Glu Ala Asp His Asp Ser Leu
    450                 455                 460

Leu Asp Leu Leu Val Thr Glu Asn Phe Cys Leu Leu Thr Asp Ser Ala
465                 470                 475                 480

Ala Trp Leu Glu Lys His Lys Lys Tyr Phe Ala Leu Gly Leu Leu Tyr
                485                 490                 495

His Tyr Asn Asn Gln Asp Ala Ala Ala Val Gln Leu Trp Val Asn Ile
            500                 505                 510

Val Asn Gly Asp Val Gln Asp Ser Thr Arg Ser Asp Leu Tyr Glu Tyr
        515                 520                 525

Ile Val Asp Phe Leu Thr Tyr Cys Leu Asp Glu Glu Leu Val Trp Ala
    530                 535                 540

Tyr Ala Asp Trp Val Leu Gln Lys Ser Glu Glu Val Gly Val Gln Val

545                 550                 555                 560

Phe Thr Lys Arg Pro Leu Asp Glu Gln Gln Lys Asn Ser Phe Asn Pro
                565                 570                 575

Asp Asp Ile Ile Asn Cys Leu Lys Lys Tyr Pro Lys Ala Leu Val Lys
            580                 585                 590

Tyr Leu Glu His Leu Val Ile Asp Lys Arg Leu Gln Lys Glu Glu Tyr
        595                 600                 605

His Thr His Leu Ala Val Leu Tyr Leu Glu Glu Val Leu Leu Gln Arg
    610                 615                 620

Ala Ser Ala Ser Gly Lys Gly Ala Glu Ala Thr Glu Thr Gln Ala Lys
625                 630                 635                 640

Leu Arg Arg Leu Leu Gln Lys Ser Asp Leu Tyr Arg Val His Phe Leu
                645                 650                 655

Leu Glu Arg Leu Gln Gly Ala Gly Leu Pro Met Glu Ser Ala Ile Leu
            660                 665                 670

His Gly Lys Leu Gly Glu His Glu Lys Ala Leu His Ile Leu Val His
        675                 680                 685

Glu Leu Gln Asp Phe Ala Ala Ala Glu Asp Tyr Cys Leu Trp Cys Ser
    690                 695                 700

Glu Gly Arg Asp Pro Pro His Arg Gln Gln Leu Phe His Thr Leu Leu
705                 710                 715                 720

Ala Ile Tyr Leu His Ala Gly Pro Thr Ala His Glu Leu Ala Val Ala
                725                 730                 735

Ala Val Asp Leu Leu Asn Arg His Ala Thr Glu Phe Asp Ala Ala Gln
            740                 745                 750

Val Leu Gln Met Leu Pro Asp Thr Trp Ser Val Gln Leu Leu Cys Pro
        755                 760                 765

Phe Leu Met Gly Ala Met Arg Asp Ser Ile His Ala Arg Arg Thr Met
    770                 775                 780

Gln Val Ala Leu Gly Leu Ala Arg Ser Glu Asn Leu Ile Tyr Thr Tyr
785                 790                 795                 800

Asp Lys Met Lys Leu Lys Gly Ser Ser Ile Gln Leu Ser Asp Lys Lys
                805                 810                 815

Leu Cys Gln Ile Cys Gln Asn Pro Phe Cys Glu Pro Val Phe Val Arg
            820                 825                 830

Tyr Pro Asn Gly Gly Leu Val His Thr His Cys Ala Ala Ser Arg His
        835                 840                 845

Thr Asn Pro Ser Ser Ser Ser Pro Gly Thr Arg Thr
    850                 855                 860

<210>  64
<211>  2495
<212>  DNA
<213>  Homo sapiens

<220>
<221> CDS
<222> (196)..(1881)

<400> 64
   gggtggtgga tctgtcggtc ccgttttccc gtcgcacgtg gtggccactg ttggcttctg 60
   aatggtttgc aaggcggata tccacgccaa ggcctttgga tcggccgtgg gtacatccgt 120
   ctgagccgtt cctttccatc gcagagcggc ggcctccggc ggcgctctcc agtcatggac 180
   taccggcggc ttctc atg agc cgg gtg gtc ccc ggg caa ttc gac gac gcg 231
   gac tcc tct gac agt gaa aac aga gac ttg aag aca gtc aaa gag aag 279
   gat gac att ctg ttt gaa gac ctt caa gac aat gtg aat gag aat ggt 327
   gaa ggt gaa ata gaa gat gag gag gag gag ggt tat gac gat gat gat 375
   gat gac tgg gac tgg gat gaa gga gtt gga aaa ctc gcc aag ggt tat 423
   gtc tgg aat gga gga agc aac cca cag gca aat cga cag acc tcc gac 471
   agc agt tca gcc aaa atg tct act cca gca gac aag gtc tta cgg aaa 519
   ttt gag aat aaa att aat tta gat aag cta aat gtt act gat tcc gtc 567
   ata aat aaa gtc acc gaa aag tct aga caa aag gaa gca gat atg tat 615
   cgc atc aaa gat aag gca gac aga gca act gta gaa cag gtg ttg gat 663
   ccc aga aca aga atg att tta ttc aag atg ttg act aga gga atc ata 711
   aca gag ata aat ggc tgc att agc aca gga aaa gaa gct aat gta tac 759
   cat gct agc aca gca aat gga gag agc aga gca atc aaa att tat aaa 807
   act tct att ttg gtg ttc aaa gat cgg gat aaa tat gta agt gga gaa 855
   ttc aga ttt cgt cat ggc tat tgt aaa gga aac cct agg aaa atg gtg 903
   aaa act tgg gca gaa aaa gaa atg agg aac tta atc agg cta aac aca 951
   gca gag ata cca tgt cca gaa cca ata atg cta aga agt cat gtt ctt 999
   gtc atg agt ttc atc ggt aaa gat gac atg cct gca cca ctc ttg aaa 1047
   aat gtc cag tta tca gaa tcc aag gct cgg gag ttg tac ctg cag gtc 1095
   att cag tac atg aga aga atg tat cag gat gcc aga ctt gtc cat gca 1143
   gat ctc agt gaa ttt aac atg ctg tac cac ggt gga ggc gtg tat atc 1191
   att gac gtg tct cag tcc gtg gag cac gac cac cca cat gcc ttg gag 1239
   ttc ttg aga aag gat tgc gcc aac gtc aat gat ttc ttt atg agg cac 1287
   agt gtt gct gtc atg act gtg cgg gag ctc ttt gaa ttt gtc aca gat 1335
   cca tcc att aca cat gag aac atg gat gct tat ctc tca aag gcc atg 1383
   gaa ata gca tct caa agg acc aag gaa gaa cgg tct agc caa gat cat 1431
   gtg gat gaa gag gtg ttt aag cga gca tat att cct aga acc ttg aat 1479
   gaa gtg aaa aat tat gag agg gat atg gac ata att atg aaa ttg aag 1527
   gaa gag gac atg gcc atg aat gcc caa caa gat aat att cta tac cag 1575
   act gtt aca gga ttg aag aaa gat ttg tca gga gtt cag aag gtc cct      1623
   gca ctc cta gaa aat caa gtg gag gaa agg act tgt tct gat tca gaa      1671
   gat att gga agc tct gag tgc tct gac aca gac tct gaa gag cag gga      1719
   gac cat gcc cgc ccc aag aaa cac acc acg gac cct gac att gat aaa      1767
   aaa gaa aga aaa aag atg gtc aag gaa gcc cag aga gag aaa aga aaa      1815
   aac aaa att cct aaa cat gtg aaa aaa aga aag gag aag aca gcc aag      1863
   acg aaa aaa ggc aaa tag aatgagaacc atattatgta cagtcatttt             1911
   cctcagttcc ttttctcgcc tgaactctta agctgcatct ggaagatggc ttattggttt    1971
   taaccagatt gtcatcgtgg cactgtctgt gaagacggat tcaaatgttt tcatgtaact    2031
   atgtaaaaag ctctaagctc tagagtctag atccagtcac tgactctgtc tggtgttgac    2091
   agaggattta tttaagctat tattttaata aagaactttg tacattttta tttttatatt    2151
   tttttctctt acaaatatgt ttttggaagc atgataaatg tttaaatgta gtcaacatct    2211
   gtaactctta catgagtgtc cagaggcact catgggaaaa ttggttttgc tttctttgta    2271
   cacaccagag acccatctga ggtcatctga ttataaggcc atgtttatat aaagggaatt 2331
   tcacccacag ttcagctggc tgttgatttt cactgcaact ctgcctttgt gtgtattggc 2391
   gatcatttgt aatgctctta cacttcgtct ttaatgttct ttttggagtt aggacctctc 2451
   agttcataaa gttttttaca attcaaaaaa aaaaaaaaaa aaaa 2495

<210> 65
<211> 561
<212> PRT
<213> Homo sapiens

<400> 65

<210> 66
<211> 3254
<212> DNA
<213> Homo sapiens

<220>
<221> CDS

<222>  (241)..(1527)

<400>  66
ggcggcggcc gcgcgctgct cggggcccga ctgcggcgcg agggcggcgc gggagcgacg      60

ctggcccgga ccgaggaaac tatgccgcag acgtccgttg tcttctccag catccttggg     120

cccagctgta gcggacaggt gcagcctggc atgggggagc gtggaggcgg ggccggtggc     180

ggctccgggg acctcatctt ccaagatgga cacctcatct ctgggtccct ggaggccctg     240

atg gag cac ctt gtt ccc acg gtg gac tat tac ccc gat agg acg tac       288
Met Glu His Leu Val Pro Thr Val Asp Tyr Tyr Pro Asp Arg Thr Tyr
1               5                   10                  15

atc ttc acc ttt ctc ctg agc tcc cgg gtc ttt atg ccc cct cat gac       336
Ile Phe Thr Phe Leu Leu Ser Ser Arg Val Phe Met Pro Pro His Asp
            20                  25                  30

ctg ctg gcc cgc gtg ggg cag atc tgc gtg gag cag aag cag cag ctg       384
Leu Leu Ala Arg Val Gly Gln Ile Cys Val Glu Gln Lys Gln Gln Leu
        35                  40                  45

gaa gcc ggg cct gaa aag gcc aag ctg aag tct ttc tca gcc aag atc       432
Glu Ala Gly Pro Glu Lys Ala Lys Leu Lys Ser Phe Ser Ala Lys Ile
    50                  55                  60

gtg cag ctc ctg aag gag tgg acc gag gcc ttc ccc tat gac ttc cag       480
Val Gln Leu Leu Lys Glu Trp Thr Glu Ala Phe Pro Tyr Asp Phe Gln
65                  70                  75                  80

gat gag aag gcc atg gcc gag ctg aaa gcc atc aca cac cgt gtc acc       528
Asp Glu Lys Ala Met Ala Glu Leu Lys Ala Ile Thr His Arg Val Thr
                85                  90                  95

cag tgt gat gag gag aat ggc aca gtg aag aag gcc att gcc cag atg       576
Gln Cys Asp Glu Glu Asn Gly Thr Val Lys Lys Ala Ile Ala Gln Met
            100                 105                 110

aca cag agc ctg ttg ctg tcc ttg gct gcc cgg agc cag ctc cag gaa       624
Thr Gln Ser Leu Leu Leu Ser Leu Ala Ala Arg Ser Gln Leu Gln Glu
        115                 120                 125

ctg cga gag aag ctc cgg cca ccg gct gta gac aag ggg ccc atc ctc 672
Leu Arg Glu Lys Leu Arg Pro Pro Ala Val Asp Lys Gly Pro Ile Leu
    130                 135                 140

aag acc aag cca cca gcc gcc cag aag gac atc ctg ggc gtg tgc tgc 720
Lys Thr Lys Pro Pro Ala Ala Gln Lys Asp Ile Leu Gly Val Cys Cys
145                 150                 155                 160

gac ccc ctg gtg ctg gcc cag cag ctg act cac att gag ctg gac agg 768
Asp Pro Leu Val Leu Ala Gln Gln Leu Thr His Ile Glu Leu Asp Arg
                165                 170                 175

gtc agc agc att tac cct gag gac ttg atg cag atc gtc agc cac atg 816
Val Ser Ser Ile Tyr Pro Glu Asp Leu Met Gln Ile Val Ser His Met
            180                 185                 190

gac tca ttg gac aac cac agg tgc cga ggg gac ctg acc aag acc tac 864
Asp Ser Leu Asp Asn His Arg Cys Arg Gly Asp Leu Thr Lys Thr Tyr
        195                 200                 205

agc ctg gag gcc tat gac aac tgg ttc aac tgc ctg agc atg ctg gtg 912
Ser Leu Glu Ala Tyr Asp Asn Trp Phe Asn Cys Leu Ser Met Leu Val
    210                 215                 220

gcc act gag gtg tgc cgg gtg gtg aag aag aaa cac cgg acc cgc atg 960
Ala Thr Glu Val Cys Arg Val Val Lys Lys Lys His Arg Thr Arg Met
225                 230                 235                 240

ttg gag ttc ttc att gat gtg gcc cgg gag tgc ttc aac atc ggg aac 1008
Leu Glu Phe Phe Ile Asp Val Ala Arg Glu Cys Phe Asn Ile Gly Asn
                245                 250                 255

ttc aac tcc atg atg gcc atc atc tct ggc atg aac ctc agt cct gtg 1056
Phe Asn Ser Met Met Ala Ile Ile Ser Gly Met Asn Leu Ser Pro Val
            260                 265                 270

gca agg ctg aag aaa act tgg tcc aag gtc aag aca gcc aag ttt gat 1104
Ala Arg Leu Lys Lys Thr Trp Ser Lys Val Lys Thr Ala Lys Phe Asp
        275                 280                 285

gtc ttg gag cat cac atg gac ccg tcc agc aac ttc tgc aac tac cgt 1152

Val Leu Glu His His Met Asp Pro Ser Ser Asn Phe Cys Asn Tyr Arg
    290                 295                 300

aca gcc ctg cag ggg gcc acg cag agg tcc cag atg gcc aac agc agc 1200
Thr Ala Leu Gln Gly Ala Thr Gln Arg Ser Gln Met Ala Asn Ser Ser
305                 310                 315                 320

cgt gaa aag atc gtc atc cct gtg ttc aac ctc ttc gtt aag gac atc 1248
Arg Glu Lys Ile Val Ile Pro Val Phe Asn Leu Phe Val Lys Asp Ile
                325                 330                 335

tac ttc ctg cac aaa atc cat acc aac cac ctg ccc aac ggg cac att 1296
Tyr Phe Leu His Lys Ile His Thr Asn His Leu Pro Asn Gly His Ile
            340                 345                 350

aac ttt aag aaa ttc tgg gag atc tcc aga cag atc cat gag ttc atg 1344
Asn Phe Lys Lys Phe Trp Glu Ile Ser Arg Gln Ile His Glu Phe Met
        355                 360                 365

aca tgg aca cag gta gag tgt cct ttc gag aag gac aag aag att cag 1392
Thr Trp Thr Gln Val Glu Cys Pro Phe Glu Lys Asp Lys Lys Ile Gln
    370                 375                 380

agt tac ctg ctc acg gcg ccc atc tac agc gag gaa gct ctc ttc gtc 1440
Ser Tyr Leu Leu Thr Ala Pro Ile Tyr Ser Glu Glu Ala Leu Phe Val
385                 390                 395                 400

gcc tcc ttt gaa agt gag ggt ccc gag aac cac gtg gaa aaa gac agc 1488
Ala Ser Phe Glu Ser Glu Gly Pro Glu Asn His Val Glu Lys Asp Ser
                405                 410                 415

tgg aag acc ctc agg acc acc ctt ctg aac aga gcc tga ggcggatgca 1537
Trp Lys Thr Leu Arg Thr Thr Leu Leu Asn Arg Ala
            420                 425

gcccgcgacg ccagaggaag cacgtgcact aactgggttt aaattttgac tgatgtgggt 1597

tgagatgagg aggcctcact ggttggggtc cattttgtat ataactttta tgagaaaaaa 1657

atggtaatta tttcacgcat caacctttgg cacttacaaa gttttttttg tttattttaa 1717

ataacagggc agggccctgc tttggggagg gggaggggag agtatcatgg gagatggtat 1777

ccatgataac atcttattct aatgaaatgt agatttttat tttctacttt tgattattga 1837

catcttatga aaaaaatatt ttaaaaaacc cagccaaaac caacgtgagc cctgcctgct 1897

cggacgcctt tccagccagt gtctctgacg tcggggttag tgccttagag ggtactgggg 1957

tctggtcttc ctgctctgtg gtttgggctg cggtgagtcc cactccacct gggcgcctgc 2017

cctcaggagc ctgggctgcg aggctccata ggagggctgg tggctgggag gtcgcgtccg 2077

cacacttctg gaagtgagcc tttgagtacg ggctgtccaa agtttacatt ttcattttcc 2137

tttcagggat ttgcggggtc agggaggggc agggggcacc tggcagcata ttttctgtga 2197

caatgtgtcc agcaaatcat tcttcaacta cattttagaa aggaggaaat ctaaaataag 2257

gtaagggagg gaagcatgga gttgtcagtt ttctgggctg tgactgaaag acacactgag 2317

ctgtgatgaa gaaaaataca tggccgactc cagggtggtg acatttagag ctagtcttga 2377

aacctatcat ctacagaggg gagggcagcc aacagccctc ttcccacctg ggtaggcagc 2437

gccctaattg gaattggaaa cagaaaattc gccaggccat actgctggag cccattcaga 2497

taaaactgcc caaatactga gaggtgtttt ctacacccag ctagaggagc acactccatt 2557

ttcccatgtc tgacttcgtg gtgtgagccc tgggccctac tgaccatggc gcaggacagc 2617

tgtccttcag aaagcacacg gtcaatccac gtggaccgtc tccctcgcag gaactccgca 2677

tccttgtccc tctctgcatt cccagtttcc gcaggagcct tgatcaatgg ggaagcctgg 2737

gtgaggatgg gccaggtccc aattcccaaa gctcctggaa gagcctgaag acattgggaa 2797

aggctgggcc tggggaggag gcagccctgg gcccgttgcc catgcctctg gtcctgggtg 2857

gagcaggaat agttccactg tattgtcaca gtgtgtttgc actttctgag gttctagcta 2917

gtacagattg tatattgata gtacatattg ctttgtttat gtctttgaga tgagaaaggc 2977

ttaaaacttg agaatatata tttggaatac agccttagaa cggtttctgt acacatccac 3037

gtgcacttca cgggtgatca gttctagtac ctacttgaaa cagtgtctgt ctgctacttt 3097

attttcccaa tttgatacat accctgattt gatgttttgg tatttgagat gaactctgag 3157

tatgaagctg taccataatg cagggcgtca gttttggtgt gactggacat acttgcttca 3217

ataaaagaat acatcactcc caaaaaaaaa aaaaaaa 3254

<210> 67
<211> 428
<212> PRT
<213> Homo sapiens

<400> 67

Met Glu His Leu Val Pro Thr Val Asp Tyr Tyr Pro Asp Arg Thr Tyr
1               5                   10                  15

Ile Phe Thr Phe Leu Leu Ser Ser Arg Val Phe Met Pro Pro His Asp
            20                  25                  30

Leu Leu Ala Arg Val Gly Gln Ile Cys Val Glu Gln Lys Gln Gln Leu
        35                  40                  45

Glu Ala Gly Pro Glu Lys Ala Lys Leu Lys Ser Phe Ser Ala Lys Ile
    50                  55                  60

Val Gln Leu Leu Lys Glu Trp Thr Glu Ala Phe Pro Tyr Asp Phe Gln
65                  70                  75                  80

Asp Glu Lys Ala Met Ala Glu Leu Lys Ala Ile Thr His Arg Val Thr
                85                  90                  95

Gln Cys Asp Glu Glu Asn Gly Thr Val Lys Lys Ala Ile Ala Gln Met

            100                 105                 110

Thr Gln Ser Leu Leu Leu Ser Leu Ala Ala Arg Ser Gln Leu Gln Glu
        115                 120                 125

Leu Arg Glu Lys Leu Arg Pro Pro Ala Val Asp Lys Gly Pro Ile Leu
    130                 135                 140

Lys Thr Lys Pro Pro Ala Ala Gln Lys Asp Ile Leu Gly Val Cys Cys
145                 150                 155                 160

Asp Pro Leu Val Leu Ala Gln Gln Leu Thr His Ile Glu Leu Asp Arg
                165                 170                 175

Val Ser Ser Ile Tyr Pro Glu Asp Leu Met Gln Ile Val Ser His Met
            180                 185                 190

Asp Ser Leu Asp Asn His Arg Cys Arg Gly Asp Leu Thr Lys Thr Tyr
        195                 200                 205

Ser Leu Glu Ala Tyr Asp Asn Trp Phe Asn Cys Leu Ser Met Leu Val
    210                 215                 220

Ala Thr Glu Val Cys Arg Val Val Lys Lys Lys His Arg Thr Arg Met
225                 230                 235                 240

Leu Glu Phe Phe Ile Asp Val Ala Arg Glu Cys Phe Asn Ile Gly Asn
                245                 250                 255

Phe Asn Ser Met Met Ala Ile Ile Ser Gly Met Asn Leu Ser Pro Val
            260                 265                 270

Ala Arg Leu Lys Lys Thr Trp Ser Lys Val Lys Thr Ala Lys Phe Asp
        275                 280                 285

Val Leu Glu His His Met Asp Pro Ser Ser Asn Phe Cys Asn Tyr Arg
    290                 295                 300

Thr Ala Leu Gln Gly Ala Thr Gln Arg Ser Gln Met Ala Asn Ser Ser
305                 310                 315                 320

Arg Glu Lys Ile Val Ile Pro Val Phe Asn Leu Phe Val Lys Asp Ile
                325                 330                 335

Tyr Phe Leu His Lys Ile His Thr Asn His Leu Pro Asn Gly His Ile
            340                 345                 350

Asn Phe Lys Lys Phe Trp Glu Ile Ser Arg Gln Ile His Glu Phe Met
        355                 360                 365

Thr Trp Thr Gln Val Glu Cys Pro Phe Glu Lys Asp Lys Lys Ile Gln
    370                 375                 380

Ser Tyr Leu Leu Thr Ala Pro Ile Tyr Ser Glu Glu Ala Leu Phe Val
385                 390                 395                 400

Ala Ser Phe Glu Ser Glu Gly Pro Glu Asn His Val Glu Lys Asp Ser
                405                 410                 415

Trp Lys Thr Leu Arg Thr Thr Leu Leu Asn Arg Ala
            420                 425

<210>  68

<211>  51
<212>  DNA
<213>  Artificial

<220>
<221>  Source
<223>  /note="Description of Artificial Sequence: An artificially synthesized
oligonucleotide for siRNA"

<400>  68
tcccggaaga attggttctt gaattcaaga gattcaagaa ccaattcttc c          51

<210>  69
<211>  51
<212>  DNA
<213>  Artificial

<220>
<221>  Source
<223>  /note="Description of Artificial Sequence: An artificially synthesized
oligonucleotide for siRNA"

<400>  69
aaaaggaaga attggttctt gaatctcttg aattcaagaa ccaattcttc c          51

<210>  70
<211>  47
<212>  DNA
<213>  Artificial

<220>
<221>  Source
<223>  /note="Description of Artificial Sequence: siRNA hairpin design"

<400>  70
ggaagaattg gttcttgaat tcaagagatt caagaaccaa ttcttcc          47

<210>  71
<211>  51

<212>  DNA
<213>  Artificial

<220>
<221>  Source
<223>   /note="Description of Artificial Sequence: An artificially synthesized
oligonucleotide for siRNA"

<400>  71
tcccgatgtg gctcaactca aagttcaaga gactttgagt tgagccacat c              51

<210>  72
<211>  51
<212>  DNA
<213>  Artificial

<220>
<221>  Source
<223>   /note="Description of Artificial Sequence: An artificially synthesized
oligonucleotide for siRNA"

<400>  72
aaaagatgtg gctcaactca aagtctcttg aactttgagt tgagccacat c              51

<210>  73
<211>  47
<212>  DNA
<213>  Artificial

<220>
<221>  Source
<223>  /note="Description of Artificial Sequence: siRNA hairpin design"

<400>  73
gatgtggctc aactcaaagt tcaagagact ttgagttgag ccacatc                   47

<210>  74
<211>  51
<212>  DNA

<213>  Artificial

<220>
<221>  Source
<223>  /note="Description of Artificial Sequence: An artificially synthesized
oligonucleotide for siRNA"

<400>  74
tcccgcagct gcgaagtgtt gtattcaaga gatacaacac ttcgcagctg c          51

<210>  75
<211>  51
<212>  DNA
<213>  Artificial

<220>
<221>  Source
<223>  /note="Description of Artificial Sequence: An artificially synthesized
oligonucleotide for siRNA"

<400>  75
aaaagcagct gcgaagtgtt gtatctcttg aatacaacac ttcgcagctg c          51

<210>  76
<211>  47
<212>  DNA
<213>  Artificial

<220>
<221>  Source
<223>  /note="Description of Artificial Sequence: siRNA hairpin design"

<400>  76
gcagctgcga agtgttgtat tcaagagata caacacttcg cagctgc          47

<210>  77
<211>  51
<212>  DNA
<213>  Artificial

<220>
<221>  Source
<223>   /note="Description of Artificial Sequence: An artificially synthesized
oligonucleotide for siRNA"

<400>  77
tcccgatacg aaagcagctg cgattcaaga gatcgcagct gctttcgtat c          51

<210>  78
<211>  51
<212>  DNA
<213>  Artificial

<220>
<221>  Source
<223>   /note="Description of Artificial Sequence: An artificially synthesized
oligonucleotide for siRNA"

<400>  78
aaaagatacg aaagcagctg cgatctcttg aatcgcagct gctttcgtat c          51

<210>  79
<211>  47
<212>  DNA
<213>  Artificial

<220>
<221>  Source
<223>  /note="Description of Artificial Sequence: siRNA hairpin design"

<400>  79
gatacgaaag cagctgcgat tcaagagatc gcagctgctt tcgtatc               47

<210>  80
<211>  51
<212>  DNA
<213>  Artificial

<220>
<221> Source
<223> /note="Description of Artificial Sequence: An artificially synthesized
oligonucleotide for siRNA"

<400> 80
tcccggtgaa gaagagcctg ccattcaaga gatggcaggc tcttcttcac c 51

<210> 81
<211> 51
<212> DNA
<213> Artificial

<220>
<221> Source
<223> /note="Description of Artificial Sequence: An artificially synthesized
oligonucleotide for siRNA"

<400> 81
aaaaggtgaa gaagagcctg ccatctcttg aatggcaggc tcttcttcac c 51

<210> 82
<211> 47
<212> DNA
<213> Artificial

<220>
<221> Source
<223> /note="Description of Artificial Sequence: siRNA hairpin design"

<400> 82
ggtgaagaag agcctgccat tcaagagatg gcaggctctt cttcacc 47

<210> 83
<211> 51
<212> DNA
<213> Artificial

<220>

<221>  Source
<223>  /note="Description of Artificial Sequence: An artificially synthesized
oligonucleotide for siRNA"

<400>  83
tccccctgaa actagcacac tgcttcaaga gagcagtgtg ctagtttcag g          51

<210>  84
<211>  51
<212>  DNA
<213>  Artificial

<220>
<221>  Source
<223>  /note="Description of Artificial Sequence: An artificially synthesized
oligonucleotide for siRNA"

<400>  84
aaaacctgaa actagcacac tgctctcttg aagcagtgtg ctagtttcag g          51

<210>  85
<211>  47
<212>  DNA
<213>  Artificial

<220>
<221>  Source
<223>  /note="Description of Artificial Sequence: siRNA hairpin design"

<400>  85
cctgaaacta gcacactgct tcaagagagc agtgtgctag tttcagg          47

<210>  86
<211>  3969
<212>  DNA
<213>  Homo sapiens

<220>

<221>  CDS
<222>  (250)..(2130)

<400>  86
agaatcttcc ggcgtctttc cggtggtggt cgtttttgct gcctgcgagc gcgtccgcgg      60

gctgggcgtt tccggctcgc tgggtccggg ccaggtaact ggagccggaa accggtggag     120

gtggtgtccg cccgcagagg agcttgcctg gtctcggtct gagcgtcgcc cagcgatttg     180

ccaccgcacg cacgccggat cccgggcttt accgcccgcc tttccaggcc ccgccccgcc     240

taaagtccc atg gcc gag gca gcg cta gtg aat acg ccg cag ggc cat gtg     291
          Met Ala Glu Ala Ala Leu Val Asn Thr Pro Gln Gly His Val
          1               5                   10

acc ttt gag gat att gct gtg tac ttc tcc cag gag gag tgg ggc ctc     339
Thr Phe Glu Asp Ile Ala Val Tyr Phe Ser Gln Glu Glu Trp Gly Leu
15                  20                  25                  30

ctt gat gaa gct caa agg tgc ctg tat cat gat gtg atg ctg gag aac     387
Leu Asp Glu Ala Gln Arg Cys Leu Tyr His Asp Val Met Leu Glu Asn
                35                  40                  45

ttt tcg ctt atg gcc tca gta ggt tgt ttg cat gga ata gag gct gag     435
Phe Ser Leu Met Ala Ser Val Gly Cys Leu His Gly Ile Glu Ala Glu
            50                  55                  60

gag gcc cct tct gag cag act ctt cct gcg caa gga gtg tca cag gcc     483
Glu Ala Pro Ser Glu Gln Thr Leu Pro Ala Gln Gly Val Ser Gln Ala
        65                  70                  75

agg act cca aag cta ggt cct tcc atc cca aat gct cat tct tgt gag     531
Arg Thr Pro Lys Leu Gly Pro Ser Ile Pro Asn Ala His Ser Cys Glu
    80                  85                  90

atg tgt atc ctg gtc atg aaa gac att ttg tac ctc agt gag cat cag     579
Met Cys Ile Leu Val Met Lys Asp Ile Leu Tyr Leu Ser Glu His Gln
95                  100                 105                 110

ggg aca ctt ccc tgg cag aaa cct tat acg tct gtg gcc agt ggg aaa     627
Gly Thr Leu Pro Trp Gln Lys Pro Tyr Thr Ser Val Ala Ser Gly Lys

                115                 120                 125

tgg ttt tca ttt ggt tct aac ctg caa cag cac cag aac cag gac agt 675
Trp Phe Ser Phe Gly Ser Asn Leu Gln Gln His Gln Asn Gln Asp Ser
            130                 135                 140

gga gag aaa cac atc aga aag gag gag agc agt gcc ttg ctt ctg aat 723
Gly Glu Lys His Ile Arg Lys Glu Glu Ser Ser Ala Leu Leu Leu Asn
        145                 150                 155

agc tgc aaa att cct ctg tca gac aat ctt ttc cca tgc aaa gat gtt 771
Ser Cys Lys Ile Pro Leu Ser Asp Asn Leu Phe Pro Cys Lys Asp Val
    160                 165                 170

gag aag gat ttt cca acc atc ctg ggc ctt ctc caa cac cag acc acc 819
Glu Lys Asp Phe Pro Thr Ile Leu Gly Leu Leu Gln His Gln Thr Thr
175                 180                 185                 190

cac agc aga caa gag tat gca cat aga agc agg gag acc ttt caa caa 867
His Ser Arg Gln Glu Tyr Ala His Arg Ser Arg Glu Thr Phe Gln Gln
                195                 200                 205

aga cgt tac aaa tgt gag caa gtt ttc aat gag aaa gtt cat gtt act 915
Arg Arg Tyr Lys Cys Glu Gln Val Phe Asn Glu Lys Val His Val Thr
            210                 215                 220

gag cat cag aga gtc cac act gga gaa aaa gct tat aag cgt agg gaa 963
Glu His Gln Arg Val His Thr Gly Glu Lys Ala Tyr Lys Arg Arg Glu
        225                 230                 235

tat ggg aaa tcc ttg aac tct aaa tac tta ttt gtt gaa cac cag aga 1011
Tyr Gly Lys Ser Leu Asn Ser Lys Tyr Leu Phe Val Glu His Gln Arg
    240                 245                 250

acc cat aat gca gaa aag cct tat gtg tgc aat ata tgt ggg aaa tca 1059
Thr His Asn Ala Glu Lys Pro Tyr Val Cys Asn Ile Cys Gly Lys Ser
255                 260                 265                 270

ttc ctc cat aaa caa aca ctc gtt ggg cac cag cag aga att cac act 1107
Phe Leu His Lys Gln Thr Leu Val Gly His Gln Gln Arg Ile His Thr
                275                 280                 285

aga gaa agg tct tat gtg tgc atc gaa tgt ggg aaa tcc ttg agc tcc 1155
Arg Glu Arg Ser Tyr Val Cys Ile Glu Cys Gly Lys Ser Leu Ser Ser
            290                 295                 300

aaa tac tca ctt gtg gaa cac cag aga acc cat aat gga gaa aag cct 1203
Lys Tyr Ser Leu Val Glu His Gln Arg Thr His Asn Gly Glu Lys Pro
        305                 310                 315

tat gtg tgc aat gta tgt ggg aaa tca ttc cgc cac aaa caa aca ttt 1251
Tyr Val Cys Asn Val Cys Gly Lys Ser Phe Arg His Lys Gln Thr Phe
    320                 325                 330

gtt ggc cat cag cag aga atc cac act gga gag agg cct tat gtg tgt 1299
Val Gly His Gln Gln Arg Ile His Thr Gly Glu Arg Pro Tyr Val Cys
335                 340                 345                 350

atg gaa tgt ggg aaa tct ttt att cat tcc tat gac cgc att cga cac 1347
Met Glu Cys Gly Lys Ser Phe Ile His Ser Tyr Asp Arg Ile Arg His
                355                 360                 365

cag aga gtt cac act gga gaa ggg gct tat cag tgc agt gaa tgt ggg 1395
Gln Arg Val His Thr Gly Glu Gly Ala Tyr Gln Cys Ser Glu Cys Gly
            370                 375                 380

aaa tcc ttc ata tac aaa cag tca ctt ctt gat cac cat aga atc cac 1443
Lys Ser Phe Ile Tyr Lys Gln Ser Leu Leu Asp His His Arg Ile His
        385                 390                 395

acg gga gaa agg cct tat gag tgc aaa gaa tgt ggg aag gcc ttc att 1491
Thr Gly Glu Arg Pro Tyr Glu Cys Lys Glu Cys Gly Lys Ala Phe Ile
    400                 405                 410

cac aaa aaa aga ctt ctt gag cac cag aga att cat act gga gaa aag 1539
His Lys Lys Arg Leu Leu Glu His Gln Arg Ile His Thr Gly Glu Lys
415                 420                 425                 430

cct tat gtg tgc atc ata tgt ggg aaa tca ttt atc cgc tcg tct gac 1587
Pro Tyr Val Cys Ile Ile Cys Gly Lys Ser Phe Ile Arg Ser Ser Asp
                435                 440                 445

tac atg cga cac cag aga att cac act gga gaa agg gct tat gaa tgc 1635
Tyr Met Arg His Gln Arg Ile His Thr Gly Glu Arg Ala Tyr Glu Cys

            450                 455                 460

agt gac tgt ggg aaa gcc ttc atc tcc aaa caa aca ctt ctt aag cat 1683
Ser Asp Cys Gly Lys Ala Phe Ile Ser Lys Gln Thr Leu Leu Lys His
        465                 470                 475

cac aaa atc cac act aga gaa agg cct tat gaa tgc agt gaa tgt gga 1731
His Lys Ile His Thr Arg Glu Arg Pro Tyr Glu Cys Ser Glu Cys Gly
    480                 485                 490

aaa ggc ttc tac ctt gag gtt aaa ctt ctt cag cac caa aga atc cat 1779
Lys Gly Phe Tyr Leu Glu Val Lys Leu Leu Gln His Gln Arg Ile His
495                 500                 505                 510

act aga gaa caa ctt tgt gag tgc aat gaa tgt gga aaa gtc ttc agc 1827
Thr Arg Glu Gln Leu Cys Glu Cys Asn Glu Cys Gly Lys Val Phe Ser
                515                 520                 525

cac caa aaa aga ctt ctt gag cac cag aaa gtt cac act ggc gaa aag 1875
His Gln Lys Arg Leu Leu Glu His Gln Lys Val His Thr Gly Glu Lys
            530                 535                 540

ccc tgt gag tgc agt gaa tgt ggg aaa tgc ttt aga cac cgc acc agc 1923
Pro Cys Glu Cys Ser Glu Cys Gly Lys Cys Phe Arg His Arg Thr Ser
        545                 550                 555

ctc att caa cac cag aaa gtt cac agt gga gag agg cct tat aac tgc 1971
Leu Ile Gln His Gln Lys Val His Ser Gly Glu Arg Pro Tyr Asn Cys
    560                 565                 570

act gca tgt gag aag gcc ttt atc tat aaa aac aaa ctt gtt gag cat 2019
Thr Ala Cys Glu Lys Ala Phe Ile Tyr Lys Asn Lys Leu Val Glu His
575                 580                 585                 590

cag cga atc cac acc gga gaa aag ccg tat gaa tgt ggt aaa tgt ggg 2067
Gln Arg Ile His Thr Gly Glu Lys Pro Tyr Glu Cys Gly Lys Cys Gly
                595                 600                 605

aaa gcc ttc aac aaa aga tat tcc ctt gtc agg cac cag aag gta cat 2115
Lys Ala Phe Asn Lys Arg Tyr Ser Leu Val Arg His Gln Lys Val His
            610                 615                 620

ata aca gaa gag ccc tagcaattgt tgggatgtgt aattgtctta ttcactgtag 2170
Ile Thr Glu Glu Pro
        625

gacaccagag agctgatttt tcaagggatc caacagacag aaattcaccc tcatacatct 2230

gcatatcact agttgaaaga ttcactacaa ggtccaagta cttgggaagc tttctagaga 2290

ttacttgtac tttctaatct gcccagtgtt acaacagaca ctaccatgtg gcatatcctc 2350

accgttttca tcagtcactc acatgtgctc aaggaatgca gaccactgtg cttaccaaat 2410

tctgaaggga ataatataat aaaagcctgt tgagggtcct cttccatctt gctgaactgt 2470

tcgaggcaaa gacaaaaccc aactttgacc aagaagagca atctggattc ttgtagccca 2530

tggaggttta ccttcttcat aggttttttt tttttttatg tgattgccat agtgggatat 2590

tccctctccc acattaccca agagggaaat gggctgtctt accatgtgct gatgtatgtg 2650

ctctgccagt gtgaagggtg aacagctcca actttatgtc cccccaggga catagtatga 2710

gatcggaaaa tagttcttag tcctgtttgt cttaattttt attggtttcc aaggtctcct 2770

aggaaggaga gcagagctgt gtggtcctaa tcatggtgtt gatgccagat ttatttgtag 2830

gtccagaggt ctccctgaga agaggcattt gtgacaaact caagtgctgg gacctgcatg 2890

aattgtttat ttgcagtgat gcttcatatt tcccagcact gttcatggta tcttatttcc 2950

caggtccttc attgtagcca tgggcactga aggactgaat tggtaggtag gtcactggtt 3010

gtaagaccta ctggggccag gtaagaacag taattggccc agacaggtag gtgtgataaa 3070

cagagtagaa aagattgtgg ccaagaagag tgaatgtgtc ccatccaaag gtgcatccag 3130

atatgtctgt gttactatgg cttcatggtt tgggggtata tagaaattct caggacctca 3190

gacctttgat tctcctttag caacacacat cgtcagcata aggcatctaa agattttgtg 3250

gactcataac ttatgtactg tttttgagat gattgctgca cgggcaggaa agcaggattt 3310

gagagaacat acaccttgcc ttccagatat gtggtttttg tgattcaccc agatctgttg 3370

tgccagttag gaattatctt tattacttcc cgtttattgc cactgctgag aaggctgtcc 3430

ttcctaactt gtgaggagat gaatccttaa taataagtaa atcttgtgtt atcctatagt 3490

ctggttttcc aaaaggagag gtagatgcat atttttgtgt ggaaccattt atcttaaaac 3550

attgaggtgc agttttcata cacaaaacct tctatctttt taagaactgt ctgtatggtt 3610

tattgcccag ttaatgggtt ttgatagtca tgttatctca gtaagtgtct tgtcttagaa 3670

atatgtgtgt ataggctggg tgcggtggct catgcctgta atcccagcac tttgggaggc 3730

caaggcaggt ggatcacctg aggtcaggag tttgagacca gcctgaccaa catggtgaaa 3790

tccagtctct acttaaaata caaaaattag ccaggcgtgg tggcgtgcat ttgtaatccc 3850

agctacacag gaggctgagg caggagaatt gcttgaacct gggggcggga ggttgcaatg 3910

agccaaggtc gcgccattgc actccagcct gggcgacaga gtgacacttc tcaaaaaac 3969

<210> 87
<211> 627
<212> PRT
<213> Homo sapiens

<400> 87

Met Ala Glu Ala Ala Leu Val Asn Thr Pro Gln Gly His Val Thr Phe
1               5                   10                  15

Glu Asp Ile Ala Val Tyr Phe Ser Gln Glu Glu Trp Gly Leu Leu Asp
            20                  25                  30

Glu Ala Gln Arg Cys Leu Tyr His Asp Val Met Leu Glu Asn Phe Ser
        35                  40                  45

Leu Met Ala Ser Val Gly Cys Leu His Gly Ile Glu Ala Glu Glu Ala
    50                  55                  60

Pro Ser Glu Gln Thr Leu Pro Ala Gln Gly Val Ser Gln Ala Arg Thr
65                  70                  75                  80

Pro Lys Leu Gly Pro Ser Ile Pro Asn Ala His Ser Cys Glu Met Cys
                85                  90                  95

Ile Leu Val Met Lys Asp Ile Leu Tyr Leu Ser Glu His Gln Gly Thr
            100                 105                 110

Leu Pro Trp Gln Lys Pro Tyr Thr Ser Val Ala Ser Gly Lys Trp Phe
        115                 120                 125

Ser Phe Gly Ser Asn Leu Gln Gln His Gln Asn Gln Asp Ser Gly Glu
    130                 135                 140

Lys His Ile Arg Lys Glu Glu Ser Ser Ala Leu Leu Leu Asn Ser Cys
145                 150                 155                 160

Lys Ile Pro Leu Ser Asp Asn Leu Phe Pro Cys Lys Asp Val Glu Lys
                165                 170                 175

Asp Phe Pro Thr Ile Leu Gly Leu Leu Gln His Gln Thr Thr His Ser
            180                 185                 190

Arg Gln Glu Tyr Ala His Arg Ser Arg Glu Thr Phe Gln Gln Arg Arg
        195                 200                 205

Tyr Lys Cys Glu Gln Val Phe Asn Glu Lys Val His Val Thr Glu His
    210                 215                 220

Gln Arg Val His Thr Gly Glu Lys Ala Tyr Lys Arg Arg Glu Tyr Gly
225                 230                 235                 240

Lys Ser Leu Asn Ser Lys Tyr Leu Phe Val Glu His Gln Arg Thr His
                245                 250                 255

Asn Ala Glu Lys Pro Tyr Val Cys Asn Ile Cys Gly Lys Ser Phe Leu
            260                 265                 270

His Lys Gln Thr Leu Val Gly His Gln Gln Arg Ile His Thr Arg Glu
        275                 280                 285

Arg Ser Tyr Val Cys Ile Glu Cys Gly Lys Ser Leu Ser Ser Lys Tyr
    290                 295                 300

Ser Leu Val Glu His Gln Arg Thr His Asn Gly Glu Lys Pro Tyr Val
305                 310                 315                 320

Cys Asn Val Cys Gly Lys Ser Phe Arg His Lys Gln Thr Phe Val Gly
                325                 330                 335

His Gln Gln Arg Ile His Thr Gly Glu Arg Pro Tyr Val Cys Met Glu
            340                 345                 350

Cys Gly Lys Ser Phe Ile His Ser Tyr Asp Arg Ile Arg His Gln Arg
        355                 360                 365

Val His Thr Gly Glu Gly Ala Tyr Gln Cys Ser Glu Cys Gly Lys Ser
    370                 375                 380

Phe Ile Tyr Lys Gln Ser Leu Leu Asp His His Arg Ile His Thr Gly
385                 390                 395                 400

Glu Arg Pro Tyr Glu Cys Lys Glu Cys Gly Lys Ala Phe Ile His Lys
                405                 410                 415

Lys Arg Leu Leu Glu His Gln Arg Ile His Thr Gly Glu Lys Pro Tyr
            420                 425                 430

Val Cys Ile Ile Cys Gly Lys Ser Phe Ile Arg Ser Ser Asp Tyr Met
        435                 440                 445

Arg His Gln Arg Ile His Thr Gly Glu Arg Ala Tyr Glu Cys Ser Asp
    450                 455                 460

Cys Gly Lys Ala Phe Ile Ser Lys Gln Thr Leu Leu Lys His His Lys
465                 470                 475                 480

Ile His Thr Arg Glu Arg Pro Tyr Glu Cys Ser Glu Cys Gly Lys Gly
                485                 490                 495

Phe Tyr Leu Glu Val Lys Leu Leu Gln His Gln Arg Ile His Thr Arg
            500                 505                 510

Glu Gln Leu Cys Glu Cys Asn Glu Cys Gly Lys Val Phe Ser His Gln
        515                 520                 525

Lys Arg Leu Leu Glu His Gln Lys Val His Thr Gly Glu Lys Pro Cys
    530                 535                 540

Glu Cys Ser Glu Cys Gly Lys Cys Phe Arg His Arg Thr Ser Leu Ile
545                 550                 555                 560

Gln His Gln Lys Val His Ser Gly Glu Arg Pro Tyr Asn Cys Thr Ala
                565                 570                 575

Cys Glu Lys Ala Phe Ile Tyr Lys Asn Lys Leu Val Glu His Gln Arg
            580                 585                 590

Ile His Thr Gly Glu Lys Pro Tyr Glu Cys Gly Lys Cys Gly Lys Ala
        595                 600                 605

Phe Asn Lys Arg Tyr Ser Leu Val Arg His Gln Lys Val His Ile Thr
    610                 615                 620

Glu Glu Pro
625

<210> 88
<211> 2203
<212> DNA
<213> Homo sapiens

<220>
<221> CDS
<222> (60)..(1913)

<400> 88
acatggctgc gtgaccgcgg gatgctgtct gtccccttgc tcagggcggc gtgccggcg 59

atg gag cgc gcg gta gag cct tgg ggc cca gat ctc cac cgc ccg gag 107
Met Glu Arg Ala Val Glu Pro Trp Gly Pro Asp Leu His Arg Pro Glu
1               5                   10                  15

gag agg gag cca cag aga ggc gcc cgc aca ggt cta ggg agt gag aac 155
Glu Arg Glu Pro Gln Arg Gly Ala Arg Thr Gly Leu Gly Ser Glu Asn
            20                  25                  30

gtg att tct cag ccg aat gag ttt gaa cat acc cca cag gaa gat gac     203
Val Ile Ser Gln Pro Asn Glu Phe Glu His Thr Pro Gln Glu Asp Asp
        35                  40                  45

ttg ggg ttc aag gaa gaa gat ttg gct cca gat cat gaa gta gga aat     251
Leu Gly Phe Lys Glu Glu Asp Leu Ala Pro Asp His Glu Val Gly Asn
    50                  55                  60

gtc tct ctc aaa cct gaa ggc atc cag aac tgg gat gac tta tgg gtc     299
Val Ser Leu Lys Pro Glu Gly Ile Gln Asn Trp Asp Asp Leu Trp Val
65                  70                  75                  80

cag aga gag ggt cta gga aag cct cag cct cgg gac aga ggc ccc cgg     347
Gln Arg Glu Gly Leu Gly Lys Pro Gln Pro Arg Asp Arg Gly Pro Arg
                85                  90                  95

ctc ctg ggt gaa cca cgc tgg ggc cag gct agt agt gat cgg gcc gct     395
Leu Leu Gly Glu Pro Arg Trp Gly Gln Ala Ser Ser Asp Arg Ala Ala
            100                 105                 110

gtg tgt ggt gag tgt ggc aaa agc ttc agg cag atg tca gat ctg gtg     443
Val Cys Gly Glu Cys Gly Lys Ser Phe Arg Gln Met Ser Asp Leu Val
        115                 120                 125

aaa cat cag cgg acc cac aca ggg gag aaa ccc tac aag tgt ggg gtc     491
Lys His Gln Arg Thr His Thr Gly Glu Lys Pro Tyr Lys Cys Gly Val
    130                 135                 140

tgt ggc aag ggc ttt ggg gat agc tct gcc cgg atc aaa cac cag cgg     539
Cys Gly Lys Gly Phe Gly Asp Ser Ser Ala Arg Ile Lys His Gln Arg
145                 150                 155                 160

act cat agt ggg gag aag ccc tat aga gcc cgg cca cca gcc cag ggt     587
Thr His Ser Gly Glu Lys Pro Tyr Arg Ala Arg Pro Pro Ala Gln Gly
                165                 170                 175

ccc cca aag att cct cgg tcc cgg atc cct gct ggt gag cgc ccc act     635
Pro Pro Lys Ile Pro Arg Ser Arg Ile Pro Ala Gly Glu Arg Pro Thr
            180                 185                 190

atc tgt ggt gaa tgt ggc aag agc ttc cgg ctg agt tct gac ctg gtg     683

Ile Cys Gly Glu Cys Gly Lys Ser Phe Arg Leu Ser Ser Asp Leu Val
        195                 200                 205

aaa cac cag cgg aca cac act ggt gag aag ccc tac aag tgt ggc ata     731
Lys His Gln Arg Thr His Thr Gly Glu Lys Pro Tyr Lys Cys Gly Ile
    210                 215                 220

tgt ggc aag ggc ttt ggc gac agt tcc gcc cgc atc aag cac cag cgg     779
Cys Gly Lys Gly Phe Gly Asp Ser Ser Ala Arg Ile Lys His Gln Arg
225                 230                 235                 240

aca cac cgg ggg gag cag ccc ccc cga cca gtg gtg ccc cga cgg cag     827
Thr His Arg Gly Glu Gln Pro Pro Arg Pro Val Val Pro Arg Arg Gln
                245                 250                 255

cca tct cgg gca gcc acg gca gct acc cag gga ccg aag gcc cag gac     875
Pro Ser Arg Ala Ala Thr Ala Ala Thr Gln Gly Pro Lys Ala Gln Asp
            260                 265                 270

aag cca tat atc tgc act gat tgc ggc aag agg ttt gtg ctc agc tgc     923
Lys Pro Tyr Ile Cys Thr Asp Cys Gly Lys Arg Phe Val Leu Ser Cys
        275                 280                 285

agc ctc ctg agt cac cag cgt agt cac ttg ggg ccc aag ccc ttt ggc     971
Ser Leu Leu Ser His Gln Arg Ser His Leu Gly Pro Lys Pro Phe Gly
    290                 295                 300

tgt gat gtg tgt gga aag gag ttt gcc cgg gga tcc gac ctg gtg aag     1019
Cys Asp Val Cys Gly Lys Glu Phe Ala Arg Gly Ser Asp Leu Val Lys
305                 310                 315                 320

cac ctg cgg gtg cac acg ggt gag aag ccc tac ctc tgc cca gag tgc     1067
His Leu Arg Val His Thr Gly Glu Lys Pro Tyr Leu Cys Pro Glu Cys
                325                 330                 335

ggc aaa ggt ttc gcg gac agc tcc gcc cga gtc aaa cac ctc cgc acc     1115
Gly Lys Gly Phe Ala Asp Ser Ser Ala Arg Val Lys His Leu Arg Thr
            340                 345                 350

cac agt ggc gag agg ccc cat gcc tgc ccg gaa tgc gac cgt acc ttc     1163
His Ser Gly Glu Arg Pro His Ala Cys Pro Glu Cys Asp Arg Thr Phe
        355                 360                 365

agc ctc agc tcc acc ctt ctt cgc cac cgc ctc act cac atg gag ccc     1211
Ser Leu Ser Ser Thr Leu Leu Arg His Arg Leu Thr His Met Glu Pro
    370                 375                 380

cag gac ttc agc ttc cca ggc tat ccc cta ccc gct ctg atc ccc agc     1259
Gln Asp Phe Ser Phe Pro Gly Tyr Pro Leu Pro Ala Leu Ile Pro Ser
385                 390                 395                 400

cca ccc cca cct cct ctg ggc acc agc ccc ccg ctg aca cct cga agt     1307
Pro Pro Pro Pro Pro Leu Gly Thr Ser Pro Pro Leu Thr Pro Arg Ser
                405                 410                 415

ccc tca cac tcg ggt gag cct ttt ggc ctg cct ggc ttg gag cca gag     1355
Pro Ser His Ser Gly Glu Pro Phe Gly Leu Pro Gly Leu Glu Pro Glu
            420                 425                 430

cct ggg ggc cca cag gct ggg gag cca ccc cca cca ctg gcg ggc gac     1403
Pro Gly Gly Pro Gln Ala Gly Glu Pro Pro Pro Pro Leu Ala Gly Asp
        435                 440                 445

aag ccc cac aag tgc cct gag tgt ggc aag ggc ttc cgc cga agc tct     1451
Lys Pro His Lys Cys Pro Glu Cys Gly Lys Gly Phe Arg Arg Ser Ser
    450                 455                 460

gac ctg gtg aaa cac cat cgt gtg cac aca ggg gag aaa ccc tac ctc     1499
Asp Leu Val Lys His His Arg Val His Thr Gly Glu Lys Pro Tyr Leu
465                 470                 475                 480

tgt cct gaa tgc ggc aag ggt ttt gct gac agc tca gcc cga gtc aag     1547
Cys Pro Glu Cys Gly Lys Gly Phe Ala Asp Ser Ser Ala Arg Val Lys
                485                 490                 495

cac ctc cgc acc cac cgt ggt gaa cgg gcc cgg cca cca cca cca tcc     1595
His Leu Arg Thr His Arg Gly Glu Arg Ala Arg Pro Pro Pro Pro Ser
            500                 505                 510

act ctg ctg cgg cca cat aac cca cct ggc cca gta ccc atg gcc cct     1643
Thr Leu Leu Arg Pro His Asn Pro Pro Gly Pro Val Pro Met Ala Pro
        515                 520                 525

cga ccc cga gtt cgg gcc cag cct tct gga ccc agc cag ccc cac gtg     1691

Arg Pro Arg Val Arg Ala Gln Pro Ser Gly Pro Ser Gln Pro His Val
    530                 535                 540

tgt ggc ttc tgt ggg aag gag ttc ccc cgg agc tca gat ctg gtc aaa      1739
Cys Gly Phe Cys Gly Lys Glu Phe Pro Arg Ser Ser Asp Leu Val Lys
545                 550                 555                 560

cac agg cgt aca cac acg ggg gag aag cca tac aag tgt gca gag tgt      1787
His Arg Arg Thr His Thr Gly Glu Lys Pro Tyr Lys Cys Ala Glu Cys
                565                 570                 575

ggc aag ggt ttt ggt gac agt tct gcc cgc atc aag cac cag cgt ggg      1835
Gly Lys Gly Phe Gly Asp Ser Ser Ala Arg Ile Lys His Gln Arg Gly
            580                 585                 590

cac ctg gtc ctg acg ccc ttt ggg ata ggg gat ggt agg gca agg ccc      1883
His Leu Val Leu Thr Pro Phe Gly Ile Gly Asp Gly Arg Ala Arg Pro
        595                 600                 605

ctc aag cag gag gca gca aca gga ctg gaa tgacgcggtc cagggagggt      1933
Leu Lys Gln Glu Ala Ala Thr Gly Leu Glu
    610                 615

ggaggcccag gagaccaaag ggaggggctc tgccgcttag cagagaagaa agggcctggg      1993

aggtggtggg agggagaagg aagggaagaa aggggaggaa gaatagatag aaatagggat      2053

tggagacagt aaccttgaag ctcaggaaac tgtcctggct gggctgagtc aggaccttgc      2113

cgggacgggc tgtacccctg gcttctagaa gactgcctag cacacagtag gcattcaata      2173

cttgttgaat aaataaactg gctttcacct                                      2203

<210>  89
<211>  618
<212>  PRT
<213>  Homo sapiens

<400>  89

Met Glu Arg Ala Val Glu Pro Trp Gly Pro Asp Leu His Arg Pro Glu

1               5                   10                  15

Glu Arg Glu Pro Gln Arg Gly Ala Arg Thr Gly Leu Gly Ser Glu Asn
            20                  25                  30

Val Ile Ser Gln Pro Asn Glu Phe Glu His Thr Pro Gln Glu Asp Asp
        35                  40                  45

Leu Gly Phe Lys Glu Glu Asp Leu Ala Pro Asp His Glu Val Gly Asn
    50                  55                  60

Val Ser Leu Lys Pro Glu Gly Ile Gln Asn Trp Asp Asp Leu Trp Val
65                  70                  75                  80

Gln Arg Glu Gly Leu Gly Lys Pro Gln Pro Arg Asp Arg Gly Pro Arg
                85                  90                  95

Leu Leu Gly Glu Pro Arg Trp Gly Gln Ala Ser Ser Asp Arg Ala Ala
            100                 105                 110

Val Cys Gly Glu Cys Gly Lys Ser Phe Arg Gln Met Ser Asp Leu Val
        115                 120                 125

Lys His Gln Arg Thr His Thr Gly Glu Lys Pro Tyr Lys Cys Gly Val
    130                 135                 140

Cys Gly Lys Gly Phe Gly Asp Ser Ser Ala Arg Ile Lys His Gln Arg
145                 150                 155                 160

Thr His Ser Gly Glu Lys Pro Tyr Arg Ala Arg Pro Pro Ala Gln Gly
                165                 170                 175

Pro Pro Lys Ile Pro Arg Ser Arg Ile Pro Ala Gly Glu Arg Pro Thr
            180                 185                 190

Ile Cys Gly Glu Cys Gly Lys Ser Phe Arg Leu Ser Ser Asp Leu Val
        195                 200                 205

Lys His Gln Arg Thr His Thr Gly Glu Lys Pro Tyr Lys Cys Gly Ile
    210                 215                 220

Cys Gly Lys Gly Phe Gly Asp Ser Ser Ala Arg Ile Lys His Gln Arg
225                 230                 235                 240

Thr His Arg Gly Glu Gln Pro Pro Arg Pro Val Val Pro Arg Arg Gln
                245                 250                 255

Pro Ser Arg Ala Ala Thr Ala Ala Thr Gln Gly Pro Lys Ala Gln Asp
            260                 265                 270

Lys Pro Tyr Ile Cys Thr Asp Cys Gly Lys Arg Phe Val Leu Ser Cys
        275                 280                 285

Ser Leu Leu Ser His Gln Arg Ser His Leu Gly Pro Lys Pro Phe Gly
    290                 295                 300

Cys Asp Val Cys Gly Lys Glu Phe Ala Arg Gly Ser Asp Leu Val Lys
305                 310                 315                 320

His Leu Arg Val His Thr Gly Glu Lys Pro Tyr Leu Cys Pro Glu Cys
                325                 330                 335

Gly Lys Gly Phe Ala Asp Ser Ser Ala Arg Val Lys His Leu Arg Thr

            340                 345                 350

His Ser Gly Glu Arg Pro His Ala Cys Pro Glu Cys Asp Arg Thr Phe
        355                 360                 365

Ser Leu Ser Ser Thr Leu Leu Arg His Arg Leu Thr His Met Glu Pro
    370                 375                 380

Gln Asp Phe Ser Phe Pro Gly Tyr Pro Leu Pro Ala Leu Ile Pro Ser
385                 390                 395                 400

Pro Pro Pro Pro Pro Leu Gly Thr Ser Pro Pro Leu Thr Pro Arg Ser
                405                 410                 415

Pro Ser His Ser Gly Glu Pro Phe Gly Leu Pro Gly Leu Glu Pro Glu
            420                 425                 430

Pro Gly Gly Pro Gln Ala Gly Glu Pro Pro Pro Pro Leu Ala Gly Asp
        435                 440                 445

Lys Pro His Lys Cys Pro Glu Cys Gly Lys Gly Phe Arg Arg Ser Ser
    450                 455                 460

Asp Leu Val Lys His His Arg Val His Thr Gly Glu Lys Pro Tyr Leu
465                 470                 475                 480

Cys Pro Glu Cys Gly Lys Gly Phe Ala Asp Ser Ser Ala Arg Val Lys
                485                 490                 495

His Leu Arg Thr His Arg Gly Glu Arg Ala Arg Pro Pro Pro Pro Ser
            500                 505                 510

Thr Leu Leu Arg Pro His Asn Pro Pro Gly Pro Val Pro Met Ala Pro
        515                 520                 525

Arg Pro Arg Val Arg Ala Gln Pro Ser Gly Pro Ser Gln Pro His Val
    530                 535                 540

Cys Gly Phe Cys Gly Lys Glu Phe Pro Arg Ser Ser Asp Leu Val Lys
545                 550                 555                 560

His Arg Arg Thr His Thr Gly Glu Lys Pro Tyr Lys Cys Ala Glu Cys
                565                 570                 575

Gly Lys Gly Phe Gly Asp Ser Ser Ala Arg Ile Lys His Gln Arg Gly
            580                 585                 590

His Leu Val Leu Thr Pro Phe Gly Ile Gly Asp Gly Arg Ala Arg Pro
        595                 600                 605

Leu Lys Gln Glu Ala Ala Thr Gly Leu Glu
    610                 615

<210>  90
<211>  2670
<212>  DNA
<213>  Homo sapiens

<220>
<221>  CDS
<222>  (192)..(2186)

<400>  90
ggccgagggc gggcggacgc gggagctgcg gacgtgaggc atgagcggcg ccctcctccg      60

gcccgcgagc gtcctgctgg ttccccgagc gagggtctcg cggcgcgggg cctagcggag 120

ggcatcgaag gcctccgcgt gcgcacgggt tgctgcggcc gcgccgggcg ccggggaggg 180

cggcggccgc c atg gag gtg agc ggg ccg gaa gac gac ccc ttc ctt tcg 230
Met Glu Val Ser Gly Pro Glu Asp Asp Pro Phe Leu Ser
1               5                   10

cag ctg cac cag gtg cag tgc ccc gtg tgc cag cag atg atg ccc gcc 278
Gln Leu His Gln Val Gln Cys Pro Val Cys Gln Gln Met Met Pro Ala
    15                  20                  25

gcg cac atc aac tcg cac ctg gac cgc tgt ctg ctg ctc cac ccg gcg 326
Ala His Ile Asn Ser His Leu Asp Arg Cys Leu Leu Leu His Pro Ala
30                  35                  40                  45

ggg cac gcg gag ccc gcg gcc ggg tcg cac cgc gcc ggg gag cgg gcc 374
Gly His Ala Glu Pro Ala Ala Gly Ser His Arg Ala Gly Glu Arg Ala
                50                  55                  60

aag ggg ccc tcg ccg ccc ggc gcc aag agg cgg cgg ctg tcg gag agc 422
Lys Gly Pro Ser Pro Pro Gly Ala Lys Arg Arg Arg Leu Ser Glu Ser
            65                  70                  75

tcg gcg ctg aag cag cca gcc acc ccg acg gca gcc gag agc agc gag 470
Ser Ala Leu Lys Gln Pro Ala Thr Pro Thr Ala Ala Glu Ser Ser Glu
        80                  85                  90

ggc gag ggt gag gag ggc gac gac ggc ggc gag acc gag agc cgc gag 518
Gly Glu Gly Glu Glu Gly Asp Asp Gly Gly Glu Thr Glu Ser Arg Glu
    95                  100                 105

agc tac gac gcg ccg ccc aca ccc agc ggc gcc cgc ctt atc ccc gac 566
Ser Tyr Asp Ala Pro Pro Thr Pro Ser Gly Ala Arg Leu Ile Pro Asp
110                 115                 120                 125

ttc ccg gtg gcc cgc tcc agc agc ccc ggg agg aag ggg tcg ggg aag 614
Phe Pro Val Ala Arg Ser Ser Ser Pro Gly Arg Lys Gly Ser Gly Lys
                130                 135                 140

agg ccg gcg gcc gcc gcc gcg gcg ggg agc gcg tct ccg cgc agc tgg 662
Arg Pro Ala Ala Ala Ala Ala Ala Gly Ser Ala Ser Pro Arg Ser Trp

            145                 150                 155

gac gag gcg gag gcg cag gag gag gag gag gcc gtg ggc gac ggc gat 710
Asp Glu Ala Glu Ala Gln Glu Glu Glu Glu Ala Val Gly Asp Gly Asp
        160                 165                 170

ggc gac ggg gac gcg gac gcg gac ggc gag gac gac ccg ggg cac tgg 758
Gly Asp Gly Asp Ala Asp Ala Asp Gly Glu Asp Asp Pro Gly His Trp
    175                 180                 185

gac gcg gac gct gcc gaa gcc gcc acc gcc ttc ggg gcc agt ggc ggg 806
Asp Ala Asp Ala Ala Glu Ala Ala Thr Ala Phe Gly Ala Ser Gly Gly
190                 195                 200                 205

ggc cgc ccg cac ccc cgg gcg ctg gct gcc gag gag atc cga cag atg 854
Gly Arg Pro His Pro Arg Ala Leu Ala Ala Glu Glu Ile Arg Gln Met
                210                 215                 220

cta cag ggc aag ccg ctg gcc gac acg atg cgt cct gac acg ctg cag 902
Leu Gln Gly Lys Pro Leu Ala Asp Thr Met Arg Pro Asp Thr Leu Gln
            225                 230                 235

gat tac ttc ggg cag agc aag gcc gtg ggc cag gat acc ctg ctg cgc 950
Asp Tyr Phe Gly Gln Ser Lys Ala Val Gly Gln Asp Thr Leu Leu Arg
        240                 245                 250

tcg ctc ctg gag acc aac gaa atc ccc tcg ctt atc ctg tgg ggg ccg 998
Ser Leu Leu Glu Thr Asn Glu Ile Pro Ser Leu Ile Leu Trp Gly Pro
    255                 260                 265

ccg ggc tgc ggc aag acc act ctg gct cac atc ata gcc agc aac agc 1046
Pro Gly Cys Gly Lys Thr Thr Leu Ala His Ile Ile Ala Ser Asn Ser
270                 275                 280                 285

aag aaa cat agc ata agg ttt gtg aca tta tct gca aca aat gcc aag 1094
Lys Lys His Ser Ile Arg Phe Val Thr Leu Ser Ala Thr Asn Ala Lys
                290                 295                 300

aca aat gat gtg cga gat gtc ata aaa caa gct caa aat gaa aag agc 1142
Thr Asn Asp Val Arg Asp Val Ile Lys Gln Ala Gln Asn Glu Lys Ser
            305                 310                 315

ttt ttc aaa agg aaa acc atc ctt ttt att gat gag att cat cgg ttc 1190
Phe Phe Lys Arg Lys Thr Ile Leu Phe Ile Asp Glu Ile His Arg Phe
        320                 325                 330

aat aaa tct cag cag gac act ttc ctt cct cac gtg gaa tgt ggg acg 1238
Asn Lys Ser Gln Gln Asp Thr Phe Leu Pro His Val Glu Cys Gly Thr
    335                 340                 345

atc act ctg att ggg gca acc act gaa aac cct tcc ttc cag gtc aac 1286
Ile Thr Leu Ile Gly Ala Thr Thr Glu Asn Pro Ser Phe Gln Val Asn
350                 355                 360                 365

gct gct ctt ctg agc cgc tgt cga gtg att gtt ctt gag aag ctt cca 1334
Ala Ala Leu Leu Ser Arg Cys Arg Val Ile Val Leu Glu Lys Leu Pro
                370                 375                 380

gta gag gca atg gtg act att tta atg cga gcg atc aac tcc ctg gga 1382
Val Glu Ala Met Val Thr Ile Leu Met Arg Ala Ile Asn Ser Leu Gly
            385                 390                 395

atc cac gtc cta gac tct agc cgt ccc act gac cct ctg agc cac agc 1430
Ile His Val Leu Asp Ser Ser Arg Pro Thr Asp Pro Leu Ser His Ser
        400                 405                 410

agc aac agc agc tca gag ccc gcc atg ttc ata gag gat aaa gca gta 1478
Ser Asn Ser Ser Ser Glu Pro Ala Met Phe Ile Glu Asp Lys Ala Val
    415                 420                 425

gac acc ctg gct tac ctc agt gac ggt gac gcc cga gct ggg ttg aac 1526
Asp Thr Leu Ala Tyr Leu Ser Asp Gly Asp Ala Arg Ala Gly Leu Asn
430                 435                 440                 445

gga ctg cag ctg gcg gtg ctg gct agg tta agc tct agg aag atg ttc 1574
Gly Leu Gln Leu Ala Val Leu Ala Arg Leu Ser Ser Arg Lys Met Phe
                450                 455                 460

tgt aag aag agt ggg caa tcc tat tct ccc agt aga gtt ctg atc aca 1622
Cys Lys Lys Ser Gly Gln Ser Tyr Ser Pro Ser Arg Val Leu Ile Thr
            465                 470                 475

gag aat gac gtg aag gag ggc cta cag cga tcc cac att tta tat gac 1670
Glu Asn Asp Val Lys Glu Gly Leu Gln Arg Ser His Ile Leu Tyr Asp

        480                 485                 490

cgg gca ggt gag gag cat tac aac tgc atc tcc gcc ctg cac aag tcc 1718
Arg Ala Gly Glu Glu His Tyr Asn Cys Ile Ser Ala Leu His Lys Ser
    495                 500                 505

atg cgg ggc tca gac cag aac gcc tcc ctc tac tgg ctg gct cgc atg 1766
Met Arg Gly Ser Asp Gln Asn Ala Ser Leu Tyr Trp Leu Ala Arg Met
510                 515                 520                 525

ctc gag gga gga gag gac cca ctc tac gtg gca cgg agg ctt gtc agg 1814
Leu Glu Gly Gly Glu Asp Pro Leu Tyr Val Ala Arg Arg Leu Val Arg
                530                 535                 540

ttt gcc agc gag gac ata ggt ctg gca gac ccg tct gcg tta aca caa 1862
Phe Ala Ser Glu Asp Ile Gly Leu Ala Asp Pro Ser Ala Leu Thr Gln
            545                 550                 555

gcg gtt gct gcc tac caa ggc tgt cat ttt ata ggc atg cct gaa tgt 1910
Ala Val Ala Ala Tyr Gln Gly Cys His Phe Ile Gly Met Pro Glu Cys
        560                 565                 570

gag gtg ctt ctg gcc cag tgt gtg gtc tac ttt gcc aga gcc cca aag 1958
Glu Val Leu Leu Ala Gln Cys Val Val Tyr Phe Ala Arg Ala Pro Lys
    575                 580                 585

tcc att gag gtg tac agc gcc tac aac aac gtc aaa gcc tgc ctg agg 2006
Ser Ile Glu Val Tyr Ser Ala Tyr Asn Asn Val Lys Ala Cys Leu Arg
590                 595                 600                 605

aac cac cag ggg cca ctg ccc ccc gtg ccc ctg cac ctg agg aac gcg 2054
Asn His Gln Gly Pro Leu Pro Pro Val Pro Leu His Leu Arg Asn Ala
                610                 615                 620

ccc act agg ctg atg aag gat ttg ggc tat ggc aaa ggc tac aag tac 2102
Pro Thr Arg Leu Met Lys Asp Leu Gly Tyr Gly Lys Gly Tyr Lys Tyr
            625                 630                 635

aac ccc atg tac agc gag cct gtg gat cag gag tac ctg cct gaa gag 2150
Asn Pro Met Tyr Ser Glu Pro Val Asp Gln Glu Tyr Leu Pro Glu Glu
        640                 645                 650

ttg agg ggg gta gat ttc ttc aag cag agg agg tgc tgactcctca 2196
Leu Arg Gly Val Asp Phe Phe Lys Gln Arg Arg Cys
    655                 660                 665

gggcacgaca gcagaaggat gttgcttttt taagggaggg ccagaaagaa agttagtgga 2256

ttgcaaagtt ggttgcctgg tggaagttag aacagaccaa cattttgtgc cagaaattta 2316

agagttccat aggtggaggc gcagttcttt cgaataaatg tgtaactttg aaattgtgtt 2376

catttgcact cggtgcagcg gttatgctta tgaaaatacc tggcagcttt gtgcaatgaa 2436

ttaatgttat aaggaattat ctattttgtc atagtattta agtcataatg tcatttcaga 2496

attcagttct gtaggatttt cttttcttta aaaaatgtat attctgggta gttttaattg 2556

gtaaaaaaat gtaattgtga tttaatactg catagtgttt tgggtatttt ttttatatgc 2616

aaaggtctta cgagccaata aaactatttc aaagtaaaaa aaaaaaaaaa aaaa 2670

<210> 91
<211> 665
<212> PRT
<213> Homo sapiens

<400> 91

Met Glu Val Ser Gly Pro Glu Asp Asp Pro Phe Leu Ser Gln Leu His
1               5                   10                  15

Gln Val Gln Cys Pro Val Cys Gln Gln Met Met Pro Ala Ala His Ile
            20                  25                  30

Asn Ser His Leu Asp Arg Cys Leu Leu Leu His Pro Ala Gly His Ala
        35                  40                  45

Glu Pro Ala Ala Gly Ser His Arg Ala Gly Glu Arg Ala Lys Gly Pro
    50                  55                  60

Ser Pro Pro Gly Ala Lys Arg Arg Arg Leu Ser Glu Ser Ser Ala Leu
65                  70                  75                  80

Lys Gln Pro Ala Thr Pro Thr Ala Ala Glu Ser Ser Glu Gly Glu Gly
                85                  90                  95

Glu Glu Gly Asp Asp Gly Gly Glu Thr Glu Ser Arg Glu Ser Tyr Asp
            100                 105                 110

Ala Pro Pro Thr Pro Ser Gly Ala Arg Leu Ile Pro Asp Phe Pro Val
        115                 120                 125

Ala Arg Ser Ser Ser Pro Gly Arg Lys Gly Ser Gly Lys Arg Pro Ala
    130                 135                 140

Ala Ala Ala Ala Ala Gly Ser Ala Ser Pro Arg Ser Trp Asp Glu Ala
145                 150                 155                 160

Glu Ala Gln Glu Glu Glu Glu Ala Val Gly Asp Gly Asp Gly Asp Gly
                165                 170                 175

Asp Ala Asp Ala Asp Gly Glu Asp Asp Pro Gly His Trp Asp Ala Asp
            180                 185                 190

Ala Ala Glu Ala Ala Thr Ala Phe Gly Ala Ser Gly Gly Gly Arg Pro
        195                 200                 205

His Pro Arg Ala Leu Ala Ala Glu Glu Ile Arg Gln Met Leu Gln Gly
    210                 215                 220

Lys Pro Leu Ala Asp Thr Met Arg Pro Asp Thr Leu Gln Asp Tyr Phe
225                 230                 235                 240

Gly Gln Ser Lys Ala Val Gly Gln Asp Thr Leu Leu Arg Ser Leu Leu
                245                 250                 255

Glu Thr Asn Glu Ile Pro Ser Leu Ile Leu Trp Gly Pro Pro Gly Cys
            260                 265                 270

Gly Lys Thr Thr Leu Ala His Ile Ile Ala Ser Asn Ser Lys Lys His
        275                 280                 285

Ser Ile Arg Phe Val Thr Leu Ser Ala Thr Asn Ala Lys Thr Asn Asp
    290                 295                 300

Val Arg Asp Val Ile Lys Gln Ala Gln Asn Glu Lys Ser Phe Phe Lys
305                 310                 315                 320

Arg Lys Thr Ile Leu Phe Ile Asp Glu Ile His Arg Phe Asn Lys Ser
                325                 330                 335

Gln Gln Asp Thr Phe Leu Pro His Val Glu Cys Gly Thr Ile Thr Leu
            340                 345                 350

Ile Gly Ala Thr Thr Glu Asn Pro Ser Phe Gln Val Asn Ala Ala Leu
        355                 360                 365

Leu Ser Arg Cys Arg Val Ile Val Leu Glu Lys Leu Pro Val Glu Ala
    370                 375                 380

Met Val Thr Ile Leu Met Arg Ala Ile Asn Ser Leu Gly Ile His Val
385                 390                 395                 400

Leu Asp Ser Ser Arg Pro Thr Asp Pro Leu Ser His Ser Ser Asn Ser
                405                 410                 415

Ser Ser Glu Pro Ala Met Phe Ile Glu Asp Lys Ala Val Asp Thr Leu
            420                 425                 430

Ala Tyr Leu Ser Asp Gly Asp Ala Arg Ala Gly Leu Asn Gly Leu Gln
        435                 440                 445

Leu Ala Val Leu Ala Arg Leu Ser Ser Arg Lys Met Phe Cys Lys Lys
    450                 455                 460

Ser Gly Gln Ser Tyr Ser Pro Ser Arg Val Leu Ile Thr Glu Asn Asp
465                 470                 475                 480

Val Lys Glu Gly Leu Gln Arg Ser His Ile Leu Tyr Asp Arg Ala Gly
                485                 490                 495

Glu Glu His Tyr Asn Cys Ile Ser Ala Leu His Lys Ser Met Arg Gly
            500                 505                 510

Ser Asp Gln Asn Ala Ser Leu Tyr Trp Leu Ala Arg Met Leu Glu Gly
        515                 520                 525

Gly Glu Asp Pro Leu Tyr Val Ala Arg Arg Leu Val Arg Phe Ala Ser
    530                 535                 540

Glu Asp Ile Gly Leu Ala Asp Pro Ser Ala Leu Thr Gln Ala Val Ala
545                 550                 555                 560

Ala Tyr Gln Gly Cys His Phe Ile Gly Met Pro Glu Cys Glu Val Leu
                565                 570                 575

Leu Ala Gln Cys Val Val Tyr Phe Ala Arg Ala Pro Lys Ser Ile Glu
            580                 585                 590

Val Tyr Ser Ala Tyr Asn Asn Val Lys Ala Cys Leu Arg Asn His Gln
        595                 600                 605

Gly Pro Leu Pro Pro Val Pro Leu His Leu Arg Asn Ala Pro Thr Arg
    610                 615                 620

Leu Met Lys Asp Leu Gly Tyr Gly Lys Gly Tyr Lys Tyr Asn Pro Met
625                 630                 635                 640

Tyr Ser Glu Pro Val Asp Gln Glu Tyr Leu Pro Glu Glu Leu Arg Gly
                645                 650                 655

Val Asp Phe Phe Lys Gln Arg Arg Cys
            660                 665

<210> 92
<211> 5384
<212> DNA
<213> Homo sapiens

<400> 92
aaaatgttgg agatctgcct gaagctggtg ggctgcaaat ccaagaaggg gctgtcctcg      60

tcctccagct gttatctgga agaagccctt cagcggccag tagcatctga ctttgagcct     120

cagggtctga gtgaagccgc tcgttggaac tccaaggaaa accttctcgc tggacccagt     180

gaaaatgacc ccaacctttt cgttgcactg tatgattttg tggccagtgg agataacact     240

ctaagcataa ctaaaggtga aaagctccgg gtcttaggct ataatcacaa tggggaatgg 300

tgtgaagccc aaaccaaaaa tggccaaggc tgggtcccaa gcaactacat cacgccagtc 360

aacagtctgg agaaacactc ctggtaccat gggcctgtgt cccgcaatgc cgctgagtat 420

ctgctgagca gcgggatcaa tggcagcttc ttggtgcgtg agagtgagag cagtcctggc 480

cagaggtcca tctcgctgag atacgaaggg agggtgtacc attacaggat caacactgct 540

tctgatggca agctctacgt ctcctccgag agccgcttca acaccctggc cgagttggtt 600

catcatcatt caacggtggc cgacgggctc atcaccacgc tccattatcc agccccaaag 660

cgcaacaagc ccactgtcta tggtgtgtcc cccaactacg acaagtggga gatggaacgc 720

acggacatca ccatgaagca caagctgggc gggggccagt acggggaggt gtacgagggc 780

gtgtggaaga aatacagcct gacggtggcc gtgaagacct tgaaggagga caccatggag 840

gtggaagagt tcttgaaaga agctgcagtc atgaaagaga tcaaacaccc taacctggtg 900

cagctccttg gggtctgcac ccgggagccc ccgttctata tcatcactga gttcatgacc 960

tacgggaacc tcctggacta cctgagggag tgcaaccggc aggaggtgaa cgccgtggtg 1020

ctgctgtaca tggccactca gatctcgtca gccatggagt acctggagaa gaaaaacttc 1080

atccacagag atcttgctgc ccgaaactgc ctggtagggg agaaccactt ggtgaaggta 1140

gctgattttg gcctgagcag gttgatgaca ggggacacct acacagccca tgctggagcc 1200

aagttcccca tcaaatggac tgcacccgag agcctggcct acaacaagtt ctccatcaag 1260

tccgacgtct gggcatttgg agtattgctt tgggaaattg ctacctatgg catgtcccct 1320

tacccgggaa ttgacctgtc ccaggtgtat gagctgctag agaaggacta ccgcatggag 1380

cgcccagaag gctgcccaga gaaggtctat gaactcatgc gagcatgttg gcagtggaat 1440

ccctctgacc ggccctcctt tgctgaaatc caccaagcct ttgaaacaat gttccaggaa 1500

tccagtatct cagacgaagt ggaaaaggag ctggggaaac aaggcgtccg tggggctgtg 1560

agtaccttgc tgcaggcccc agagctgccc accaagacga ggacctccag gagagctgca 1620

gagcacagag acaccactga cgtgcctgag atgcctcact ccaagggcca gggagagagc 1680

gatcctctgg accatgagcc tgccgtgtct ccattgctcc ctcgaaaaga gcgaggtccc 1740

ccggagggcg gcctgaatga agatgagcgc cttctcccca aagacaaaaa gaccaacttg 1800

ttcagcgcct tgatcaagaa gaagaagaag acagccccaa cccctcccaa acgcagcagc 1860

tccttccggg agatggacgg ccagccggag cgcagagggg ccggcgagga agagggccga 1920

gacatcagca acggggcact ggctttcacc cccttggaca cagctgaccc agccaagtcc 1980

ccaaagccca gcaatggggc tggggtcccc aatggagccc tccgggagtc cgggggctca 2040

ggcttccggt ctccccacct gtggaagaag tccagcacgc tgaccagcag ccgcctagcc 2100

accggcgagg aggagggcgg tggcagctcc agcaagcgct tcctgcgctc ttgctccgcc 2160

tcctgcgttc cccatggggc caaggacacg gagtggaggt cagtcacgct gcctcgggac 2220

ttgcagtcca cgggaagaca gtttgactcg tccacatttg gagggcacaa aagtgagaag 2280

ccggctctgc ctcggaagag ggcaggggag aacaggtctg accaggtgac ccgaggcaca 2340

gtaacgcctc cccccaggct ggtgaaaaag aatgaggaag ctgctgatga ggtcttcaaa 2400

gacatcatgg agtccagccc gggctccagc ccgcccaacc tgactccaaa acccctccgg 2460

cggcaggtca ccgtggcccc tgcctcgggc ctcccccaca aggaagaagc tggaaagggc 2520

agtgccttag ggacccctgc tgcagctgag ccagtgaccc ccaccagcaa agcaggctca 2580

ggtgcaccag ggggcaccag caagggcccc gccgaggagt ccagagtgag gaggcacaag 2640

cactcctctg agtcgccagg gagggacaag gggaaattgt ccaggctcaa acctgccccg 2700

ccgcccccac cagcagcctc tgcagggaag gctggaggaa agccctcgca gagcccgagc 2760

caggaggcgg ccggggaggc agtcctgggc gcaaagacaa aagccacgag tctggttgat 2820

gctgtgaaca gtgacgctgc caagcccagc cagccgggag agggcctcaa aaagcccgtg 2880

ctcccggcca ctccaaagcc acagtccgcc aagccgtcgg ggacccccat cagcccagcc 2940

cccgttccct ccacgttgcc atcagcatcc tcggccctgg caggggacca gccgtcttcc 3000

accgccttca tccctctcat atcaacccga gtgtctcttc ggaaaacccg ccagcctcca 3060

gagcggatcg ccagcggcgc catcaccaag ggcgtggtcc tggacagcac cgaggcgctg 3120

tgcctcgcca tctctaggaa ctccgagcag atggccagcc acagcgcagt gctggaggcc 3180

ggcaaaaacc tctacacgtt ctgcgtgagc tatgtggatt ccatccagca aatgaggaac 3240

aagtttgcct tccgagaggc catcaacaaa ctggagaata atctccggga gcttcagatc 3300

tgcccggcga cagcaggcag tggtccagcg gccactcagg acttcagcaa gctcctcagt 3360

tcggtgaagg aaatcagtga catagtgcag aggtagcagc agtcaggggt caggtgtcag 3420

gcccgtcgga gctgcctgca gcacatgcgg gctcgcccat acccgtgaca gtggctgaca 3480

agggactagt gagtcagcac cttggcccag gagctctgcg ccaggcagag ctgagggccc 3540

tgtggagtcc agctctacta cctacgtttg caccgcctgc cctcccgcac cttcctcctc 3600

cccgctccgt ctctgtcctc gaattttatc tgtggagttc ctgctccgtg gactgcagtc 3660

ggcatgccag gacccgccag ccccgctccc acctagtgcc ccagactgag ctctccaggc 3720

caggtgggaa cggctgatgt ggactgtctt tttcattttt ttctctctgg agcccctcct 3780

cccccggctg ggcctccttc ttccacttct ccaagaatgg aagcctgaac tgaggccttg 3840

tgtgtcaggc cctctgcctg cactccctgg ccttgcccgt cgtgtgctga agacatgttt 3900

caagaaccgc atttcgggaa gggcatgcac gggcatgcac acggctggtc actctgccct 3960

ctgctgctgc ccggggtggg gtgcactcgc catttcctca cgtgcaggac agctcttgat 4020

ttgggtggaa aacagggtgc taaagccaac cagcctttgg gtcctgggca ggtgggagct 4080

gaaaaggatc gaggcatggg gcatgtcctt tccatctgtc cacatcccca gagcccagct 4140

cttgctctct tgtgacgtgc actgtgaatc ctggcaagaa agcttgagtc tcaagggtgg 4200

caggtcactg tcactgccga catccctccc ccagcagaat ggaggcaggg gacaagggag 4260

gcagtggcta gtggggtgaa cagctggtgc caaatagccc cagactgggc ccaggcaggt 4320

ctgcaagggc ccagagtgaa ccgtcctttc acacatctgg gtgccctgaa agggcccttc 4380

ccctccccca ctcctctaag acaaagtaga ttcttacaag gccctttcct ttggaacaag 4440

acagccttca cttttctgag ttcttgaagc atttcaaagc cctgcctctg tgtagccgcc 4500

ctgagagaga atagagctgc cactgggcac ctgcgcacag gtgggaggaa agggcctggc 4560

cagtcctggt cctggctgca ctcttgaact gggcgaatgt cttatttaat taccgtgagt 4620

gacatagcct catgttctgt gggggtcatc agggagggtt aggaaaacca caaacggagc 4680

ccctgaaagc ctcacgtatt tcacagagca cgcctgccat cttctccccg aggctgcccc 4740

aggccggagc ccagatacgg gggctgtgac tctgggcagg gacccggggt ctcctggacc 4800

ttgacagagc agctaactcc gagagcagtg ggcaggtggc cgcccctgag gcttcacgcc 4860

gggagaagcc accttcccac cccttcatac cgcctcgtgc cagcagcctc gcacaggccc 4920

tagctttacg ctcatcacct aaacttgtac tttatttttc tgatagaaat ggtttcctct 4980

ggatcgtttt atgcggttct tacagcacat cacctctttg cccccgacgg ctgtgacgca 5040

gccggaggga ggcactagtc accgacagcg gccttgaaga cagagcaaag cgcccaccca 5100

ggtcccccga ctgcctgtct ccatgaggta ctggtccctt ccttttgtta acgtgatgtg 5160

ccactatatt ttacacgtat ctcttggtat gcatctttta tagacgctct tttctaagtg 5220

gcgtgtgcat agcgtcctgc cctgccccct cgggggcctg tggtggctcc ccctctgctt 5280

ctcggggtcc agtgcatttt gtttctgtat atgattctct gtggtttttt ttgaatccaa 5340

atctgtcctc tgtagtattt tttaaataaa tcagtgttta catt 5384

<210> 93
<211> 1149
<212> PRT
<213> Homo sapiens

<400> 93

<210> 94
<211> 23
<212> DNA
<213> Artificial

<220>
<221> Source
<223> /note="Description of Artificial Sequence: An artificially synthesized
primer for PCR"

<400> 94
taatagtacc agccatcgct cag                                              23

<210> 95
<211> 23
<212> DNA
<213> Artificial

<220>
<221> Source
<223> /note="Description of Artificial Sequence: An artificially synthesized
primer for PCR"

<400> 95
atcctacggc tttattgaca cct                                              23

<210> 96
<211> 21
<212> RNA
<213> Artificial

<220>
<221> Source
<223> /note="Description of Artificial Sequence: An artificially synthesized
oligonucleotide for siRNA"

<400> 96
cuaggaagau guucuguaau u 21

<210> 97
<211> 21
<212> RNA
<213> Artificial

<220>
<221> Source
<223> /note="Description of Artificial Sequence: An artificially synthesized
oligonucleotide for siRNA"

<400> 97
ccacuaggcu gaugaaggau u 21

<210> 98
<211> 167
<212> PRT
<213> Artificial

<220>
<221> Source
<223> /note="Description of Artificial Sequence: An artificially synthesized
antigen polypeptide"

<400> 98

Ala Val Val Ala Glu Glu Asp Thr Glu Leu Arg Asp Leu Leu Val Gln
1               5                   10                  15

Thr Leu Glu Asn Ser Gly Val Leu Asn Arg Ile Lys Ala Glu Leu Arg
            20                  25                  30

Ala Ala Val Phe Leu Ala Leu Glu Glu Gln Glu Lys Val Glu Asn Lys
        35                  40                  45

Thr Pro Leu Val Asn Glu Ser Leu Lys Lys Phe Leu Asn Thr Lys Asp
    50                  55                  60

Gly Arg Leu Val Ala Ser Leu Val Ala Glu Phe Leu Gln Phe Phe Asn
65                  70                  75                  80

Leu Asp Phe Thr Leu Ala Val Phe Gln Pro Glu Thr Ser Thr Leu Gln
                85                  90                  95

Gly Leu Glu Gly Arg Glu Asn Leu Ala Arg Asp Leu Gly Ile Ile Glu
            100                 105                 110

Ala Glu Gly Thr Val Gly Gly Pro Leu Leu Leu Glu Val Ile Arg Arg
        115                 120                 125

Cys Gln Gln Lys Glu Lys Gly Pro Thr Thr Gly Glu Gly Ala Leu Asp
    130                 135                 140

Leu Ser Asp Val His Ser Pro Pro Lys Ser Pro Glu Gly Lys Thr Ser
145                 150                 155                 160

Ala Gln Thr Thr Pro Ser Lys
                165

<210>  99
<211>  126
<212>  PRT
<213>  Artificial

<220>
<221> Source
<223> /note="Description of Artificial Sequence: An artificially synthesized
antigen polypeptide"

<400> 99

Ala Gly Ala Gln Asp Phe Gly Pro Thr Arg Phe Ile Cys Thr Ser Val
1               5                   10                  15

Pro Val Asp Ala Asp Met Cys Ala Ala Ser Val Ala Ala Gly Gly Ala
            20                  25                  30

Glu Glu Leu Arg Ser Ser Val Leu Gln Leu Arg Glu Thr Val Leu Gln
        35                  40                  45

Gln Lys Glu Thr Ile Leu Ser Gln Lys Glu Thr Ile Arg Glu Leu Thr
    50                  55                  60

Ala Lys Leu Gly Arg Cys Glu Ser Gln Ser Thr Leu Asp Pro Gly Ala
65                  70                  75                  80

Gly Glu Ala Arg Ala Gly Gly Gly Arg Lys Gln Pro Gly Ser Gly Lys
                85                  90                  95

Asn Thr Met Gly Asp Leu Ser Arg Thr Pro Ala Ala Glu Thr Leu Ser
            100                 105                 110

Gln Leu Gly Gln Thr Leu Gln Ser Leu Lys Thr Arg Leu Glu
        115                 120                 125

<210> 100
<211> 76

<212> PRT
<213> Artificial

<220>
<221> Source
<223> /note="Description of Artificial Sequence: An artificially synthesized
antigen polypeptide"

<400> 100

Gln Glu Gln Asp Thr Leu Gly Gly Gly Phe Asp Ala Thr Gln Ala Phe
1               5                   10                  15

Val Gly Glu Leu Ala His Phe Asn Ile Trp Asp Arg Lys Leu Thr Pro
            20                  25                  30

Gly Glu Val Tyr Asn Leu Ala Thr Cys Ser Thr Lys Ala Leu Ser Gly
        35                  40                  45

Asn Val Ile Ala Trp Ala Glu Ser His Ile Glu Ile Tyr Gly Gly Ala
    50                  55                  60

Thr Lys Trp Thr Phe Glu Ala Cys Arg Gln Ile Asn
65                  70                  75

<210> 101
<211> 77
<212> DNA
<213> Homo sapiens

<400> 101
gggtggtgga tctgtcggtc ccgttttccc gtcgcacgtg gtggccactg ttggcttctg 60

aatggtttgc aaggcgg 77